# EUROPEAN PATENT APPLICATION

(11) **EP 4 722 208 A1**
(43) Date of publication of application: **08.04.2026**
(21) Application number: 24814732.4
(22) Date of filing: 29.05.2024
(51) Int. Cl.: C07D 403/12, A61K 31/4155, A61K 31/437, A61K 31/4439, A61K 31/415, A61K 31/5386, C07D 231/12, C07D 471/04, C07D 401/12, C07D 401/14

(54) **NOVEL BENZAMIDE DERIVATIVE COMPOUND AND PHARMACEUTICAL COMPOSITION COMPRISING SAME**

(30) Priority: 30.05.2023 KR 20230069604
(71) Applicant: Samjin Pharmaceutical Co., Ltd., Seoul 04054 (KR)
(72) Inventor: AHN, Soyeon, Seoul 07605 (KR); KIM, Jiwon, Seoul 07791 (KR); LEE, Goeun, Seoul 07537 (KR); HWANG, Kyungrim, Seoul 07791 (KR); WOO, Juhyun, Goyang-si Gyeonggi-do 10595 (KR); LEE, Wonje, Seoul 06660 (KR); LEE, Soo Min, Seoul 06277 (KR); KIM, Jincheul, Seoul 07982 (KR); SOHN, Te-ik, Seoul 07794 (KR); HA, Haechan, Seoul 06262 (KR); KWON, Ho Seok, Suwon-Si Gyeonggi-do 16557 (KR); LEE, Jae Woong, Suwon-Si Gyeonggi-do 16713 (KR); KIM, Hyun Tae, Suwon-si Gyeonggi-do 16687 (KR)
(74) Representative: Witthoff Jaekel Steinecke Patentanwälte PartG mbB
(86) International application number: PCT/IB2024/055205
(87) International publication number: WO 2024/246764

(57) **Abstract**

The present invention relates to a novel benzamide compound, a stereoisomer thereof, a pharmaceutically acceptable salt of them, or a hydrate or solvate of them. The novel compound exhibits NAMPT inhibitory activity, can exhibit a preventive or therapeutic effect on diseases that can be treated by inhibiting NAMPT, and can effectively prevent or treat cancer.

## Description

### Technical Field

The present invention relates to a compound represented by formula I below, a stereoisomer thereof, a pharmaceutically acceptable salt of them, or a solvate or hydrate of them, and a use thereof.

### Background

Nicotinamide phosphoribosyltransferase (NAMPT) is an enzyme which induces the synthesis of nicotinamide mononucleotide (NMN) from nicotinamide (NAM) and 5'-phosphoribosyl-1'-pyrophosphate (PRPP), playing a key role in the cyclic biosynthetic pathway of nicotinamide adenine dinucleotide (NAD+). NAD is essential for several signaling pathways including mono-ADP-ribosylation in both the immune system and G-protein-coupled receptor signaling, among other poly-ADP-ribosylation in DNA repair, and NAD is also essential for the deacetylase activity of sirtuin.

NAD+ is produced by two distinct biosynthetic pathways: salvage and de novo synthesis pathways. As a rate-limiting enzyme in the NAD+ salvage pathway, NAMPT is biologically essential and may be associated with a number of diverse diseases.

Thus, there is a need to develop a drug capable of more effectively inhibiting NAMPT.

### Related Art References

### Patent Documents

Korean Patent Publication 10-2015-0014250

### Detailed Description of the Invention

### Technical Problem

The present invention may provide a benzamide compound represented by formula I below, a stereoisomer thereof, a pharmaceutically acceptable salt of them, or a hydrate or solvate of them.

The present invention may provide a pharmaceutical composition including a benzamide compound represented by formula I below, a stereoisomer thereof, a pharmaceutically acceptable salt of them, or a hydrate or solvate of them. The present invention may provide a pharmaceutical composition for preventing or treating diseases (e.g., cancer) that may be treated by inhibiting NAMPT, including the benzamide compound represented by formula I below, the stereoisomer thereof, a pharmaceutically acceptable salt of them, or a hydrate or solvate of them as an active ingredient.

The present invention may provide a method for preventing or treating diseases (e.g., cancer) that may be treated by inhibiting NAMPT, including administering a benzamide compound represented by formula I below, a stereoisomer thereof, a pharmaceutically acceptable salt of them, or a hydrate or solvate of them into an individual.

The present invention may provide a benzamide compound represented by formula I below, a stereoisomer thereof, a pharmaceutically acceptable salt of them, or a hydrate or solvate of them for preventing or treating diseases (e.g., cancer) that may be treated by inhibiting NAMPT.

The present invention may provide a use of a benzamide compound represented by formula I below, a stereoisomer thereof, a pharmaceutically acceptable salt of them, or a hydrate or solvate of them for preparing a medication for preventing or treating diseases (e.g., cancer) that may be treated by inhibiting NAMPT.

### Technical Solution

The present inventors have identified a compound having a novel structure with NAMPT inhibitory activity and have used the same in preventing and/or treating diseases that may be treated by inhibiting NAMPT, thereby completing the present invention.

Hereinafter, the present invention will be described in more detail. All the combinations of various elements disclosed in the present invention may fall within the scope of the present invention. In addition, it may not be seen that the scope of the present invention is limited to the specific description below.
1) The present invention may provide a benzamide compound represented by formula I below, a stereoisomer thereof, a pharmaceutically acceptable salt of them, or a hydrate or solvate of them.

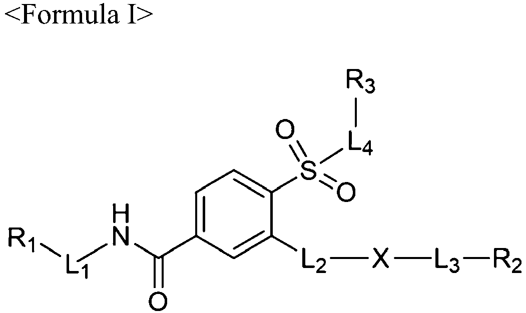

In above formula I,
R₁ is 6- to 14-membered aryl, C2-C8 heterocycloalkenyl, or 5- to 12-membered heteroaryl, in which at least one H of said R₁ may be substituted with halogen (F, Cl, Br, or I), -NRaRb, -NO₂ or -OH;
L₁ is C1-C6 alkylene;
L₂ is a single bond or C2-C6 alkynylene;
X is 6- to 14-membered arylene, C2-C8 heterocycloalkenylene, or 5- to 12-membered heteroarylene;
at least one H of said X may be substituted with C1-C6 alkyl, CF₃, halogen (F, Cl, Br or I), -C1-C6 alkoxy, or OH, -CH₂- of said X may be substituted with -(C=O)-, specifically at least one H of 6- to 14-membered arylene or C2-C8 heterocycloalkenylene of said X may be substituted with C1-C6 alkyl, CF₃, halogen (F, Cl, Br, or I), C1-C6 alkoxy, or OH, and at least one -CH₂- of C2-C8 heterocycloalkenylene of said X may each independently be substituted with - (C=O)-;
L₃ is a single bond, C1-C6 alkylene, C2-C6 alkenylene, C=O, (C=O)NRc, (C=O)NRd-(C1-C6 alkylene), Nry(C=O)-(C1-C6 alkylene), or O(C=O)-(C1-C6 alkylene);
R₂ is H, halogen (F, Cl, Br or I), C1-C6 alkyl, C1-C6 alkoxy, C3-C8 cycloalkyl, C1-C8 heterocycloalkyl, -NRz₁Rz₂, 6- to 14-membered aryl, or 5- to 12-membered heteroaryl;
at least one H of said R₂ may be substituted with C1-C6 alkyl, C1-C6 alkoxy, OH, COOH, (C1-C6 alkylene)-NreRf, NRgRh, NO₂, CF₃, (C=O)NriRj, or halogen (F, Cl, Br, or I);
L₄ is a single bond, C1-C6 alkylene, -NRm-(C1-C6 alkylene)-, or NRn;
R₃ is H, C1-C6 alkyl, C3-C8 cycloalkyl, 6- to 14-membered aryl, C1-C8 heterocycloalkyl, C2-C8 heterocycloalkenyl, 5- to 12-membered heteroaryl, 6- to 14-membered arylene-C1-C8 heterocycloalkyl, 6- to 14-membered arylene-5- to 12-membered heteroaryl, 5- to 12-membered heteroarylene-6- to 14-membered aryl, 5- to 12-membered heteroarylene-C1-C8 heterocycloalkyl, or 5- to 12-membered heteroarylene-5- to 12-membered heteroaryl;
at least one H of said R₃ may be substituted with C1-C6 alkyl, C1-C6 alkoxy, phenoxy, OH, (C1-C6 alkylene)-OH, COOH, (C1-C6 alkylene)-COOH, (C1-C6 alkylene)-CN, NroRp, (C1-C6 alkylene)-NRqRr, (C=O)-(C1-C6 alkyl), (C=O)-(C3-C8 cycloalkyl), (C=O)-(6- to 14-membered aryl), 6- to 14-membered aryl, 6- to 14-membered heteroaryl, 6- to 14-membered heterocycloaryl, halogen-substitutable (C=O)NRsRt, (C=O)-(C1-C6 alkylene)-NruRv, NRwRx, (C=O)O-(C1-C6 alkyl), NO₂, CHF₂, CF₃, OCF₃ or halogen (F, Cl, Br, or I), and at least one -CH₂- of heterocycloalkenyl of said R₃ may be substituted with -(C=O)-; and
said Ra to Rj, Rm to Ry, Rz₁ and Rz₂ are each independently H or C1-C6 alkyl in each case. For example, Ra, Rb, Rc, Rd, Re, Rf, Rg, Rh, Ri, Rj, Rm, Rn, Ro, Rp, Rq, Rr, Rs, Rt, Ru, Rv, Rw, Rx, Ry, Rz₁ and Rz₂ may be each independently H or C1-C6 alkyl in each case.

In the present invention, the term "Cm-Cn" (in which m and n are each independently an integer of 1 or more) may mean the number of carbons and, for example, "C1-C5 alkyl" may refer to alkyl having one to five carbon atoms.

In the present invention, "substitution" or "substituted with~" may be defined to include implicit conditions, in which the substitution follows a permitted valency of a substituted atom and a substituent and induces a compound stabilized by substitution, for example, a compound which is not naturally modified by rearrangement, cyclization, removal, etc.

In the present invention, a "single bond" may mean a case in which adjacent atoms or groups of atoms are directly bonded to each other.

In the present invention, "alkyl" may mean a linear (or straight-chain) saturated hydrocarbon group or a branched (or side-chain) saturated hydrocarbon group, unless otherwise specified. Examples of alkyl may include at least one selected from methyl, ethyl, n-propyl, isopropyl, n-butyl, sec-butyl, isobutyl, tert-butyl, n-pentyl, n-hexyl, n-heptyl, and the like, but are not limited thereto.

In the present invention, "alkylene" may mean a divalent functional group which is induced from alkyl as defined above, unless otherwise stated, and may include at least one selected from methylene, ethylene, n-propylene, isopropylene, n-butylene, sec-butylene, isobutylene, tert-butylene, n-pentylene, n-hexylene, n-heptylene and the like, but is not limited thereto.

In the present invention, "alkynylene" may mean a divalent functional group which is induced from alkynyl including at least one carbon-carbon triple bond in said alkyl, unless otherwise stated. Examples of alkynylene may include 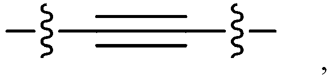 hydrocarbon including at least one 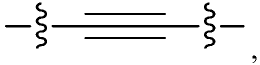 or the like, but are not limited thereto.

In the present invention, "alkenyl" may mean an unsaturated hydrocarbon group including at least one carbon-carbon double bond in said alkyl, unless otherwise stated.

In the present invention, "alkenylene" may mean a divalent functional group which is induced from alkenyl defined as above, unless otherwise stated. In the present invention, "alkoxy" may mean a monovalent group which is derived from a straight-chain or branched-chain saturated hydrocarbon moiety represented by OCnH2n+1, unless otherwise stated. Examples of alkoxy may include at least one selected from methoxy, ethoxy, propoxy, isopropoxy, n-butoxy, sec-butoxy, tert-butoxy, pentoxy, hexoxy, and the like, but are not limited thereto.

In the present invention, "cycloalkyl" may mean a saturated hydrocarbon ring having at least three carbon atoms, unless otherwise stated, and the saturated hydrocarbon ring may include both monocyclic and polycyclic structures. In addition, it may be meant to include all of a double ring structure such as a bridged ring or a spiro structure. Examples of cycloalkyl may include at least one selected from cyclopropyl, cyclobutyl, cyclopentyl, cyclohexyl, cycloheptyl, cyclooctyl, decahydronaphthalenyl, spiro[4.4]nonanyl, spiro[3.3]heptanyl, bicyclo[3.1.0]hexanyl, bicyclo[3.1.1]heptanyl, bicyclo[2.2.1]heptanyl, and the like, but are not limited thereto.

In the present invention, "cycloalkenyl" may refer to a cyclic group including at least one carbon-carbon double bond in said cycloalkyl, unless otherwise stated, and cycloalkenyl is not an aromatic ring. Examples of cycloalkenyl may include at least one selected from cyclopropenyl, cyclobutenyl, cyclopentenyl, cyclohexenyl, cycloheptenyl, cyclooctenyl, and the like, but are not limited thereto.

In the present invention, "heterocycloalkyl" may refer to a cyclic group in which at least one carbon atom constituting a ring in the cycloalkyl is each independently substituted with a heteroatom or a functional group selected from the group consisting of N, O, S, SO, and SO₂, unless otherwise stated. Examples of heterocycloalkyl may include at least one selected from oxiranyl, oxetanyl, tetrahydrofuranyl, tetrahydropyranyl, tetrahydrothiopyranyl, tetrahydrothiophenyl, oxepanyl, piperidinyl, piperazinyl, morpholinyl, thiomorpholinyl, pyrrolidinyl, azetidinyl, aziridinyl, azepanyl, 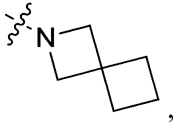 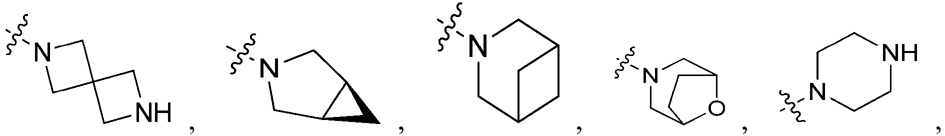 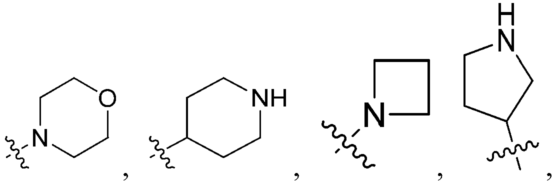 etc., but are not limited thereto.

In the present invention, "heterocycloalkenyl" may refer to a cyclic group in which at least one carbon atom constituting a ring in the cycloalkenyl is each independently substituted with a heteroatom or a functional group selected from the group consisting of N, O, S, SO, and SO₂, unless otherwise stated, and heterocycloalkenyl may not be an aromatic ring. Examples of heterocycloalkenyl may include at least one selected from 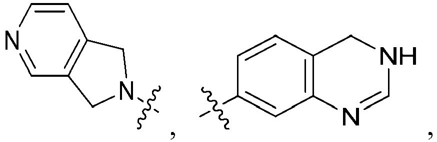 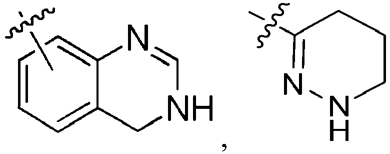 and the like, but are not limited thereto.

In the present invention, "heterocycloalkenylene" may mean a divalent functional group which is induced from heterocycloalkenyl defined as above, unless otherwise stated.

In the present invention, "aryl" may mean a monocyclic or polycyclic aromatic cyclic group having at least one fused or non-fused aromatic ring, unless otherwise stated. Examples of aryl may include at least one selected from phenyl, naphthalenyl, indenyl, anthracenyl and the like, but are not limited thereto.

In the present invention, "arylene" may mean a divalent functional group which is induced from aryl defined as above, unless otherwise stated.

In the present invention, "heteroaryl" may mean a monocyclic or polycyclic aromatic hetero ring which includes at least one heteroatom selected from the group consisting of N, O, and S in the ring and has at least one fused or non-fused aromatic ring, unless otherwise stated. Examples of heteroaryl may include at least one selected from thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, indolyl, benzothiophenyl, benzofuranyl, benzothiazolyl, benzimidazolyl, benzoxazolyl, benzisoxazolyl, benzthiazolyl, benzthiadiazolyl, benztriazolyl, quinolinyl, isoquinolinyl, purinyl, and the like, but are not limited thereto.

In the present invention, "heteroarylene" may mean a divalent functional group which is induced from heteroaryl defined as above, unless otherwise stated.

In the present invention, "halogen" may be F, Cl, Br, or I, unless otherwise stated.

In the present invention, when a divalent functional group is described as -AB-, it may include both -AB- and -BA- and, for example, a description of -(C1-C6 alkylene)-NRm- may include both X-(C1-C6 alkylene)-NRm-Y and X-NRm-(C1-C6 alkylene)-Y.

In the present invention, 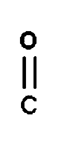 may be represented by CO, C=O, C(=O) or the like, 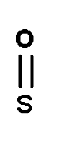 may be represented by SO, S=O, S(=O) or the like, and 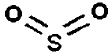 may be represented by SO₂, S=O₂, S(=O)₂, etc.

Besides, the terms and abbreviations used in the present specification may have their original meanings, unless defined otherwise.

2) According to embodiments of the present invention, in above 1), it may provide a benzamide derivative compound represented by above formula I, a stereoisomer thereof, a pharmaceutically acceptable salt of them, or a hydrate or solvate of them.

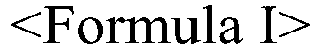

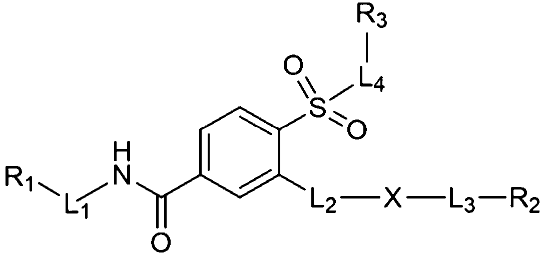

In above formula I,
R₁ is 6- to 14-membered aryl, C2-C8 heterocycloalkenyl including at least one heteroatom or heterofunctional group selected from the group consisting of N, O, S, S(=O), and S(=O)₂, or 5- to 12-membered heteroaryl including at least one heteroatom selected from the group consisting of N, O, and S, in which -H of said R₁ is unsubstituted or at least one H thereof is each independently substituted with halogen (F, Cl, Br, or I), -NRaRb, -NO₂, or -OH;
L₁ is C1-C6 alkylene;
L₂ is a single bond or C2-C6 alkynylene;
X is 6- to 14-membered arylene, C2-C8 heterocycloalkenylene including at least one heteroatom or heterofunctional group selected from the group consisting of N, O, S, S(=O), and S(=O)₂, or 5- to 12-membered heteroarylene including at least one heteroatom selected from the group consisting of N, O and S, in which at least one -H of said X may be substituted with -C1-C6 alkyl, -CF₃, -halogen (F, Cl, Br or I), -C1-C6 alkoxy, or -OH, at least one -CH₂- of said X may each independently be substituted with -(C=O)-, and specifically -H of said X is unsubstituted or, when said X is 6- to 14-membered arylene or C2-C8 heterocycloalkenylene, at least one -H thereof may each independently be substituted with C1-C6 alkyl, -CF₃, halogen (F, Cl, Br or I), -C1-C6 alkoxy, or OH, and when X is C2-C8 heterocycloalkenylene, -CH₂- is unsubstituted or at least one -CH₂- is each independently substituted with -(C=O)-;
L₃ is a single bond, C1-C6 alkylene, C2-C6 alkenylene, -C=O, - C(=O)NRc, -C(=O)NRd-(C1-C6 alkylene), -NRyC(=O)-(C1-C6 alkylene), - NHC(=O), or -OC(=O)-(C1-C6alkylene);
R₂ is H, halogen (F, Cl, Br or I), -NRz₁Rz₂, C1-C6 alkyl, C1-C6 alkoxy, C3-C8 cycloalkyl, C1-C8 heterocycloalkyl including at least one heteroatom or heterofunctional group selected from the group consisting of N, O, S, SO and SO₂, 6- to 14-membered aryl, or 5- to 12-membered heteroaryl including at least one heteroatom selected from the group consisting of N, O and S, in which -H of said R₂ is unsubstituted or at least one -H of R₂ is each independently substituted with -C1-C6 alkyl, -C1-C6 alkoxy, -OH, -COOH, -(C1-C6 alkylene)-NReRf, -NRgRh, -NO₂, -CF₃, -C(=O)NRiRj or halogen (F, Cl, Br or I);
L₄ is a single bond, -C1-C6 alkylene-, -NRm-(C1-C6 alkylene)-, or -NRn-, in which -H of said L₄ is unsubstituted or at least one -H of L₄ is each independently substituted with -C1-C6 alkyl;
R₃ is H, C1-C6 alkyl, C3-C8 cycloalkyl, 6- to 14-membered aryl, C1-C8 heterocycloalkyl including at least one heteroatom or heterofunctional group selected from the group consisting of N, O, S, SO, and SO₂, C2-C8 heterocycloalkenyl including at least one heteroatom or heterofunctional group selected from the group consisting of N, O, S, SO, and SO₂, 5- to 12-membered heteroaryl including at least one heteroatom selected from the group consisting of N, O, and S, 6- to 14-membered arylene-C1-C8 heterocycloalkyl (in which said heterocycloalkyl includes at least one heteroatom or heterofunctional group selected from the group consisting of N, O, S, SO and SO₂), 6- to 14-membered arylene-5- to 12-membered heteroaryl (in which heteroaryl includes at least one heteroatom selected from the group consisting of N, O and S), 5- to 12-membered heteroarylene-6- to 14-membered aryl (in which said heteroarylene includes at least one heteroatom selected from the group consisting of N, O and S), 5- to 12-membered heteroarylene-C1-C8 heterocycloalkyl (in which said heteroarylene includes at least one heteroatom selected from the group consisting of N, O and S, and said heterocycloalkyl includes at least one heteroatom or heterofunctional group selected from the group consisting of N, O, S, SO and SO₂), or 5- to 12-membered heteroarylene-5- to 12-membered heteroaryl (in which said heteroarylene or heteroaryl includes at least one heteroatom selected from the group consisting of N, O and S);
R₃ is H, C1-C6 alkyl, C3-C8 cycloalkyl, 6- to 14-membered aryl, C1-C8 heterocycloalkyl including at least one heteroatom or heterofunctional group selected from the group consisting of N, O, S, SO, and SO₂, C2-C8 heterocycloalkenyl including at least one heteroatom or heterofunctional group selected from the group consisting of N, O, S, SO, and SO₂, 5- to 12-membered heteroaryl including at least one heteroatom selected from the group consisting of N, O, and S, 6- to 14-membered arylene-C1-C8 heterocycloalkyl (in which said heterocycloalkyl includes at least one heteroatom or heterofunctional group selected from the group consisting of N, O, S, SO and SO₂), 6- to 14-membered arylene-5- to 12-membered heteroaryl (in which said heteroaryl includes at least one heteroatom selected from the group consisting of N, O and S), 5- to 12-membered heteroarylene-6- to 14-membered aryl (in which said heteroarylene includes at least one heteroatom selected from the group consisting of N, O and S), 5- to 12-membered heteroarylene-C1-C8 heterocycloalkyl (in which said heteroarylene includes at least one heteroatom selected from the group consisting of N, O and S, and said heterocycloalkyl includes at least one heteroatom or heterofunctional group selected from the group consisting of N, O, S, SO and SO₂), or 5- to 12-membered heteroarylene-5- to 12-membered heteroaryl (in which said heteroarylene or heteroaryl includes at least one heteroatom selected from the group consisting of N, O and S), in which -H of said R₃ is unsubstituted or at least one H of R₃ is each independently substituted with -C1-C6 alkyl, -C1-C6 alkoxy, phenoxy, -OH, -(C1-C6 alkylene)-OH, -COOH, -(C1-C6 alkylene)-COOH, (C1-C6 alkylene)-CN, -NRoRp, (C1-C6 alkylene)-NRqRr, -C(=O)-(C1-C6 alkyl), -C(=O)-(C3-C8 cycloalkyl), -C=(O)-(6- to 14-membered aryl), -NO₂, -CHF₂, -CF₃, -OCF₃, 6- to 14-membered aryl, 6- to 14-membered heteroaryl including at least one heteroatom selected from the group consisting of N, O and S, 6- to 14-membered heterocycloaryl including at least one heteroatom or heterofunctional group selected from the group consisting of N, O, S, SO and SO₂ , -C(=O)NRsRt (in which -H of -C(=O)NRsRt is unsubstituted or at least one -H thereof is each independently substituted with halogen (F, Cl, Br or I)), - C(=O)-(C1-C6 alkylene)-NRuRv, -NRwRx, or halogen (F, Cl, Br or I), and when said R₃ is heterocycloalkenyl, at least one -CH₂- may each independently be substituted with -(C=O)-; and
said Ra to Rj, Rm to Ry, Rz₁ and Rz₂ are each independently H or C1-C6 alkyl in each case. For example, Ra, Rb, Rc, Rd, Re, Rf, Rg, Rh, Ri, Rj, Rm, Rn, Ro, Rp, Rq, Rr, Rs, Rt, Ru, Rv, Rw, Rx, Ry, Rz₁ and Rz₂ may be each independently H or C1-C6 alkyl in each case.

3) According to embodiments of the present invention, in above 1) or 2), it may be provided that said R₁ is 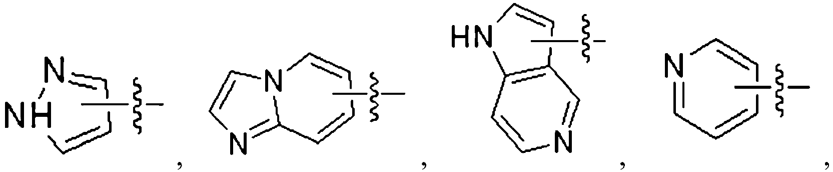 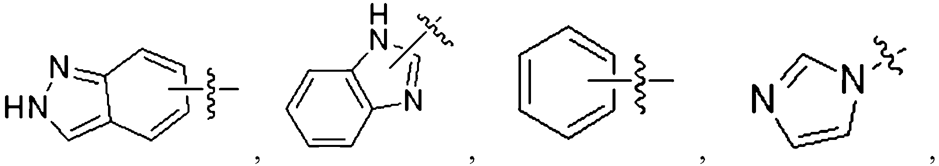 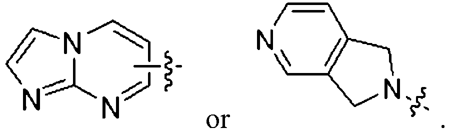 In this case, it may be provided that -H of said R₁ is unsubstituted or at least one H thereof is each independently substituted with halogen (F, Cl, Br, or I), -NRaRb, -NO₂ or -OH, in which Ra and Rb are each independently -H or C1-C6 alkyl, and specifically -H of said R₁ is unsubstituted or at least one -H of R₁ is each independently substituted with halogen (F, Cl, Br, or I), -NH₂, -NO₂, -NHCH₃, -N(CH₃)₂ or -OH.

4) According to embodiments of the present invention, in above 1) to 3), it may be provided that said L₁ is C1-C6 alkylene, specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), sec-butylene (-CHCH₃CH₂CH₂-), isobutylene (-CH₂CHCH₃CH₂-), tert-butylene (-CCH₃CH₃CH₂-), n-pentylene (-CH₂CH₂CH₂CH₂CH₂-), n-hexylene (-CH₂CH₂CH₂CH₂CH₂CH₂-) or the like, and specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), n-pentylene (-CH₂CH₂CH₂CH₂CH₂-) or n-hexylene (-CH₂CH₂CH₂CH₂CH₂CH₂-).

5) According to embodiments of the present invention, in above 1) to 4), it may be provided that said L₂ is a single bond or C2-C5 alkynylene, and specifically L₂ is a single bond or ethynylnene

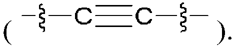

6) According to embodiments of the present invention, in above 1) to 5), it may be provided that said X is 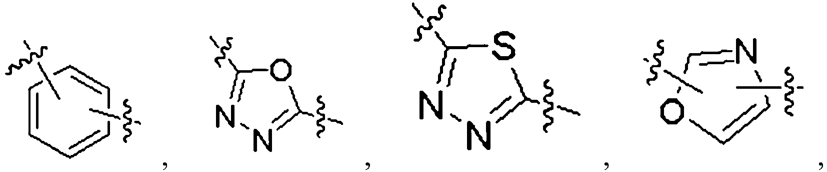 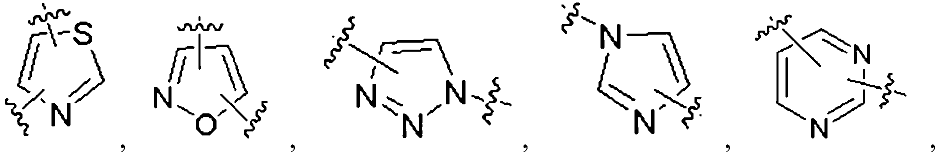 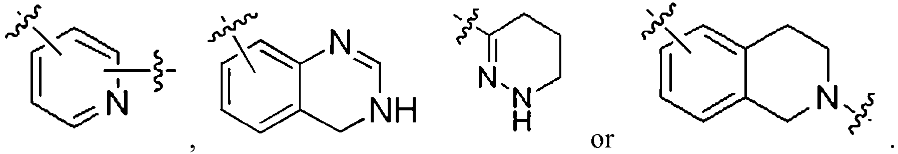 In this case, it may be provided that -H of said X is unsubstituted or at least one -H thereof is each independently substituted with C1-C6 alkyl, halogen (F, Br, Cl, or I), -OH, -C1-C6 alkoxy, or -CF₃, and -CH₂- of X is unsubstituted or at least one - CH₂- is each independently substituted with -(C=O)-, and specifically when said X is 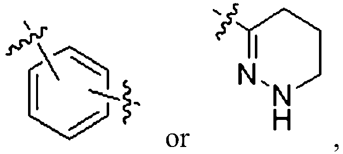 -H is unsubstituted or at least one -H thereof is each independently substituted with C1-C6 alkyl, halogen (F, Br, Cl, or I), -C1-C6 alkoxy, -OH, or -CF₃, and when X is 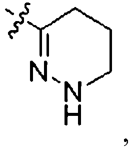 -CH₂- is unsubstituted or at least one -CH₂- is each independently substituted with -(C=O)-.

7) According to embodiments of the present invention, in above 1) to 6), it may be provided that said L₃ is a single bond, C1-C6 alkylene, C2-C6 alkenylene, -(C=O)-, -(C=O)-NRc-, -C(=O)NRd-(C1-C6 alkylene)-, - NRyC(=O)-(C1-C6 alkylene), -NHC(=O), or -O(C=O)-(C1-C6 alkylene), in which Rc, Rd or Ry is each independently -H or C1-C6 alkyl. Specifically, it may be provided that L₃ is a single bond, -(CH₂)-, -(CH₂)₂-, -(CH₂)₃-, -(CH₂)₄-, - (CH₂)₅-, -(C=O)-NH(CH₂)n, -NH(C=O)-, -NH(C=O)-(CH₂)m, -(C=O)NH-, - (CH=CH)-CH₂-, -O(C=O)CH₂, or -(C=O)-, in which n and m are each independently 1, 2, 3 or 4.

8) According to embodiments of the present invention, in above 1) to 7), it may be provided that said R₂ is H, halogen (F, Cl, Br or I), C1-C6 alkyl, C1-C6 alkoxy, -OH, -NH₂, 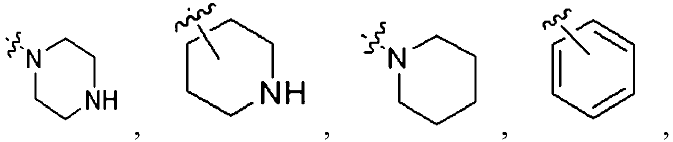 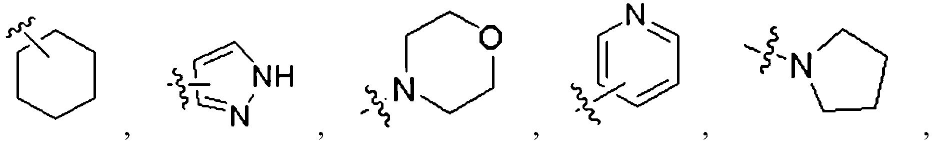 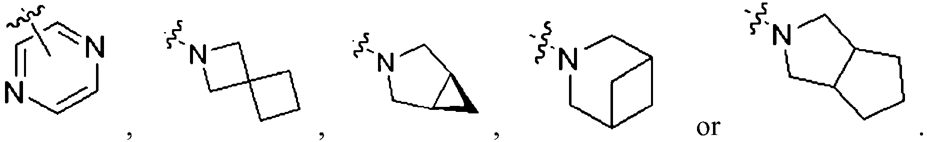 In this case, it may be provided that -H of said R₂ is unsubstituted or at least one -H of R₂ is each independently substituted with halogen (F, Cl, Br or I), -C1-C6 alkoxy, -NH₂, -OH, -CF₃, -NO₂, -C1-C6 alkyl, -(CH₂)₂-NH₂, -(CH₂)₃-NH₂, - (CH₂)-NH₂, -(C=O)NH₂, -NHCH₃, -NHCH(CH₃)₂, or -COOH.

9) According to embodiments of the present invention, in above 1) to 8), it may be provided that said L₄ is
a single bond, -C1-C6 alkylene-, -NRm-(C1-C6 alkylene)-, or -NRn-, in which Rm and Rn are each independently -H or C1-C6 alkyl. Specifically, it may be provided that L₄ is a single bond, -(CH₂)-, -(CH₂)₂-, -(CH₂)₃- , -(CH₂)₄-, - (CH₂)₅-, -NH-, -NHCH₂-, -NH(CH₂)₂-, -NH(CH₂)₃-, -CH(CH₃)-, -CH₂NH-, - (CH₂)₂NH-, or -(CH₂)₃NH-. In this case, it may be provided that -H of said L₄ is unsubstituted or at least one -H of L₄ is each independently substituted with -C1-C6 alkyl.

10) According to embodiments of the present invention, in above 1) to 9), it may be provided that said R₃ is
H, C1-C6 alkyl, 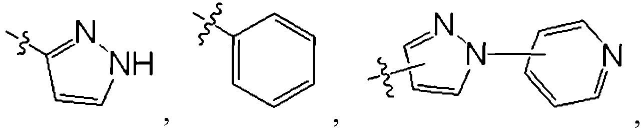 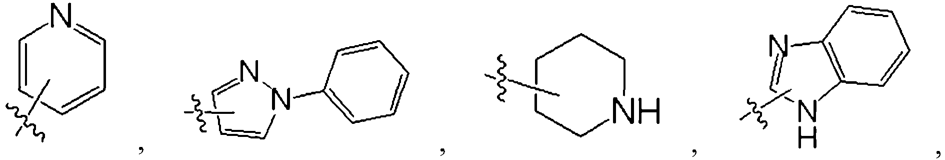 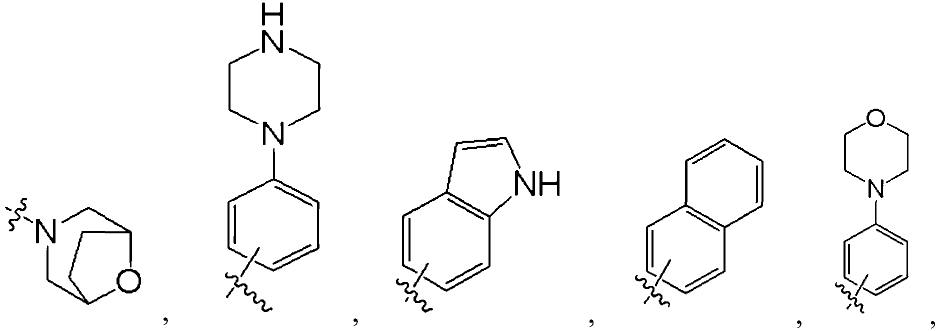 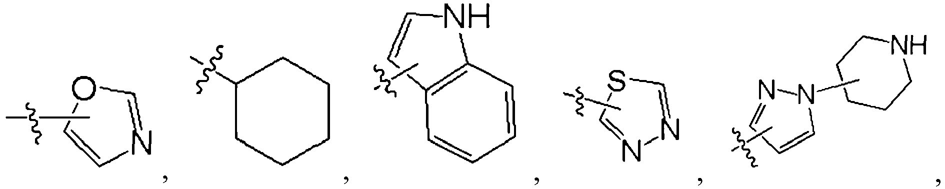 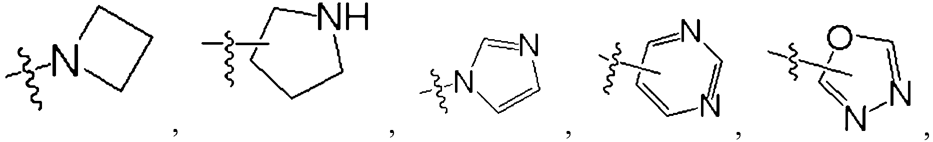 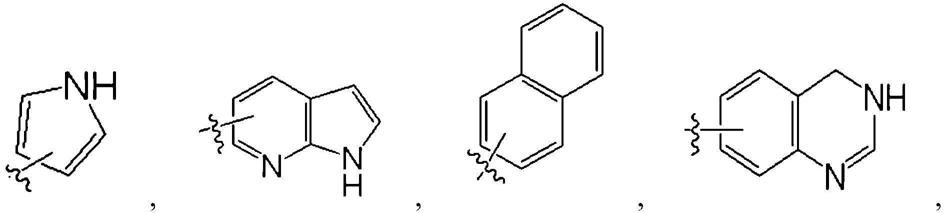 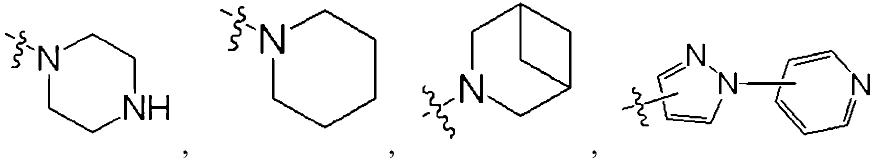 or 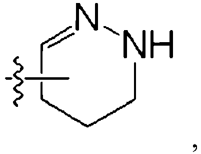 in which -H of said R₃ is unsubstituted or at least one H of R₃ is each independently substituted with -C1-C6 alkyl, -C1-C6 alkoxy, -phenoxy, - OH, -(C1-C6 alkylene)-OH, -COOH, -(C1-C6 alkylene)-COOH, -(C1-C6 alkylene)-CN, -NRoRp, (C1-C6 alkylene)-NRqRr, -C(=O)-(C1-C6 alkyl), - C(=O)-(C3-C8 cycloalkyl), -C=(O)-(6- to 14-membered aryl), -NO₂, -CHF₂, -CF₃, -OCF₃, -C(=O)NRsRt (in which -H of -C(=O)NRsRt is unsubstituted or at least one -H thereof is each independently substituted with halogen (F, Cl, Br or I)), - C(=O)-(C1-C6 alkylene)-NRuRv, -NRwRx, or halogen (F, Cl, Br or I), and when said R₃ is heterocycloalkenyl , CH₂- is unsubstituted or at least one -CH₂- is each substituted with -(C=O)-, in which Ro, Rp, Rq, Rr, Rs, Rt, Ru, Rv, Rw or Rx is each independently H or C1-C6 alkyl in each case. Specifically, it may be provided that -H of said R₃ is unsubstituted or at least one -H of R₃ is each independently substituted with -C1-C6 alkyl (for example, -CH₃, -CH₂CH₃, - CH₂CH₂CH₃, -CH(CH₃)₂, -CH₂CH(CH₃)₂, etc.) -C1-C6 alkoxy (for example, - OCH3, -OCH₂CH₃, -OCH₂CH₂CH₃, -OCH(CH₃)₂), -phenoxy, -OH, -CH₂F, - CHF₂, -CF₃, -OCF₃, halogen (F, Cl, Br or I), -(C=O)OCH₂CH₃, -(C=O)CH₃,-(C=O)CH(CH₃)₂, -(C=O)CHCH₃NH₂, -NH₂, -NHCH₃, -N(CH₃)₂, -COOH, - (C=O)NH₂, -(C=O)NHCH₃, -(C=O)N(CH₃)₂, -NO₂, -(C=O)NHCH₂CF₃, - CH(CH₃)OH, -CH₂(CH₃)OH, -CH₂CN, -CH(CH₃)CN, -C(CH₃)CH₂CN, - (CH₂)₂NH₂, -(CH₂)NH₂, -(CH₂)₃NH₂, -(CH₂)₄NH₂, -(CH₂)₂OH, -(CH₂)OH, - (CH₂)₃OH, -(CH₂)₄OH, -(CH₂)₂COOH, -(CH₂)COOH, -(CH₂)₃COOH, 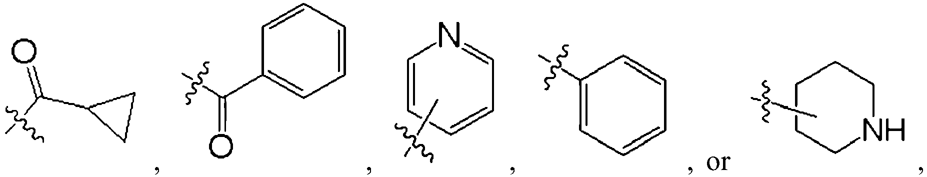 in which when said R₃ is 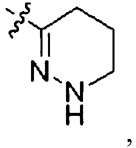 -CH₂- is unsubstituted or at least one - CH₂- is each independently substituted with -(C=O).

11) According to embodiments of the present invention, in above 1) to 10), it may be provided that the compound represented by above formula I is represented by formula I-1 below.

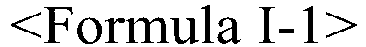

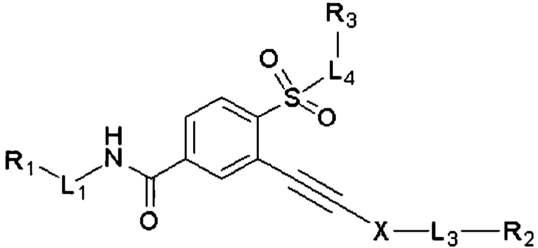

In above formula I-1, it may be provided that R₁, L₁, X, L₃, R₂, L₄, R₃ are substantially the same as defined in above formula I.

12) According to embodiments of the present invention, in above 1) to 11), it may be provided that the compound represented by above formula I is represented by formula I-2 below.

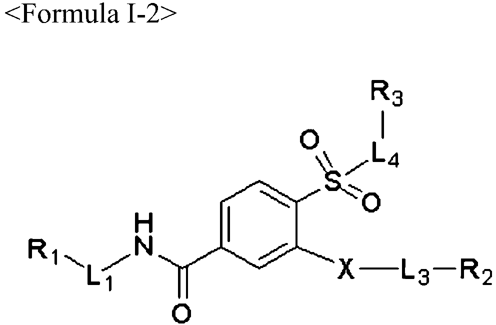

In above formula 1-2, it may be provided that R₁, L₁, X, L₃, R₂, L₄, R₃ are substantially the same as defined in above formula I.

13) According to embodiments of the present invention, in above 1) to 12), it may provide a compound represented by formula I-1 below, a stereoisomer thereof, a pharmaceutically acceptable salt of them, or a hydrate or solvate of them:

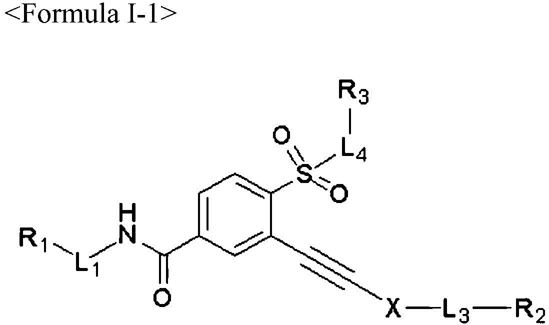

In above formula I-1, it may be provided that R₁, L₁, X, L₃, R₂, L₄, R₃ are substantially the same as defined in above formula I.

According to embodiments of the present invention, in above formula I-1, it may be provided that R₁ is 5- to 12-membered heteroaryl including at least one heteroatom selected from the group consisting of N, O and S, in which -H of said R₁ is unsubstituted or at least one H thereof is each independently substituted with halogen (F, Cl, Br, I), -NH₂, -NHCH₃, -N(CH₃)₂, -NO₂ or -OH.

14) According to embodiments of the present invention, in above 1) to 13), it may be provided that in above formula I-1, R₁ is 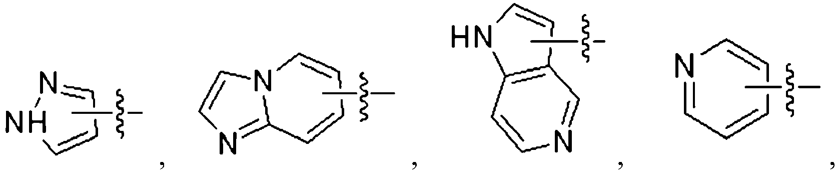 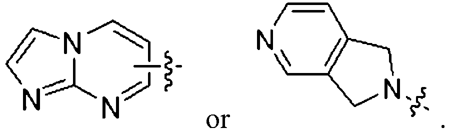 In this case, it may be provided that -H of said R₁ is unsubstituted or at least one H thereof is each independently substituted with halogen (F, Cl, Br, or I), -NH₂, -NO₂, -NHCH₃, -N(CH₃)₂ or -OH.

15) According to embodiments of the present invention, in above 1) to 14), it may be provided that in above formula I-1, L₁ is C1-C6 alkylene, specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), sec-butylene (-CHCH₃CH₂CH₂-), isobutylene (-CH₂CHCH₃CH₂-), tert-butylene (-CCH₃CH₃CH₂-), n-pentylene (-CH₂CH₂CH₂CH₂CH₂-), n-hexylene (-CH₂CH₂CH₂CH₂CH₂CH₂-) or the like, and specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), n-pentylene (-CH₂CH₂CH₂CH₂CH₂-) or n-hexylene (-CH₂CH₂CH₂CH₂CH₂CH₂-).

16) According to embodiments of the present invention, in above 1) to 15), it may be provided that in above formula I-1, X is 6- to 14-membered arylene, C2-C8 heterocycloalkenylene including at least one heteroatom or heterofunctional group selected from the group consisting of N, O, S, S(=O), and S(=O)₂, or 5- to 12-membered heteroarylene including at least one heteroatom selected from the group consisting of N, O and S, in which -H of said X is unsubstituted, or when said X is 6- to 14-membered arylene or C2-C8 heterocycloalkenylene, at least one -H thereof is each independently substituted with C1-C6 alkyl, -CF₃, halogen (F, Cl, Br or I), -C1-C6 alkoxy or OH, and when said X is C2-C8 heterocycloalkenylene, -CH₂- is unsubstituted or at least one - CH₂- is each independently substituted with -(C=O)-.

17) According to embodiments of the present invention, in above 1) to 16), it may be provided that in above formula 1-1, X is 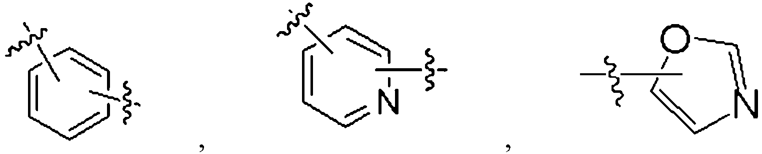 or 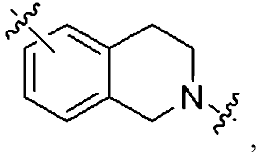 in which -H of said X is unsubstituted or at least one -H thereof is each independently substituted with C1-C6 alkyl, halogen (F, Br, Cl, or I), -OH, -C1-C6 alkoxy, or -CF₃, and specifically when said X is 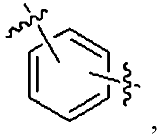 H is unsubstituted or at least one -H thereof is each independently substituted with C1-C6 alkyl, halogen (F, Br, Cl, or I), -C1-C6 alkoxy, -OH, or -CF₃.

18) According to embodiments of the present invention, in above 1) to 17), it may be provided that in above formula I-1, said L₃ is a single bond, C1-C6 alkylene, -(C=O)-, or -C(=O)NRd-(C1-C6 alkylene)-, in which Rd is -H or C1-C6 alkyl. Specifically, it may be provided that L₃ is a single bond, -(CH₂)-, - (CH₂)₂-, -(CH₂)₃-, -(CH₂)₄-, -(CH₂)₅-, -(C=O)-NH(CH₂)n, -(C=O)NH-, or - (C=O)-, in which n is 0, 1, 2, 3 or 4.

19) According to embodiments of the present invention, in above 1) to 18), it may be provided that in above formula I-1, said R₂ is H, halogen (F, Cl, Br or I), -NRz₁Rz₂, C1-C6 alkyl, C1-C6 alkoxy, or C1-C8 heterocycloalkyl including at least one heteroatom or heterofunctional group selected from the group consisting of N, O, S, SO and SO₂, in which -H of said R₂ is unsubstituted or at least one -H of R₂ is each independently substituted with -C1-C6 alkyl, -C1-C6 alkoxy, -NRgRh, or halogen (F, Cl, Br or I), in which Rg, Rh, Rz₁ and Rz₂ are each independently H or C1-C6 alkyl.

20) According to embodiments of the present invention, in above 1) to 19), it may be provided that in above formula I-1, said R₂ is H, halogen (F, Cl, Br or I), C1-C6 alkyl, C1-C6 alkoxy, -NH₂, 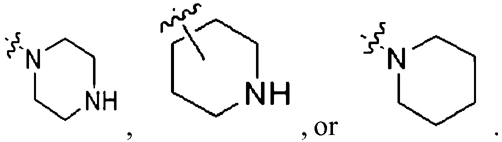 In this case, it may be provided that -H of said R₂ is unsubstituted or at least one -H of R₂ is each independently substituted with halogen (F, Cl, Br or I), -C1-C6 alkoxy, -NH₂, or -C1-C6 alkyl.

21) According to embodiments of the present invention, in above 1) to 20), it may be provided that in above formula I-1, L₄ is a single bond, -C1-C6 alkylene-, -NRm-(C1-C6 alkylene), or -NRn-, in which -H of said L₄ is unsubstituted or at least one -H of L₄ is each independently substituted with -C1-C6 alkyl, in which Rm and Rn are each independently H or C1-C6 alkyl.

22) According to embodiments of the present invention, in above 1) to 21), it may be provided that in above formula I-1, L₄ is a single bond, -(CH₂)-, -(CH₂)₂-, -(CH₂)₃- , -(CH₂)₄- , -(CH₂)₅-, -NH-, -NHCH₂-, -NH(CH₂)₂-, -NH(CH₂)₃-, in which -H of said L₄ is unsubstituted or at least one -H of L₄ is each independently substituted with -C1-C6 alkyl.

23) According to embodiments of the present invention, in above 1) to 22), it may be provided that in above formula I-1, R₃ is H, C1-C6 alkyl, 6- to 14-membered aryl, 5- to 12-membered heteroaryl including at least one heteroatom selected from the group consisting of N, O, and S, 5- to 12-membered heteroarylene-6- to 14-membered aryl (in which said heteroarylene or heteroaryl includes at least one heteroatom selected from the group consisting of N, O, and S), or 5- to 12-membered heteroarylene-5- to 12-membered heteroaryl (in which said heteroarylene or heteroaryl includes at least one heteroatom selected from the group consisting of N, O and S), in which -H of said R₃ is unsubstituted or at least one H of R₃ is each independently substituted with -C1-C6 alkyl, -C1-C6 alkoxy, - 6- to 14-membered heteroaryl including at least one heteroatom selected from the group consisting of -N, O and S, -NRwRx, or halogen (F, Cl, Br or I), in which Rw and Rx are each independently H or C1-C6 alkyl.

24) According to embodiments of the present invention, in above 1) to 23), it may be provided that in above formula I-1, said R₃ is H, C1-C6 alkyl, 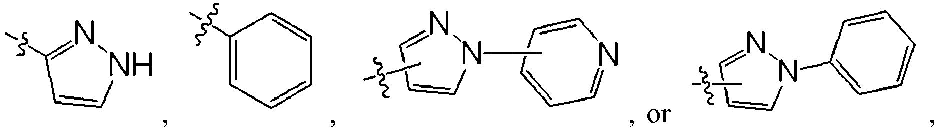 in which -H of said R₃ is unsubstituted or at least one -H of R₃ is each independently substituted with -C1-C6 alkyl (for example, -CH₃, -CH₂CH₃, - CH₂CH₂CH₃, -CH(CH₃)₂, -CH₂CH(CH₃)₂, etc.) -C1-C6 alkoxy (for example, - OCH3, -OCH₂CH₃, -OCH₂CH₂CH₃, -OCH(CH₃)₂), -NH₂, -CF₃, -OCF₃, halogen (F, Cl, Br or I), or

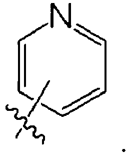

25) According to embodiments of the present invention, in above 1) to 11), it may provide a compound represented by formula I-2 below, a stereoisomer thereof, a pharmaceutically acceptable salt of them, or a hydrate or solvate of them:

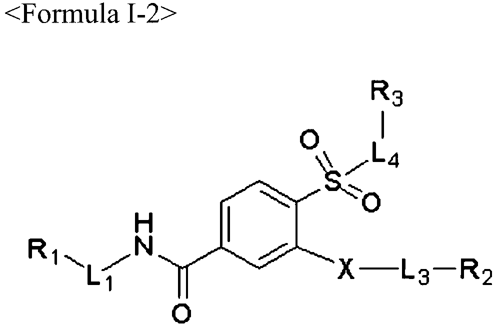

In above formula 1-2, it may be provided that R₁, L₁, X, L₃, R₂, L₄, R₃ are substantially the same as defined in above formula I.

26) According to embodiments of the present invention, in above 1) to 11) and 25), it may be provided that in above formula I-2, R₁ is 5- to 12-membered heteroaryl including at least one heteroatom selected from the group consisting of N, O and S, in which -H of said R₁ is unsubstituted or at least one H thereof is each independently substituted with halogen (F, Cl, Br, I), -NH₂, -NHCH₃, - N(CH₃)₂, -NO₂ or -OH.

27) According to embodiments of the present invention, in above 1) to 11), 25) and 26), it may be provided that in above formula 1-2, R₁ is 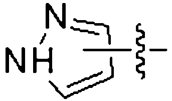 or 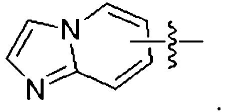 In this case, it may be provided that -H of said R₁ is unsubstituted or at least one H thereof is each independently substituted with halogen (F, Cl, Br, or I), -NH₂, -NO₂, -NHCH₃, -N(CH₃)₂ or -OH.

28) According to embodiments of the present invention, in above 1) to 11) and 25) to 27), it may be provided that in above formula I-2, L₁ is C1-C6 alkylene, specifically methylene (-CH₂-), ethylene (-CH₂CH₂-), n-propylene (-CH₂CH₂CH₂-), isopropylene (-CHCH₃CH₂), n-butylene (-CH₂CH₂CH₂CH₂-), sec-butylene (-CHCH₃CH₂CH₂-), isobutylene (-CH₂CHCH₃CH₂-), tert-butylene (-CCH₃CH₃CH₂-), n-pentylene (-CH₂CH₂CH₂CH₂CH₂-), n-hexylene (-CH₂CH₂CH₂CH₂CH₂CH₂-) or the like, and specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), n-pentylene (-CH₂CH₂CH₂CH₂CH₂-) or n-hexylene (-CH₂CH₂CH₂CH₂CH₂CH₂-).

29) According to embodiments of the present invention, in above 1) to 11) and 25) to 28), it may be provided that in above formula 1-2, X is C2-C8 heterocycloalkenylene including at least one heteroatom or heterofunctional group selected from the group consisting of N, O, S, S(=O), and S(=O)₂, or 5- to 12-membered heteroarylene including at least one heteroatom selected from the group consisting of N, O and S, in which -H of said X is unsubstituted, or when said X is C2-C8 heterocycloalkenylene, at least one -H thereof is each independently substituted with C1-C6 alkyl, -CF₃, halogen (F, Cl, Br or I), -C1-C6 alkoxy or OH, and when said X is C2-C8 heterocycloalkenylene, -CH₂- is unsubstituted or at least one -CH₂- is each independently substituted with -(C=O)-.

30) According to embodiments of the present invention, in above 1) to 11) and 25) to 29), it may be provided that in above formula I-2, said X is 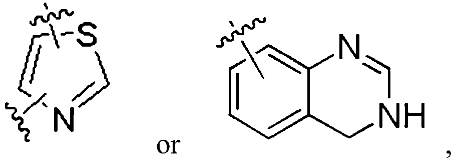 in which -H of said X is unsubstituted or at least one -H thereof is each independently substituted with C1-C6 alkyl, halogen (F, Br, Cl, or I), -OH, -C1-C6 alkoxy, or -CF₃, and when said X is 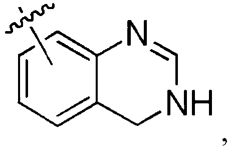 -CH₂- is unsubstituted or at least one -CH₂- is each independently substituted with -(C=O)-.

31) According to embodiments of the present invention, in above 1) to 11) and 25) to 30), it may be provided that in above formula I-2, said L₃ is a single bond or C1-C6 alkylene, and specifically L₃ is a single bond, -(CH₂)-, -(CH₂)₂-, - (CH₂)₃-, -(CH₂)₄- or -(CH₂)₅-.

32) According to embodiments of the present invention, in above 1) to 11) and 25) to 31), it may be provided that in above formula I-2, said R₂ is H, halogen (F, Cl, Br or I), -NRz₁Rz₂, or 6- to 14-membered aryl, in which -H of said R₂ is unsubstituted or at least one -H of R₂ is each independently substituted with -C1-C6 alkyl, -C1-C6 alkoxy, -NRgRh, or halogen (F, Cl, Br or I), in which Rg, Rh, Rz₁ and Rz₂ are each independently H or C1-C6 alkyl.

33) According to embodiments of the present invention, in above 1) to 11) and 25) to 32), it may be provided that in above formula I-2, said R₂ is H, halogen (F, Cl, Br or I), -NH₂, or 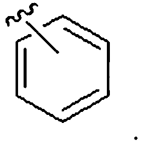 In this case, it may be provided that -H of said R₂ is unsubstituted or at least one -H of R₂ is each independently substituted with halogen (F, Cl, Br or I), -C1-C6 alkoxy, -NH₂, or -C1-C6 alkyl.

34) According to embodiments of the present invention, in above 1) to 11) and 25) to 32), it may be provided that in above formula I-2, L₄ is a single bond, -C1-C6 alkylene-, in which -H of said L₄ is unsubstituted or at least one -H of L₄ is each independently substituted with -C1-C6 alkyl.

35) According to embodiments of the present invention, in above 1) to 11) and 25) to 33), it may be provided that in above formula I-2, L₄ is a single bond, -(CH₂)-, -(CH₂)₂-, -(CH₂)₃- , -(CH₂)₄- or -(CH₂)₅-, in which -H of said L₄ is unsubstituted or at least one -H of L₄ is each independently substituted with -C1-C6 alkyl.

36) According to embodiments of the present invention, in above 1) to 11) and 25) to 34), it may be provided that in above formula I-2, R₃ is 5- to 12-membered heteroaryl including at least one heteroatom selected from the group consisting of N, O and S, in which -H of said R₃ is unsubstituted or at least one H of R₃ is each independently substituted with -C1-C6 alkyl, -C1-C6 alkoxy, or halogen (F, Cl, Br or I).

37) According to embodiments of the present invention, in above 1) to 11) and 25) to 35), it may be provided that in above formula I-2, said R₃ is 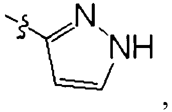 in which -H of said R₃ is unsubstituted or at least one -H of R₃ is each independently substituted with -C1-C6 alkyl (for example, -CH₃, -CH₂CH₃, -CH₂CH₂CH₃, -CH(CH₃)₂, -CH₂CH(CH₃)₂, etc.) -C1-C6 alkoxy (for example, - OCH3, -OCH₂CH₃, -OCH₂CH₂CH₃, -OCH(CH₃)₂), or halogen (F, Cl, Br or I).

38) According to embodiments of the present invention, in above 1) to 37), the present invention may provide a compound described in table 1 below, a stereoisomer thereof, a pharmaceutically acceptable salt of them, or a hydrate or solvate of them.

**[Table 1]**

| NO | Compound |
|---|---|
| 1 | 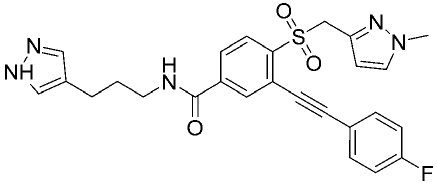 |
| 2 | 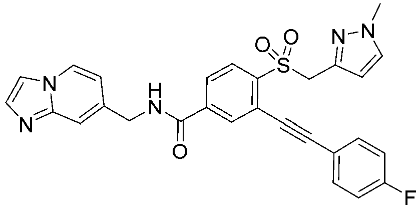 |
| 3 | 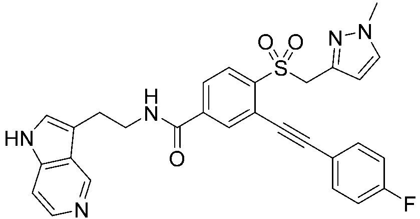 |
| 4 | 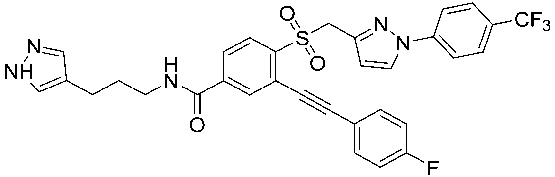 |
| 5 | 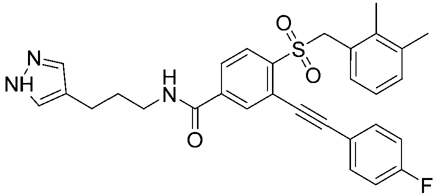 |
| 6 | 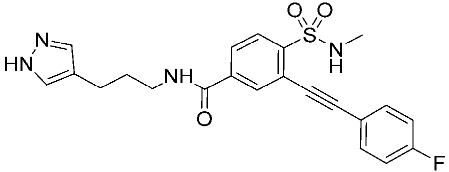 |
| 7 | 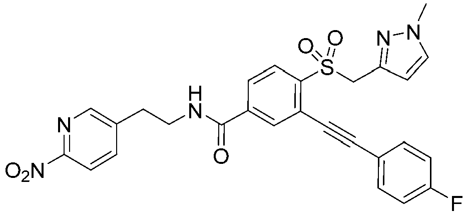 |
| 8 | 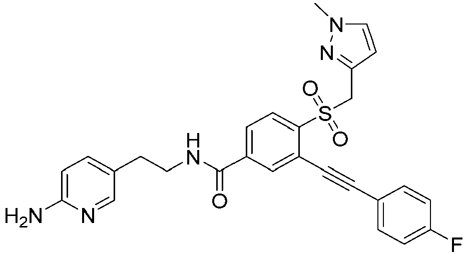 |
| 9 | 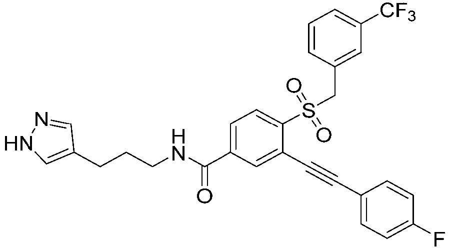 |
| 10 | 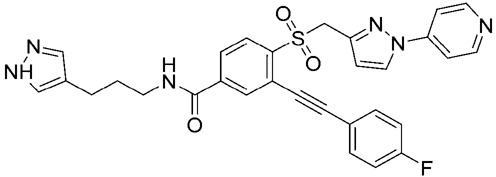 |
| 11 | 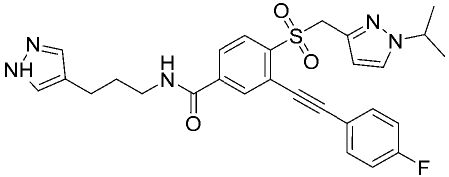 |
| 12 | 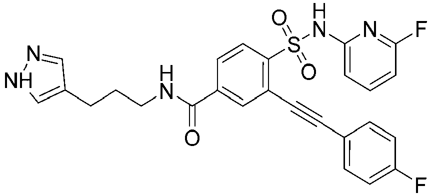 |
| 13 | 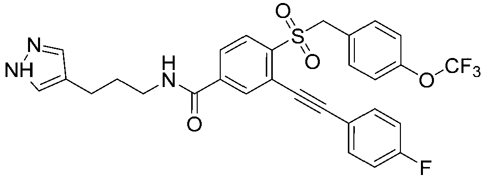 |
| 14 | 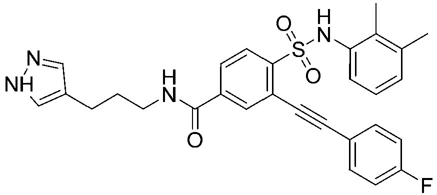 |
| 15 | 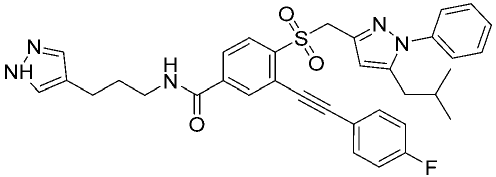 |
| 16 | 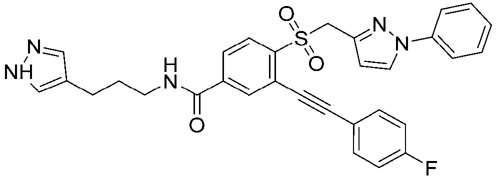 |
| 17 | 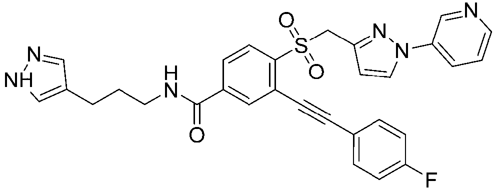 |
| 18 | 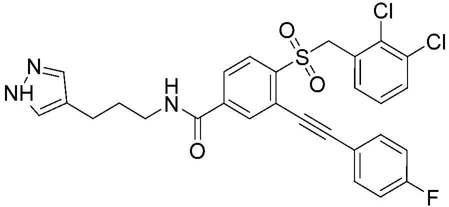 |
| 19 | 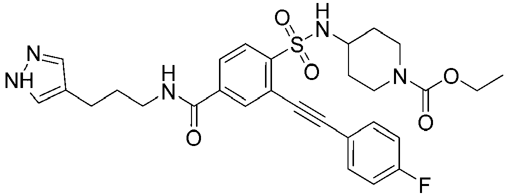 |
| 20 | 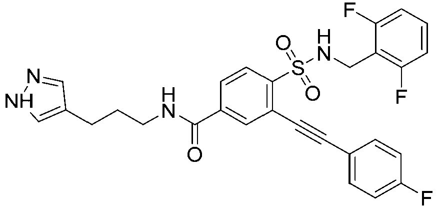 |
| 21 | 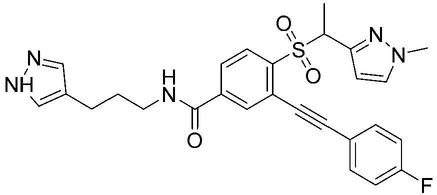 |
| 22 | 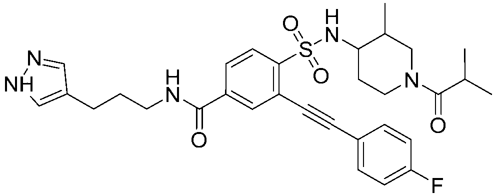 |
| 23 | 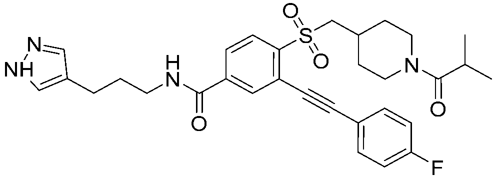 |
| 24 | 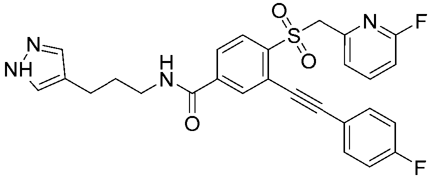 |
| 25 | 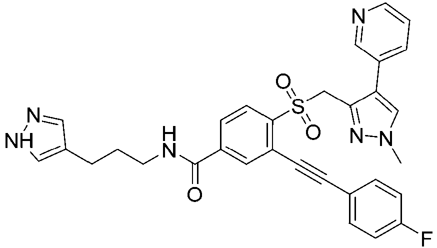 |
| 26 | 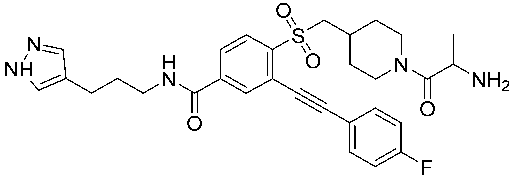 |
| 27 | 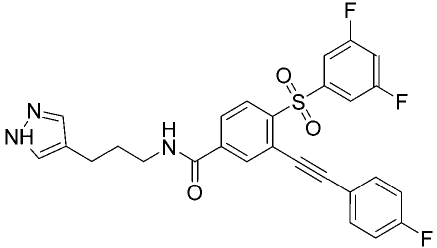 |
| 28 | 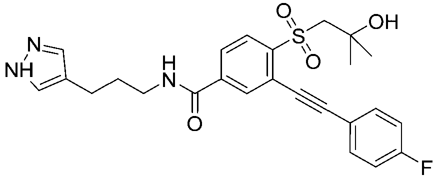 |
| 29 | 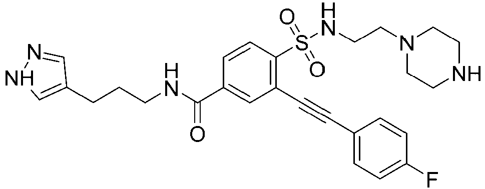 |
| 30 | 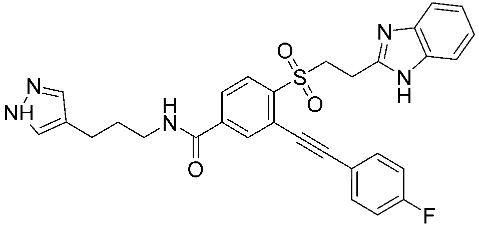 |
| 31 | 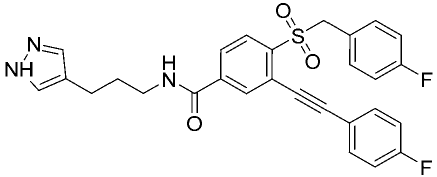 |
| 32 | 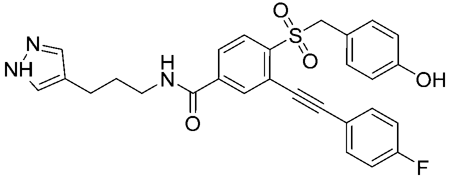 |
| 33 | 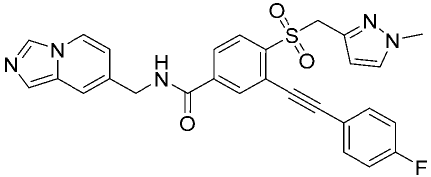 |
| 34 | 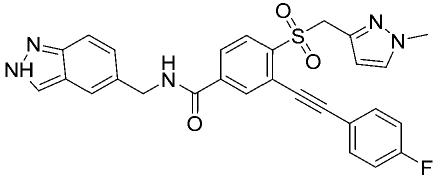 |
| 35 | 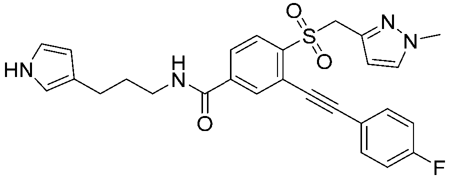 |
| 36 | 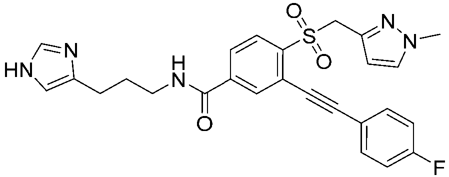 |
| 37 | 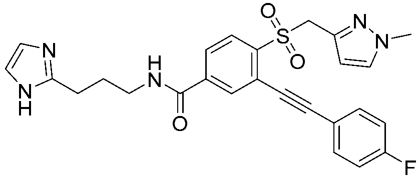 |
| 38 | 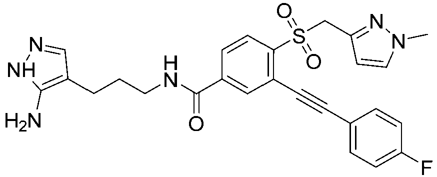 |
| 39 | 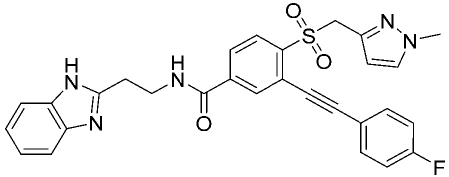 |
| 40 | 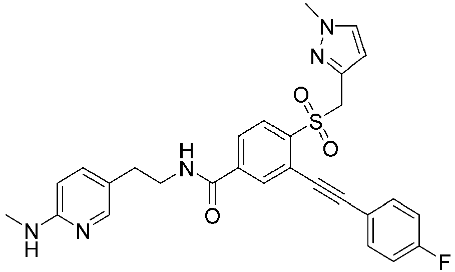 |
| 41 | 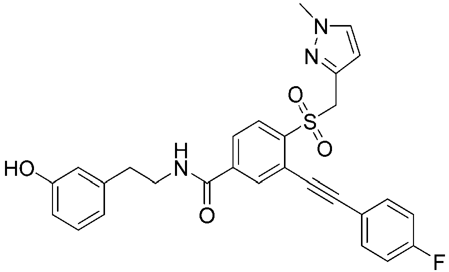 |
| 42 | 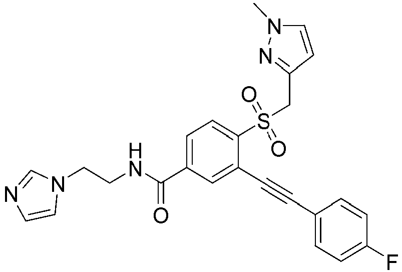 |
| 43 | 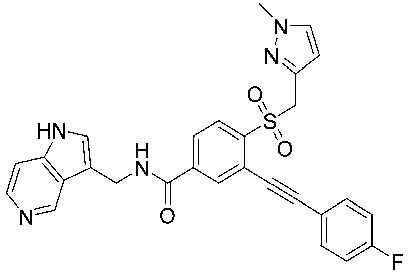 |
| 44 | 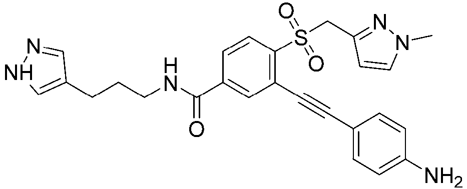 |
| 45 | 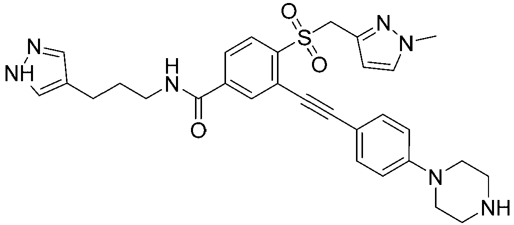 |
| 46 | 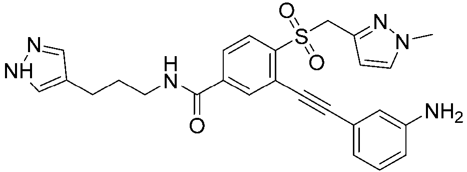 |
| 47 | 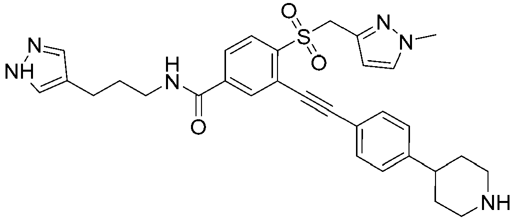 |
| 48 | 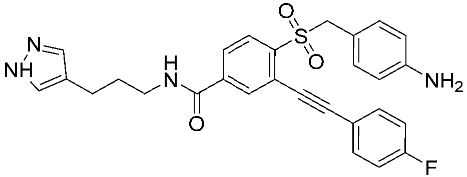 |
| 49 | 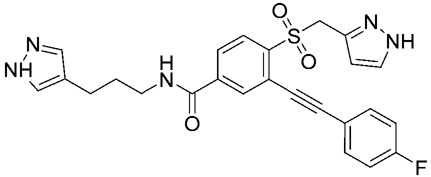 |
| 50 | 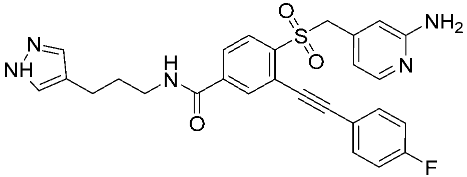 |
| 51 | 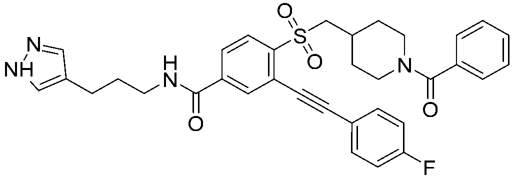 |
| 52 | 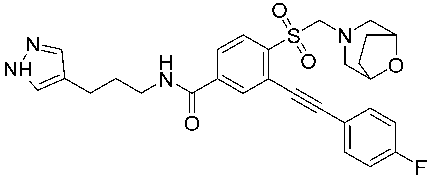 |
| 53 | 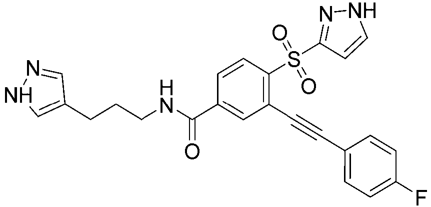 |
| 54 | 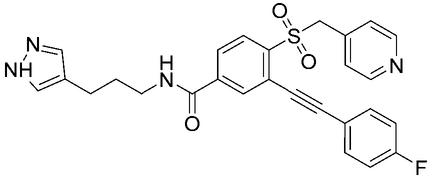 |
| 55 | 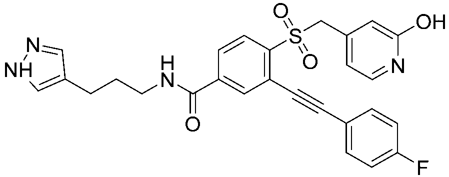 |
| 56 | 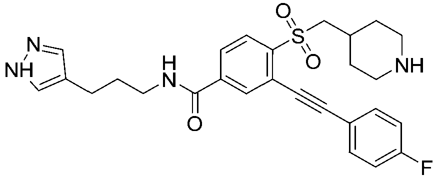 |
| 57 | 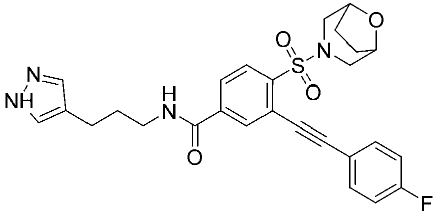 |
| 58 | 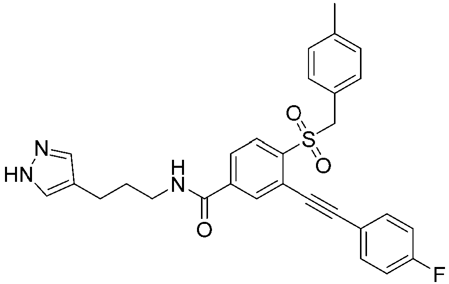 |
| 59 | 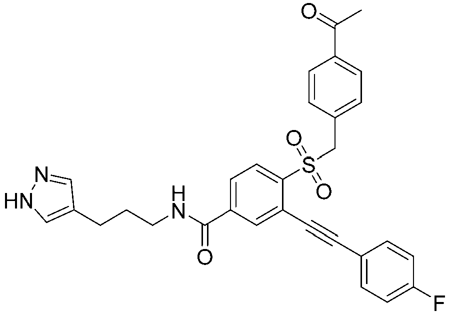 |
| 60 | 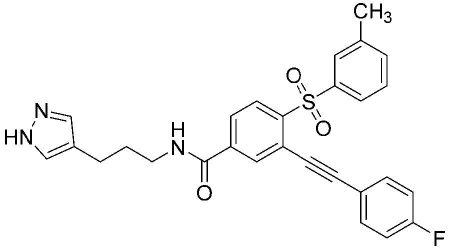 |
| 61 | 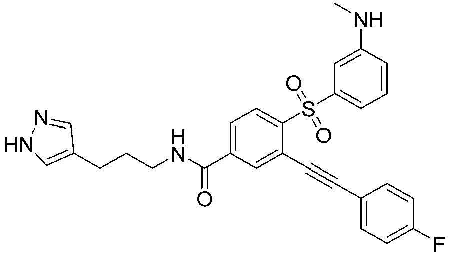 |
| 62 | 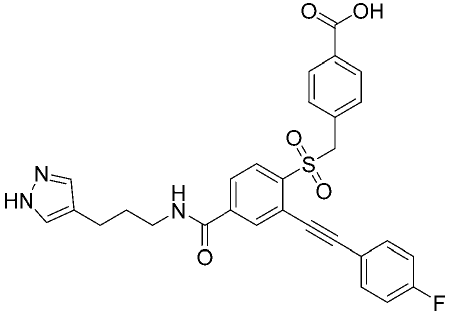 |
| 63 | 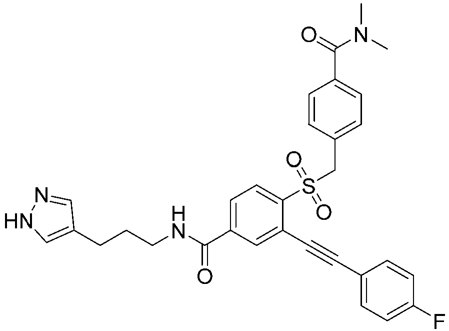 |
| 64 | 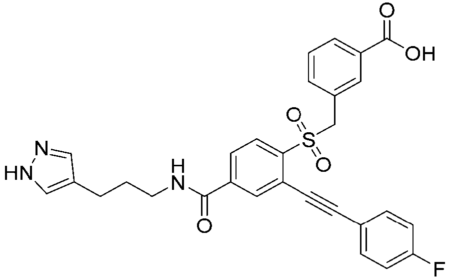 |
| 65 | 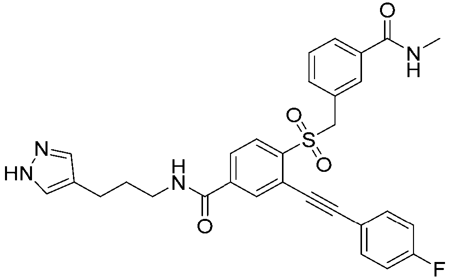 |
| 66 | 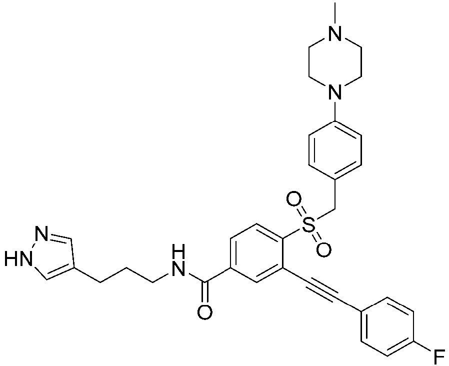 |
| 67 | 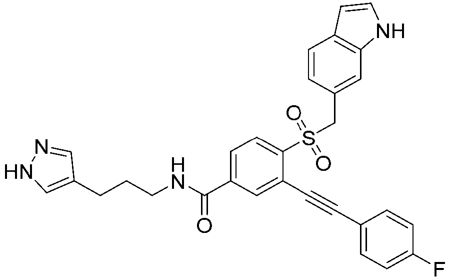 |
| 68 | 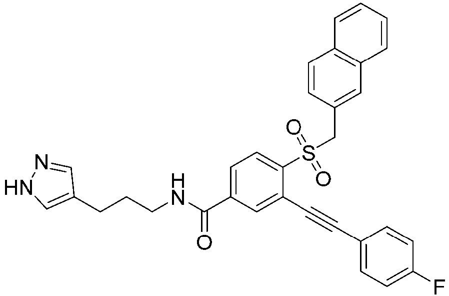 |
| 69 | 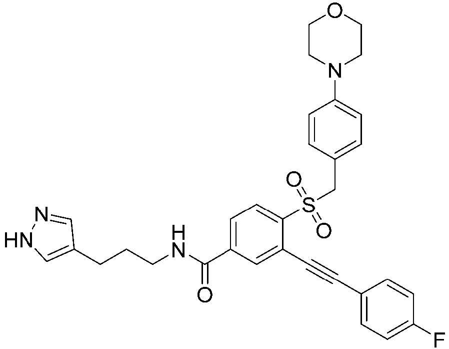 |
| 70 | 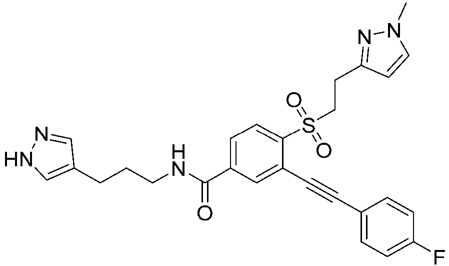 |
| 71 | 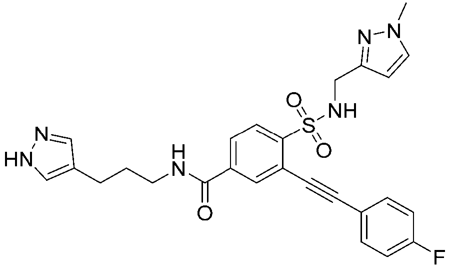 |
| 72 | 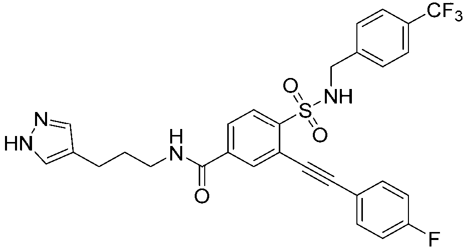 |
| 73 | 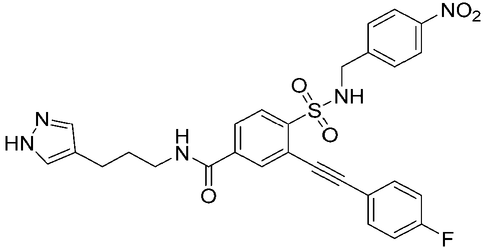 |
| 74 | 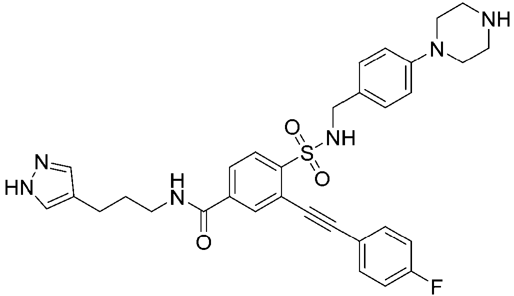 |
| 75 | 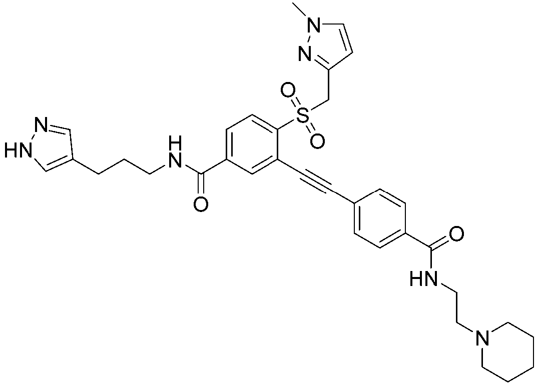 |
| 76 | 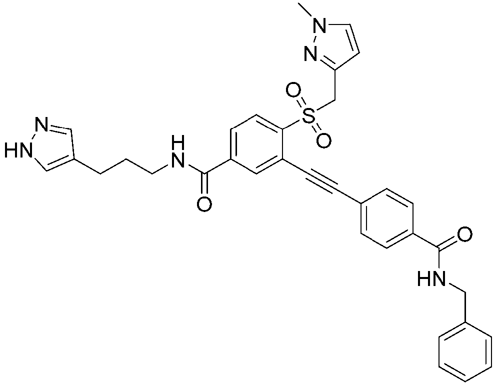 |
| 77 | 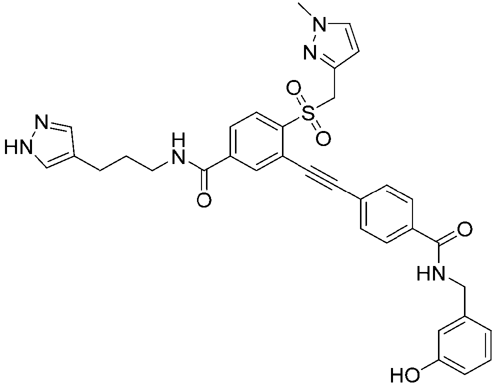 |
| 78 | 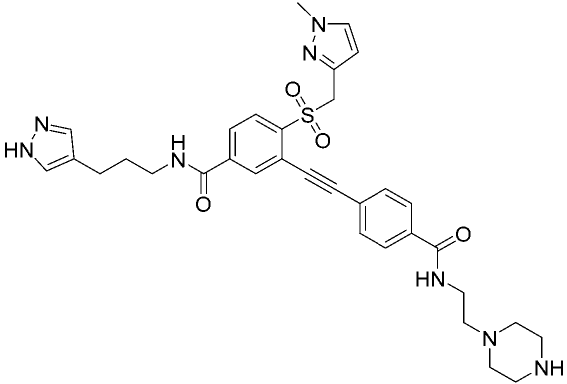 |
| 79 | 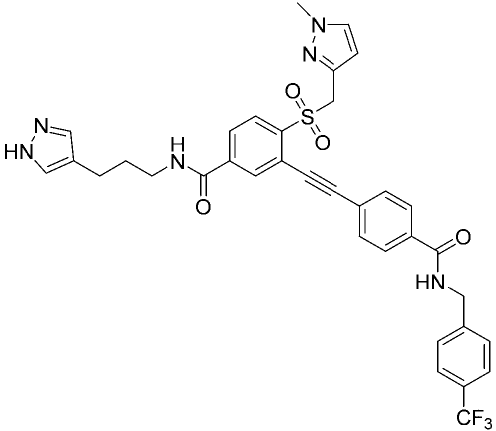 |
| 80 | 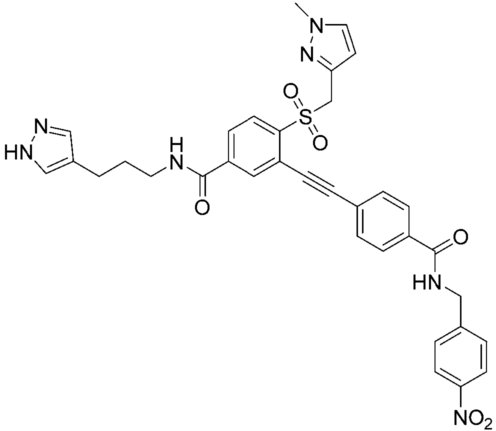 |
| 81 | 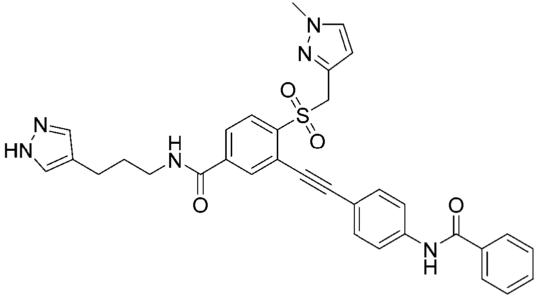 |
| 82 | 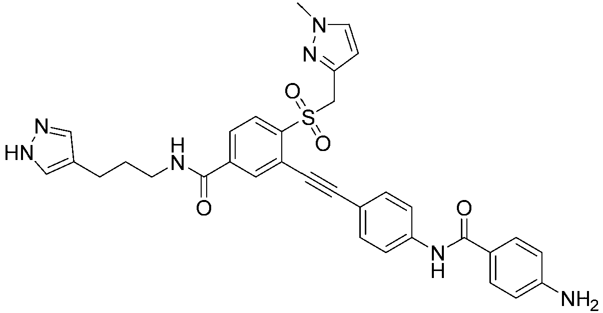 |
| 83 | 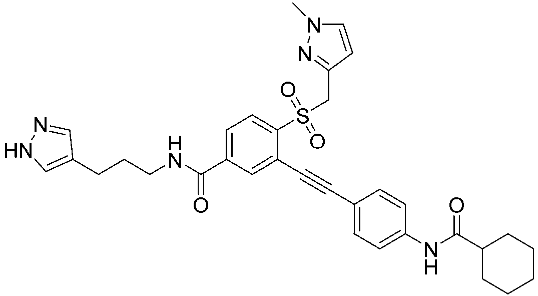 |
| 84 | 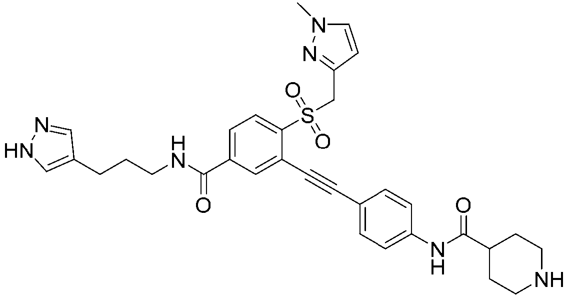 |
| 85 | 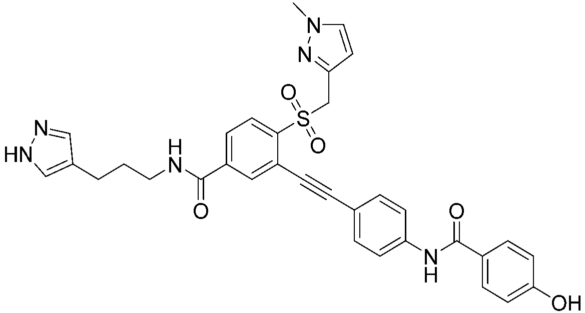 |
| 86 | 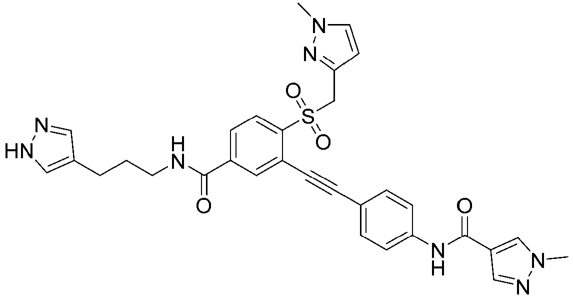 |
| 87 | 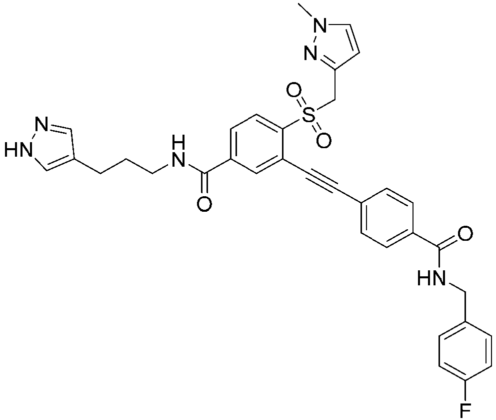 |
| 88 | 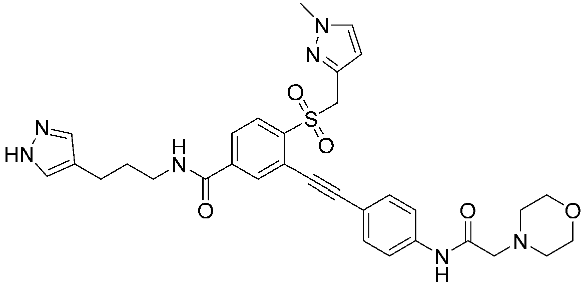 |
| 89 | 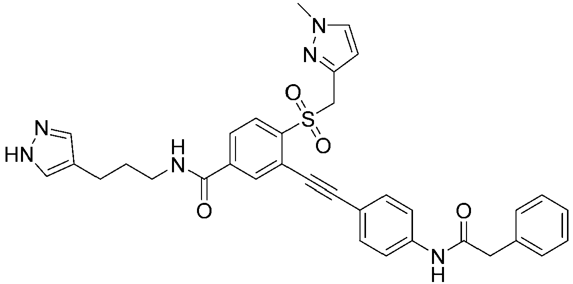 |
| 90 | 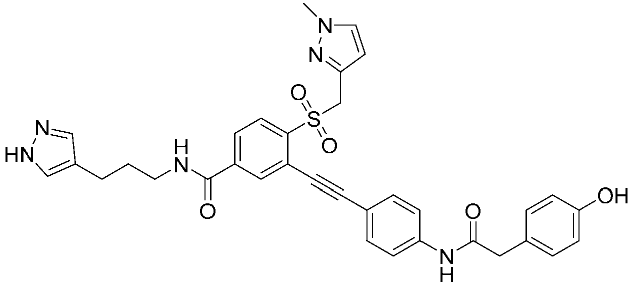 |
| 91 | 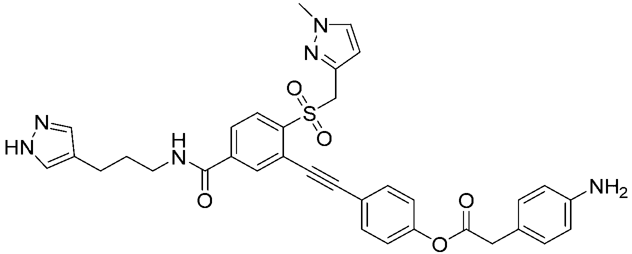 |
| 92 | 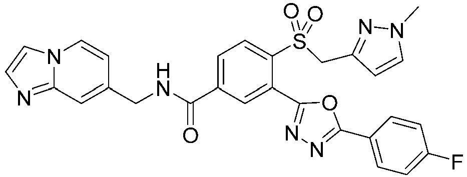 |
| 93 | 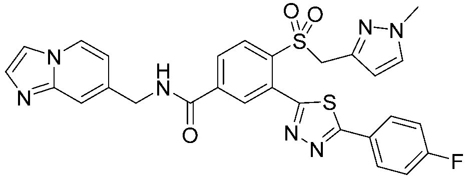 |
| 94 | 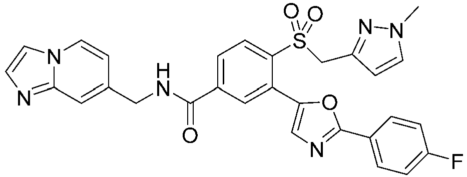 |
| 95 | 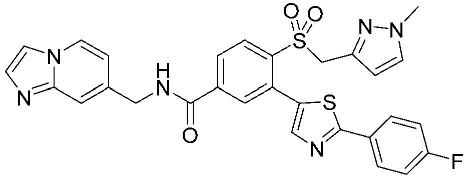 |
| 96 | 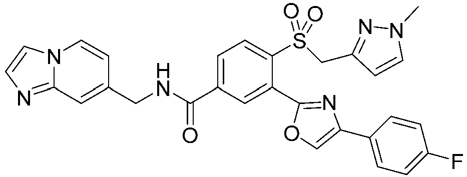 |
| 97 | 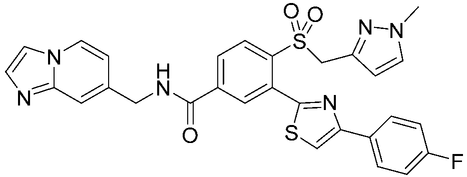 |
| 98 | 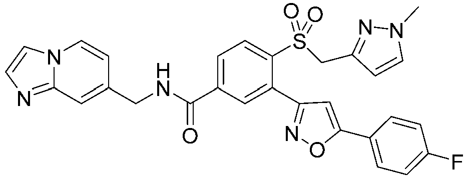 |
| 99 | 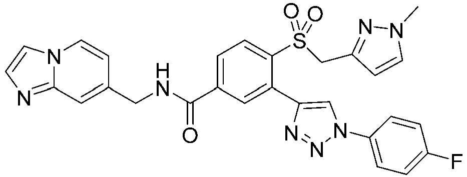 |
| 100 | 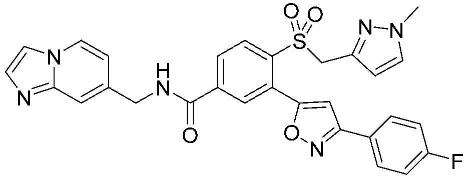 |
| 101 | 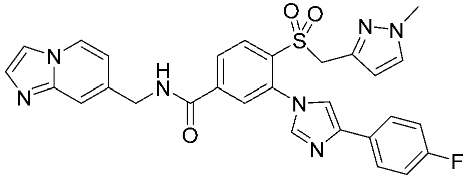 |
| 102 | 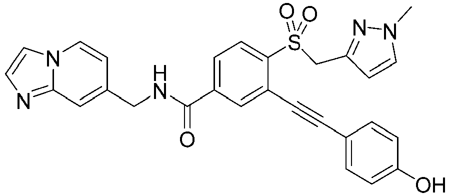 |
| 103 | 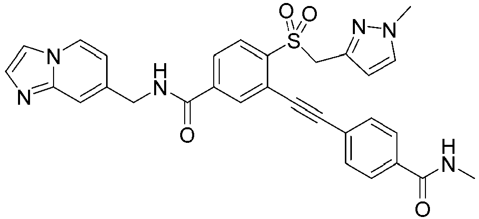 |
| 104 | 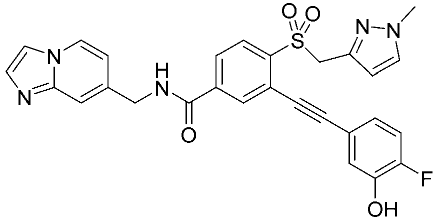 |
| 105 | 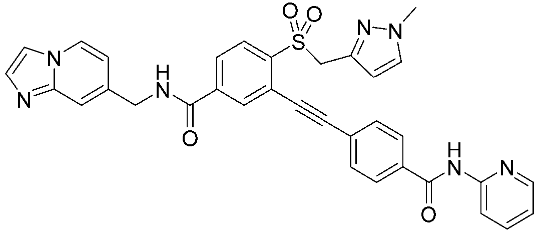 |
| 106 | 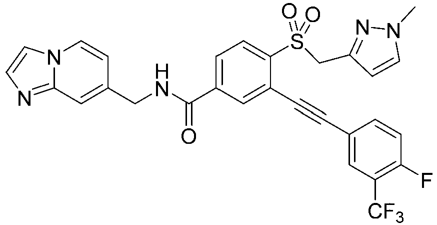 |
| 107 | 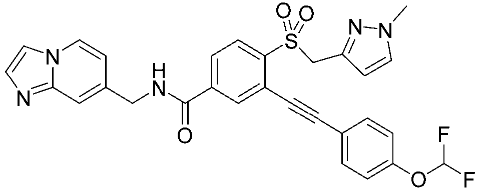 |
| 108 | 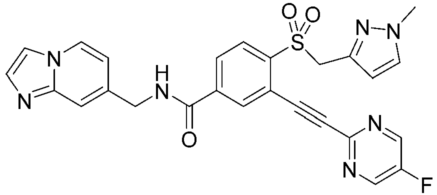 |
| 109 | 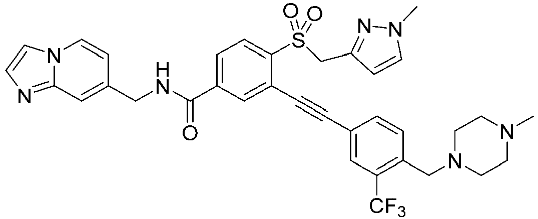 |
| 110 | 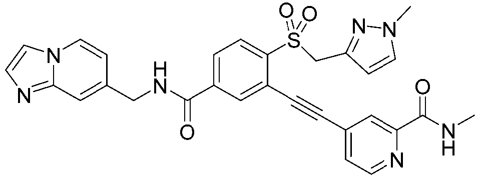 |
| 111 | 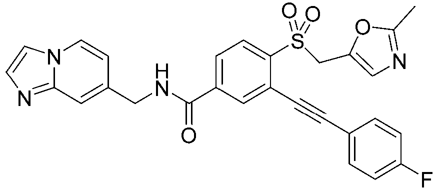 |
| 112 | 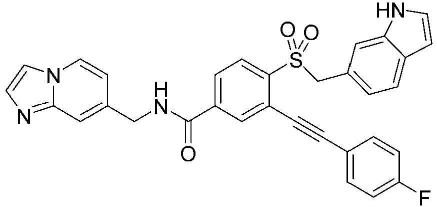 |
| 113 | 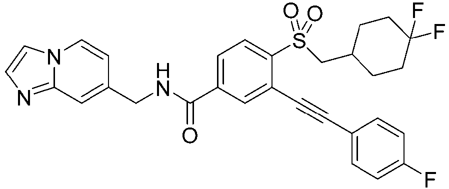 |
| 114 | 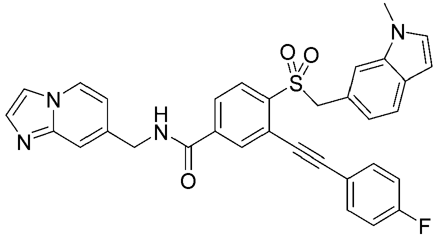 |
| 115 | 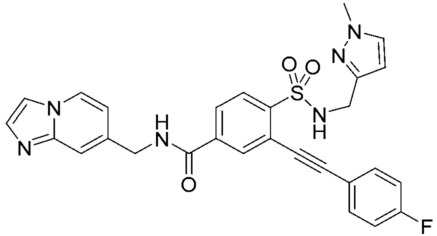 |
| 116 | 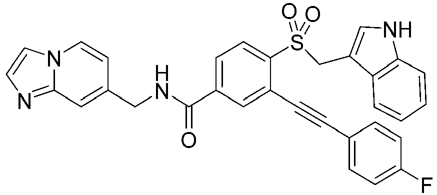 |
| 117 | 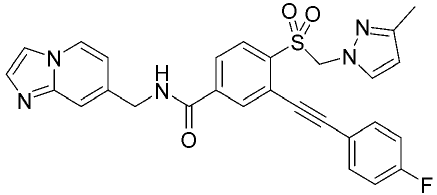 |
| 118 | 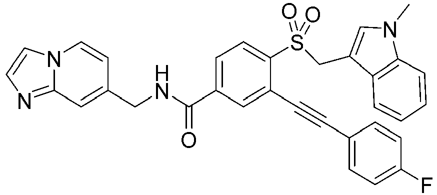 |
| 119 | 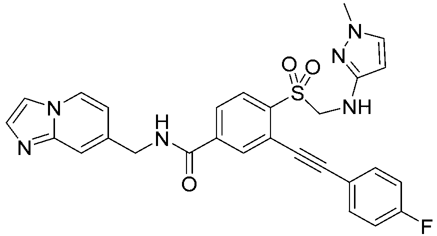 |
| 120 | 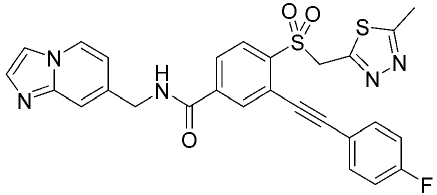 |
| 121 | 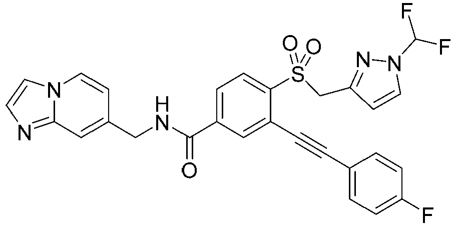 |
| 122 | 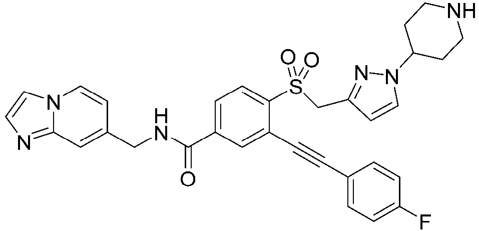 |
| 123 | 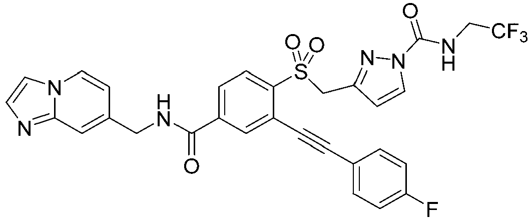 |
| 124 | 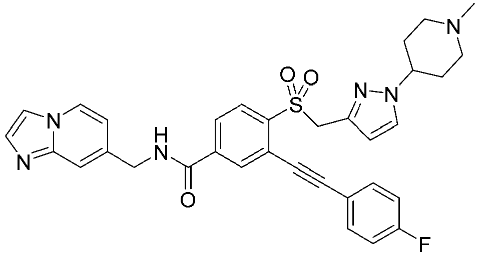 |
| 125 | 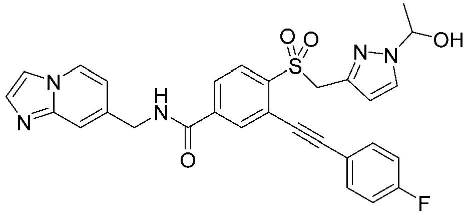 |
| 126 | 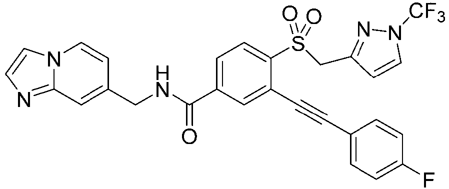 |
| 127 | 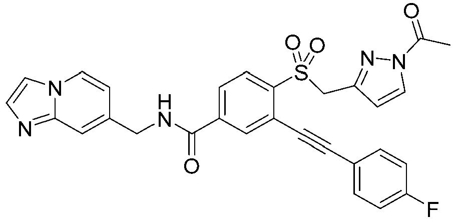 |
| 128 | 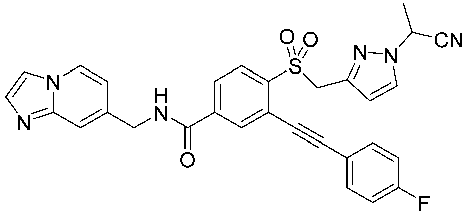 |
| 129 | 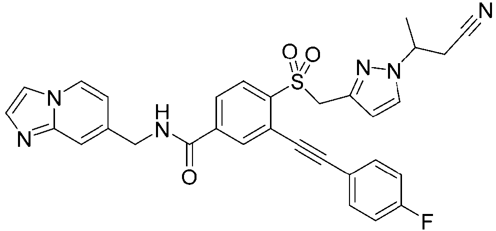 |
| 130 | 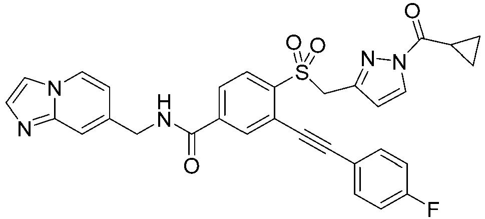 |
| 131 | 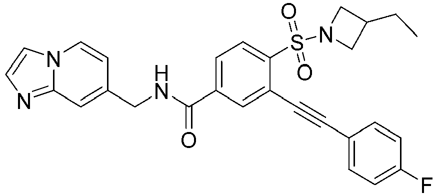 |
| 132 | 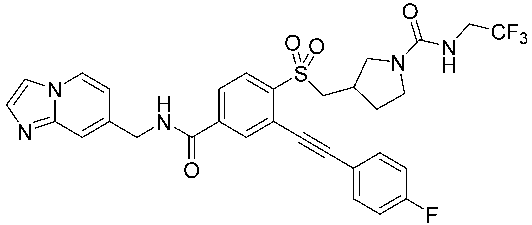 |
| 133 | 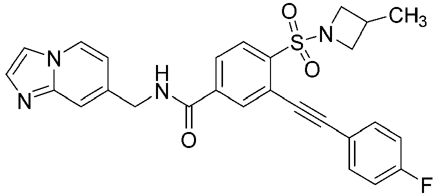 |
| 134 | 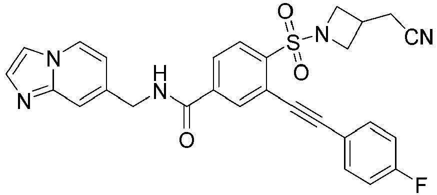 |
| 135 | 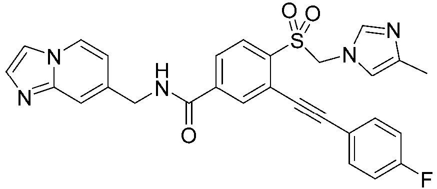 |
| 136 | 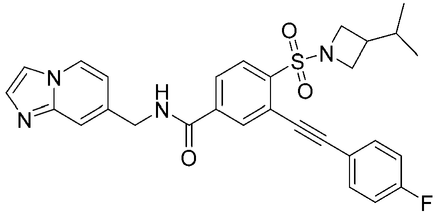 |
| 137 | 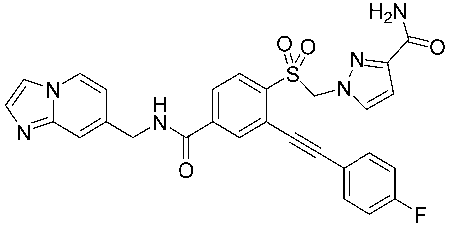 |
| 138 | 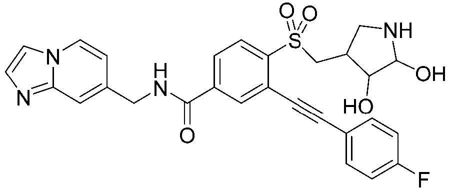 |
| 139 | 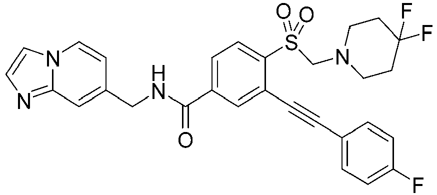 |
| 140 | 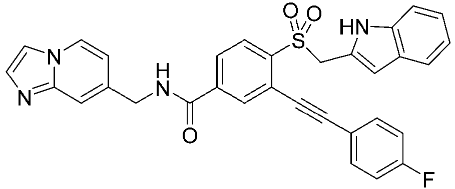 |
| 141 | 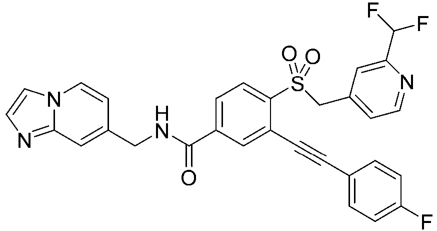 |
| 142 | 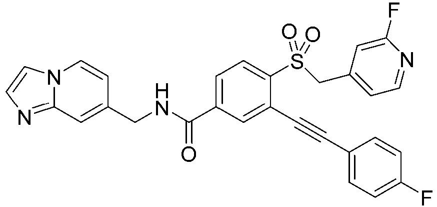 |
| 143 | 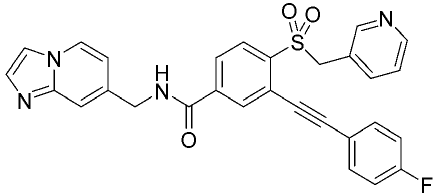 |
| 144 | 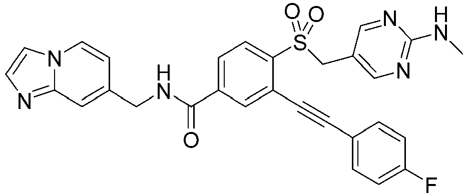 |
| 145 | 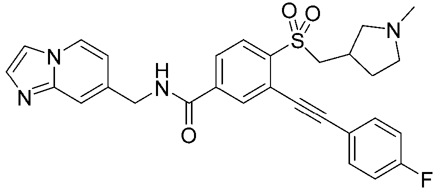 |
| 146 | 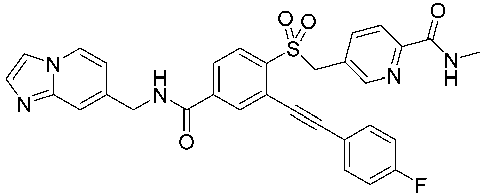 |
| 147 | 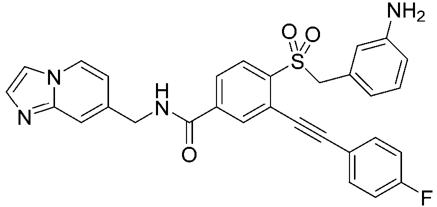 |
| 148 | 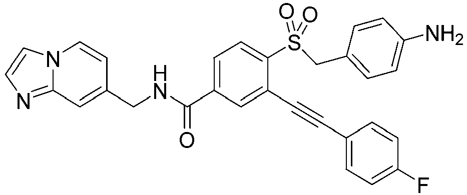 |
| 149 | 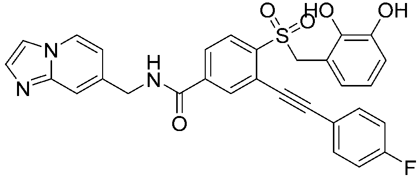 |
| 150 | 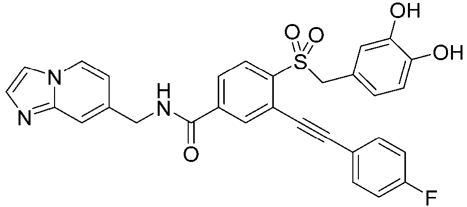 |
| 151 | 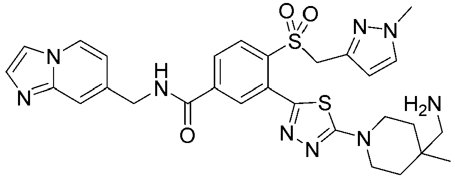 |
| 152 | 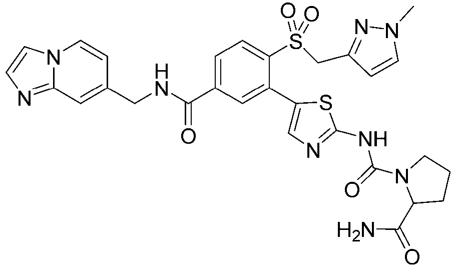 |
| 153 | 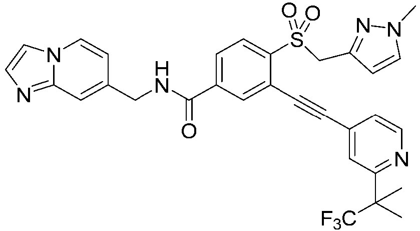 |
| 154 | 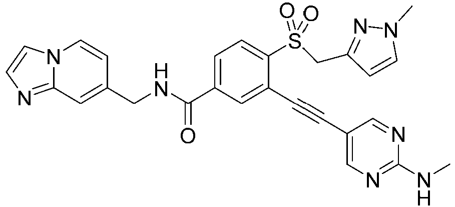 |
| 155 | 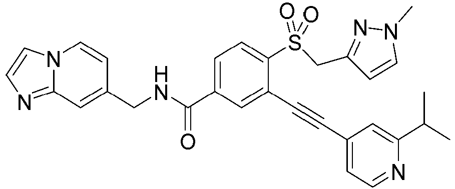 |
| 156 | 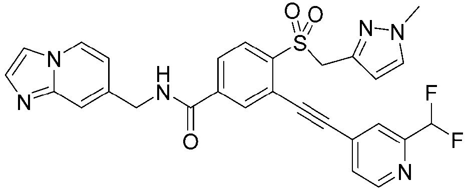 |
| 157 | 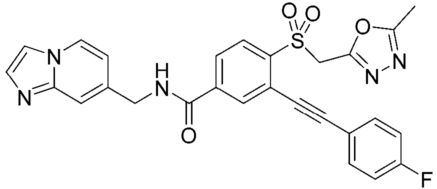 |
| 158 | 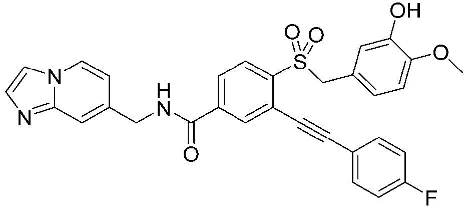 |
| 159 | 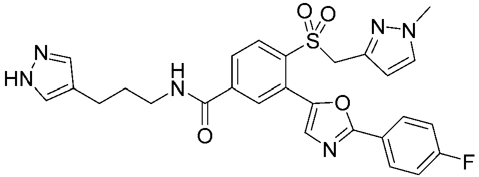 |
| 160 | 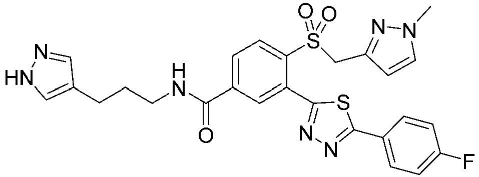 |
| 161 | 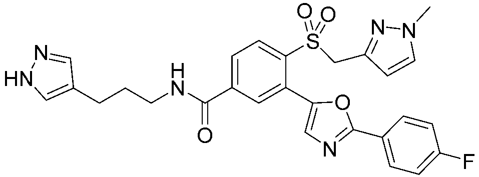 |
| 162 | 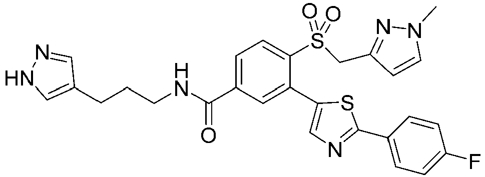 |
| 163 | 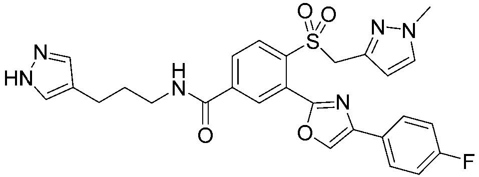 |
| 164 | 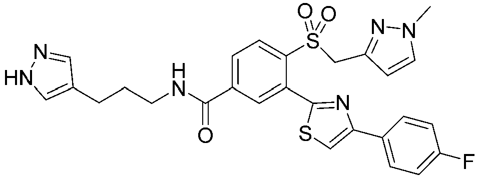 |
| 165 | 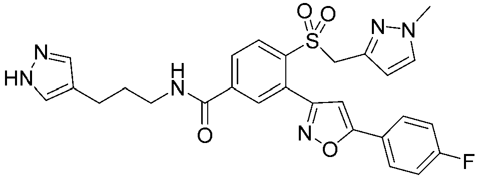 |
| 166 | 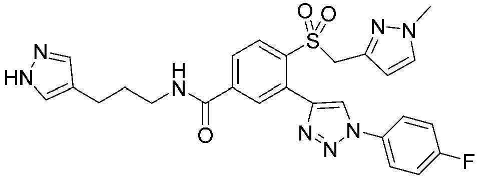 |
| 167 | 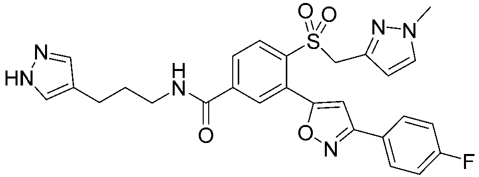 |
| 168 | 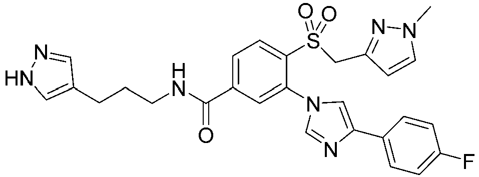 |
| 169 | 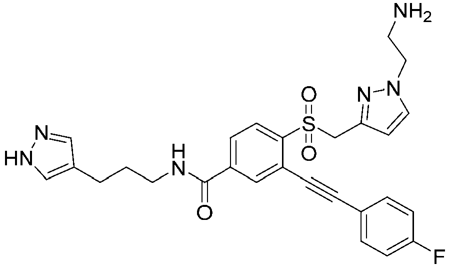 |
| 170 | 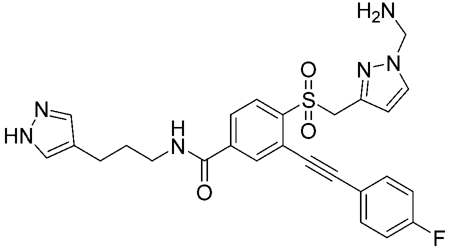 |
| 171 | 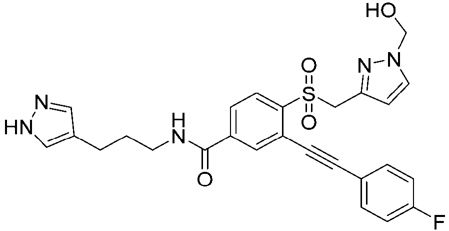 |
| 172 | 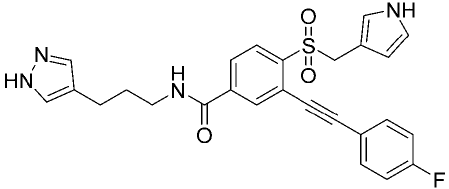 |
| 173 | 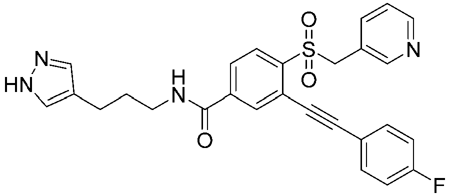 |
| 174 | 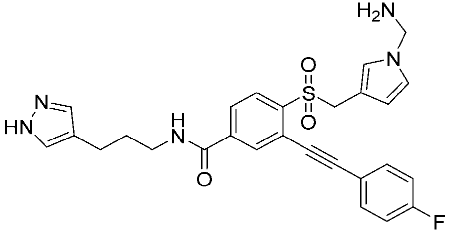 |
| 175 | 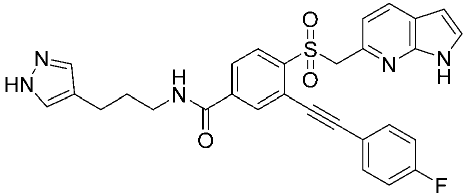 |
| 176 | 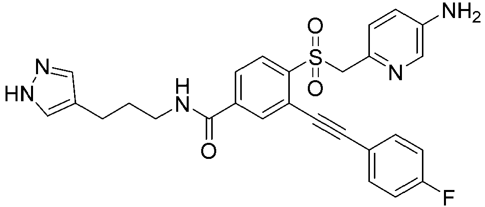 |
| 177 | 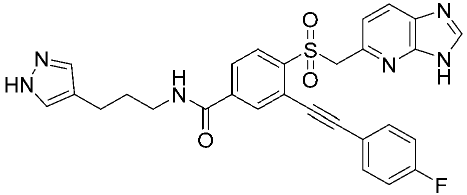 |
| 178 | 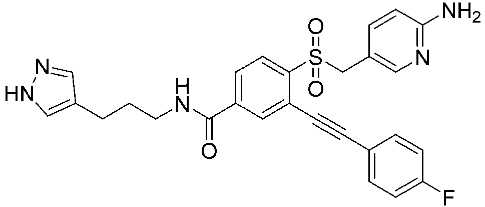 |
| 179 | 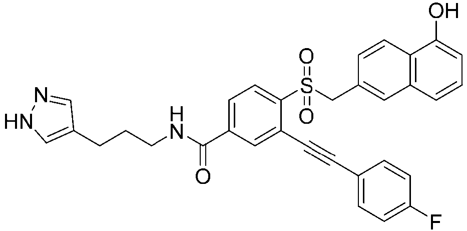 |
| 180 | 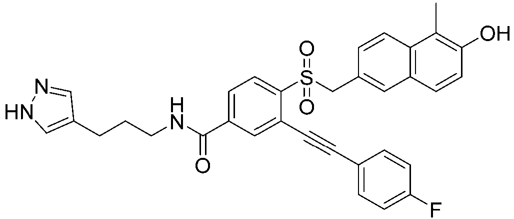 |
| 181 | 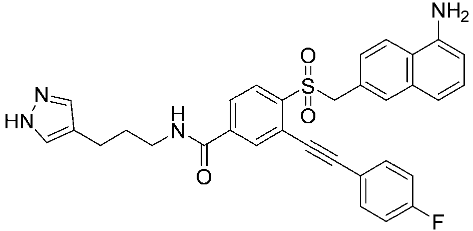 |
| 182 | 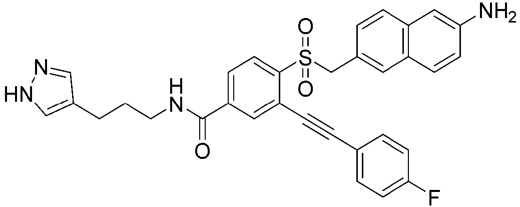 |
| 183 | 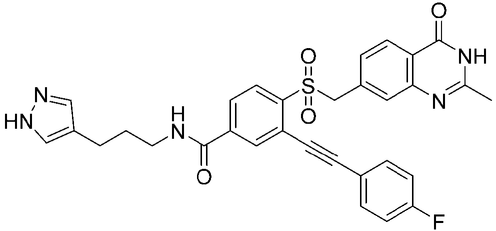 |
| 184 | 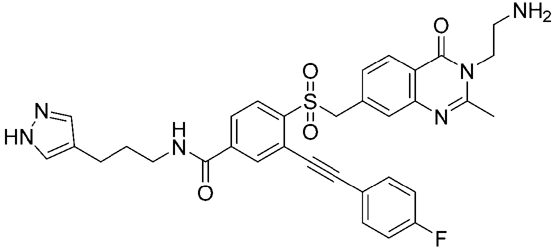 |
| 185 | 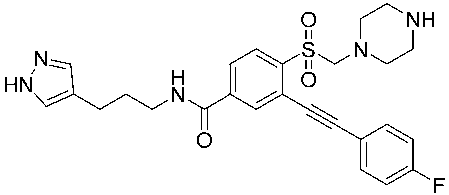 |
| 186 | 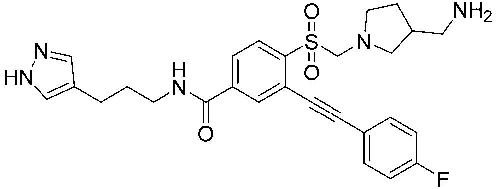 |
| 187 | 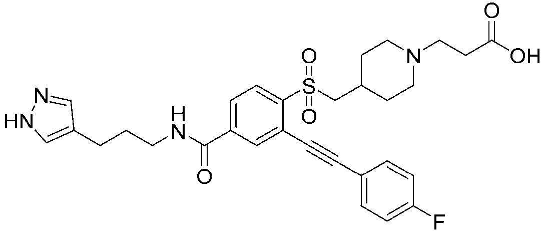 |
| 188 | 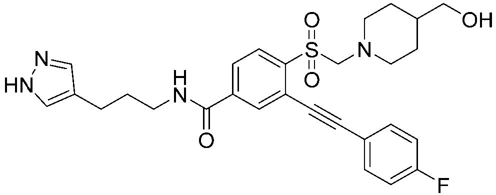 |
| 189 | 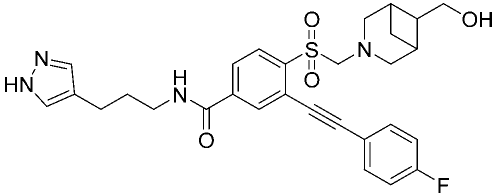 |
| 190 | 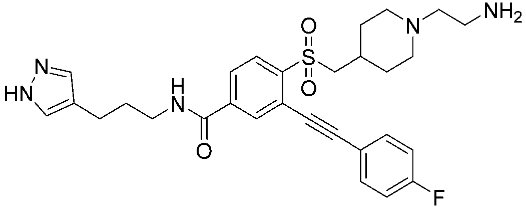 |
| 191 | 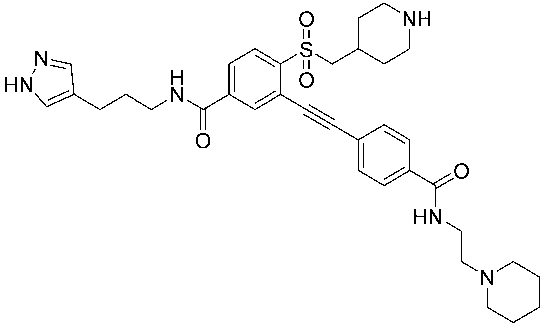 |
| 192 | 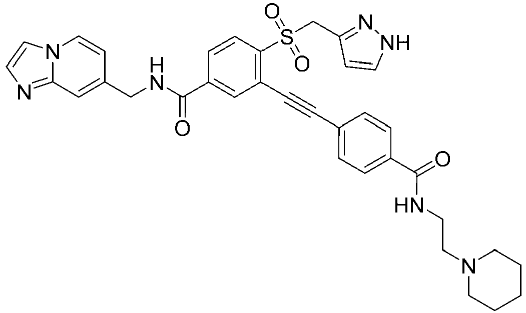 |
| 193 | 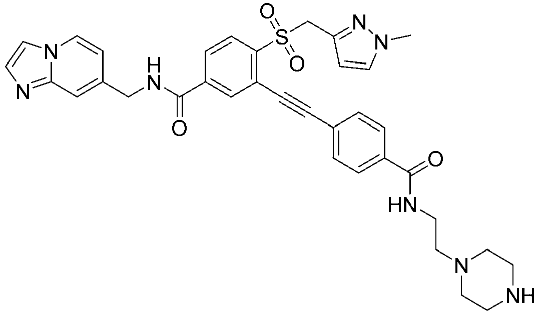 |
| 194 | 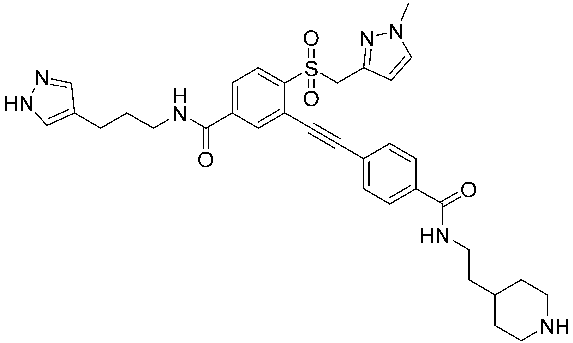 |
| 195 | 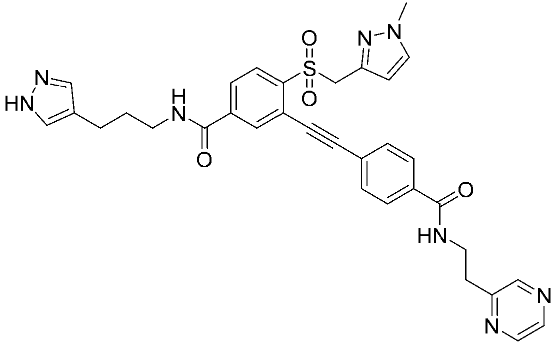 |
| 196 | 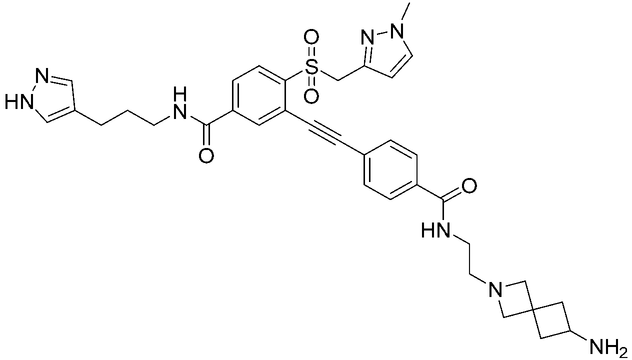 |
| 197 | 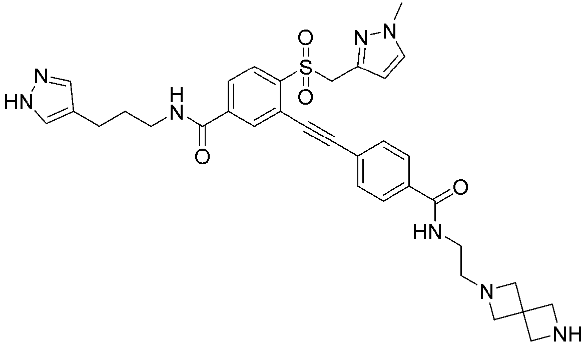 |
| 198 | 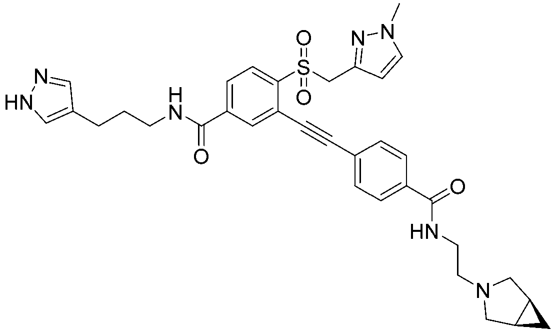 |
| 199 | 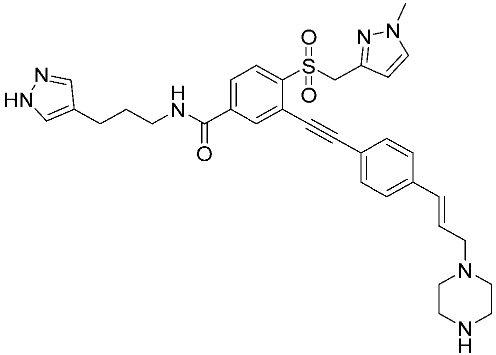 |
| 200 | 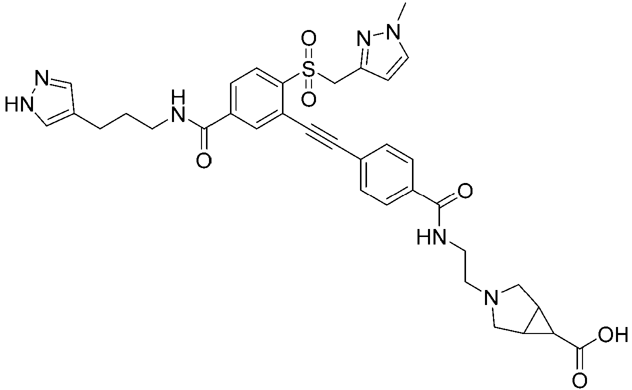 |
| 201 | 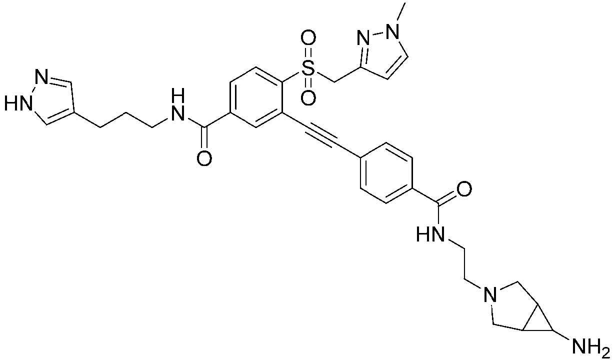 |
| 202 | 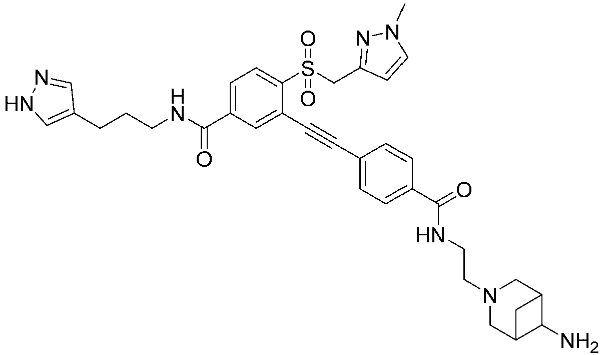 |
| 203 | 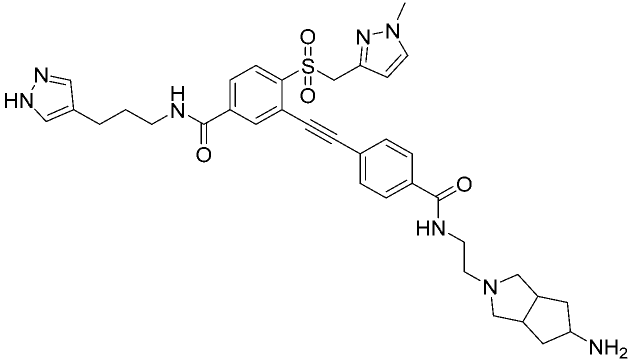 |
| 204 | 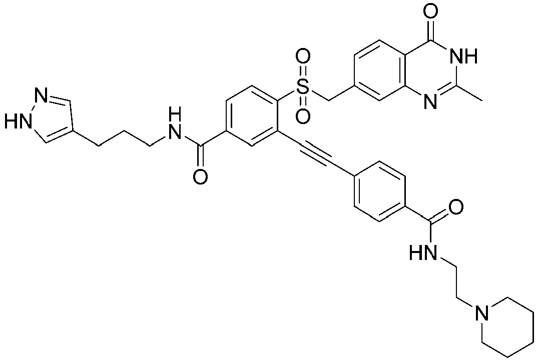 |
| 205 | 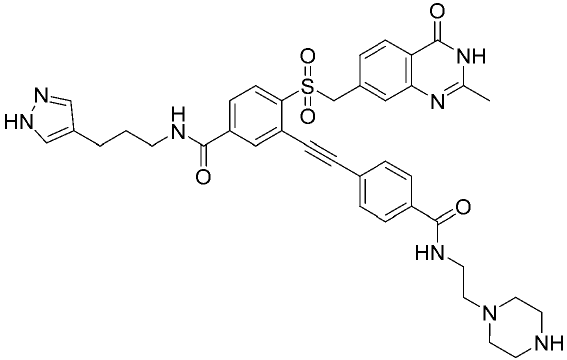 |
| 206 | 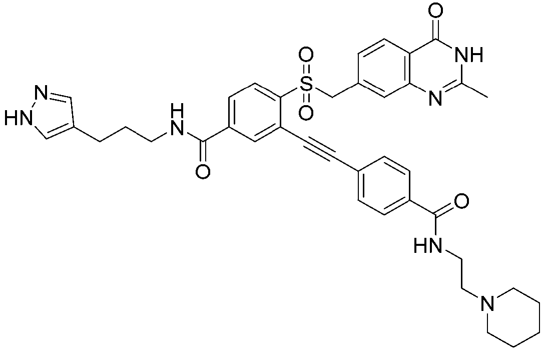 |
| 207 | 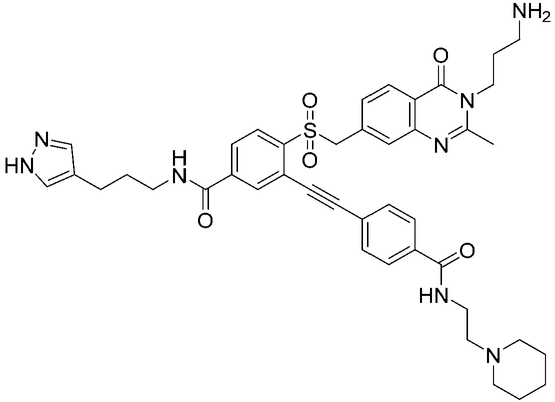 |
| 208 | 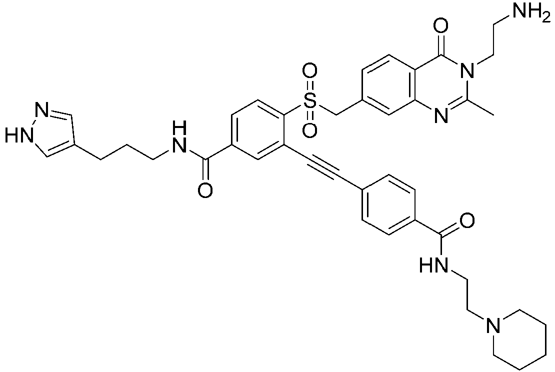 |
| 209 | 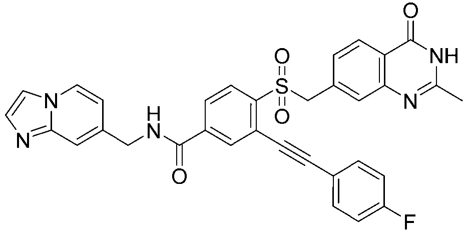 |
| 210 | 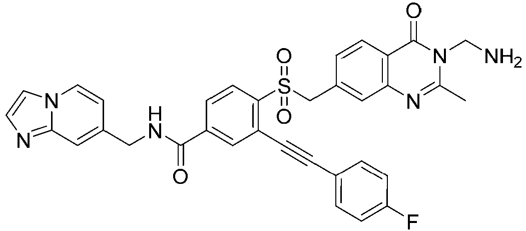 |
| 211 | 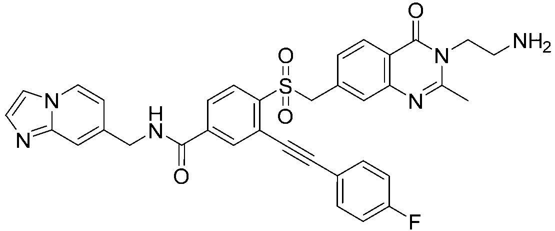 |
| 212 | 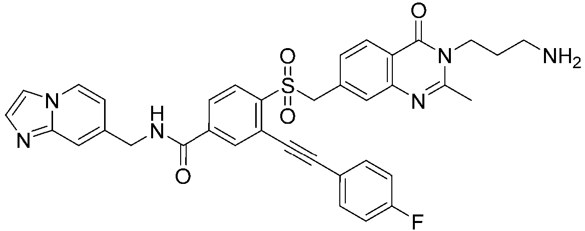 |
| 213 | 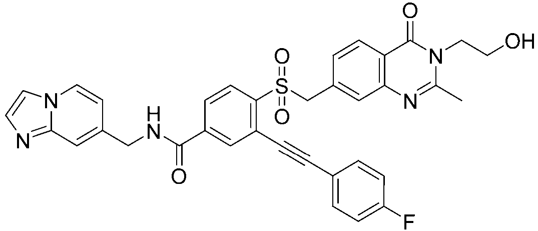 |
| 214 | 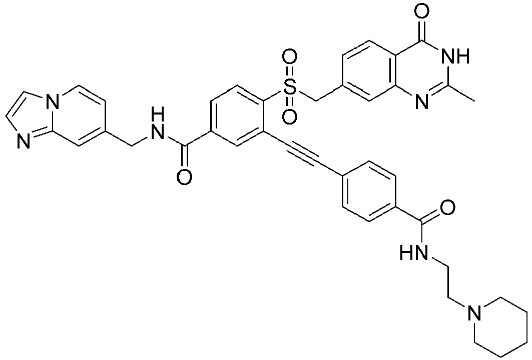 |
| 215 | 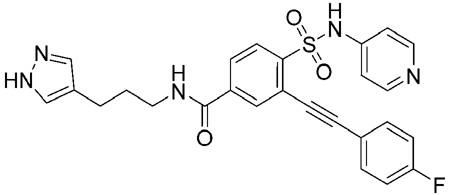 |
| 216 | 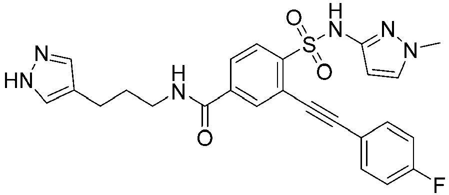 |
| 217 | 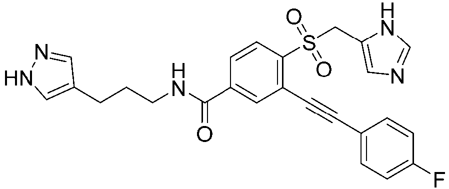 |
| 218 | 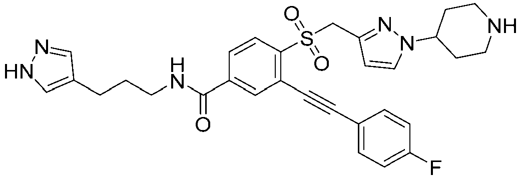 |
| 219 | 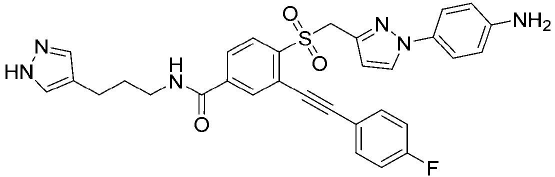 |
| 220 | 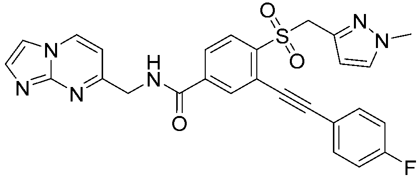 |
| 221 | 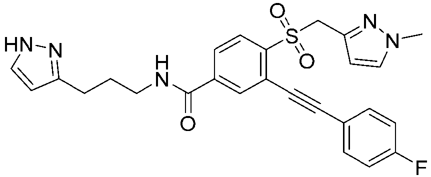 |
| 222 | 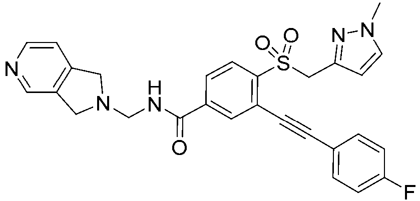 |
| 223 | 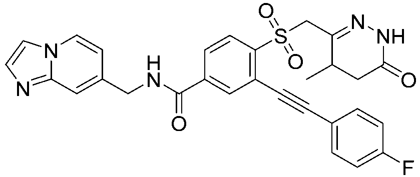 |
| 224 | 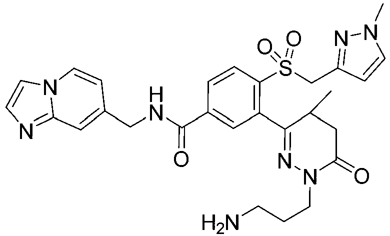 |
| 225 | 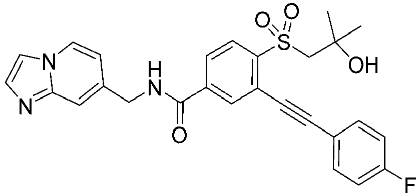 |
| 226 | 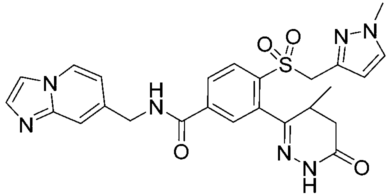 |
| 227 | 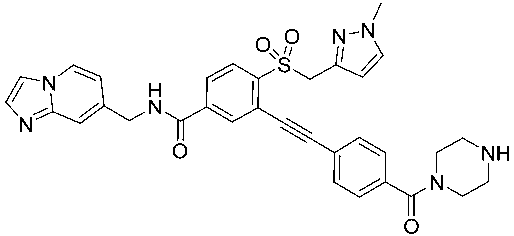 |
| 228 | 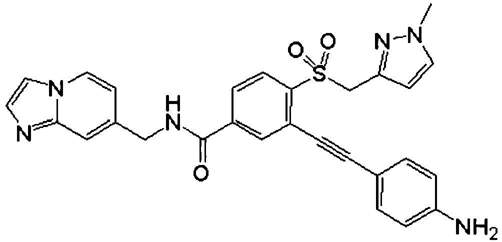 |
| 229 | 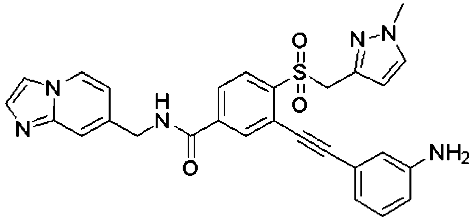 |
| 230 | 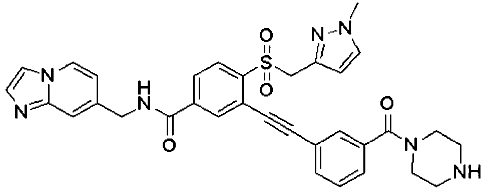 |
| 231 | 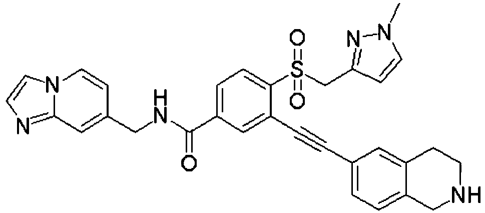 |
| 232 | 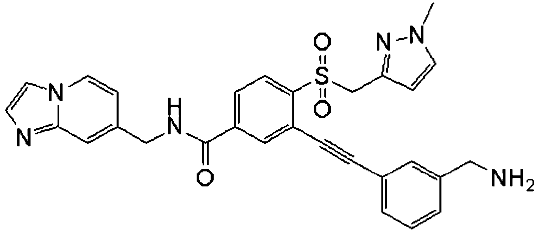 |
| 233 | 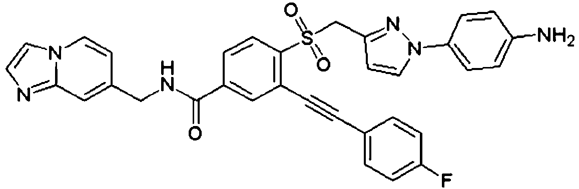 |
| 234 | 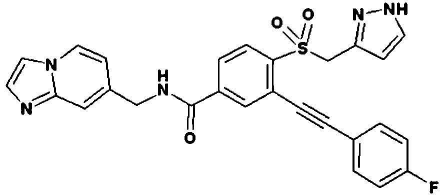 |
| 235 | 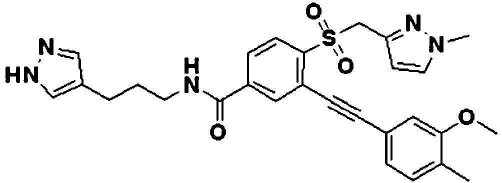 |
| 236 | 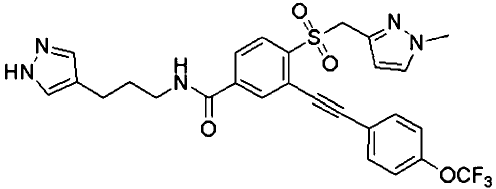 |
| 237 | 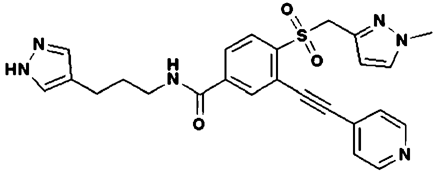 |
| 238 | 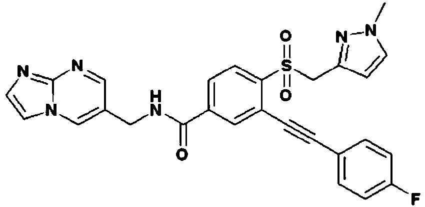 |
| 239 | 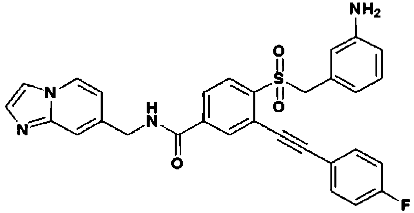 |
| 240 | 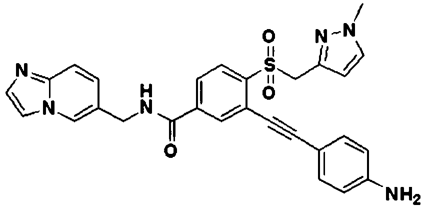 |
| 241 | 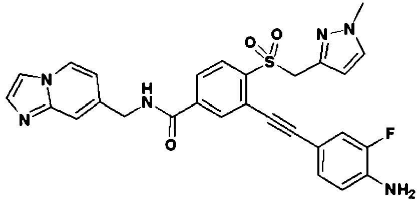 |
| 242 | 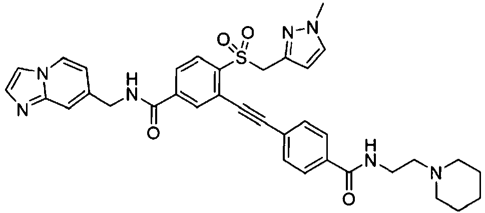 |
| 243 | 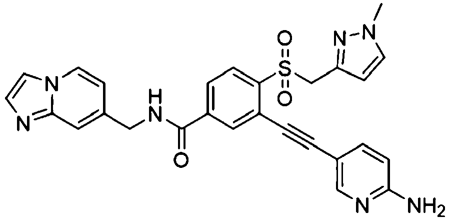 |
| 244 | 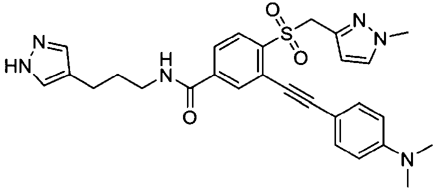 |
| 245 | 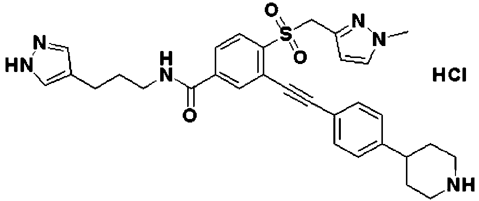 |
| 246 | 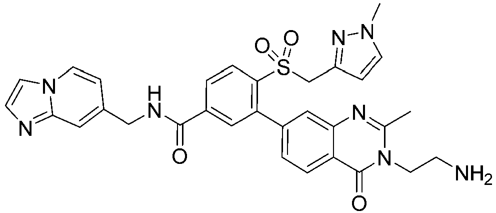 |
| 247 | 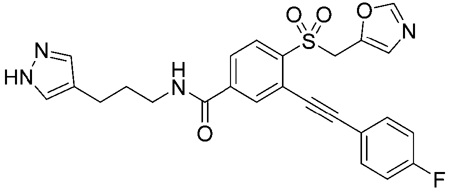 |
| 248 | 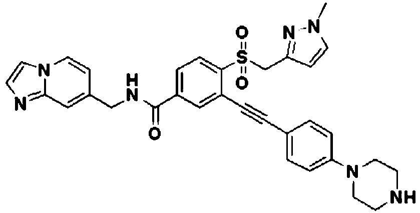 |
| 249 | 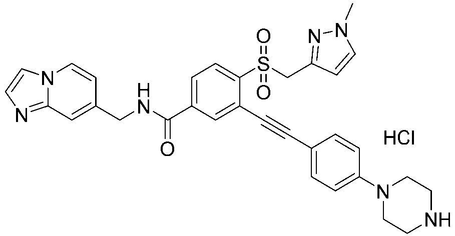 |

39) According to embodiments of the present invention, in above 1) to 38), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may exhibit NAMPT inhibitory activity.

40) According to embodiments of the present invention, in above 1) to 39), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may effectively inhibit NAMPT at low concentrations and may effectively prevent or treat diseases that may be treated by inhibiting NAMPT.

41) According to embodiments of the present invention, in above 1) to 40), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may sufficiently lower a NAD concentration in the cells.

42) According to embodiments of the present invention, in above 1) to 41), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may exhibit high cytotoxicity to cancer cells and may sufficiently kill cancer cells at a low concentration.

43) According to embodiments of the present invention, in above 1) to 42), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may exhibit high cytotoxicity to gastric cancer cells or colorectal cancer cells, and may sufficiently kill gastric cancer cells or colorectal cancer cells at a low concentration.

44) According to embodiments of the present invention, in above 1) to 43), a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may have little or no side effects and may exhibit excellent pharmacokinetic properties. For example, a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may exhibit an excellent absorption rate (e.g., high bioavailability), may be appropriately distributed in each tissue according to administration to exhibit a desired pharmacological effect (e.g., a preventive or therapeutic effect on diseases that may be treated by inhibiting NAMPT) in each tissue, and may be effectively metabolized in the body.

In the present invention, "stereoisomers" may include diastereomers and optical isomers, and the optical isomers may include not only enantiomers, but also a mixture of the enantiomers as well as racemates.

In the present invention, "pharmaceutically acceptable" may mean that it is physiologically acceptable and does not usually cause an allergic reaction, such as gastrointestinal disorder or dizziness, or a reaction similar thereto when administered to an individual.

45) According to embodiments of the present invention, in above 1) to 44), in the present invention, "pharmaceutically acceptable salts" may mean salts conventionally used in a pharmaceutical industry, for example, inorganic ion salts prepared from calcium, potassium, sodium, magnesium or the like; inorganic acid salts prepared from hydrochloric acid, nitric acid, phosphoric acid, bromic acid, iodic acid, perchloric acid, sulfuric acid or the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbric acid, carbonic acid, vanillic acid or the like; sulfonic acid salts prepared from methanesulfonic acid, ethanesulfonic acid, benzenesulfonic acid, p-toluenesulfonic acid, naphthalenesulfonic acid or the like; amino acid salts prepared from glycine, arginine, lysine, or the like; amine salts prepared from trimethylamine, triethylamine, ammonia, pyridine, picoline, or the like; and the like, but types of salt meant in the present invention are not limited to those listed salts.

46) According to embodiments of the present invention, in above 1) to 45), said pharmaceutically acceptable salt may be a hydrochloride salt. For example, said pharmaceutically acceptable salt may be a hydrochloride salt of a benzamide compound represented by above formula I, a benzamide compound represented by above formula I-1, a benzamide compound represented by above formula I-2, benzamide compounds 1 to 249 of above table 1, or stereoisomers thereof.

The pharmaceutically acceptable salt of the present invention may be prepared by conventional methods known to those skilled in the art.

The "hydrate" of the present invention may refer to one in which a compound represented by formula I or a pharmaceutically acceptable salt thereof and water are bound by a non-covalent intermolecular force, and may include a stoichiometric or non-stoichiometric amount of water. Specifically, said hydrate may include water at a ratio of about 0.25 mol to about 10 mol based on 1 mol of an active ingredient, and more specifically include about 0.5 mol, about 1 mol, about 1.5 mol, about 2 mol, about 2.5 mol, about 3 mol, about 5 mol, etc.

The "solvate" of the present invention may refer to one in which a compound represented by formula I, a stereoisomer thereof or a pharmaceutically acceptable salt thereof and a solvent other than water are bound by a non-covalent intermolecular force, and may include a stoichiometric or non-stoichiometric amount of the solvent. Specifically, said solvate may include a solvent molecule at a ratio of about 0.25 mol to about 10 mol based on 1 mol of an active component, and more specifically include about 0.5 mol, about 1 mol, about 1.5 mol, about 2 mol, about 2.5 mol, about 3 mol, about 5 mol, etc.

### Pharmaceutical composition including compound represented by formula I

47) The present invention may provide a pharmaceutical composition including a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof according to 1) to 46) as an active ingredient.

48) According to embodiments of the present invention, in above 33), a pharmaceutical composition including a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof as an active ingredient may exhibit NAMPT inhibitory activity and may be used for preventing or treating NAMPT enzyme-associated diseases.

49) According to embodiments of the present invention, in above 47) or 48), a pharmaceutical composition including a benzamide compound represented by above formula I of the present invention, a compound represented by formula I-1, compounds 1 to 245 of above table 14, stereoisomers thereof, pharmaceutically acceptable salts thereof, or hydrates or solvates thereof as an active ingredient may sufficiently lower a NAD concentration in the cells.

50) According to embodiments of the present invention, in above 47) to 49), a pharmaceutical composition including a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof as an active ingredient may exhibit a preventive or therapeutic effect on cancer.

51) According to embodiments of the present invention, in above 47) to 50), cancer may be a solid cancer or blood cancer, and said cancer may be skin cancer (e.g., melanoma), lymphoma, breast cancer, cervical cancer, uterine cancer, gastrointestinal cancer, lung cancer, ovarian cancer, prostate cancer, colon cancer, rectal cancer, oral cancer, brain cancer, head and neck cancer, throat cancer, testicular cancer, kidney cancer, pancreatic cancer, bone cancer, spleen cancer, liver cancer, bladder cancer, larynx cancer, nasal cavity cancer, AIDS-related cancer, esophageal cancer, gastric cancer, pancreatic cancer, colorectal cancer, virus-related cancer (one selected from nasopharyngeal cancer, vaginal cancer, vulvar cancer, penile cancer, Kaposi sarcoma, Burkitt lymphoma, T-cell lymphoma, Merkel cell carcinoma and the like), cancer of blood and bone marrow, such as multiple myeloma, acute and chronic leukemia, for example, lymphoblastic leukemia, myeloid leukemia, lymphocytic leukemia, and myelocytic leukemia, and the like.

52) According to embodiments of the present invention, in above 47) to 51), the pharmaceutical composition of the present invention may further include at least one pharmaceutically acceptable carrier in addition to a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof.

In this case, the pharmaceutically acceptable carrier may be one commonly used in the art in the preparation, and may be at least one selected from lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate, mineral oil and the like, but is not limited thereto.

In addition, the pharmaceutical composition of the present invention may further include lubricant, humectant, a sweetening agent, a flavoring agent, emulsifier, a suspending agent, preservative, or the like in addition to said ingredients.

Furthermore, the pharmaceutical composition of the present invention may be formulated into an oral dosage form such as tablet, powder, granule, pill, capsule, suspension, emulsion, liquid for internal use, emulsion, syrup, etc., as well as a form of external application, suppository and sterile solution for injection by using a pharmaceutically acceptable carrier and excipient, and thus may be prepared in a unit dose form or prepared by being inserted into a multidose container. The preparations may be prepared according to a conventional method used for formulation in the art or a method disclosed in Remington's Pharmaceutical Science (19th ed., 1995), and may be formulated into various preparations depending on each disease or ingredient. The pharmaceutical composition of the present invention may be orally or parenterally administered (for example, applied intravenously, hypodermically, intraperitoneally or locally) according to an intended method, in which a dosage thereof varies depending on a patient's condition and weight, a degree of disease, a drug form, and an administration route and time, but may be appropriately selected by those skilled in the art. Specifically, a daily dosage of a benzamide compound represented by above formula I of the present invention, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof may be about 0.1 to about 1000 mg/kg based on the weight of the compound represented by above formula I, and may be administered at one time a day or several times a day by dividing the daily dosage thereof.

The pharmaceutical composition of the present invention may further include at least one component which may exhibit the same or similar effect in addition to or may synergize a medicinal effect in combination with a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof.

53) According to embodiments of the present invention, in above 47) to 51), the present invention may provide a method for preventing or treating NAMPT enzyme-associated diseases, including administering a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, hydrates or solvates thereof, or a pharmaceutical composition including the same into an individual.

54) According to embodiments of the present invention, in above 53), the method for preventing or treating NAMPT enzyme-associated diseases of the present invention may include administering a therapeutically effective amount of a benzamide compound represented by above formula I, a benzamide compound represented by above formula I-1, a benzamide compound represented by above formula I-2, benzamide compounds 1 to 249 of above table 1, stereoisomers thereof, pharmaceutically acceptable salts thereof, hydrates or solvates thereof, or a pharmaceutical composition including the same.

55) According to embodiments of the present invention, in above 53) or 54), in the present invention, the "therapeutically effective amount" may refer to an amount enough to treat a disease at a reasonable benefit/risk ratio applicable to medical treatment, and a level of effective dose may be determined according to factors including a patient's disease type, severity, activity of a drug, sensitivity to the drug, an administration time, an administration route and excretion rate, a treatment period and a concurrently used drug, as well as other factors well known in a medical field. Specifically, it may refer to an amount effective in preventing or treating diseases (e.g., cancer) that may be treated by inhibiting NAMPT.

In the present invention, the "individual" may refer to a subject, whose disease needs to be prevented or treated, and more specifically to a mammal such as a human, monkey, mouse, dog, cat, horse, cow, etc., but is not limited thereto.

56) According to embodiments of the present invention, in above 53) to 55), in the present invention, the "prevention" may mean all the acts, which inhibit NAMPT enzyme-associated diseases or delay the occurrence thereof by administering a benzamide derivative compound represented by formula I of the present invention, a benzamide compound, a benzamide compound represented by above formula I-1, a benzamide compound represented by above formula I-2, benzamide compounds 1 to 249 of above table 1, stereoisomers thereof, pharmaceutically acceptable salts thereof, or hydrates or solvates thereof.

57) According to embodiments of the present invention, in above 53) to 56), in the present invention, the "treatment" may refer to all the acts, by which a symptom of NAMPT-associated diseases gets better or takes a favorable turn by administering a benzamide derivative compound represented by formula I of the present invention, a benzamide compound, a benzamide compound represented by above formula I-1, a benzamide compound represented by above formula 1-2, benzamide compounds 1 to 249 of above table 1, stereoisomers thereof, pharmaceutically acceptable salts thereof, or hydrates or solvates thereof.

58) The present invention may provide a use of a benzamide derivative compound represented by formula I according to above 47) to 51), a benzamide compound, a benzamide compound represented by above formula I-1, a benzamide compound represented by above formula 1-2, benzamide compounds 1 to 249 of above table 1, stereoisomers thereof, pharmaceutically acceptable salts thereof, hydrates or solvates thereof, or a pharmaceutical composition including the same for preventing or treating NAMPT enzyme-associated diseases.

59) The present invention may provide a use of a benzamide derivative compound represented by above formula I according to above 47) to 51), a benzamide compound represented by above formula I-1, a benzamide compound represented by above formula I-2, benzamide compounds 1 to 249 of above table 1, stereoisomers thereof, pharmaceutically acceptable salts thereof, hydrates or solvates thereof, or a pharmaceutical composition including the same for preparing a medication for preventing or treating NAMPT enzyme-associated diseases.

Matters mentioned in respective items of the present invention, that is, the benzamide compound, use, composition and therapeutic method may be equally applied, if not contradictory to each other. Besides, the terms and abbreviations used in the present specification may have their original meanings, unless defined otherwise.

### Advantageous Effects

A benzamide derivative compound of the present invention, a stereoisomer thereof, a pharmaceutically acceptable salt of them, or a hydrate or solvate of them can inhibit NAMPT to achieve a remarkable effect of preventing or treating NAMPT-associated diseases. Thus, the urea compound of the present invention, the stereoisomer thereof, the pharmaceutically acceptable salt of them, or the hydrate or solvate of them can be advantageously used for preventing or treating diseases (e.g., cancer) that may be treated by inhibiting NAMPT.

### Mode for Invention

Hereinafter, the present invention will be described in more detail through preparation examples and exemplary examples. However, the following preparation examples and exemplary examples are provided for the purpose of illustrating the present invention, and thus the present invention is not limited to the preparation examples and exemplary examples.

Each of the compounds according to the present invention was synthesized by a method described below, and a conventional method derived by combining the following specific synthesis methods may be used by those skilled in the art.

Each of the compounds used in the synthesis was purchased from a supplier outside or synthesized by using an organic synthesis method apparent to those skilled in the art or, and was used without a separate purification process. The compound of each exemplary example was identified through 400 MHz 1H-NMR (BRUKER, 400-MR), LC-Mass (Waters, SQD2) analysis.

### <Examples>

### Example 1: Synthesis of compound 1

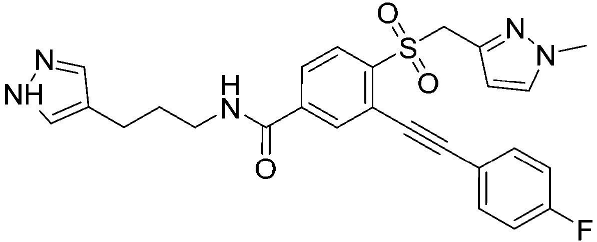

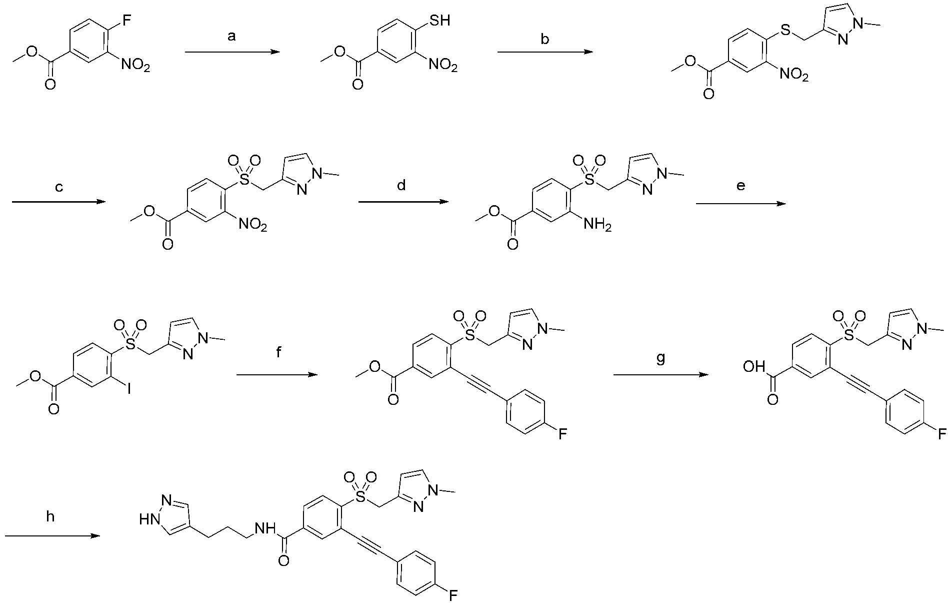

Reagents and conditions (a) Na₂S, DMF, room temperature (r.t), 16 hours (16 h) (b) 3-(chloromethyl)-1-methyl-1H-pyrazole, K₂CO₃, DMF, r.t, 16 hours (16 h) (c) mCPBA, DCM, room temperature (r.t), 16 hours (16 h) (d) Fe, AcOH, 80°C, two hours (2 h) (e) HCl, NaNO₂, KI, H₂O, 0°C to room temperature (0°C-r.t), one hour (1 h) (f) PdCl₂[PPh₃]₂, CuI, TEA, ACN, room temperature (r.t), 16 hours (16 h) (g) NaOH, H₂O, MeOH, 55°C, two hours (2 h) (h) 3-(1H-pyrazol-4-yl)propan-1-amine dihydrochloride, TBTU, TEA, DCM, 12 hours (12 h)

### Step 1) Synthesis of methyl 4-mercapto-3-nitrobenzoate

Methyl 4-fluoro-3-nitrobenzoate (10 g, 46.4 mmol, 1 eq) was dissolved in DMF (80 ml), and sodium disulfide (4.32 g, 55.3 mmol, 1.2 eq) was added thereto and stirred at room temperature for 16 hours. After completion of a reaction, water (200 ml) was added to a reaction product and stirred while pH was adjusted to 5 with 1 N HCl. While being stirred, a yellow solid was produced, filtered, and washed with 25% EA/HX in suspension to obtain a target compound (6.13 g, 28.7 mmol, 62%).

### Step 2) Synthesis of methyl 4-(((1-methyl-1H-pyrazol-3-yl)methyl)thio)-3-nitrobenzoate

Methyl 4-mercapto-3-nitrobenzoate (4 g, 18.8 mmol, 1 eq) was dissolved in DMF (40 ml), and K₂CO₃ (5.18 g, 37.52 mmol, 2 eq) was added thereto and stirred. When a reaction product turned dark red, 3-(chloromethyl)-1-methyl-1H-pyrazole (2.45 g, 18.8 mmol, 1 eq) was added to a reaction product and stirred for 16 hours. When a reaction was completed by confirming the reaction through TLC, water was added to a reaction product, and a resulting precipitate was filtered to obtain a target compound (5.14 g, 16.7 mmol, 89%).

### Step 3) Synthesis of methyl 4-(((1-methyl-1H-pyrazol-3-yl)methyl)sulfonyl)-3-nitrobenzoate

Methyl 4-(((1-methyl-1H-pyrazol-3-yl)methyl)thio)-3-nitrobenzoate (5.14 g, 16.7 mmol, 1 eq) was dissolved in DCM (100 ml), and mCPBA (12.2 g, 50.2 mmol, 3 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure to obtain a target compound which was not purified.

### Step 4) Synthesis of methyl 3-amino-4-(((1-methyl-1H-pyrazol-3-yl)methyl)sulfonyl)benzoate

AcOH (100 ml) and iron (9.32 g, 16.7 mmol, 10 eq) were added to methyl 4-(((1-methyl-1H-pyrazol-3-yl)methyl)sulfonyl)-3-nitrobenzoate (16.7 mmol, 1 eq) and stirred at 80°C for two hours. A catalyst was removed by celite filtration, and a filtrate was concentrated under reduced pressure. A residue was basified with a potassium carbonate aqueous solution and extracted with ethyl acetate, and then an organic layer was concentrated under reduced pressure. A target compound was obtained as a crude product.

### Step 5) Synthesis of methyl 3-iodo-4-(((1-methyl-1H-pyrazol-3-yl)methyl)sulfonyl)benzoate

A solution (1 g, 4.65 mmol, 1 eq) of sodium nitrite (1.73 g, 1.5 eq) dissolved in water (40 ml) was slowly added dropwise to a suspension of methyl 3-amino-4-(((1-methyl-1H-pyrazol-3-yl)methyl)sulfonyl)benzoate (16.7 mmol), a concentrated hydrochloric acid aqueous solution (40 ml) and water (40 ml) at 0°C. A resulting mixture was stirred in ice-bath for 30 minutes, after which a solution of potassium iodide (8.3 g, 3 eq) dissolved in water (40 mL) was added thereto at 0°C. A reaction mixture was stirred at room temperature for one hour, treated with a saturated potassium carbonate aqueous solution (pH>8), and extracted with ethyl acetate. An organic layer was washed with water, dried over magnesium sulfate, and concentrated under reduced pressure. A resulting residue was purified by column chromatography (silica gel, 30-50% EA/HX) to obtain a target compound (3.24 g, three steps[(c),(d),(e)] yield 46%).

### Step 6) Synthesis of methyl 3-((4-fluorophenyl)ethynyl)-4-(((1-methyl-1H-pyrazol-3-yl)methyl)sulfonyl)benzoate

MeCN (10 ml, 0.2 M) was added to methyl 3-iodo-4-(((1-methyl-1H-pyrazol-3-yl)methyl)sulfonyl)benzoate (0.84 g, 2 mmol, 1 eq) and TEA (0.84 ml, 6 mmol, 3 eq) was added thereto. PdCl₂[PPh₃]₂ (42 mg, 3 mol%) and CuI (11 mg, 3 mol%) were added to a reaction product and stirred at room temperature for five minutes under a nitrogen atmosphere. Subsequently, 4-fluorophenyl-acetylene (0.29 g, 1.2 eq) was added thereto. A reaction mixture was stirred at room temperature for 16 hours and concentrated under reduced pressure. A resulting residue was purified by column chromatography (silica gel, 40-50% EA/HX) to obtain a target compound (1.7 mmol, 85%).

### Step 7) Synthesis of 3-((4-fluorophenyl)ethynyl)-4-(((1-methyl-1H-pyrazol-3-yl)methyl)sulfonyl)benzoic acid

MeOH (17 ml, 0.1 M), H₂O (8.5 ml, 0.2 M), and NaOH (0.17 g, 4.3 mmol, 2.5 eq) were added to methyl 3-((4-fluorophenyl)ethynyl)-4-(((1-methyl-1H-pyrazol-3-yl)methyl)sulfonyl)benzoate (0.7 g, 1.7 mmol, 1 eq) and stirred at 55°C for two hours. When a reaction was completed by confirming the reaction through TLC, the pH of a reaction product was adjusted to 1 with a 1 N HCl aqueous solution and extracted with EA. An organic layer was washed with water, dried over magnesium sulfate, and concentrated under reduced pressure to obtain a target compound (0.62 g, 92%).

### Step 8) Synthesis of N-(3-(1H-pyrazol-4-yl)propyl)-3-((4-fluorophenyl)ethynyl)-4-(((1-methyl-1H-pyrazol-3-yl)methyl)sulfonyl)benzamide

DCM (5 ml) and TBTU (0.14 g, 0.45 mmol, 1.5 eq) were added to 3-((4-fluorophenyl)ethynyl)-4-(((1-methyl-1H-pyrazol-3-yl)methyl)sulfonyl)benzoic acid (0.12 g, 0.3 mmol, 1 eq) and stirred at room temperature for 50 minutes. Subsequently, TEA (0.15 ml, 1.11 mmol, 3.7 eq) and 3-(1H-pyrazol-4-yl)propan-1-amine dihydrochloride (71 mg, 0.36 mmol, 1.2 eq) were added to a reaction product and stirred for 12 hours. Water was added to a reaction product and extracted with EA. An organic layer was dried over magnesium sulfate and concentrated under reduced pressure, and a resulting residue was purified by column chromatography (silica gel, 3-4% MeOH/DCM) to obtain a target compound (0.1 g, 0.20 mmol, 66%).

¹H NMR (400 MHz, CDCl₃) δ 8.01 (d, *J =* 1.6 Hz, 1H), 7.94 (d, *J =* 8.2 Hz, 1H), 7.73 - 7.66 (m, 3H), 7.49 (s, 2H), 7.23 (d, *J* = 2.2 Hz, 1H), 7.13 (t, *J =* 8.7 Hz, 2H), 6.35 - 6.32 (m, 1H), 6.17 (d, *J =* 2.2 Hz, 1H), 4.79 (s, 2H), 3.79 (s, 3H), 3.55 (q, *J =* 6.4 Hz, 2H), 2.66 (t, *J =* 7.4 Hz, 2H), 1.97 (p, *J =* 7.4 Hz, 2H).

MS(ESI) m/e MH⁺ 506.2

The reagents may be changed in substantially the same manner as the synthesis method of above Example 1 to synthesize compounds 11, 13, 18, 21, 23, 24, 26 to 28, 30 to 43, and 48 to 70.

### Example 2: Synthesis of compound 2

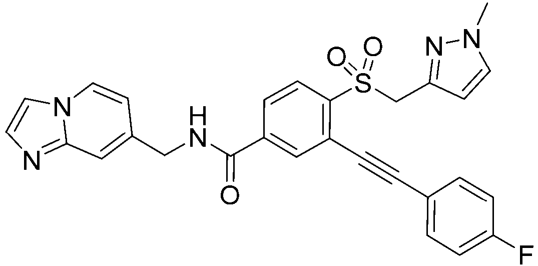

### Synthesis of 3-((4-fluorophenyl)ethynyl)-N-(imidazo[1,2-a]pyridin-7-ylmethyl)-4-(((1-methyl-1H-pyrazol-3-yl)methyl)sulfonyl)benzamide

1-{imidazo[1,2-a]pyridin-7-yl}methanamine dihydrochloride was used to synthesize a target compound in the same manner as in Step 8) of Example 1.

¹H NMR (400 MHz, DMSO) δ 9.47 - 9.44 (m, 1H), 8.52 (d, *J =* 7.1 Hz, 1H), 8.33 (s, 1H), 8.05 (d, *J =* 8.2 Hz, 1H), 7.97 - 7.88 (m, 2H), 7.82 - 7.76 (m, 2H), 7.60 (s, 1H), 7.54 (s, 1H), 7.46 (s, 1H), 7.39 (t, *J =* 8.4 Hz, 2H), 6.89 (d, *J =* 6.7 Hz, 1H), 6.10 (s, 1H), 4.88 (s, 2H), 4.55 (d, *J =* 5.4 Hz, 2H), 3.72 (s, 3H).

MS(ESI) m/e MH⁺ 528.4

The reagents may be changed in substantially the same manner as the method of Example 2 to synthesize compounds 220, 221, 222 and 227.

### Example 3: Synthesis of compound 3

### Synthesis of N-(2-(1H-pyrrolo[3,2-c]pyridin-3-yl)ethyl)-3-((4-fluorophenyl)ethynyl)-4-(((1-methyl-1H-pyrazol-3-yl)methyl)sulfonyl)benzamide

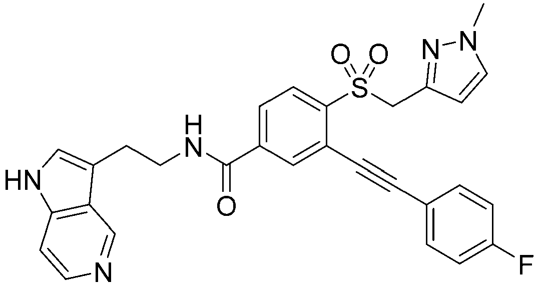

1-{1H-pyrrolo[3,2-c]pyridin-2-yl}methanamine dihydrochloride was used to synthesize a target compound in the same manner as in Step 8) of Example 1.

¹H NMR (400 MHz, MeOD) δ 8.91 (s, 1H), 8.15 (d, *J =* 5.8 Hz, 1H), 8.10 (s, 1H), 7.90 (d, *J =* 8.3 Hz, 1H), 7.82 (d, *J =* 8.3 Hz, 1H), 7.78 - 7.72i (m, 2H), 7.45 (m, 2H), 7.32 (s, 1H), 7.24 (t, *J =* 8.3 Hz, 2H), 6.17 (s, 1H), 4.83 (s, 2H), 3.78 - 3.72 (m, 5H), 3.19 (t, *J =* 7.1 Hz, 2H).

MS(ESI) m/e [M-H]⁻ 540.4

### Example 4: Synthesis of 3-(chloromethyl)-1-(4-(trifluoromethyl)phenyl)-1H-pyrazole

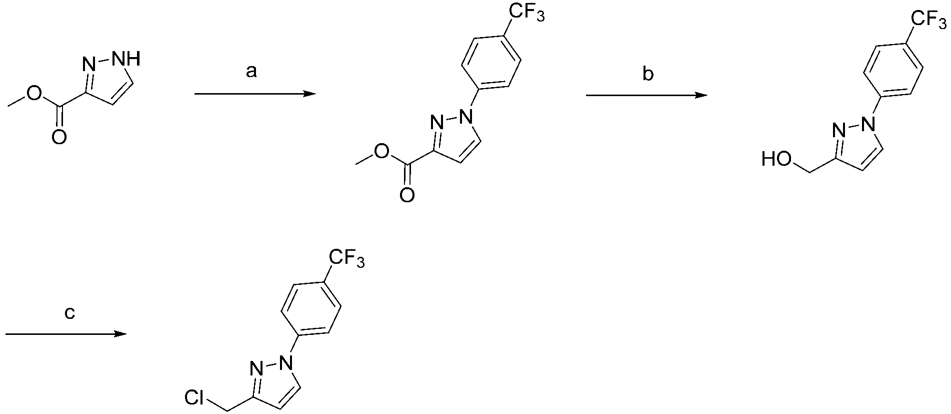

Reagents and conditions. (a) 1-iodo-4-(trifluoromethyl)benzene, CuI, K₃PO₄, (1S,2S)-(+)-N,N'-dimethylcyclohexan-1,2-diamine, 110°C, 16 hour (16 h) (b) LiAlH₄, THF, 0°C to room temperature (0°C-r.t), one hour (1 h) (c) SOCl₂, DCM, room temperature (r.t), 12 hours (12 h)

### Step 1) Synthesis of methyl 1-(4-(trifluoromethyl)phenyl)-1H-pyrazole-3-carboxylate

Methyl 1H-pyrazole-3-carboxylate (1 g, 7.9 mmol, 1 eq), 1-iodo-4-(trifluoromethyl)benzene (2.15 g, 1 eq), CuI (0.24 g, 1.27 mmol, 0.16 eq), (1S,2S)-(+)-N,N'-dimethylcyclohexan-1,2-diamine (0.28 ml, 1.7 mmol, 0.22 eq), K₃PO₄(4 g, 19 mmol, 2.4 eq), and toluene (10 ml, 0.8 M) were added to a pressure tube and stirred to 110°C for 16 hours. A reaction product was cooled to room temperature, and water and EA were added and separated. An organic layer was dried over magnesium sulfate and concentrated under reduced pressure, and a resulting residue was purified by column chromatography (silica gel, 10% EA/HX) to obtain a target compound (1.4 g, 5.2 mmol, 65%).

### Step 2) Synthesis of (1-(4-(trifluoromethyl)phenyl)-1H-pyrazol-3-yl)methanol

Methyl 1-(4-(trifluoromethyl)phenyl)-1H-pyrazole-3-carboxylate (1 g, 3.7 mmol, 1 eq) was dissolved in anhydrous THF, and a lithium aluminum hydride solution (3.7 ml, 1.0 M in a THF solution) was added dropwise at 0°C. A reaction product was stirred at room temperature for one hour, and cooled to 0°C. Water (1 ml), a 1 N NaOH aqueous solution (1 ml), and water (2 ml) were sequentially added to a reaction product, and a reaction was completed and filtered through celite. A filtrate was concentrated under reduced pressure to obtain a target compound which was not purified.

### Step 3) Synthesis of 3-(chloromethyl)-1-(4-(trifluoromethyl)phenyl)-1H-pyrazole

(1-(4-(trifluoromethyl)phenyl)-1H-pyrazol-3-yl)methanol was dissolved in DCM (20 ml) and SOCl₂ (0.54 ml, 2 eq) was added thereto, stirred at room temperature for 12 hours, and concentrated under reduced pressure to obtain a target compound which was not purified.

### Example 5: Synthesis of compound 4

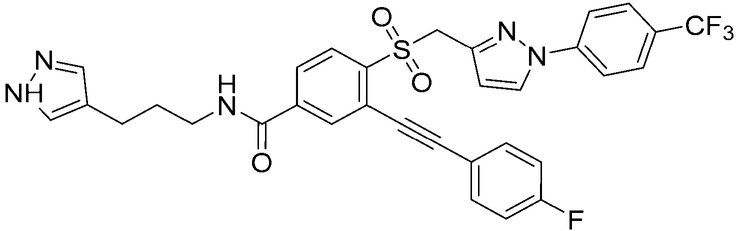

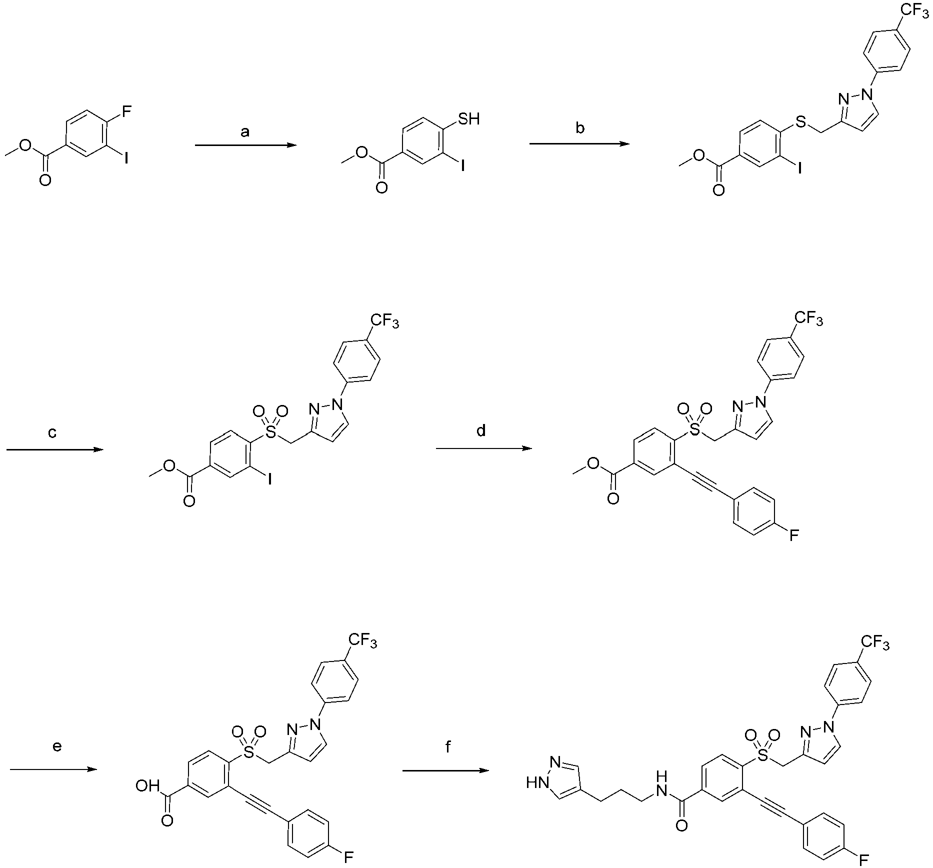

Reagents and conditions. (a) Na₂S, DMF, reflux, two hours (2 h) (b) 3-(chloromethyl)-1-(4-(trifluoromethyl)phenyl)-1H-pyrazole, K₂CO₃, DMF, room temperature (r.t), 0.5 hours (0.5 h) (c) mCPBA, DCM, room temperature (r.t), 16 hours (16 h) (d) PdCl₂[PPh₃]₂, CuI, TEA, ACN, room temperature (r.t), 16 hours (16 h) (e) NaOH, H₂O, MeOH, 60°C, 12 hours (12 h) (f) 3-(1H-pyrazol-4-yl)propan-1-amine dihydrochloride, TBTU, TEA, DCM, 12 hours (12 h)

### Step 1) Synthesis of methyl 3-iodo-4-mercaptobenzoate

Methyl 4-fluoro-3-iodobenzoate (5 g, 17.8 mmol, 1 eq) was dissolved in DMF (20 ml), and sodium disulfide (1.53 g, 19.6 mmol, 1.1 eq) was added thereto and stirred under reflux for two hours. Water was added to a reaction product, acidified with 1 N HCl, and extracted several times with EA. An organic layer was dried over magnesium sulfate and concentrated under reduced pressure to obtain a resulting residue which was a target compound as a crude product.

### Step 2) Synthesis of methyl 3-iodo-4-(((1-(4-(trifluoromethyl)phenyl)-1H-pyrazol-3-yl)methyl)thio)benzoate

3-(chloromethyl)-1-(4-(trifluoromethyl)phenyl)-1H-pyrazole (94 mg) was dissolved in DMF (2 ml), and K₂CO₃ (0.098 g, 0.71 mmol, 2 eq) was added thereto. Then, methyl 3-iodo-4-mercaptobenzoate (0.1 g) was added to a reaction product and stirred for 30 minutes. Water was added to a reaction product and extracted several times with EA. An organic layer was washed with a saturated NH₄Cl aqueous solution, separated, dried over magnesium sulfate, and then concentrated under reduced pressure. A resulting residue was purified by column chromatography (silica gel, 10% EA/HX) to obtain a target compound (78 mg, 0.15 mmol, 42%).

### Step 3) Synthesis of methyl 3-iodo-4-(((1-(4-(trifluoromethyl)phenyl)-1H-pyrazol-3-yl)methyl)sulfonyl)benzoate

Methyl 3-iodo-4-(((1-(4-(trifluoromethyl)phenyl)-1H-pyrazol-3-yl)methyl)thio)benzoate (0.1 g, 0.61 mmol, 1 eq) was dissolved in DCM (30 ml), and mCPBA (0.45 g, 1.83 mmol, 3 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure, and a resulting residue was purified by column chromatography (silica gel, 25% EA/HX) to obtain a target compound (0.19 g, 0.34 mmol, 57%).

### Step 4) Synthesis of methyl 3-((4-fluorophenyl)ethynyl)-4-(((1-(4-(trifluoromethyl)phenyl)-1H-pyrazol-3-yl)methyl)sulfonyl)benzoate

MeCN (1 ml, 0.2 M) was added to methyl 3-iodo-4-(((1-(4-(trifluoromethyl)phenyl)-1H-pyrazol-3-yl)methyl)sulfonyl)benzoate (0.1 g, 0.19 mmol, 1 eq) and TEA (0.078 ml, 0.56 mmol, 3 eq) was added thereto. PdCl₂[PPh₃]₂ (4 mg, 3 mol%) and CuI (1 mg, 3 mol%) were added to a reaction product and stirred at room temperature for five minutes under a nitrogen atmosphere. Subsequently, 4-fluorophenyl-acetylene (27 mg, 0.22mmol, 1.2 eq) was added thereto. A reaction mixture was stirred at room temperature for 16 hours and concentrated under reduced pressure. A resulting residue was purified by column chromatography (silica gel, 30% EA/HX) to obtain a target compound (86 mg, 0.16 mmol, 85%).

### Step 5) Synthesis of 3-((4-fluorophenyl)ethynyl)-4-(((1-(4-(trifluoromethyl)phenyl)-1H-pyrazol-3-yl)methyl)sulfonyl)benzoic acid

MeOH (1 ml, 0.1 M), H₂O (0.5 ml, 0.2 M), and NaOH (0.016 g, 0.4 mmol, 2.5 eq) were added to methyl 3-((4-fluorophenyl)ethynyl)-4-(((1-(4-(trifluoromethyl)phenyl)-1H-pyrazol-3-yl)methyl)sulfonyl)benzoate (0.085 g, 0.16 mmol, 1 eq) and stirred at 60°C for two hours. NaOH (0.016 g, 0.4 mmol, 2.5 eq) was further added and stirred for 12 hours. When a reaction was completed by confirming the reaction through TLC, the pH of a reaction product was adjusted to 1 with a 1 N HCl aqueous solution and extracted with EA. An organic layer was washed with water, separated, dried over magnesium sulfate, and concentrated under reduced pressure to obtain a target compound (0.075 g, 90%).

### Step 6) Synthesis of compound 4: Synthesis of N-(3-(1H-pyrazol-4-yl)propyl)-3-((4-fluorophenyl)ethynyl)-4-(((1-(4-(trifluoromethyl)phenyl)-1H-pyrazol-3-yl)methyl)sulfonyl)benzamide

DCM (2.5 ml) and TBTU (0.037 g, 0.21 mmol, 1.5 eq) were added to 3-((4-fluorophenyl)ethynyl)-4-(((1-(4-(trifluoromethyl)phenyl)-1H-pyrazol-3-yl)methyl)sulfonyl)benzoic acid (0.075 g, 0.14 mmol, 1 eq) and stirred at room temperature for 50 minutes. Subsequently, TEA (0.072 ml, 0.52 mmol, 3.7 eq) and 3-(1H-pyrazol-4-yl)propan-1-amine dihydrochloride (0.034 g, 0.17 mmol, 1.2 eq) were added to a reaction product and stirred for 12 hours. Water was added to a reaction product and extracted with EA. An organic layer was dried over magnesium sulfate and concentrated under reduced pressure, and a resulting residue was purified by column chromatography (silica gel, 2% MeOH/DCM) to obtain a target compound.

¹H NMR (400 MHz, MeOD) δ 8.25 - 8.22 (m, 2H), 7.98 (d, *J=* 8.1 Hz, 1H), 7.87 (d, *J =* 8.1 Hz, 1H), 7.73 (m, 6H), 7.49 (m, 2H), 7.20 (t, *J =* 8.5 Hz, 2H), 6.53 (s, 1H), 4.99 (s, 2H), 3.45 (d, *J =* 6.8 Hz, 2H), 2.63 (t, *J =* 7.4 Hz, 2H), 1.98 - 1.92 (m, 2H).

MS(ESI) m/e [M-H]⁻ 634.4

The reagents may be changed in substantially the same manner as in Example 5 to synthesize compounds 111, 112, 113, 114, 116, 117, 118, 120, 121, 123, 124, 125, 126, 127, 128, 129, 130, 132, 135, 137, 138, 139, 140, 141, 142, 143, 144, 145, 146, 149, 219, 157, 158, 209, 210, 201, 212, 213, 223, 225, 169, 170, 171, 218, 172, 173, 174, 175, 176, 177, 178, 179, 180, 181, 182, 183, 184, 185, 186, 187, 188, 189, 190, 217, 191, 192, 204, 205, 206, 207, 208, and 214.

### Example 6: Synthesis of compound 5

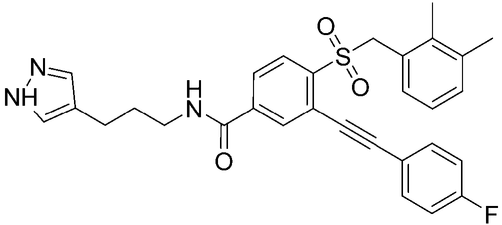

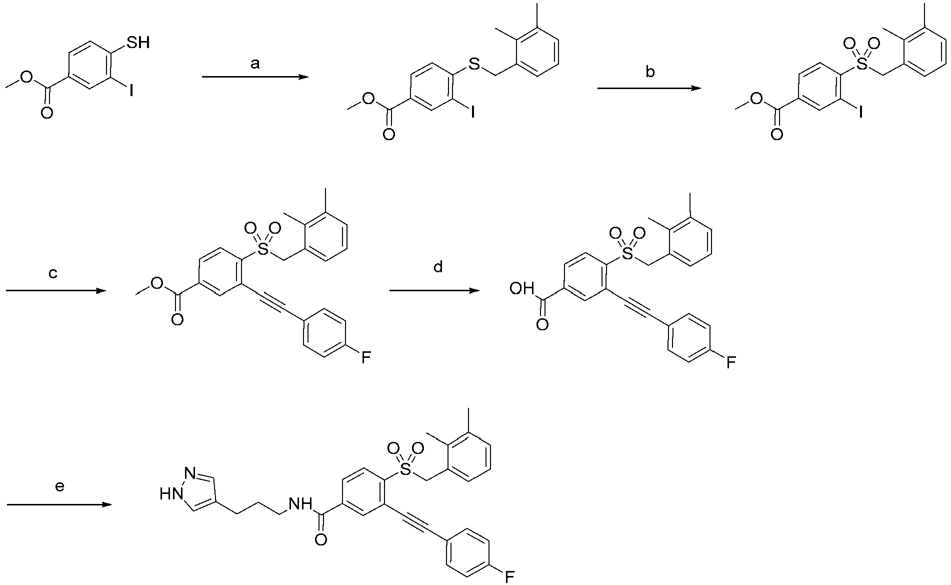

Scheme Reagents and Conditions (a) 1-(bromomethyl)-2,3-dimethylbenzene, K₂CO₃, DMF, room temperature (r.t), 0.5 hours (0.5 h) (b) mCPBA, DCM, room temperature (r.t), 16 hours (16 h) (c) PdCl₂[PPh₃]₂, CuI, TEA, ACN, room temperature (r.t), 16 hours (16 h) (d) NaOH, H₂O, MeOH, 55°C, 12 hours (12 h) (e) 3-(1H-pyrazol-4-yl)propan-1-amine dihydrochloride, TBTU, TEA, DCM, 12 hours (12 h)

### Step 1) Synthesis of methyl 4-((2,3-dimethylbenzyl)thio)-3-iodobenzoate

1-(bromomethyl)-2,3-dimethylbenzene (0.07 g, 0.36 mmol, 1 eq) was dissolved in DMF (2 ml), and K₂CO₃ (0.098 g, 0.71 mmol, 2 eq) was added thereto. Then, methyl 3-iodo-4-mercaptobenzoate (0.1 g) was added to a reaction product and stirred for 30 minutes. Water was added to a reaction product and extracted several times with EA. An organic layer was washed with a saturated NH₄Cl aqueous solution, separated, dried over magnesium sulfate, and then concentrated under reduced pressure. A resulting residue was purified by column chromatography (silica gel, 10% EA/HX) to obtain a target compound.

### Step 2) Synthesis of methyl 4-((2,3-dimethylbenzyl)sulfonyl)-3-iodobenzoate

Methyl 4-((2,3-dimethylbenzyl)thio)-3-iodobenzoate (0.31 g, 0.75 mmol, 1 eq) was dissolved in DCM (30 ml), and mCPBA (0.55 g, 2.3 mmol, 3 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure, and a resulting residue was purified by column chromatography (silica gel, 20% EA/HX) to obtain a target compound (0.17 g, 0.39 mmol, 52%).

### Step 3) Synthesis of methyl 4-((2,3-dimethylbenzyl)sulfonyl)-3-((4-fluorophenyl)ethynyl)benzoate

MeCN (2 ml, 0.2 M) was added to methyl 4-((2,3-dimethylbenzyl)sulfonyl)-3-iodobenzoate (0.17 g, 0.38 mmol, 1eq) and TEA (0.16 ml, 1.15 mmol, 3 eq) was added thereto. PdCl₂[PPh₃]₂ (8 mg, 3 mol%) and CuI (2 mg, 3 mol%) were added to a reaction product and stirred at room temperature for five minutes under a nitrogen atmosphere. Subsequently, 4-fluorophenyl-acetylene (55 mg, 0.45mmol, 1.2 eq) was added thereto. A reaction mixture was stirred at room temperature for 16 hours and concentrated under reduced pressure. A resulting residue was purified by column chromatography to obtain a target compound (0.165 g, 0.38 mmol, 99%).

### Step 4) Synthesis of 4-(((2,3-dimethylbenzyl)sulfonyl)-3-((4-fluorophenyl)ethynyl)benzoic acid

MeOH (1 ml, 0.1 M), H₂O (0.5 ml, 0.2 M), and NaOH (0.016 g, 0.4 mmol, 2.5 eq) were added to methyl 4-((2,3-dimethylbenzyl)sulfonyl)-3-((4-fluorophenyl)ethynyl)benzoate (0.165 g, 0.38 mmol, 1 eq) and stirred at 85°C for 12 hours. When a reaction was completed by confirming the reaction through TLC, the pH of a reaction product was adjusted to 1 with a 1 N HCl aqueous solution and extracted with EA. An organic layer was washed with water, separated, dried over magnesium sulfate, and concentrated under reduced pressure to obtain a target compound as a crude product.

### Step 5) Synthesis of compound 5: Synthesis of N-(3-(1H-pyrazol-4-yl)propyl)-4-((2,3-dimethylbenzyl)sulfonyl)-3-((4-fluorophenyl)ethynyl)benzamide

DCM (5 ml) and TBTU (0.17 g, 0.53 mmol, 1.5 eq) were added to 4-((2,3-dimethylbenzyl)sulfonyl)-3-((4-fluorophenyl)ethynyl)benzoic acid (0.15 g, 0.35 mmol, 1 eq) and stirred at room temperature for 50 minutes. Subsequently, TEA (0.18 ml, 1.3 mmol, 3.7 eq) and 3-(1H-pyrazol-4-yl)propan-1-amine dihydrochloride (0.083 g, 0.42 mmol, 1.2 eq) were added to a reaction product and stirred for 12 hours. Water was added to a reaction product and extracted with EA. An organic layer was dried over magnesium sulfate and concentrated under reduced pressure, and a resulting residue was purified by column chromatography (silica gel, 3-5% MeOH/DCM) to obtain a target compound (0.07 g, 0.13 mmol, 38%).

¹H NMR (400 MHz, DMSO) δ 12.51 (s, 1H), 8.83 (m, 1H), 8.27 (s, 1H), 8.00 (d, *J* = 8.3 Hz, 1H), 7.91 (d, *J =* 8.1 Hz, 1H), 7.77 - 7.70 (m, 2H), 7.52 (br, 1H), 7.37 (d, *J =* 8.5 Hz, 2H), 7.10 (d, *J =* 7.4 Hz, 1H), 6.95 (t, *J =* 7.5 Hz, 1H), 6.85 (d, *J =* 7.6 Hz, 1H), 4.91 (s, 2H), 3.32 (m, 2H), 2.48 (m, 2H), 2.19 (s, 6H), 1.80 (p, *J =* 7.0 Hz, 2H).

MS(ESI) m/e [M-H]⁻ 528.4

### Example 7: Synthesis of compound 6

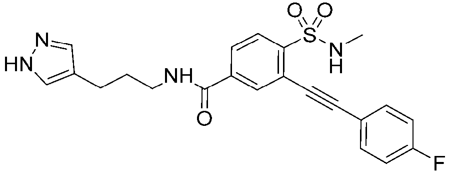

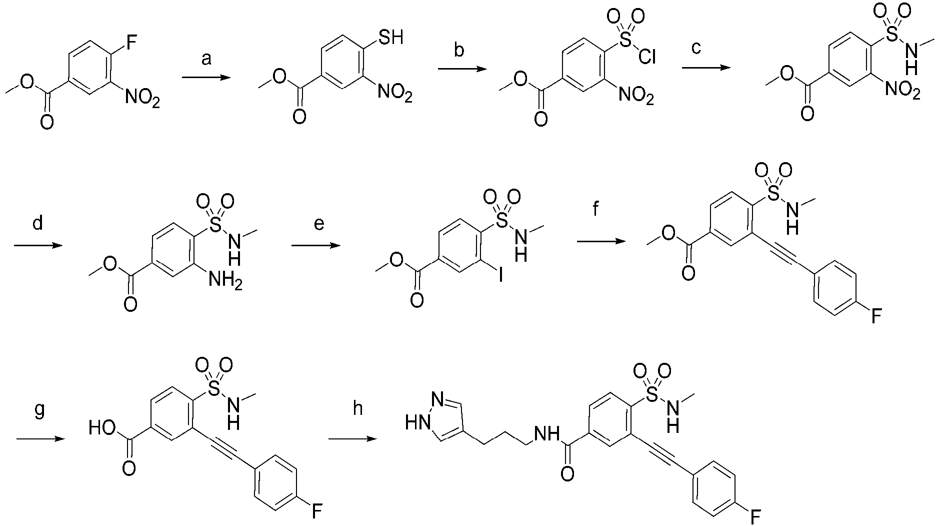

Scheme Reagents and Conditions (a) Na₂S, DMF, room temperature (r.t), 16 hours (16 h) (b) n-chlorosuccinimide, 2 N HCl (aq.), MeCN, room temperature (r.t), 16 hours (16 h) (c) methylamine, EtOH, MeCN, room temperature (r.t), one hour (1 h) (d) Fe, AcOH, 80°C, one hour (1 h) (e) HCl, NaNO₂, KI, H₂O, 0°C room temperature (r.t), one hour (1 h) (f) PdCl₂[PPh₃]₂, CuI, TEA, ACN, room temperature (r.t), one hour (1 h) (g) NaOH, H₂O, MeOH, 55°C, four hours (4 h) (h) 3-(1H-pyrazol-4-yl)propan-1-amine dihydrochloride, TBTU, TEA, DCM, 12 hours (12 h)

### Step 1) Synthesis of methyl 4-mercapto-3-nitrobenzoate

Methyl 4-fluoro-3-nitrobenzoate (10 g, 46.4 mmol, 1 eq) was dissolved in DMF (80 ml), and sodium sulfide (4.32 g, 55.3 mmol, 1.2 eq) was added thereto and stirred at room temperature for 16 hours. After completion of a reaction, water (200 ml) was added to a reaction product and stirred while pH was adjusted to 5 with 1 N HCl. While being stirred, the yellow solid was produced, filtered, and washed with 25% EA/HX in suspension to obtain a target compound (6.13 g, 28.7 mmol, 62%).

### Step 2) Synthesis of methyl 4-(chlorosulfonyl)-3-nitrobenzoate

Methyl 4-mercapto-3-nitrobenzoate (1 g, 4.9 mmol, 1 eq) was added to a reaction product of n-chlorosuccinimide (3.3 g, 24.5 mmol, 5 eq), a 2 N HCl aqueous solution (5.2 ml, 2 eq) and MeCN (50 ml) in ice-bath and stirred at room temperature for 16 hours. Upon completion of a reaction, water (20 ml) was added thereto and MeCN was concentrated under reduced pressure. A residue was extracted several times with EA and washed several times with saturated NaHCO₃ aqueous solution and brine. An organic layer was dried over magnesium sulfate and concentrated under reduced pressure to obtain a resulting residue which was a target compound as a crude product.

### Step 3) Synthesis of methyl 4-(N-methylsulfamoyl)-3-nitrobenzoate

Methyl 4-(chlorosulfonyl)-3-nitrobenzoate (0.2 g, 0.71 mmol, 1 eq) was dissolved in MeCN, and a methylamine solution (33 wt. % in absolute ethanol) was added thereto and stirred at room temperature for one hour. Water was added to a reaction product, and MeCN was concentrated under reduced pressure to obtain a resulting residue, which was then extracted several times with EA. An organic layer was dried over magnesium sulfate and concentrated under reduced pressure, and a resulting residue was purified by column chromatography (silica gel, 20-50% EA/HX) to obtain a target compound (0.13 g, 0.47 mmol, 66%).

### Step 4) Synthesis of methyl 3-amino-4-(N-methylsulfamoyl)benzoate

AcOH (5 ml) and iron (0.26 g, 4.67 mmol, 10 eq) were added to methyl 3-amino-4-(N-methylsulfamoyl)benzoate (0.128 g, 0.47 mmol, 1 eq) and stirred at 80°C for one hour. A catalyst was removed by celite filtration, and a filtrate was concentrated under reduced pressure. A residue was basified with a potassium carbonate aqueous solution and extracted with ethyl acetate, and then an organic layer was concentrated under reduced pressure to obtain a target compound as a crude product.

### Step 5) Synthesis of methyl 3-iodo-4-(N-methylsulfamoyl)benzoate

A solution of sodium nitrite (0.03 g, 0.4 mmol, 1.5 eq) dissolved in water (1 ml) was slowly added dropwise to a suspension of methyl 3-amino-4-(N-methylsulfamoyl)benzoate (0.065 g, 0.27 mmol, 1 eq), a concentrated hydrochloric acid aqueous solution (1 ml) and water (1 ml) at 0°C. A resulting mixture was stirred in ice-bath for 30 minutes, after which a solution of potassium iodide (0.13 g, 0.8 mmol, 3 eq) dissolved in water (1 mL) was added thereto at 0°C. A reaction mixture was stirred at room temperature for one to two hours, treated with a saturated potassium carbonate aqueous solution (pH>8) and extracted with ethyl acetate. An organic layer was dried over magnesium sulfate and concentrated under reduced pressure, and a resulting residue was purified by column chromatography (silica gel, 20-60% EA/HX) to obtain a target compound (0.06 g, 0.17 mmol, 63%).

### Step 6) Synthesis of methyl 3-((4-fluorophenyl)ethynyl)-4-(N-methylsulfamoyl)benzoate

MeCN (1 ml) was added to methyl 3-iodo-4-(N-methylsulfamoyl)benzoate (0.06 g, 0.17 mmol, 1 eq) and TEA (0.071 ml, 0.51 mol, 3 eq) was added thereto. PdCl₂[PPh₃]₂ (0.0036 mg, 3 mol%) and CuI (0.001 mg, 3 mol%) were added to a reaction product and stirred at room temperature for five minutes under a nitrogen atmosphere. Subsequently, 4-fluorophenyl-acetylene (0.024 g, 0.2mmol, 1.2 eq) was added thereto. A reaction mixture was stirred at room temperature for one hour and concentrated under reduced pressure. A resulting residue was purified by column chromatography (silica gel, 40% EA/HX) to obtain a target compound (0.059 g, 0.17 mmol, 99%).

### Step 7) Synthesis of 3-((4-fluorophenyl)ethynyl)-4-(N-methylsulfamoyl)benzoic acid

MeOH (2.6 ml, 0.1 M), H₂O (1.3 ml, 0.2 M), and NaOH (0.026 g, 0.64 mmol, 2.5 eq) were added to methyl 3-((4-fluorophenyl)ethynyl)-4-(N-methylsulfamoyl)benzoate (0.089 g, 0.26 mmol, 1 eq) and stirred at 55°C for four hours. When a reaction was completed by confirming the reaction through TLC, the pH of a reaction product was adjusted to 1 with a 1 N HCl aqueous solution and extracted with EA. An organic layer was washed with water, separated, dried over magnesium sulfate, and concentrated under reduced pressure to obtain a target compound as a crude product.

### Step 8) Synthesis of compound 6: Synthesis of N-(3-(1H-pyrazol-4-yl)propyl)-3-((4-fluorophenyl)ethynyl)-4-(N-methylsulfamoyl)benzamide

DCM (5 ml) and TBTU (0.12 g, 0.38 mmol, 1.5 eq) were added to 3-((4-fluorophenyl)ethynyl)-4-(N-methylsulfamoyl)benzoic acid (0.084 g, 0.25 mmol, 1 eq) and stirred at room temperature for 50 minutes. Subsequently, TEA (0.13 ml, 0.93 mmol, 3.7 eq) and 3-(1H-pyrazol-4-yl)propan-1-amine dihydrochloride (0.060 g, 0.3 mmol, 1.2 eq) were added to a reaction product and stirred for 12 hours. Water was added to a reaction product and extracted with EA. An organic layer was dried over magnesium sulfate and concentrated under reduced pressure, and a resulting residue was purified by reverse phase column chromatography (C18 Aq, 40% ACN/0.1 % formic acid in water) to obtain a target compound (0.02 g, 0.045 mmol, 18%).

¹H NMR (400 MHz, MeOD) δ 8.42 (s, 1H), 8.05 - 7.92 (m, 2H), 7.56 - 7.48 (m, 4H), 7.18 (t, *J =* 8.8 Hz, 2H), 6.95 (s, 1H), 3.47 (t, *J =* 7.1 Hz, 2H), 2.94 (s, 3H), 2.63 (t, *J =* 7.5 Hz, 2H), 1.94 (p, *J =* 7.3 Hz, 2H).

MS(ESI) m/e MH⁺ 441.2

The reagents may be changed in substantially the same manner as in Example 7 to synthesize compounds 12, 14, 19, 20, 22, 29, 71 to 74, 215, and 216.

### Example 8: Synthesis of compound 7

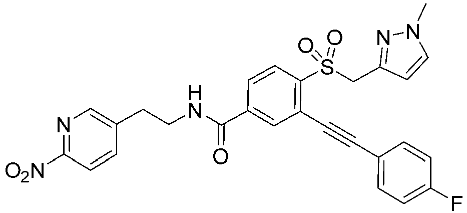

### Synthesis of 3-((4-fluorophenyl)ethynyl)-4-(((1-methyl-1H-pyrazol-3-yl)methyl)sulfonyl)-N-(2-(6-nitropyridin-3-yl)ethyl)benzamide

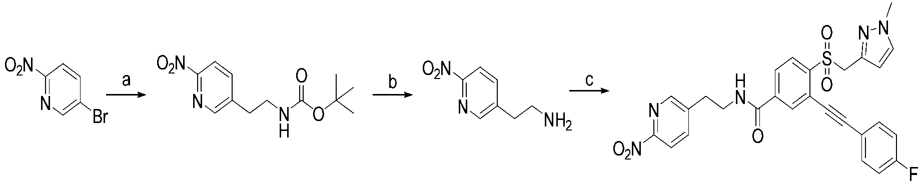

Scheme reagents and conditions (a) potassium tert-butyl N-[2-(trifluoroboranuidyl)ethyl]carbamate, Pd(dppf)Cl₂ ·CH₂Cl₂, Cs₂CO₃, H₂O, toluene, 80°C, overnight; (b) 4 N HCl in dioxane, room temperature (rt), overnight; (c) TBTU, TEA, DCM, room temperature (rt), overnight

### Step 1) Synthesis of tert-butyl (2-(6-nitropyridin-3-yl)ethyl)carbamate

5-bromo-2-nitropyridine (1 g, 4.93 mmol, 1.0 eq) was dissolved in toluene (20 ml, 0.25 M), and potassium tert-butyl N-[2-(trifluoroboranuidyl)ethyl]carbamate (1.36 g, 5.41 mmol, 1.1 eq), [1,1'-bis(diphenylphosphino)ferrocene]dichloropalladium(II) complexed with dichloromethane (0.302 g, 0.369 mmol, 0.07 eq), Cs₂CO₃ (4.815 g, 14.8 mmol, 3 eq), and H₂O (5 ml) were added thereto, heated to 80°C and stirred overnight. After cooling to room temperature, water and EA were added and an organic layer was extracted. The organic layer was dried over MgSO₄, filtered, concentrated, and purified by column chromatography (silica gel, EA/HX). (0.842 g, 64%)

### Step 2) Synthesis of 2-(6-nitropyridin-3-yl)ethan-1-amine

Tert-butyl (2-(6-nitropyridin-3-yl)ethyl)carbamate (0.842 g, 3.15 mmol, 1 eq) was dissolved in 4 N HCl in dioxane (8 ml, 31.5 mmol, 10 eq) and stirred at room temperature overnight. A reaction mixture was concentrated, and diethyl ether was added thereto and precipitated. A precipitated solid was washed with diethyl ether and filtered under reduced pressure. (0.507 g, 79%)

### Step 3) Synthesis of compound 7: Synthesis of 3-((4-fluorophenyl)ethynyl)-4-(((1-methyl-1H-pyrazol-3-yl)methyl)sulfonyl)-N-(2-(6-nitropyridin-3-yl)ethyl)benzamide

3-((4-fluorophenyl)ethynyl)-4-(((1-methyl-1H-pyrazol-3-yl)methyl)sulfonyl)benzoic acid (0.1 g, 0.25 mmol, 1 eq) was dissolved in DCM (0.5 ml, 0.5 M), and then 2-(6-nitropyridin-3-yl)ethan-1-amine (0.057 g, 0.28 mmol, 1.1 eq), TBTU (0.12 g, 0.375 mmol, 1.5 eq), and TEA (0.076 ml, 0.55 mmol, 2.2 eq) were added thereto and stirred at room temperature overnight. A reaction mixture was diluted in DCM, and then a saturated NaHCO₃ aqueous solution, H₂O, and brine were added thereto, and an organic layer was extracted. The organic layer was dried over MgSO₄, filtered, concentrated, and then precipitated with MC. A precipitated solid was washed with MC and filtered under reduced pressure. (0.052 g, 38%)

¹H NMR (400 MHz, MeOD) δ 8.51 (s, 1H), 8.27 (d, *J* = 8.3 Hz, 1H), 8.10 (d, *J* = 12.5 Hz, 2H), 7.88 (d, *J =* 8.2 Hz, 1H), 7.79 (d, *J =* 8.2 Hz, 1H), 7.75 - 7.69 (m, 2H), 7.43 (s, 1H), 7.21 (t, *J =* 8.4 Hz, 2H), 6.15 (s, 1H), 4.81 (s, 2H), 3.76 - 3.70 (m, 5H), 3.14 (t, *J =* 6.8 Hz, 2H). ; MS(ESI) m/e MH⁺ 548.4

### Example 9: Synthesis of compound 8

### Synthesis of N-(2-(6-aminopyridin-3-yl)ethyl)-3-((4-fluorophenyl)ethynyl)-4-(((1-methyl-1H-pyrazol-3-yl)methyl)sulfonyl)benzamide

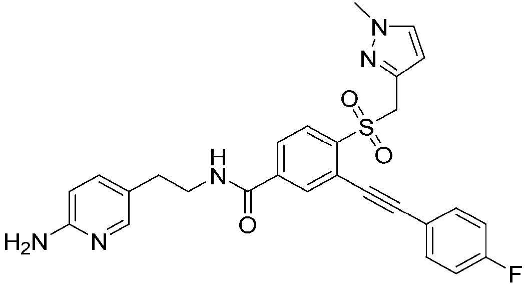

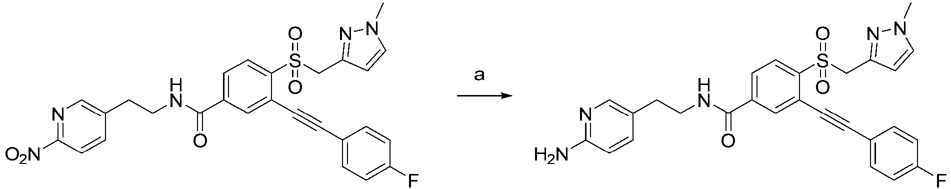

Scheme reagents and conditions (a) Zn, NH₄Cl, 1,4-dioxane, H₂O, room temperature (rt), overnight

3-((4-fluorophenyl)ethynyl)-4-(((1-methyl-1H-pyrazol-3-yl)methyl)sulfonyl)-N-(2-(6-nitropyridin-3-yl)ethyl)benzamide (0.046 g, 0.084 mmol, 1 eq) was dissolved in 1,4-dioxane : water (H₂O) (3:1 (v/v) (1 ml, 0.084 M), and then zinc (0.055 g, 0.84 mmol, 10 eq) and NH₄Cl (0.045 g, 0.84 mmol, 10 eq) were added thereto and stirred at room temperature overnight. A reaction mixture was washed with MeOH and filtered under reduced pressure through celite. A filtrate was concentrated and purified by reverse phase column chromatography (0.1% formic acid in H₂O/acetonitrile). (0.021 g, 47%)

¹H NMR (400 MHz, MeOD) δ 8.13 (s, 1H), 7.90 (d, *J =* 8.6 Hz, 2H), 7.83 (d, *J* = 8.3 Hz, 1H), 7.78 - 7.73 (m, 2H), 7.63 (d, *J =* 8.6 Hz, 1H), 7.45 (s, 1H), 7.23 (t, *J =* 8.3 Hz, 2H), 6.74 (d, *J =* 8.8 Hz, 1H), 6.16 (s, 1H), 4.84 (s, 2H), 3.74 (s, 3H), 3.60 (t, *J =* 6.7 Hz, 2H), 2.82 (t, *J* = 6.7 Hz, 2H). MS(ESI) m/e MH⁺ 518.4

### Example 10: Synthesis of compound 9

### Synthesis of N-(3-(1H-pyrazol-4-yl)propyl)-3-((4-fluorophenyl)ethynyl)-4-((3-(trifluoromethyl)benzyl)sulfonyl)benzamide

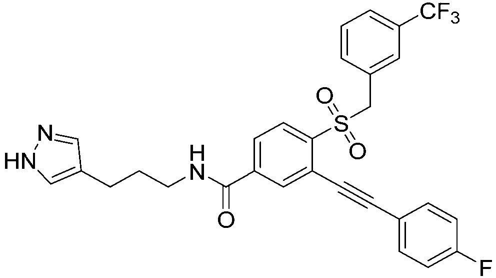

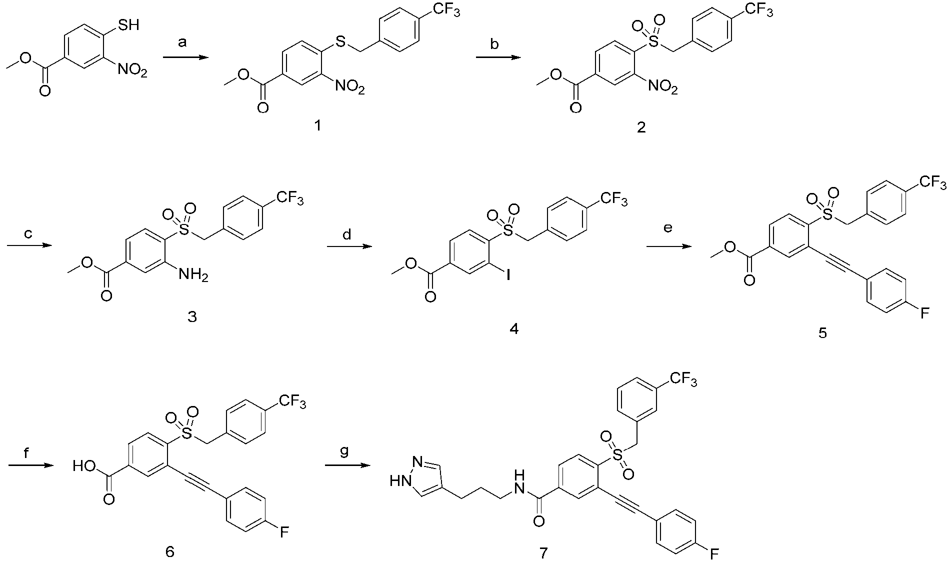

Scheme reagents and conditions (a) 1-(chloromethyl)-4-(trifluoromethyl)benzene, TEA, ACN, reflux, one hour (1 h); (b) mCPBA, MC, room temperature (RT), overnight; (c) Fe, NH₄Cl, EtOH, water (H₂O), reflux, one hour (1 h); (d) sodium nitrite (NaNO₂), potassium iodide (KI), pTSA, ACN, 0°C, one hour (1 h); (e) 1-ethynyl-4-fluorobenzene, TEA, CuI, PdCl₂(PPh₃)₂, ACN, reflux, one hour (1 h); (f) NaOH, EtOH, H₂O, reflux, one hour (1 h); (g) 3-(1H-pyrazol-4-yl)propan-1-amine dihydrochloride, TBTU, TEA, MC, room temperature (rt), overnight

### Step 1) Synthesis of methyl 3-nitro-4-((4-(trifluoromethyl)benzyl)thio)benzoate

Methyl 4-mercapto-3-nitrobenzoate (0.1 g, 0.469 mmol, 1 eq) was dissolved in ACN (5 ml, 0.1 M), and then 1-(chloromethyl)-4-(trifluoromethyl)benzene (0.07 ml, 0.469 mmol, 1eq) and TEA (0.09 ml, 0.61 mmol, 1.3 eq) were added and stirred under reflux for one hour. After completion of a reaction, a resulting mixture was concentrated, and water was added thereto and extracted with EA. An organic layer was dried over MgSO₄, filtered and concentrated to obtain a resulting product as a crude product. (0.15 g, 85%)

¹H NMR (400 MHz, CDCl₃) δ 8.90 (s, 1H), 8.19 (d, *J =* 8.4 Hz, 1H), 7.71 (s, 1H), 7.64 (dd, *J =* 16.9, 7.6 Hz, 2H), 7.52 (t, *J =* 8.6 Hz, 2H), 4.32 (s, 2H), 3.99 (s, 3H).

### Step 2) Synthesis of methyl 3-nitro-4-((4-(trifluoromethyl)benzyl)sulfonyl)benzoate

Methyl 3-nitro-4-((4-(trifluoromethyl)benzyl)thio)benzoate (0.15 g, 0.4 mmol, 1 eq) was dissolved in MC (2 ml, 0.2M), and then mCPBA (0.68 g, 3.96 mmol, 10 eq) was added thereto and stirred overnight. After completion of a reaction, a saturated NaHCO₃ aqueous solution was added and extracted with MC. An organic layer was dried over MgSO₄, filtered and concentrated to obtain a resulting product as a crude product. (0.138 g, 86%)

¹H NMR (400 MHz, CDCl₃) δ 8.43 (s, 1H), 8.16 (d, *J =* 8.0 Hz, 1H), 7.69 (d, *J =* 8.1 Hz, 1H), 7.61 (d, *J =* 7.6 Hz, 1H), 7.56 - 7.51 (m, 2H), 7.45 (t, *J =* 7.6 Hz, 1H), 4.89 (s, 2H), 4.00 (s, 3H).

### Step 3) Synthesis of methyl 3-amino-4-((4-(trifluoromethyl)benzyl)sulfonyl)benzoate

Methyl 3-nitro-4-((4-(trifluoromethyl)benzyl)sulfonyl)benzoate (0.14 g, 0.342 mmol, 1 eq) was dissolved in ethanol (EtOH) : water (H₂O) (7 ml, 4:1 (v:v), 0.05 M), and then Fe (0.1 g, 1.71 mmol, 5 eq) and NH₄Cl (0.091 g, 1.71 mmol, 5 eq) were added thereto and stirred under reflux for one hour. After cooling to room temperature, a resulting product was filtered through celite, and water was added to the filtrate and extracted with EA. An organic layer was dried over MgSO₄, filtered and concentrated to obtain a resulting product as a crude product. (0.12 g, 93.8 %)

¹H NMR (400 MHz, CDCl₃) δ 7.57 (d, *J =* 7.4 Hz, 1H), 7.44 - 7.32 (m, 4H), 7.28 (d, *J =* 6.1 Hz, 2H), 5.12 (s, 2H), 4.43 (s, 2H), 3.92 (s, 3H).

### Step 4) Synthesis of methyl 3-iodo-4-((4-(trifluoromethyl)benzyl)sulfonyl)benzoate

Methyl 3-amino-4-((4-(trifluoromethyl)benzyl)sulfonyl)benzoate (0.373 g, 1 mmol, 1 eq) was dissolved in ACN (10 ml, 0.1 M), pTSA (0.517 g, 3 mmol, 3 eq) was added thereto, and then sodium nitrite (0.07 g, 1.02 mmol, 1.02 eq) dissolved in 1 ml of water and KI (0.169 g, 1.02 mmol, 1.02 eq) dissolved in 1 ml of water were added thereto at 0°C. After reacting at room temperature for one hour, a saturated NaHCO₃ aqueous solution was added thereto, neutralized to PH 8 or higher, and then extracted with EA. An organic layer was dried over MgSO₄, filtered, concentrated, and purified by column chromatography (silica gel, EA/HX). (0.175 g, 36%)

¹H NMR (400 MHz, CDCl₃) δ 8.77 (s, 1H), 8.01 (d, *J =* 8.2 Hz, 1H), 7.86 (d, *J* = 8.1 Hz, 1H), 7.59 (d, *J =* 5.0 Hz, 1H), 7.44 (d, *J =* 6.3 Hz, 3H), 4.75 (s, 2H), 3.99 (s, 3H).

### Step 5) Synthesis of methyl 3-((4-fluorophenyl)ethynyl)-4-((4-(trifluoromethyl)benzyl)sulfonyl)benzoate

Methyl 3-iodo-4-((4-(trifluoromethyl)benzyl)sulfonyl)benzoate (0.194 g, 0.4 mmol, 1 eq) was dissolved in ACN (4 ml, 0.1 M), and then TEA (0.12 ml, 0.88 mmol, 2.2 eq), PdCl₂(PPh₃)₂ (8.4 mg, 0.012 mmol, 3 mol %), and CuI (3 mg, 0.016 mmol, 4 mol%) were added thereto and stirred at room temperature for five minutes. 1-ethynyl-4-fluorobenzene (0.07 ml, 0.6 mmol, 1.5 eq) was added thereto and stirred at a temperature of 90°C for one hour. After cooling to room temperature, a resulting product was concentrated and purified by column chromatography (silica gel, EA/HX). (0.183 g, 96.3%)

¹H NMR (400 MHz, CDCl₃) δ 8.40 (s, 1H), 8.01 (d, *J =* 8.1 Hz, 1H), 7.87 (d, *J =* 8.3 Hz, 1H), 7.71 (t, *J =* 6.1 Hz, 2H), 7.57 (d, *J =* 7.1 Hz, 1H), 7.47 - 7.38 (m, 3H), 7.17 (t, *J* = 7.9 Hz, 2H), 4.76 (s, 2H), 4.01 (s, 3H).

### Step 6) Synthesis of 3-((4-fluorophenyl)ethynyl)-4-((4-(trifluoromethyl)benzyl)sulfonyl)benzoic acid

Methyl 3-((4-fluorophenyl)ethynyl)-4-((4-(trifluoromethyl)benzyl)sulfonyl)benzoate (0.183 g, 0.396 mmol, 1 eq) was dissolved in EtOH (8 ml, 0.05 M), and then NaOH (0.079 g, 1.98 mmol, 5 eq) dissolved in H₂O (0.8 ml) was added thereto and stirred under reflux for one hour. After cooling to room temperature, a resulting product was concentrated and diluted with water. After adjusting to pH=5 to 6 with 1 N HCl, a resulting solid was filtered, washed with water, and dried. (0.14 g, 76.5%)

¹H NMR (400 MHz, DMSO) δ 8.28 (s, 1H), 8.04 (d, *J =* 8.2 Hz, 1H), 7.84 (d, *J =* 8.3 Hz, 1H), 7.80 - 7.73 (m, 2H), 7.68 (d, *J =* 7.6 Hz, 1H), 7.58 - 7.51 (m, 1H), 7.51 - 7.47 (m, 1H), 7.45 (s, 1H), 7.38 (t, *J =* 8.5 Hz, 2H), 5.03 (s, 2H).

### Step 7) Synthesis of N-(3-(1H-pyrazol-4-yl)propyl)-3-((4-fluorophenyl)ethynyl)-4-((3-(trifluoromethyl)benzyl)sulfonyl)benzamide

After dissolving 3-((4-fluorophenyl)ethynyl)-4-((4-(trifluoromethyl)benzyl)sulfonyl)benzoic acid (0.046 g, 0.1 mmol, 1 eq) in MC, TBTU (0.048 g, 0.15 mmol, 1.5 eq), TEA (0.03 ml, 0.22 mmol, 2.2 eq), and 3-(1H-pyrazol-4-yl)propan-1-amine dihydrochloride (0.022 g, 0.11 mmol, 1.1 eq) were added thereto and stirred at room temperature overnight. After completion of a reaction, a saturated NaHCO₃ aqueous solution was added, neutralized and extracted with MC. An organic layer was dried over MgSO₄, filtered, concentrated, and purified by column chromatography (silica gel, EA 100%). (35.7 mg, 62.7%)

¹H NMR (400 MHz, CDCl₃) δ 8.05 (s, 1H), 7.78 (d, *J =* 8.1 Hz, 1H), 7.69 - 7.63 (m, 2H), 7.61 (d, *J =* 8.0 Hz, 1H), 7.54 (d, *J =* 6.4 Hz, 1H), 7.45 (s, 2H), 7.41 - 7.34 (m, 3H), 7.13 (t, *J =* 8.1 Hz, 2H), 6.35 (s, 1H), 4.70 (s, 2H), 3.51 (dd, *J =* 12.6, 6.3 Hz, 2H), 2.62 (t, *J =* 7.2 Hz, 2Hff), 1.99 - 1.89 (m, 2H). m/e MH⁺ 570.1

### Example 11: Synthesis of compound 10

### N-(3-(1H-pyrazol-4-yl)propyl)-3-((4-fluorophenyl)ethynyl)-4-(((1-(pyridin-4-yl)-1H-pyrazol-3-yl)methyl)sulfonyl)benzamide

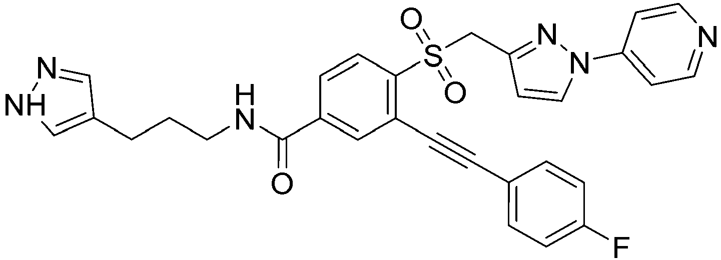

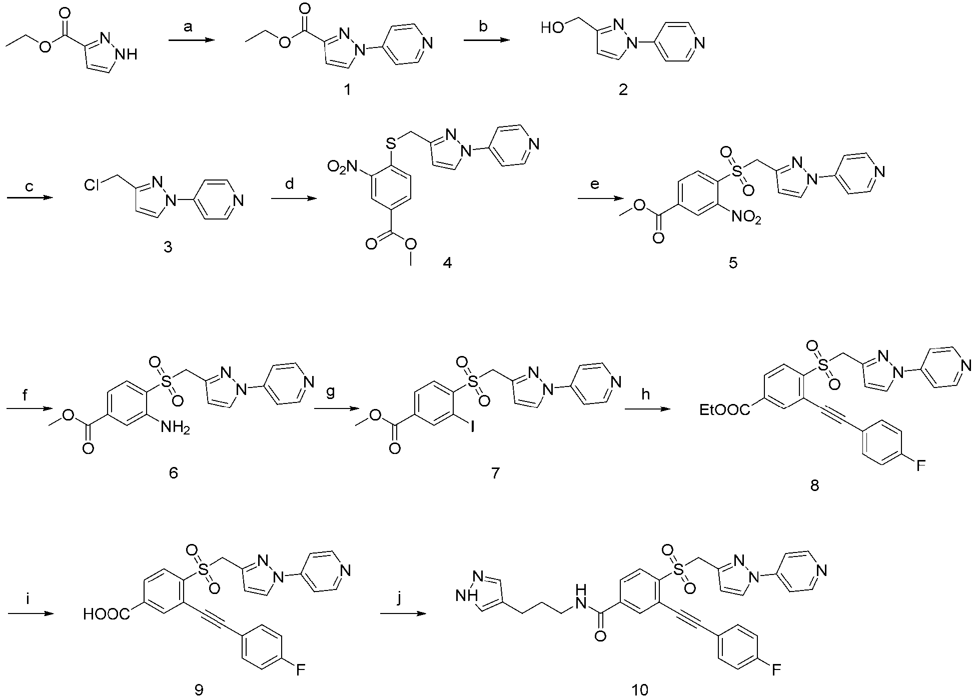

Scheme reagents and conditions (a) 4-iodopyridine, K₃PO₄, CuI, 1,2-transdimethylaminocyclohexane, 110°C, 16 hours (16 h); (b) 1.0 M LiAlH₄ in THF, THF, 0°C, one hour (1 h); (c) SOCl₂, MC, 0°C → reflux; (d) methyl 4-mercapto-3-nitrobenzoate, K₂CO₃, DMF, room temperature (rt), overnight; (e) mCPBA, MC, room temperature (RT), overnight; (f) Fe, NH₄Cl, ethanol (EtOH), water (H₂O), reflux, one hour (1 h); (g) sodium nitrite (NaNO₂), KI, pTSA, ACN, 0°C, one hour (1 h); (h) 1-ethynyl-4-fluorobenzene, TEA, CuI, PdCl₂(PPh₃)₂, ACN, reflux, one hour (1 h); (i) NaOH, EtOH, H₂O, reflux, one hour (1 h); (j) 3-(1H-pyrazol-4-yl)propan-1-amine dihydrochloride, TBTU, TEA, MC, room temperature (rt), overnight

### Step 1) Synthesis of ethyl 1-(pyridin-4-yl)-1H-pyrazole-3-carboxylate

After adding ethyl 1H-pyrazole-3-carboxylate (1 g, 7.2 mmol, 1 eq) to a pressure tube and dissolving the same in toluene (30 ml, 0.2 M), 4-iodopyridine (1.54 g, 7.56 mmol, 1.05 eq), K₃PO₄ (3.8 g, 18 mmol, 2.5 eq), CuI (0.14 g, 0.72 mmol, 0.1 eq), and 1,2-transdimethylaminocyclohexane (0.22 ml, 1.44 mmol, 0.2 eq) were added thereto and stirred at 110°C for 16 hours. After completion of a reaction, a resulting mixture was cooled to room temperature, and water was added thereto and extracted with EA. An organic layer was dried over MgSO₄, filtered, concentrated, and purified by column chromatography (silica gel, EA/HX). (1.1 g, 70.5%)

¹H NMR (400 MHz, CDCl₃) δ 8.71 (d, *J =* 4.9 Hz, 2H), 8.06 (s, 1H), 7.72 (d, *J =* 4.9 Hz, 2H), 7.04 (s, 1H), 4.46 (q, *J =* 6.8 Hz, 2H), 1.43 (t, *J =* 6.9 Hz, 3H).

### Step 2) Synthesis of (1-(pyridin-4-yl)-1H-pyrazol-3-yl)methanol

Ethyl 1-(pyridin-4-yl)-1H-pyrazole-3-carboxylate (1.1 g, 5.06 mmol, 1 eq) was dissolved in anhydrous THF (50 ml, 0.1 M), and then 1.0 M LiAlH₄ in THF (7.6 ml, 7.60 mmol, 1.5 eq) was slowly added dropwise thereto at 0°C and stirred for one hour. After completion of a reaction, water and EA were added, a resulting solid was filtered, and a filtrate was concentrated to obtain a crude product. (0.89 g, 100%)

¹H NMR (400 MHz, MeOD) δ 8.59 (d, *J =* 5.3 Hz, 2H), 8.42 (s, 1H), 7.87 (d, *J* = 5.4 Hz, 2H), 6.63 (s, 1H), 4.70 (s, 2H).

### Step 3) Synthesis of 4-(3-(chloromethyl)-1H-pyrazol-1-yl)pyridine

(1-(pyridin-4-yl)-1H-pyrazol-3-yl)methanol (0.89 g, 5.06 mmol, 1 eq) was dissolved in MC (25 ml, 0.2 M), and then SOCl₂ (1.1 ml, 15.2 mmol, 3 eq) was slowly added dropwise thereto at 0°C and stirred under reflux overnight. After completion of a reaction, a resulting product was concentrated under reduced pressure to obtain a crude product. (1.42 g, 100%)

¹H NMR (400 MHz, MeOD) δ 8.62 (d, *J =* 5.3 Hz, 2H), 8.45 (s, 1H), 7.89 (d, *J* = 5.4 Hz, 2H), 6.69 (s, 1H), 4.73 (s, 2H).

### Step 4) Synthesis of methyl 3-nitro-4-(((1-(pyridin-4-yl)-1H-pyrazol-3-yl)methyl)thio)benzoate

After dissolving 4-(3-(chloromethyl)-1H-pyrazol-1-yl)pyridine (0.091 g, 0.47 mmol, 1 eq) in DMF (5 ml, 0.1 M), K₂CO₃ (0.13 g, 0.94 mmol, 2 eq) was added thereto, and methyl 4-mercapto-3-nitrobenzoate (0.1 g, 0.47 mmol, 1 eq) dissolved in DMF (5 ml, 0.1 M) was slowly added thereto and stirred at room temperature overnight. After completion of a reaction, water was added thereto, and a resulting solid was filtered and dried to obtain a resulting product as a crude product. (0.09 g, 52%)

¹H NMR (400 MHz, MeOD) δ 8.77 (s, 1H), 8.60 (d, *J =* 5.3 Hz, 2H), 8.42 (s, 1H), 8.21 (d, *J =* 8.6 Hz, 1H), 7.98 (d, *J =* 8.6 Hz, 1H), 7.87 (d, *J =* 5.5 Hz, 2H), 6.67 (s, 1H), 4.49 (s, 2H), 3.96 (s, 3H).

### Step 5) Synthesis of methyl 3-nitro-4-(((1-(pyridin-4-yl)-1H-pyrazol-3-yl)methyl)sulfonyl)benzoate

Methyl 3-nitro-4-(((1-(pyridin-4-yl)-1H-pyrazol-3-yl)methyl)thio)benzoate (0.61 g, 1.65 mmol, 1 eq) was dissolved in MC (8 ml, 0.2 M), and then mCPBA (2.8 g, 16.46 mmol, 10 eq) was added thereto and stirred overnight. After completion of a reaction, a saturated NaHCO₃ aqueous solution was added and extracted with MC. An organic layer was dried over MgSO₄, filtered and concentrated to obtain a resulting product as a crude product. (0.685 g, 100%)

¹H NMR (400 MHz, MeOD) δ 8.50 (s, 1H), 8.43 (s, 1H), 8.36 (d, *J* = 6.5 Hz, 2H), 8.28 (d, *J =* 7.9 Hz, 1H), 7.99 (s, 1H), 7.95 (d, *J =* 7.9 Hz, 2H), 7.86 (d, *J* = 6.6 Hz, 2H), 7.60 (d, *J =* 7.8 Hz, 1H), 7.47 (t, *J =* 7.9 Hz, 1H), 6.72 (s, 1H), 5.11 (s, 2H), 4.00 (s, 3H).

### Step 6) Synthesis of methyl 3-amino-4-(((1-(pyridin-4-yl)-1H-pyrazol-3-yl)methyl)sulfonyl)benzoate

Methyl 3-nitro-4-(((1-(pyridin-4-yl)-1H-pyrazol-3-yl)methyl)sulfonyl)benzoate (0.385 g, 0.96 mmol, 1 eq) was dissolved in ethanol (EtOH) : water (H₂O) (10 ml, 4:1 (v:v), 0.1 M), and then Fe (0.27 g, 4.78 mmol, 5 eq) and NH₄Cl (0.26 g, 4.78 mmol, 5 eq) were added thereto and stirred under reflux for one hour. After cooling to room temperature, a resulting product was filtered through celite, and water was added to a filtrate and extracted with EA. An organic layer was dried over MgSO₄, filtered, concentrated, and purified by column chromatography (silica gel, EA/HX). (0.085 g, 24%)

¹H NMR (400 MHz, MeOD) δ 8.55 (d, *J =* 4.9 Hz, 2H), 8.38 (s, 1H), 7.71 (d, *J =* 5.1 Hz, 2H), 7.51 (s, 1H), 7.46 (d, *J =* 8.2 Hz, 1H), 7.16 (d, *J =* 8.2 Hz, 1H), 6.53 (s, 1H), 4.68 (s, 2H), 3.90 (s, 3H).

### Step 7) Synthesis of methyl 3-iodo-4-(((1-(pyridin-4-yl)-1H-pyrazol-3-yl)methyl)sulfonyl)benzoate

After dissolving methyl 3-amino-4-(((1-(pyridin-4-yl)-1H-pyrazol-3-yl)methyl)sulfonyl)benzoate (0.159 g, 0.427 mmol, 1 eq) in ACN (4 ml, 0.1 M), pTSA (0.22 g, 1.28 mmol, 3 eq) was added thereto, and then sodium nitrite (NaNO₂, 0.03 g, 0.436 mmol, 1.02 eq) dissolved in 0.4 ml of water and KI (0.142 g, 0.854 mmol, 2 eq) dissolved in 0.8 ml of water were added thereto at 0°C. After reacting at room temperature for one hour, a saturated NaHCO₃ aqueous solution was added thereto, neutralized to pH 8 or higher, and then extracted with EA. An organic layer was dried over MgSO₄, filtered and concentrated to obtain a resulting product as a crude product. (0.2g, 100%)

¹H NMR (400 MHz, CDCl₃) δ 8.75 (s, 1H), 8.61 (d, *J =* 4.9 Hz, 2H), 8.00 (s, 2H), 7.92 (s, 1H), 7.43 (d, *J =* 4.9 Hz, 2H), 6.55 (s, 1H), 4.88 (s, 2H), 3.95 (s, 3H).

### Step 8) Synthesis of ethyl 3-((4-fluorophenyl)ethynyl)-4-(((1-(pyridin-4-yl)-1H-pyrazol-3-yl)methyl)sulfonyl)benzoate

Methyl 3-iodo-4-(((1-(pyridin-4-yl)-1H-pyrazol-3-yl)methyl)sulfonyl)benzoate (0.2 g, 0.414 mmol, 1 eq) was dissolved in ACN (4 ml, 0.1 M), and then TEA (0.13 ml, 0.92 mmol, 2.2 eq), PdCl₂(PPh₃)₂ (8.7 mg, 0.012 mmol, 3 mol %), and CuI (3.2 mg, 0.017 mmol, 4 mol%) were added thereto and stirred at room temperature for five minutes. 1-ethynyl-4-fluorobenzene (0.071 ml, 0.62 mmol, 1.5 eq) was added thereto and stirred at a temperature of 90°C for one hour. After cooling to room temperature, a resulting product was purified by column chromatography (silica gel, EA/HX). (0.104 g, 51.5 %)

¹H NMR (400 MHz, CDCl₃) δ 8.61 (s, 2H), 8.38 (s, 1H), 8.01 (s, 2H), 7.91 (s, 1H), 7.73 - 7.65 (m, 3H), 7.43 (s, 2H), 7.11 (t, *J =* 8.2 Hz, 2H), 6.55 (s, 1H), 4.89 (s, 2H), 3.98 (s, 3H).

### Step 9) Synthesis of 3-((4-fluorophenyl)ethynyl)-4-(((1-(pyridin-4-yl)-1H-pyrazol-3-yl)methyl)sulfonyl)benzoic acid

Ethyl 3-((4-fluorophenyl)ethynyl)-4-(((1-(pyridin-4-yl)-1H-pyrazol-3-yl)methyl)sulfonyl)benzoate (0.104 g, 0.212 mmol, 1 eq) was dissolved in EtOH (4 ml, 0.05 M), and then NaOH (0.042 g, 1.062 mmol, 5 eq) dissolved in H₂O (0.4 ml) was added thereto and stirred under reflux for one hour. After cooling to room temperature, a resulting product was concentrated and diluted with water. After adjusting to pH=5 to 6 with 1 N HCl, a resulting solid was filtered, washed with water, and dried. (0.039 g, 40%)

### Step 10) Synthesis of N-(3-(1H-pyrazol-4-yl)propyl)-3-((4-fluorophenyl)ethynyl)-4-(((1-(pyridin-4-yl)-1H-pyrazol-3-yl)methyl)sulfonyl)benzamide

3-((4-fluorophenyl)ethynyl)-4-(((1-(pyridin-4-yl)-1H-pyrazol-3-yl)methyl)sulfonyl)benzoic acid (0.039 g, 0.08 mmol, 1 eq) was dissolved in MC, and then TBTU (0.039 g, 0.12 mmol, 1.5 eq), TEA (0.025 ml, 0.176 mmol, 2.2 eq), and 3-(1H-pyrazol-4-yl)propan-1-amine dihydrochloride (0.0174 g, 0.088 mmol, 1.1 eq) were added thereto and stirred at room temperature overnight. After completion of a reaction, a saturated NaHCO₃ aqueous solution was added, neutralized and extracted with MC. An organic layer was dried over MgSO₄, filtered, concentrated, and purified by column chromatography (silica gel, MeOH/MC). (16.2 mg, 35.6 %)

¹H NMR (400 MHz, MeOD) δ 8.53 (d, *J =* 5.2 Hz, 2H), 8.35 (s, 1H), 8.21 (s, 1H), 7.98 (d, *J =* 8.3 Hz, 1H), 7.86 (d, *J =* 8.3 Hz, 1H), 7.77 - 7.70 (m, 2H), 7.64 (d, *J =* 5.3 Hz, 2H), 7.49 (s, 2H), 7.21 (t, *J =* 8.2 Hz, 2H), 6.57 (s, 1H), 4.99 (s, 2H), 3.45 (t, *J =* 7.2 Hz, 2H), 2.62 (t, *J =* 7.2 Hz, 2H), 1.93 (p, *J =* 6.8 Hz, 2H). MS(ESI) m/e MH⁺ 569.2

The reagents may be changed in substantially the same manner as the method of Example 11 to synthesize compounds 15, 16, 17 and 25.

### Example 12: Compound 245 (Synthesis of hydrochloride of compound 47)

### Synthesis of N-(3-(1H-pyrazol-4-yl)propyl)-4-(((1-methyl-1H-pyrazol-3-yl)methyl)sulfonyl)-3-((4-(piperidin-4-yl)phenyl)ethynyl)benzamide hydrochloride

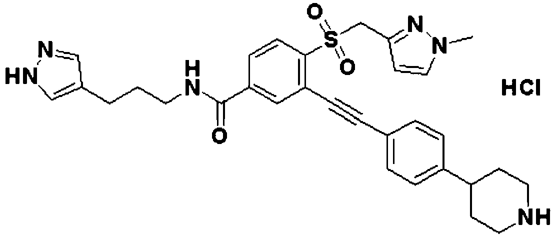

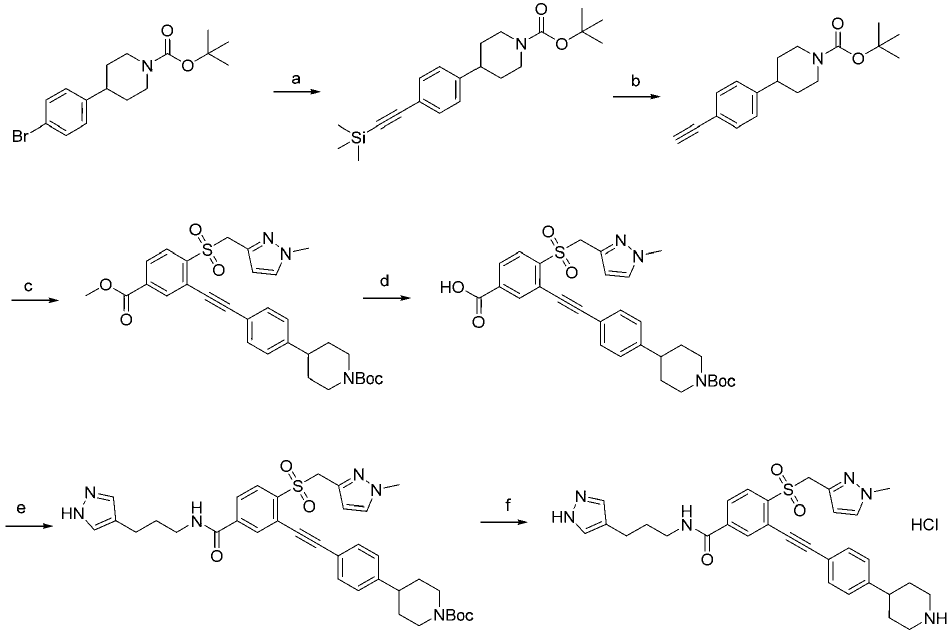

Reaction Formula 10. (a) trimethylsilylacetylene, PdCl₂[PPh₃]₂, CuI, TEA, Toluene, 100°C, 12 hours (12 h); (b) K₂CO₃, MeOH, room temperature (r.t). one hour (1 h); (c) PdCl₂[PPh₃]₂, CuI, TEA, ACN, reflux, 12 hours (12 h); (d) NaOH, H₂O, MeOH, 55°C; two hours (2 h); (e) (1H-pyrazol-4-yl)propan-1-amine dihydrochloride, TBTU, TEA, DCM, room temperature (rt), 12 hours (12 h); (f) 4 N HCl in dioxane, 1,4-dioxane, room temperature (r.t). 12 hours (12 h)

### Step 1) Synthesis of tert-butyl 4-(4-((trimethylsilyl)ethynyl)phenyl)piperidine-1-carboxylate

Tert-butyl 4-(4-bromophenyl)piperidine-1-carboxylate (0.34 g, 1 mmol, 1 eq) and trimethylsilylacetylene (0.24 ml, 1.5 mmol, 1.5 eq) were dissolved in toluene (0.1 M, 10 ml), and then PdCl₂[PPh₃]₂ (0.07 g, 0.1 mmol, 0.1 eq), CuI (0.019 g, 0.1 mmol, 0.1 eq) and triethylamine (0.6 ml, 4 mmol, 4 eq) were added thereto and was stirred at 100°C for 12 hours. A reaction mixture was extracted with DCM. An organic layer was dried over MgSO₄ and concentrated under reduced pressure to obtain a resulting residue, which was used in the next reaction without purification.

### Step 2) Synthesis of tert-butyl 4-(4-ethynylphenyl)piperidine-1-carboxylate

Tert-butyl 4-(4-((trimethylsilyl)ethynyl)phenyl)piperidine-1-carboxylate (0.31 g, 0.87 mmol, 1 eq) was dissolved in methanol (0.2 M), and then K₂CO₃ (0.6 g, 4.33 mmol, 5 eq) was added thereto and stirred at room temperature for one hour. After completion of a reaction, a resulting mixture was filtered, concentrated, and purified by column chromatography (silica gel, 10% EA/HX) to obtain a target compound (0.266 g).

### Step 3) Synthesis of tert-butyl 4-(4-((5-(methoxycarbonyl)-2-(((1-methyl-1H-pyrazol-3-yl)methyl)sulfonyl)phenyl)ethynyl)phenyl)piperidine-1-carboxylate

MeCN (2 ml, 0.1 M) was added to methyl 3-iodo-4-(((1-methyl-1H-pyrazol-3-yl)methyl)sulfonyl)benzoate (0.1 g, 0.24 mmol, 1 eq) and TEA (0.074 ml, 0.53 mmol, 2.2 eq) was added thereto. PdCl₂[PPh₃]₂ (5 mg, 3 mol%) and CuI (2 mg, 4 mol%) were added to a reaction product and stirred at room temperature for five minutes under a nitrogen atmosphere. Subsequently, tert-butyl 4-(4-ethynylphenyl)piperidine-1-carboxylate (0.1 g, 1.5 eq) was added. A reaction mixture was stirred at 90°C for 12 hours and concentrated under reduced pressure. A resulting residue was purified by column chromatography (silica gel, 40-50% EA/HX) to obtain a target compound (0.1 g).

¹H NMR (400 MHz, CDCl₃) δ 8.35 (d*, J =* 1.1 Hz, 1H), 7.97 (dd, *J* = 11.4, 4.8 Hz, 3H), 7.63 (d, *J =* 8.1 Hz, 2H), 7.20 (d, *J =* 2.0 Hz, 2H), 6.13 (d, *J* = 2.1 Hz, 1H), 4.81 (s, 2H), 4.26 (s, 2H), 3.98 (s, 3H), 3.76 (s, 3H), 2.81 (t, *J =* 12.2 Hz, 2H), 2.69 (t, *J =* 12.2 Hz, 1H), 1.83 (d, *J =* 13.0 Hz, 2H), 1.65 (d, *J =* 12.4 Hz, 2H), 1.49 (s, 9H).

### Step 4) Synthesis of 3-((4-(1-(tert-butoxycarbonyl)piperidin-4-yl)phenyl)ethynyl)-4-(((1-methyl-1H-pyrazol-3-yl)methyl)sulfonyl)benzoic acid

Ethanol (3 ml, 0.05 M), H₂O (0.5 ml), and NaOH (0.035 g, 0.87 mmol, 5 eq) were added to tert-butyl 4-(4-((5-(methoxycarbonyl)-2-(((1-methyl-1H-pyrazol-3-yl)methyl)sulfonyl)phenyl)ethynyl)phenyl)piperidine-1-carboxylate (0.1 g, 0.173 mmol, 1 eq) and stirred at 100°C for one hour. When a reaction was completed by confirming the reaction through TLC, the pH of a reaction product was adjusted to 1 with 1 N HCl aqueous solution and extracted with EA. An organic layer was washed with water, dried over magnesium sulfate, and concentrated under reduced pressure to obtain a target compound (0.082 g, 84%).

### Step 5) Synthesis of tert-butyl 4-(4-((5-((3-(1H-pyrazol-4-yl)propyl)carbamoyl)-2-(((1-methyl-1H-pyrazol-3-yl)methyl)sulfonyl)phenyl)ethynyl)phenyl)piperidine-1-carboxylate

DCM (2 ml), TBTU (0.071 g, 0.22 mmol, 1.5 eq), TEA (0.045 ml, 0.32 mmol, 2.2 eq), and 3-(1H-pyrazol-4-yl)propan-1-amine dihydrochloride (32 mg, 0.16 mmol, 1.1 eq) were added to 3-((4-(1-(tert-butoxycarbonyl)piperidin-4-yl)phenyl)ethynyl)-4-(((1-methyl-1H-pyrazol-3-yl)methyl)sulfonyl)benzoic acid (0.082 g, 0.145 mmol, 1 eq) and stirred for two hours. Water was added to a reaction product and extracted with EA. An organic layer was dried over magnesium sulfate and concentrated under reduced pressure, and a resulting residue was purified by column chromatography (silica gel, 100% EA) to obtain a target compound (0.02 g, 21%).

### Step 6) Synthesis of N-(3-(1H-pyrazol-4-yl)propyl)-4-(((1-methyl-1H-pyrazol-3-yl)methyl)sulfonyl)-3-((4-(piperidin-4-yl)phenyl)ethynyl)benzamide hydrochloride

Tert-butyl 4-(4-((5-((3-(1H-pyrazol-4-yl)propyl)carbamoyl)-2-(((1-methyl-1H-pyrazol-3-yl)methyl)sulfonyl)phenyl)ethynyl)phenyl)piperidine-1-carboxylate (0.02 g, 0.03 mmol, 1 eq) was dissolved in 1,4-dioxane (1 ml, 0.03 M), and then 4 N HCl in 1,4-dioxane (0.015 ml, 0.06 mmol, 2 eq) was added thereto and stirred at room temperature for 12 hours. After completion of a reaction, a resulting solid was filtered and washed with 1,4-dioxane to obtain a target compound (0.0066g, 39%).

¹H NMR (400 MHz, MeOD) δ 8.20 (s, 3H), 7.96 - 7.86 (m, 3H), 7.70 (d, *J* = 8.0 Hz, 2H), 7.46 (d, *J =* 1.9 Hz, 1H), 7.42 (d, *J =* 8.1 Hz, 2H), 6.18 (d, *J =* 2.0 Hz, 1H), 4.86 (s, 2H), 3.74 (s, 3H), 3.55 (d, *J =* 12.5 Hz, 2H), 3.51 - 3.44 (m, 3H), 3.19 (t, *J =* 12.6 Hz, 2H), 3.09 - 2.98 (m, 2H), 2.73 (dd, *J =* 14.4, 7.3 Hz, 3H), 2.14 (d, *J =* 14.3 Hz, 2H), 2.02 - 1.97 (m, 3H).

The reagents may be changed in substantially the same manner as in Example 12 to synthesize compounds 83, 84, 85, 86, 87, 88, 89, 90, 91, 105, and 249.

### Example 13: Synthesis of 4-ethynyl-N-(2-(piperidin-1-yl)ethyl)benzamide

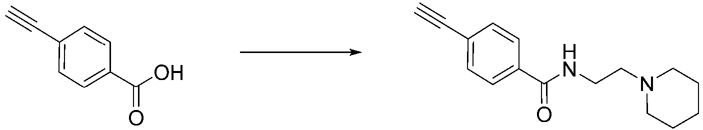

DMF (20 ml), HATU (07.8 g, 20.5 mmol, 1.5 eq), DIPEA (7 ml, 41.1 mmol, 3), and 2-(piperidin-1-yl)ethan-1-amine (2.7 ml, 15.4 mmol, 1.1 eq) were added to 4-ethynylbenzoic acid (2.0 g, 13.7 mmol, 1 eq) and stirred for 12 hours. Water was added to a reaction product, and an organic layer was extracted three times with EA, washed with a NH₄Cl aqueous solution, dried over magnesium sulfate, and then concentrated under reduced pressure to obtain a resulting residue, which was purified by column chromatography (silica gel, 10% MeOH/DCM) to obtain a target compound.

¹H NMR (400 MHz, CDCl₃) δ 7.81 (d, *J =* 7.9 Hz, 2H), 7.58 (d, *J =* 7.8 Hz, 2H), 7.39 (s, 1H), 3.68 (m, 2H), 3.23 (s, 1H), 2.89-2.99 (m, 6H), 1.80 (m, 4H), 1.62 (s, 2H).

### Example 14: Synthesis of compound 115

### Synthesis of 3-((4-fluorophenyl)ethynyl)-N-(imidazo[1,2-a]pyridin-7-ylmethyl)-4-(N-((1-methyl-1H-pyrazol-3-yl)methyl)sulfamoyl)benzamide

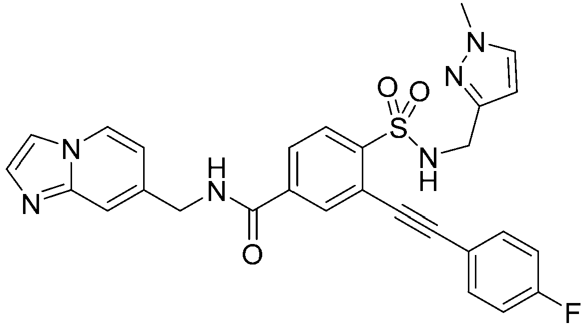

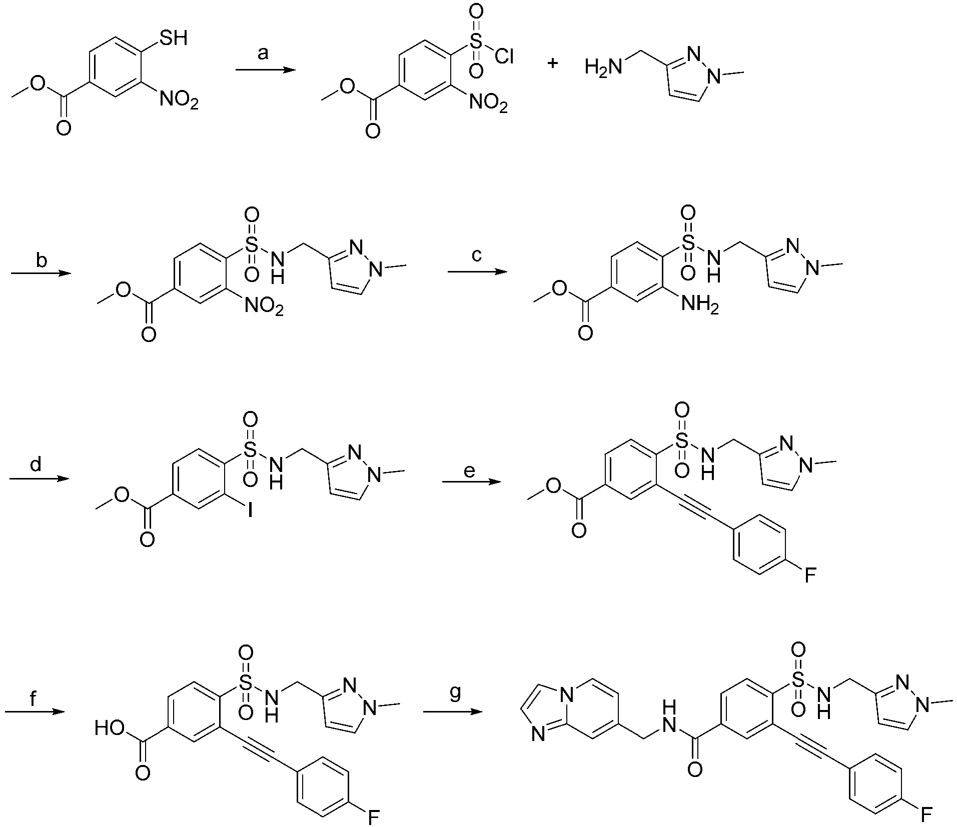

Reagents and conditions: (a) NCS, 2 N HCl (aq), ACN, 0°C to room temperature (0°C-rt), overnight; (b) ACN, four hours (4 h), room temperature (rt); (c) Zn, NH₄Cl, 1,4-dioxane, H₂O, room temperature (rt), overnight; (d) NaNO₂, KI, HCl, H₂O, 0°C to room temperature(0°C-rt), two hours (2 h); (e) PdCl₂(PPh₃)₂, CuI, TEA, ACN, 60°C, four hours (4 h); (f) 1N NaOH, EtOH, 60°C, overnight; (g) 1-{imidazo[1,2-a]pyridin-7-yl}methanamine dihydrochloride, TBTU, TEA, DCM, room temperature (rt), overnight.

### Step 1) Synthesis of methyl 4-(chlorosulfonyl)-3-nitrobenzoate

NCS (3.131 g, 23.45 mmol, 5 eq) and a 2 N HCl aqueous solution (4.69 ml, 9.38 mmol, 2 eq) were added to ACN (46 ml, 0.1 M) and stirred at 0°C for 30 minutes. Methyl 4-mercapto-3-nitrobenzoate (1 g, 4.69 mmol, 1 eq) was added thereto and stirred at room temperature overnight. After completion of a reaction, a resulting mixture was concentrated, and a resulting concentrate was diluted with EA, and then sequentially extracted with a NaHCO₃ aqueous solution and a NaCl aqueous solution. An organic layer was dried over MgSO₄, filtered and concentrated to obtain a target compound as a crude product.

### Step 2) Synthesis of methyl 4-(N-((1-methyl-1H-pyrazol-3-yl)methyl)sulfamoyl)-3-nitrobenzoate

(1-methyl-1H-pyrazol-3-yl)methanamine (1.042 g, 9.38 mmol, 2 eq) was dissolved in ACN (20 ml, 0.23 M), and methyl 4-(chlorosulfonyl)-3-nitrobenzoate (1.311 g, 4.69 mmol, 1 eq) dissolved in ACN (26 ml, 0.18 M) was added dropwise to a reaction solution. After completion of a reaction, a resulting mixture was extracted with EA and water. An organic layer was dried over MgSO₄, filtered, concentrated, and purified by column chromatography (silica gel, EA/HEX) to obtain a target compound (0.3 g, 18.4%).

### Step 3) Synthesis of methyl 3-amino-4-(N-((1-methyl-1H-pyrazol-3-yl)methyl)sulfamoyl)benzoate

Methyl 4-(N-((1-methyl-1H-pyrazol-3-yl)methyl)sulfamoyl)-3-nitrobenzoate (0.296 g, 0.835 mmol, 1 eq) was dissolved in 1,4-dioxane : H₂O (3:1 (v/v)) (8.3 ml, 0.1 M), and then Zn (0.546 g, 8.35 mmol, 10 eq) and NH₄Cl (0.447 g, 8.35 mmol, 10 eq) were added thereto and stirred at room temperature overnight. A resulting mixture was washed with MeOH, filtered under reduced pressure, and concentrated to obtain a target compound as a crude product.

### Step 4) Synthesis of methyl 3-iodo-4-(N-((1-methyl-1H-pyrazol-3-yl)methyl)sulfamoyl)benzoate

Methyl 3-amino-4-(N-((1-methyl-1H-pyrazol-3-yl)methyl)sulfamoyl)benzoate (0.255 g, 0.79 mmol, 1 eq) and HCl (7 ml, 0.1 M) were dissolved in H₂O (7 ml, 0.1 M) and stirred at 0°C. NaNO₂ (0.082 g, 1.185 mmol, 1.5 eq) dissolved in H₂O (7 ml, 0.1 M) was added dropwise thereto. A reaction solution was stirred at 0°C for 30 minutes, and then KI (0.393 g, 2.37 mmol, 3 eq) dissolved in H₂O (7 ml, 0.1 M) was added dropwise thereto. A reaction solution was stirred at room temperature for two hours, neutralized to pH 8 or higher with a NaHCO₃ aqueous solution, and then extracted with EA and water. An organic layer was dried over MgSO₄, filtered, concentrated, and purified by column chromatography (silica gel, EA/HEX) to obtain a target compound (0.167 g, 48.6%).

### Step 5) Synthesis of methyl 3-((4-fluorophenyl)ethynyl)-4-(N-((1-methyl-1H-pyrazol-3-yl)methyl)sulfamoyl)benzoate

Methyl 3-iodo-4-(N-((1-methyl-1H-pyrazol-3-yl)methyl)sulfamoyl)benzoate (0.167 g, 0.38 mmol, 1 eq) was dissolved in ACN (7.6 ml, 0.05 M), and then 1-ethynyl-4-fluorobenzene (0.068 g, 0.57 mmol, 1.5 eq), PdCl₂(PPh₃)₂ (0.008 g, 0.01 mmol, 0.03 eq), CuI (0.002 g, 0.01 mmol, 0.03 eq), and TEA (0.077 g, 0.76 mmol, 2 eq) were added and degassed with nitrogen (N₂) gas. A reaction solution was heated to 60°C and stirred for four hours. After completion of a reaction, a resulting mixture was filtered through celite and extracted with EA. An organic layer was washed with water, separated, dried over MgSO₄, filtered and concentrated to obtain a target compound as a crude product.

### Step 6) Synthesis of 3-((4-fluorophenyl)ethynyl)-4-(N-((1-methyl-1H-pyrazol-3-yl)methyl)sulfamoyl)benzoic acid

EtOH (8 ml, 0.05 M), H₂O (4.38 ml, 0.1 M), and NaOH (0.175 g, 4.38 mmol, 10 eq) were added to methyl 3-((4-fluorophenyl)ethynyl)-4-(N-((1-methyl-1H-pyrazol-3-yl)methyl)sulfamoyl)benzoate (0.187 g, 0.438 mmol, 1 eq) and stirred at 60°C overnight. After completion of a reaction, the pH of a reaction product was adjusted to 1 with 1 N HCl aqueous solution and extracted with EA. An organic layer was washed with water, separated, dried over MgSO₄, filtered and concentrated to obtain a target compound as a crude product.

### Step 7) Synthesis of 3-((4-fluorophenyl)ethynyl)-N-(imidazo[1,2-a]pyridin-7-ylmethyl)-4-(N-((1-methyl-1H-pyrazol-3-yl)methyl)sulfamoyl)benzamide

DCM (3.2 ml, 0.1 M) and TBTU (0.154 g, 0.48 mmol, 1.5 eq) were added to 3-((4-fluorophenyl)ethynyl)-4-(N-((1-methyl-1H-pyrazol-3-yl)methyl)sulfamoyl)benzoic acid (0.131 g, 0.32 mmol, 1 eq) and stirred at room temperature for 30 minutes. Subsequently, TEA (0.070 g, 0.7 mmol, 2.2 eq) and 1-{imidazo[1,2-a]pyridin-7-yl}methanamine dihydrochloride (0.077 g, 0.352 mmol, 1.1 eq) were added to a reaction product and stirred for 12 hours. Water was added to a reaction product and extracted with DCM. An organic layer was dried over MgSO₄, filtered, concentrated and then purified by column chromatography (silica gel, MeOH/DCM) to obtain a title compound (0.03 g, 17.3%).

¹H NMR (400 MHz, DMSO) δ 9.36 (s, 1H), 8.54 (s, 1H), 8.51 (d, J = 6.9 Hz, 1H), 8.12 (dd, J = 19.3, 8.1 Hz, 2H), 7.90 (s, 1H), 7.55 (dd, J = 8.3, 5.7 Hz, 3H), 7.47 (s, 1H), 7.42 (d, J = 1.8 Hz, 1H), 7.24 (t, J = 8.8 Hz, 2H), 7.10 (s, 1H), 6.89 (d, J = 6.2 Hz, 1H), 5.81 (d, J = 1.9 Hz, 1H), 4.57 (s, 2H), 3.62 (s, 3H); MS MH⁺ 543.6

### Example 15: Synthesis of compound 193

### Synthesis of N-(imidazo[1,2-a]pyridin-7-ylmethyl)-4-(((1-methyl-1H-pyrazol-3-yl)methyl)sulfonyl)-3-((4-((2-(piperazin-1-yl)ethyl)carbamoyl)phenyl)ethynyl)benzamide

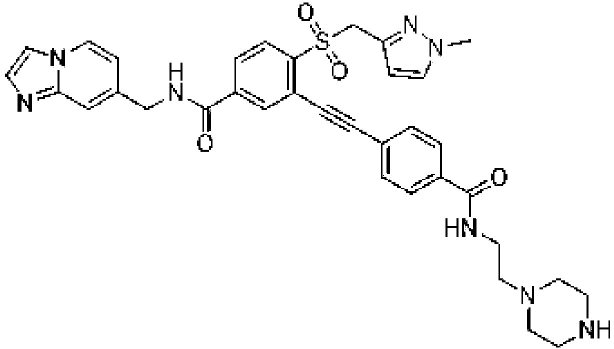

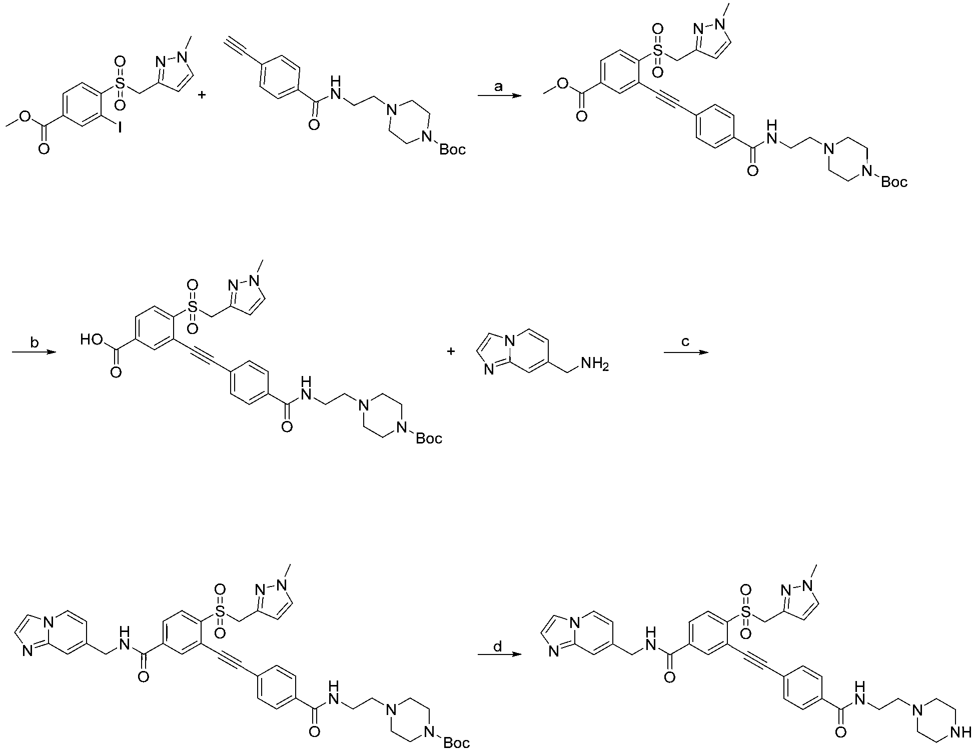

Reagents and conditions: (a) PdCl₂(PPh₃)₂, CuI, TEA, ACN, 60°C, one hour (1 h); (b) 1 N NaOH, EtOH, 60°C, one hour (1 h); (c) TBTU, TEA, DCM, room temperature (rt), overnight; (d) TFA, DCM, room temperature (rt), overnight

### Step 1) Synthesis of tert-butyl 4-(2-(4-((5-(methoxycarbonyl)-2-(((1-methyl-1H-pyrazol-3-yl)methyl)sulfonyl)phenyl)ethynyl)benzamido)ethyl)piperazine-1-carboxylate

Methyl 3-iodo-4-(((1-methyl-1H-pyrazol-3-yl)methyl)sulfonyl)benzoate (0.26 g, 0.52 mmol, 1 eq) was dissolved in ACN (3 ml, 0.2 M), and then tert-butyl 4-(2-(4-ethynylbenzamido)ethyl)piperazine-1-carboxylate (0.336 g, 0.84 mmol, 1.5 eq), PdCl₂(PPh₃)₂ (0.013 g, 0.0186 mmol, 0.03 eq), CuI (0.0035 g, 0.0186 mmol, 0.03 eq), and TEA (0.125 g, 1.24 mmol, 2 eq) were added and degassed with nitrogen (N₂) gas. A reaction solution was heated to 60°C and stirred for one hour. After completion of a reaction, a resulting mixture was filtered through celite and extracted with EA. An organic layer was washed with water, separated, dried over MgSO₄, filtered and concentrated to obtain a target compound as a crude product.

### Step 2) Synthesis of 3-((4-((2-(4-(tert-butoxycarbonyl)piperazin-1-yl)ethyl)carbamoyl)phenyl)ethynyl)-4-(((1-methyl-1H-pyrazol-3-yl)methyl)sulfonyl)benzoic acid

EtOH (12 ml, 0.05 M), H₂O (3.1 ml, 0.2 M) and NaOH (0.120 g, 3.1 mmol, 5 eq) were added to tert-butyl 4-(2-(4-((5-(methoxycarbonyl)-2-(((1-methyl-1H-pyrazol-3-yl)methyl)sulfonyl)phenyl)ethynyl)benzamido)ethyl)piperazine-1-carboxylate (0.403 g, 0.62 mmol, 1 eq) and stirred at 60°C for one hour. When a reaction was completed by confirming the reaction through TLC, the pH of a reaction product was adjusted to 1 with 1 N HCl aqueous solution and extracted with EA. An organic layer was washed with water, separated, dried over MgSO₄, filtered and concentrated to obtain a target compound as a crude product.

### Step 3) Synthesis of tert-butyl 4-(2-(4-((5-((imidazo[1,2-a]pyridin-7-ylmethyl)carbamoyl)-2-(((1-methyl-1H-pyrazol-3-yl)methyl)sulfonyl)phenyl)ethynyl)benzamido)ethyl)piperazine-1-carboxylate

DCM (3 ml, 0.2 M) and TBTU (0.298 g, 0.93 mmol, 1.5 eq) were added to 3-((4-((2-(4-(tert-butoxycarbonyl)piperazin-1-yl)ethyl)carbamoyl)phenyl)ethynyl)-4-(((1-methyl-1H-pyrazol-3-yl)methyl)sulfonyl)benzoic acid (0.394 g, 0.62 mmol, 1 eq) and stirred at room temperature for 50 minutes. Subsequently, TEA(0.137 g, 1.36 mmol, 2.2 eq) and 1-{imidazo[1,2-a]pyridin-7-yl}methanamine dihydrochloride (0.150 g, 0.682 mmol, 1.1 eq) were added to a reaction product and stirred for 12 hours. Water was added to a reaction product and extracted with DCM. An organic layer was dried over MgSO₄, filtered, concentrated and then purified by column chromatography (silica gel, MeOH/DCM) to obtain a title compound (0.27 g, 56.4%).

### Step 4) Synthesis of N-(imidazo[1,2-a]pyridin-7-ylmethyl)-4-(((1-methyl-1H-pyrazol-3-yl)methyl)sulfonyl)-3-((4-((2-(piperazin-1-yl)ethyl)carbamoyl)phenyl)ethynyl)benzamide

Tert-butyl 4-(2-(4-((5-((imidazo[1,2-a]pyridin-7-ylmethyl)carbamoyl)-2-(((1-methyl-1H-pyrazol-3-yl)methyl)sulfonyl)phenyl)ethynyl)benzamido)ethyl)piperazine-1-carboxylate (0.150 g, 0.196 mmol, 1 eq) was dissolved in DCM (2 ml, 0.1 M) and TFA (0.223 g, 1.96 mmol, 10 eq) was added thereto and stirred at room temperature overnight. After completion of a reaction, a resulting mixture was concentrated and purified by reverse phase column chromatography (0.1% formic acid in H₂O/ACN) to obtain a title compound (0.067 g, 51.5%).

¹H NMR (400 MHz, MeOD) δ 8.51 (s, 1H), 8.28 (s, 1H), 8.23 (s, 1H), 7.96 (s, 1H), 7.91 (d, J = 5.1 Hz, 3H), 7.78 (d, J = 7.5 Hz, 2H), 7.66 (s, 1H), 7.59 (s, 1H), 7.44 (s, 1H), 7.10 (d, J = 6.7 Hz, 1H), 6.17 (s, 1H), 4.85 - 4.83 (m, 2H), 4.70 (s, 2H), 3.72 (s, 3H), 3.57 (s, 3H), 3.50 (s, 1H), 3.22 (s, 2H), 2.79 (s, 2H), 2.75 - 2.57 (m, 4H); MS MH⁺ 665.58

The reagents may be changed in substantially the same manner as in Example 15 to synthesize compounds 119, 131, 133, 134 and 136.

### Example 16: Synthesis of compound 228

### Synthesis of 3-((4-aminophenyl)ethynyl)-N-(imidazo[1,2-a]pyridin-7-ylmethyl)-4-(((1-methyl-1H-pyrazol-3-yl)methyl)sulfonyl)benzamide

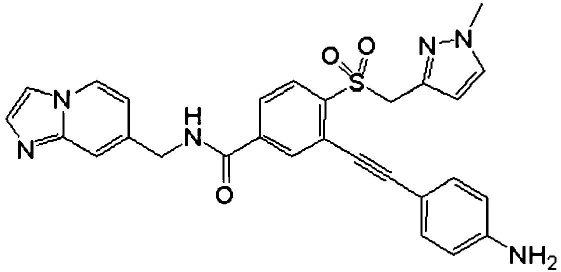

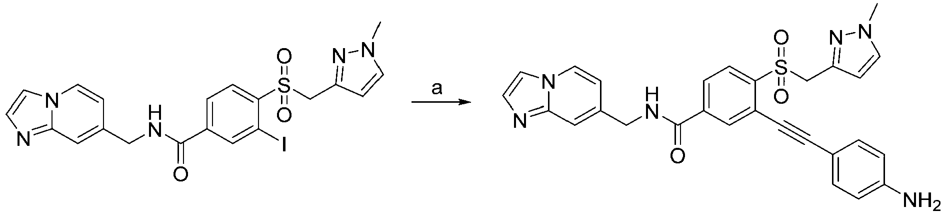

Reagents and conditions; 4-ethynylaniline, TEA, CuI, PdCl₂(PPh₃)₂, ACN, reflux, one hour (1 h);
N-(imidazo[1,2-a]pyridin-7-ylmethyl)-3-iodo-4-(((1-methyl-1H-pyrazol-3-yl)methyl)sulfonyl)benzamide (0.535 g, 1 mmol, 1 eq) was dissolved in ACN (10 ml, 0.1 M), and then TEA (0.3 ml, 2.2 mmol, 2.2 eq), PdCl₂(PPh₃)₂ (21 mg, 0.03 mmol, 3 mol %), and CuI (8 mg, 0.04 mmol, 4 mol%) were added thereto and stirred at room temperature for five minutes. 4-ethynylaniline (0.14 g, 1.2 mmol, 1.5 eq) was added and stirred at 90°C for one hour. After cooling to room temperature, a resulting product was concentrated and purified by column chromatography (silica gel, EA/HX). (0.193 g, 37%)

¹H NMR (400 MHz, CD₃OD) δ 8.41 (d, *J* = 7.0 Hz, 1H), 8.16 - 8.12 (m, 1H), 7.87 (d, *J* = 8.3 Hz, 1H), 7.84 - 7.79 (m, 2H), 7.53 (s, 1H), 7.48 (s, 1H), 7.44 - 7.37 (m, 3H), 6.95 (dd, *J* = 7.1, 1.5 Hz, 1H), 6.69 (d, *J* = 8.7 Hz, 2H), 6.12 (d, *J= 2.2* Hz, 1H), 4.85 (s, 2H), 4.64 (s, 2H), 3.72 (s, 3H).

The reagents may be changed in substantially the same manner as in Example 16 to synthesize compound 105.

### Example 17: Synthesis of compound 229

### Synthesis of 3-((3-aminophenyl)ethynyl)-N-(imidazo[1,2-a]pyridin-7-ylmethyl)-4-(((1-methyl-1H-pyrazol-3-yl)methyl)sulfonyl)benzamide

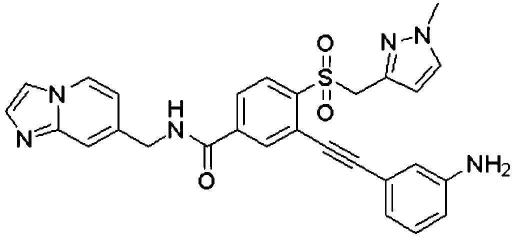

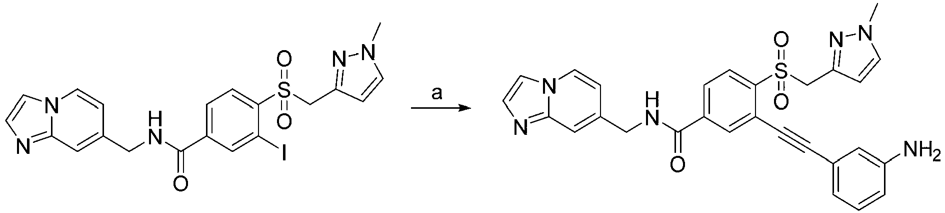

Reagents and conditions; (a) 3-ethylnylaniline, PdCl₂(PPh₃)₂, CuI, TEA, ACN, 60°C, four hours (4 h)

N-(imidazo[1,2-a]pyridin-7-ylmethyl)-3-iodo-4-(((1-methyl-1H-pyrazol-3-yl)methyl)sulfonyl)benzamide (0.050 g, 0.093 mmol, 1 eq) was dissolved in ACN (1 ml, 0.1 M), and then 3-ethylnylaniline (0.016 g, 0.139 mmol, 1.5 eq), PdCl₂(PPh₃)₂ (0.006 g, 0.01 mmol, 0.1 eq), CuI (0.002 g, 0.01 mmol, 0.1 eq), and TEA (0.037 g, 0.37 mmol, 4 eq) were added and degassed with nitrogen (N₂) gas. A reaction solution was heated to 60°C and stirred for four hours. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a title compound (0.002 g, 4%).

¹H NMR (400 MHz, MeOD) δ 8.43 (d, J = 7.0 Hz, 1H), 8.23 (s, 1H), 7.92 (d, J = 0.7 Hz, 2H), 7.82 (s, 1H), 7.56 (s, 1H), 7.51 (s, 1H), 7.44 (d, J = 2.2 Hz, 1H), 7.17 (t, J = 7.8 Hz, 1H), 7.02 (d, J = 1.8 Hz, 1H), 6.98 (dd, J = 11.8, 4.6 Hz, 2H), 6.81 (dd, J = 8.1, 1.5 Hz, 1H), 6.15 (d, J = 2.2 Hz, 1H), 4.67 (s, 2H), 3.74 (s, 3H).

The reagents may be changed in substantially the same manner as in Example 17 to synthesize compounds 102, 103, 104, 106, 107, 108, 109, 110, 153, 154, 155, 156, 193, 224 and 226.

### Example 18: Synthesis of compound 230

### Synthesis of N-(imidazo[1,2-a]pyridin-7-ylmethyl)-4-(((1-methyl-1H-pyrazol-3-yl)methyl)sulfonyl)-3-((3-(piperazine-1-carbonyl)phenyl)ethynyl)benzamide

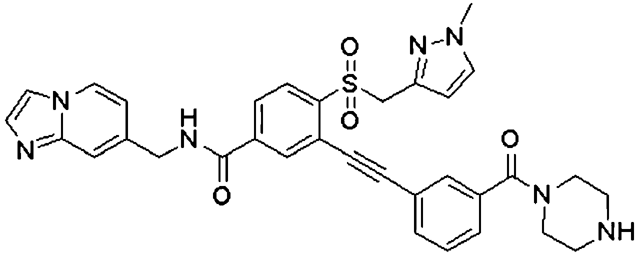

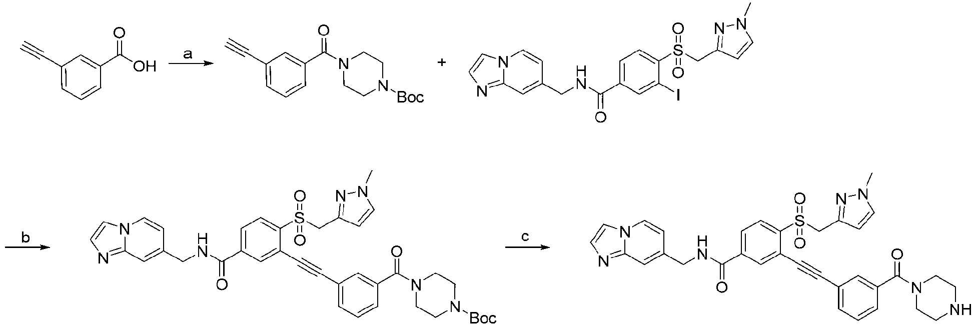

Reagents and conditions; (a) N-Boc-piperazine, EDC*HCl, HOBt, TEA, DCM, room temperature (rt), overnight; (b) PdCl₂(PPh₃)₂, CuI, TEA, ACN, reflux, one hour (1 h); (c) TFA, DCM, room temperature (rt), three hours (3 h).

### Step 1) Synthesis of tert-butyl 4-(3-ethynylbenzoyl)piperazine-1-carboxylate

3-ethynylbenzoic acid (0.2 g, 1.37 mmol, 1 eq), EDC*HCl (0.393 g, 2.05 mmol, 1.5 eq), and HOBt (0.296 g, 2.192 mmol, 1.6 eq) were added to DCM (13 ml, 0.1 M) and stirred at room temperature for 15 minutes. N-Boc-piperazine (0.331 g, 1.781 mmol, 1.3 eq) and TEA (0.416 g, 4.11 mmol, 3 eq) were added and stirred at room temperature overnight. After completion of a reaction, a reaction solution was concentrated under reduced pressure and purified by column chromatography (silica gel, EA/HEX) to obtain a target compound (0.25 g, 58%).

### Step 2) Synthesis of tert-butyl 4-(3-((5-((imidazo[1,2-a]pyridin-7-ylmethyl)carbamoyl)-2-(((1-methyl-1H-pyrazol-3-yl)methyl)sulfonyl)phenyl)ethynyl)benzoyl)piperazine-1-carboxylate

N-(imidazo[1,2-a]pyridin-7-ylmethyl)-3-iodo-4-(((1-methyl-1H-pyrazol-3-yl)methyl)sulfonyl)benzamide (0.1 g, 0.19 mmol, 1 eq) was dissolved in ACN (1.9 ml, 0.1 M), and then tert-butyl 4-(3-ethynylbenzoyl)piperazine-1-carboxylate (0.089 g, 0.285 mmol, 1.5 eq), PdCl₂(PPh₃)₂ (0.013 g, 0.02 mmol, 0.1 eq), CuI (0.004 g, 0.02 mmol, 0.1 eq), and TEA (0.077 g, 0.76 mmol, 4 eq) were added and degassed with nitrogen (N₂) gas. A reaction solution was stirred under reflux for one hour. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.052 g, 37.9%).

### Step 3) Synthesis of N-(imidazo[1,2-a]pyridin-7-ylmethyl)-4-(((1-methyl-1H-pyrazol-3-yl)methyl)sulfonyl)-3-((3-(piperazin-1-carbonyl)phenyl)ethynyl)benzamide

Tert-butyl 4-(3-((5-((imidazo[1,2-a]pyridin-7-ylmethyl)carbamoyl)-2-(((1-methyl-1H-pyrazol-3-yl)methyl)sulfonyl)phenyl)ethynyl)benzoyl)piperazine-1-carboxylate (0.050 g, 0.069 mmol, 1 eq) was dissolved in DCM (1 ml, 0.07 M) and TFA (0.079 g, 0.69 mmol, 10 eq) was added thereto and stirred at room temperature overnight. After completion of a reaction, a resulting mixture was concentrated and purified by reverse phase column chromatography (0.1% formic acid in H₂O/ACN) to obtain a title compound (0.013 g, 32.5%).

¹H NMR (400 MHz, DMSO) δ 9.45 (d, J = 5.5 Hz, 1H), 8.51 (d, J = 6.6 Hz, 1H), 8.34 (s, 1H), 8.16 (s, 1H), 8.06 (d, J = 8.2 Hz, 1H), 7.97 - 7.88 (m, 2H), 7.79 (d, J = 7.5 Hz, 1H), 7.75 (s, 1H), 7.60 (t, J = 7.4 Hz, 2H), 7.55 (d, J = 7.2 Hz, 2H), 7.46 (s, 1H), 6.89 (d, J = 6.9 Hz, 1H), 6.09 (s, 1H), 4.88 (s, 2H), 4.54 (d, J = 5.3 Hz, 2H), 3.70 (s, 3H), 3.43 (s, 4H), 2.96 (s, 4H).

### Example 19: Synthesis of compound 231

### Synthesis of N-(imidazo[1,2-a]pyridin-7-ylmethyl)-4-(((1-methyl-1H-pyrazol-3-yl)methyl)sulfonyl)-3-((1,2,3,4-tetrahydroisoquinolin-6-yl)ethynyl)benzamide

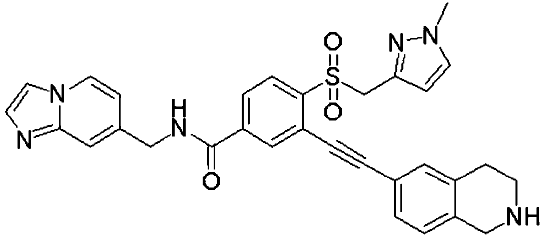

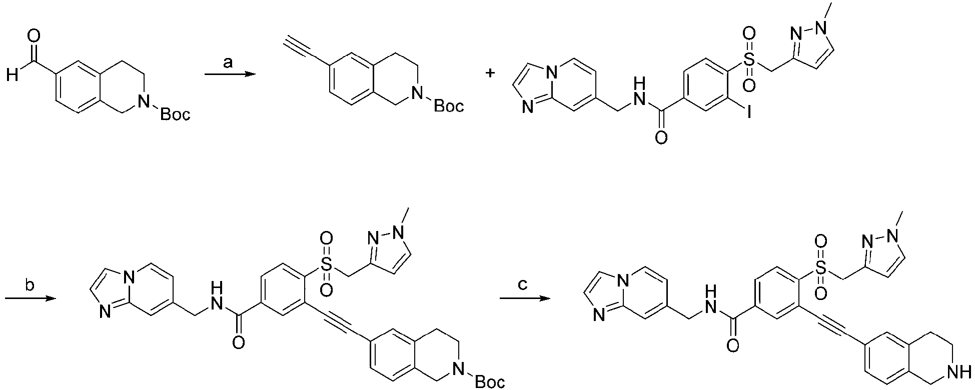

Reagents and conditions; (a) dimethyl(1-diazo-2-oxopropyl)phosphate, K₂CO₃, MeOH, room temperature (rt), overnight; (b) PdCl₂(PPh₃)₂, CuI, TEA, ACN, reflux, one hour (1 h); (c) TFA, 4 N HCl in 1,4-dioxane, DCM, room temperature (rt), overnight.

### Step 1) Synthesis of tert-butyl 6-ethynyl-3,4-dihydroisoquinoline-2(1H)-carboxylate

Tert-butyl 6-formyl-3,4-dihydroisoquinoline-2(1H)-carboxylate (0.25 g, 0.96 mmol, 1 eq) was diluted in MeOH (4 ml, 0.24 M), and dimethyl(1-diazo-2-oxopropyl)phosphate (0.221 g, 1.152 mmol, 1.2 eq), and K₂CO₃ (0.265 g, 1.92 mmol, 2 eq) were added thereto and stirred at room temperature overnight. After completion of a reaction, a resulting mixture was extracted with DCM and water. An organic layer was washed with NaCl aqueous solution, separated, dried over MgSO₄, filtered and concentrated to obtain a target compound as a crude product.

### Step 2) Synthesis of tert-butyl 6-((5-((imidazo[1,2-a]pyridin-7-ylmethyl)carbamoyl)-2-(((1-methyl-1H-pyrazol-3-yl)methyl)sulfonyl)phenyl)ethynyl)-3,4-dihydroisoquinoline-2(1H)-carboxylate

N-(imidazo[1,2-a]pyridin-7-ylmethyl)-3-iodo-4-(((1-methyl-1H-pyrazol-3-yl)methyl)sulfonyl)benzamide (0.1 g, 0.19 mmol, 1 eq) was dissolved in ACN (1.9 ml, 0.1 M), and then tert-butyl 6-ethynyl-3,4-dihydroisoquinoline-2(1H)-carboxylate (0.072 g, 0.281 mmol, 1.5 eq), PdCl₂(PPh₃)₂ (0.013 g, 0.02 mmol, 0.1 eq), CuI (0.004 g, 0.02 mmol, 0.1 eq), and TEA (0.076 g, 0.75 mmol, 4 eq) were added and degassed with nitrogen (N₂) gas. A reaction solution was stirred under reflux for one hour. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.046 g, 36.9%).

### Step 3) Synthesis of N-(imidazo[1,2-a]pyridin-7-ylmethyl)-4-(((1-methyl-1H-pyrazol-3-yl)methyl)sulfonyl)-3-((1,2,3,4-tetrahydroisoquinolin-6-yl)ethynyl)benzamide

Tert-butyl 6-((5-((imidazo[1,2-a]pyridin-7-ylmethyl)carbamoyl)-2-(((1-methyl-1H-pyrazol-3-yl)methyl)sulfonyl)phenyl)ethynyl)-3,4-dihydroisoquinoline-2(1H)-carboxylate (0.046 g, 0.069 mmol, 1 eq) was dissolved in DCM (1 ml, 0.07 M) and TFA (0.079 g, 0.69 mmol, 10 eq) was added thereto and stirred at room temperature overnight. A reaction product was concentrated, and then 4 N HCl in 1,4-dioxane (0.34 ml, 1.38 mmol, 20 eq) was added thereto and stirred at room temperature for three hours. After completion of a reaction, a reaction product was concentrated and purified by reverse phase column chromatography (0.1% formic acid in H₂O/ACN) to obtain a title compound (0.013 g, 33%).

¹H NMR (400 MHz, DMSO) δ 9.47 (s, 1H), 8.67 (s, 1H), 8.32 (s, 1H), 8.05 (d, J = 8.0 Hz, 1H), 7.93 (d, J = 8.0 Hz, 1H), 7.61 (s, 1H), 7.52 (d, J = 13.1 Hz, 3H), 7.28 (s, 1H), 6.90 (d, J = 6.3 Hz, 1H), 6.10 (s, 1H), 4.90 (s, 2H), 4.53 (s, 2H), 3.90 (s, 3H), 3.72 (s, 3H), 2.92 (s, 3H).

### Example 20: Synthesis of compound 232

### Synthesis of 3-((3-(aminomethyl)phenyl)ethynyl)-N-(imidazo[1,2-a]pyridin-7-ylmethyl)-4-(((1-methyl-1H-pyrazol-3-yl)methyl)sulfonyl)benzamide

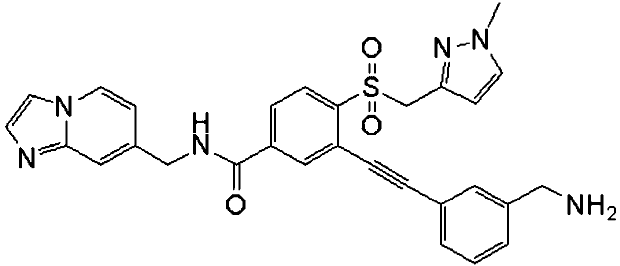

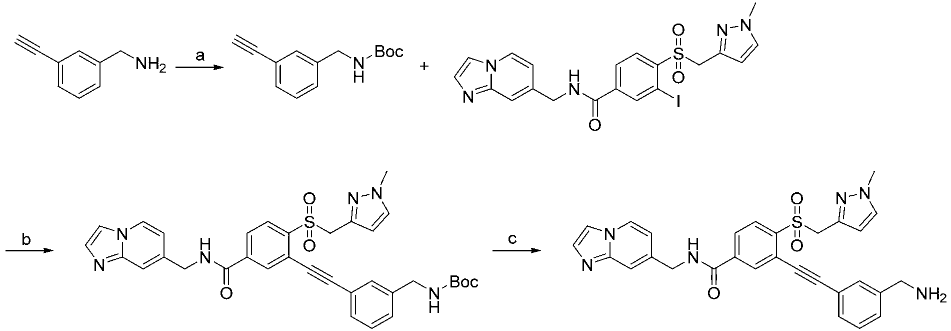

Reagents and conditions; (a) Boc₂O, DCM, 0°C to room temperature(0°C-rt), overnight; (b) PdCl₂(PPh₃)₂, CuI, TEA, ACN, reflux, four hours (4 h); (c) 4 N HCl in dioxane, room temperature (rt), overnight

### Step 1) Synthesis of tert-butyl (3-ethynylbenzyl)carbamate

(3-ethynylphenyl)methanamine (0.1 g, 0.76 mmol, 1 eq) was diluted in DCM (1.4 ml, 0.5 M), and di-tert-butyl dicarbonate (0.183 g, 0.84 mmol, 1.1 eq) was added at 0°C, gradually heated to room temperature, and stirred overnight. After completion of a reaction, a resulting mixture was extracted with DCM and H₂O. An organic layer was dried over MgSO₄, filtered, concentrated, and purified by column chromatography (silica gel, EA/HEX) to obtain a target compound (0.15 g, 85%).

### Step 2) Synthesis of tert-butyl (3-((5-((imidazo[1,2-a]pyridin-7-ylmethyl)carbamoyl)-2-(((1-methyl-1H-pyrazol-3-yl)methyl)sulfonyl)phenyl)ethynyl)benzyl)carbamate

N-(imidazo[1,2-a]pyridin-7-ylmethyl)-3-iodo-4-(((1-methyl-1H-pyrazol-3-yl)methyl)sulfonyl)benzamide (0.2 g, 0.37 mmol, 1 eq) was dissolved in ACN (3.7 ml, 0.1 M), and then tert-butyl (3-ethynylbenzyl)carbamate (0.128 g, 0.56 mmol, 1.5 eq), PdCl₂(PPh₃)₂ (0.007 g, 0.01 mmol, 0.03 eq), CuI (0.002 g, 0.01 mmol, 0.03 eq), and TEA (0.075 g, 0.74 mmol, 2 eq) were added and degassed with nitrogen (N₂) gas. A reaction solution was stirred under reflux for four hours. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.15 g, 63.5%).

### Step 3) Synthesis of 3-((3-(aminomethyl)phenyl)ethynyl)-N-(imidazo[1,2-a]pyridin-7-ylmethyl)-4-(((1-methyl-1H-pyrazol-3-yl)methyl)sulfonyl)benzamide

Tert-butyl (3-((5-((imidazo[1,2-a]pyridin-7-ylmethyl)carbamoyl)-2-(((1-methyl-1H-pyrazol-3-yl)methyl)sulfonyl)phenyl)ethynyl)benzyl)carbamate (0.05 g, 0.078 mmol, 1 eq) was dissolved in 1,4-dioxane (1 ml, 0.07 M), and then 4 N HCl in 1,4-dioxane (0.58 ml, 2.34 mmol, 30 eq) was added thereto and stirred at room temperature overnight. After completion of a reaction, a reaction product was concentrated and purified by reverse phase column chromatography (0.1% formic acid in H₂O/ACN). 4 N HCl in 1,4-dioxane (0.058 ml, 0.3 M) was added to a purified product at room temperature and stirred overnight. A resulting solid was washed with diethyl ether and filtered under reduced pressure to obtain a title compound (0.017 g, 42.3%).

¹H NMR (400 MHz, DMSO) δ 9.84 (t, J = 5.6 Hz, 1H), 8.88 (d, J = 6.9 Hz, 1H), 8.50 (s, 2H), 8.38 (d, J = 1.4 Hz, 1H), 8.34 (d, J = 1.6 Hz, 1H), 8.17 (d, J = 1.9 Hz, 1H), 8.09 (dd, J = 8.3, 1.5 Hz, 1H), 7.93 (d, J = 8.2 Hz, 1H), 7.87 (s, 1H), 7.82 (s, 1H), 7.73 (d, J = 7.6 Hz, 1H), 7.65 (d, J = 7.7 Hz, 1H), 7.61 - 7.55 (m, 2H), 7.52 (d, J = 7.0 Hz, 1H), 6.09 (d, J = 2.1 Hz, 1H), 4.91 (s, 2H), 4.71 (d, J = 5.5 Hz, 2H), 4.10 (d, J = 5.6 Hz, 2H), 3.70 (s, 3H); MS MH⁺ 537.05

### Example 21: Synthesis of compound 233

### Synthesis of 4-(((1-(4-aminophenyl)-1H-pyrazol-3-yl)methyl)sulfonyl)-3-((4-fluorophenyl)ethynyl)-N-(imidazo[1,2-a]pyridin-7-ylmethyl)benzamide

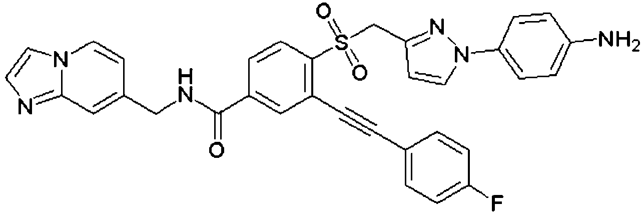

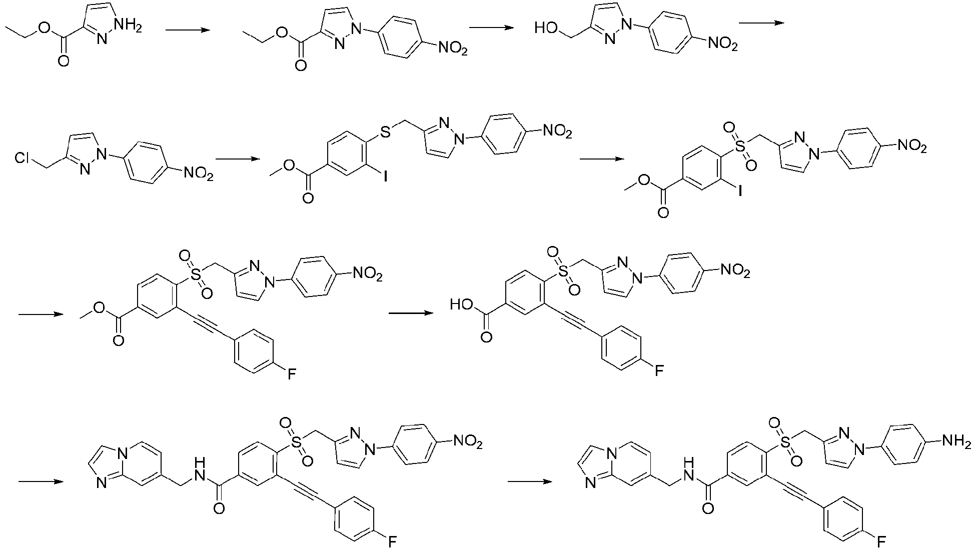

Reagents and conditions: (a) 1-fluoro-4-nitrobenzene, K₂CO₃, DMF, room temperature (rt), 16 hours (16 h); (b) 1.0 M LiAlH₄ in THF, THF, 0°C, seven hours (7 h); (c) SOCl₂, MC, 0°C → rt; (d) methyl 3-iodo-4-mercaptobenzoate, K₂CO₃, DMF, room temperature (rt), two hours (2 h); (e) mCPBA, MC, room temperature (RT), overnight; (f) 1-ethynyl-4-fluorobenzene, TEA, CuI, PdCl₂(PPh₃)₂, ACN, reflux, one hour (1 h); (g) NaOH, EtOH, H₂O, reflux, one hour (1 h); (h) 1-{imidazo[1,2-a]pyridin-7-yl}methanamine hydrochloride, TBTU, TEA, MC, room temperature (rt), seven hours (7 h), (i) Zn, NH₄Cl, 1,4-dioxane, H₂O, room temperature (rt), five hours (5 h)

### Step 1) Synthesis of ethyl 1-(4-nitrophenyl)-1H-pyrazole-3-carboxylate

Ethyl 1H-pyrazole-3-carboxylate (1.4 g, 10 mmol, 1 eq) was dissolved in DMF (100 ml, 0.1 M), and then K₂CO₃ (1.66 g, 12 mmol, 1.2 eq) and 1-fluoro-4-nitrobenzene (1.55 g, 11 mmol 1.1 eq) were added thereto and stirred at room temperature overnight. After completion of a reaction, water was added thereto, and a resulting solid was filtered and dried to obtain a resulting product as a crude product. (1.907 g, 73%)

### Step 2) Synthesis of (1-(4-nitrophenyl)-1H-pyrazol-3-yl)methanol

Ethyl 1-(4-nitrophenyl)-1H-pyrazole-3-carboxylate (1.907 g, 7.36 mmol, 1 eq) was dissolved in anhydrous THF (73 ml, 0.1 M), and then 1.0 M LiAlH₄ in THF (10.9 ml, 10.9 mmol, 1.5 eq) was slowly added dropwise thereto at 0°C and stirred for seven hours. After completion of a reaction, water and EA were added, a resulting solid was filtered, and a filtrate was concentrated to obtain a crude product. (0.468 g)

¹H NMR (400 MHz, CDCl₃) δ 8.40 - 8.33 (m, 2H), 8.03 (d, *J =* 2.6 Hz, 1H), 7.91 - 7.86 (m, 2H), 6.59 (d, *J* = 2.6 Hz, 1H), 4.84 (s, 2H).

### Step 3) Synthesis of 3-(chloromethyl)-1-(4-nitrophenyl)-1H-pyrazole

(1-(4-nitrophenyl)-1H-pyrazol-3-yl)methanol (0.468 g, 2.135 mmol, 1 eq) was dissolved in DCM (11 ml, 0.2 M), and then SOCl₂ (0.46 ml, 6.41 mmol, 3 eq) was slowly added dropwise thereto at 0°C and refluxed overnight. After completion of a reaction, a resulting product was concentrated under reduced pressure to obtain a crude product. (0.432 g, 100%)

¹H NMR (400 MHz, CDCl₃) δ 8.37 - 8.30 (m, 2H), 8.00 (d, *J* = 2.6 Hz, 1H), 7.87 (dd, *J* = 6.9, 5.0 Hz, 2H), 6.63 (d, *J* = 2.6 Hz, 1H), 4.68 (s, 2H).

### Step 4) Synthesis of methyl 3-iodo-4-(((1-(4-nitrophenyl)-1H-pyrazol-3-yl)methyl)thio)benzoate

After dissolving 3-(chloromethyl)-1-(4-nitrophenyl)-1H-pyrazole (0.15 g, 0.5 mmol, 1 eq) in DMF (4 ml, 0.1 M), K₂CO₃ (0.116 g, 0.84 mmol, 2 eq) was added thereto, and methyl 3-iodo-4-mercaptobenzoate (0.15 g, 0.5 mmol, 1.2 eq) dissolved in DMF (5 ml, 0.1 M) was slowly added thereto and stirred at room temperature for two hours. After completion of a reaction, water was added thereto, and a resulting solid was filtered, dried, and then purified by column chromatography (silica gel, EA/HX). (0.073 g, 29.4%)

¹H NMR (400 MHz, CDCl₃) δ 8.45 (d, *J* = 1.8 Hz, 1H), 8.39 - 8.34 (m, 2H), 7.98 (dd, *J* = 4.6, 3.0 Hz, 2H), 7.89 - 7.84 (m, 2H), 7.42 (d, *J=* 8.4 Hz, 1H), 6.60 (d, *J* = 2.6 Hz, 1H), 4.33 (s, 2H), 3.92 (d, *J =* 2.9 Hz, 3H

### Step 5) Synthesis of methyl 3-iodo-4-(((1-(4-nitrophenyl)-1H-pyrazol-3-yl)methyl)sulfonyl)benzoate

Methyl 3-iodo-4-(((1-(4-nitrophenyl)-1H-pyrazol-3-yl)methyl)thio)benzoate (0.073 g, 0.147 mmol, 1 eq) was dissolved in MC (8 ml, 0.2 M), and then mCPBA (0.127 g, 0.735 mmol, 5 eq) was added thereto and stirred overnight. After completion of a reaction, a saturated NaHCO₃ aqueous solution was added and extracted with MC. An organic layer was dried over MgSO₄, filtered and concentrated to obtain a resulting product as a crude product. (0.080 g, 100%)

### Step 6) Synthesis of methyl 3-((4-fluorophenyl)ethynyl)-4-(((1-(4-nitrophenyl)-1H-pyrazol-3-yl)methyl)sulfonyl)benzoate

Methyl 3-iodo-4-(((1-(4-nitrophenyl)-1H-pyrazol-3-yl)methyl)sulfonyl)benzoate (0.447 g, 0.859 mmol, 1 eq) was dissolved in ACN (8.6 ml, 0.1 M), and then TEA (0.26 ml, 1.89 mmol, 2.2 eq), PdCl₂(PPh₃)₂ (18 mg, 0.025 mmol, 3 mol %), and CuI (6.5 mg, 0.034 mmol, 4 mol%) were added thereto and stirred at room temperature for five minutes. 1-ethynyl-4-fluorobenzene (0.15 ml, 1.29 mmol, 1.5 eq) was added thereto and stirred at a temperature of 90°C for one hour. After cooling to room temperature, a resulting product was concentrated and purified by column chromatography (silica gel, EA/HX). (0.125 g, 28%)

¹H NMR (400 MHz, CDCl₃) δ 8.38 (s, 1H), 8.28 (d, *J* = 9.1 Hz, 2H), 8.02 (d, *J* = 0.8 Hz, 2H), 7.91 (d, *J* = 2.4 Hz, 1H), 7.72 - 7.65 (m, 4H), 7.11 (t, *J* = 8.6 Hz, 2H), 6.56 (d, *J* = 2.4 Hz, 1H), 4.90 (s, 2H), 3.98 (s, 3H).

### Step 7) Synthesis of 3-((4-fluorophenyl)ethynyl)-4-(((1-(4-nitrophenyl)-1H-pyrazol-3-yl)methyl)sulfonyl)benzoic acid

Methyl 3-((4-fluorophenyl)ethynyl)-4-(((1-(4-nitrophenyl)-1H-pyrazol-3-yl)methyl)sulfonyl)benzoate (0.125 g, 0.065 mmol, 1 eq) was dissolved in EtOH (1 ml, 0.05 M), and then NaOH (0.005 g, 0.13 mmol, 2 eq) dissolved in H₂O (0.14 ml) was added thereto and refluxed for one hour. After cooling to room temperature, a resulting product was concentrated and diluted with water. After adjusting to pH=5 to 6 with 1 N HCl, a resulting solid was filtered, washed with water, and dried. (0.1 g, 82%)

### Step 8) Synthesis of 3-((4-fluorophenyl)ethynyl)-N-(imidazo[1,2-a]pyridin-7-ylmethyl)-4-(((1-(4-nitrophenyl)-1H-pyrazol-3-yl)methyl)sulfonyl)benzamide

3-((4-fluorophenyl)ethynyl)-4-(((1-(4-nitrophenyl)-1H-pyrazol-3-yl)methyl)sulfonyl)benzoic acid (0.1 g, 0.2 mmol, 1 eq) was dissolved in MC (2 ml, 0.1 M), and then TBTU (0.1 g, 0.3 mmol, 1.5 eq), TEA (0.06 ml, 0.44 mmol, 2.2 eq), and 1-{imidazo[1,2-a]pyridin-7-yl}methanamine hydrochloride (0.04 g, 0.22 mmol, 1.1 eq) were added thereto and stirred at room temperature for seven hours. After completion of a reaction, a resulting product was concentrated and purified by column chromatography (silica gel, MeOH/MC). (51 mg, 40.2%)

### Step 9) Synthesis of 4-(((1-(4-aminophenyl)-1H-pyrazol-3-yl)methyl)sulfonyl)-3-((4-fluorophenyl)ethynyl)-N-(imidazo[1,2-a]pyridin-7-ylmethyl)benzamide

3-((4-fluorophenyl)ethynyl)-N-(imidazo[1,2-a]pyridin-7-ylmethyl)-4-(((1-(4-nitrophenyl)-1H-pyrazol-3-yl)methyl)sulfonyl)benzamide (0.051 g, 0.080 mmol, 1 eq) was dissolved in 1,4-dioxane : H₂O (3:1 (v:v) (1 ml, 0.084 M), and then zinc (0.055 g, 0.84 mmol, 10 eq) and NH₄Cl (0.045 g, 0.84 mmol, 10 eq) were added thereto and stirred at room temperature for five hours. A reaction mixture was washed with MeOH and filtered under reduced pressure through celite. A filtrate was concentrated and purified by reverse phase column chromatography (0.1% formic acid in H₂O/acetonitrile).

¹H NMR (400 MHz, MeOD) δ 8.48 (d, *J* = 7.1 Hz, 1H), 8.29 (s, 1H), 8.25 (s, 1H), 7.98 - 7.94 (m, 2H), 7.88 (s, 1H), 7.86 (d, *J =* 2.4 Hz, 1H), 7.78 - 7.73 (m, 2H), 7.63 (s, 1H), 7.57 (s, 1H), 7.23 - 7.16 (m, 4H), 7.04 (d, *J* = 7.1 Hz, 1H), 6.71 (d, *J* = 8.8 Hz, 2H), 6.41 (d, *J* = 2.4 Hz, 1H), 4.94 (s, 2H), 4.70 (s, 2H).

### Example 22: Synthesis of compound 122

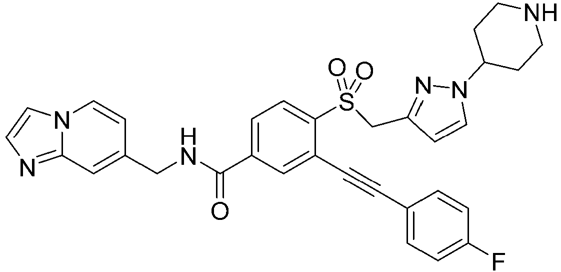

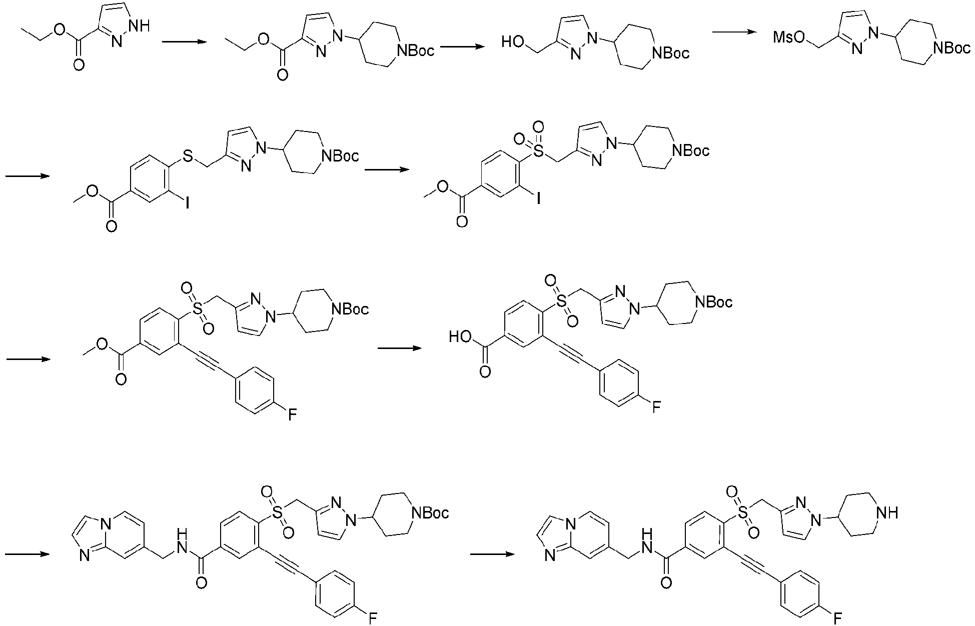

Reagents and conditions: (a) tert-butyl 4-((methylsulfonyl)oxy)piperidine-1-carboxylate, K₂CO₃, DMF, 70°C (b) 1.0 M LiAlH₄ in THF, THF, 0°C, one hour (1 h); (c) MsCl, TEA, THF, 0°C to room temperature(0°C-rt), 12 hours (12 h) (d) methyl 3-iodo-4-mercaptobenzoate, K₂CO₃, DMF, room temperature (rt), overnight; (e) mCPBA, MC, room temperature (RT), overnight; (f) 1-ethynyl-4-fluorobenzene, TEA, CuI, PdCl₂(PPh₃)₂, ACN, reflux, one hour (1 h); (g) NaOH, EtOH, H₂O, reflux, one hour (1 h); (h) 1-{imidazo[1,2-a]pyridin-7-yl}methanamine hydrochloride, TBTU, TEA, MC, room temperature (rt), overnight, (i) 4 N HCl in dioxane, DCM, room temperature (rt), two hours (2 h)

### Step 1) Synthesis of tert-butyl 4-(3-(ethoxycarbonyl)-1H-pyrazol-1-yl)piperidine-1-carboxylate

Ethyl 1H-pyrazole-3-carboxylate (1.4 g, 10 mmol, 1 eq) was dissolved in DMF (50 ml, 0.2 M), and K₂CO₃ (1.66 g, 12 mmol, 1.2. eq) and tert-butyl 4-((methylsulfonyl)oxy)piperidine-1-carboxylate (3.07 g, 11 mmol, 1.1 eq) were added thereto and stirred at 70°C for 16 hours. After completion of a reaction, a resulting mixture was cooled to room temperature, and water was added thereto and extracted with EA. An organic layer was dried over MgSO₄, filtered, concentrated, and purified by column chromatography (silica gel, EA/HX). (1.623 g, 50.2%)

### Step 2) Synthesis of tert-butyl 4-(3-(hydroxymethyl)-1H-pyrazol-1-yl)piperidine-1-carboxylate

Tert-butyl 4-(3-(ethoxycarbonyl)-1H-pyrazol-1-yl)piperidine-1-carboxylate (1.623 g, 5.02 mmol, 1 eq) was dissolved in anhydrous THF (50 ml, 0.1 M), and then 1.0 M LiAlH₄ in THF (7.6 ml, 7.60 mmol, 1.5 eq) was slowly added dropwise thereto at 0°C and stirred for one hour. After completion of a reaction, water and EA were added, a resulting solid was filtered, and a filtrate was concentrated to obtain a crude product. (1.5 g, 100%)

### Step 3) Synthesis of tert-butyl 4-(3-(((methylsulfonyl)oxy)methyl)-1H-pyrazol-1-yl)piperidine-1-carboxylate

Tert-butyl 4-(3-(hydroxymethyl)-1H-pyrazol-1-yl)piperidine-1-carboxylate (0.2 g, 0.71 mmol, 1 eq) was dissolved in THF (4 ml, 0.2 M), and then TEA (0.12 ml, 0.852 mmol, 1.2 eq) and MsCl (0.06ml, 0.781 mmol, 1.1 eq) were added thereto and stirred overnight. After completion of a reaction, water was added and extracted with DCM. An organic layer was dried over MgSO₄, filtered and concentrated to obtain a resulting product as a crude product. (0.204 g, 100%)

### Step 4) Synthesis of tert-butyl 4-(3-(((2-iodo-4-(methoxycarbonyl)phenyl)thio)methyl)-1H-pyrazol-1-yl)piperidine-1-carboxylate

After dissolving tert-butyl 4-(3-(((methylsulfonyl)oxy)methyl)-1H-pyrazol-1-yl)piperidine-1-carboxylate (0.204 g, 0.57 mmol, 1 eq) in DMF (5 ml, 0.1 M), K₂CO₃ (0.16 g, 1.134 mmol, 2 eq) was added thereto, and methyl 3-iodo-4-mercaptobenzoate (0.2 g, 0.68 mmol, 1.2 eq) dissolved in DMF (5 ml, 0.1 M) was slowly added thereto and stirred at room temperature overnight for one hour. After completion of a reaction, water was added thereto, and a resulting solid was filtered and dried to obtain a resulting product as a crude product. (0.193 g, 50.9%)

¹H NMR (400 MHz, CDCl₃) δ 8.50 (d, *J* = 1.7 Hz, 1H), 8.00 (dd, *J =* 8.2, 1.8 Hz, 1H), 7.50 (d, *J* = 1.3 Hz, 1H), 7.30 (s, 1H), 7.28 (s, 1H), 6.22 (d, *J* = 1.7 Hz, 1H), 4.32 (dd, *J* = 13.2, 8.8 Hz, 3H), 4.24 (d, *J* = 5.9 Hz, 2H), 3.93 (d, *J* = 4.6 Hz, 3H), 2.89 (t, *J* = 12.7 Hz, 2H), 2.23 (qd, *J* = 12.6, 4.2 Hz, 2H), 1.95 (d, *J* = 12.3 Hz, 2H), 1.49 (s, 9H).

### Step 5) Synthesis of tert-butyl 4-(3-(((2-iodo-4-(methoxycarbonyl)phenyl)sulfonyl)methyl)-1H-pyrazol-1-yl)piperidine-1-carboxylate

Tert-butyl 4-(3-(((2-iodo-4-(methoxycarbonyl)phenyl)thio)methyl)-1H-pyrazol-1-yl)piperidine-1-carboxylate (0.193 g, 0.346 mmol, 1 eq) was dissolved in MC (2 ml, 0.2 M), and then mCPBA (0.3 g, 1.731 mmol, 5 eq) was added thereto and stirred overnight. After completion of a reaction, a saturated NaHCO₃ aqueous solution was added and extracted with MC. An organic layer was dried over MgSO₄, filtered and concentrated to obtain a resulting product as a crude product. (0.183 g, 100%)

### Step 6) Synthesis of tert-butyl 4-(3-(((2-((4-fluorophenyl)ethynyl)-4-(methoxycarbonyl)phenyl)sulfonyl)methyl)-1H-pyrazol-1-yl)piperidine-1-carboxylate

Tert-butyl 4-(3-(((2-iodo-4-(methoxycarbonyl)phenyl)sulfonyl)methyl)-1H-pyrazol-1-yl)piperidine-1-carboxylate (0.183 g, 0.31 mmol, 1 eq) was dissolved in ACN (4 ml, 0.1 M), and then TEA (0.1 ml, 0.68 mmol, 2.2 eq), PdCl₂(PPh₃)₂ (6.5 mg, 0.009 mmol, 3 mol %), and CuI (2.4 mg, 0.012 mmol, 4 mol%) were added thereto and stirred at room temperature for five minutes. 1-ethynyl-4-fluorobenzene (0.053 ml, 0.47 mmol, 1.5 eq) was added thereto and stirred at a temperature of 90°C for one hour. After cooling to room temperature, a resulting product was concentrated and purified by column chromatography (silica gel, EA/HX). (0.1 g, 55.6%)

### Step 7) Synthesis of 4-(((1-(1-(tert-butoxycarbonyl)piperidin-4-yl)-1H-pyrazol-3-yl)methyl)sulfonyl)-3-((4-fluorophenyl)ethynyl)benzoic acid

Tert-butyl 4-(3-(((2-((4-fluorophenyl)ethynyl)-4-(methoxycarbonyl)phenyl)sulfonyl)methyl)-1H-pyrazol-1-yl)piperidine-1-carboxylate (0.10 g, 0.172 mmol, 1 eq) was dissolved in EtOH (4 ml, 0.05 M), and then NaOH (0.014 g, 0.344 mmol, 2 eq) dissolved in H₂O (0.1 ml) was added thereto and refluxed for one hour. After cooling to room temperature, a resulting product was concentrated to obtain a target compound as a crude product.

### Step 8) Synthesis of tert-butyl 4-(3-(((2-((4-fluorophenyl)ethynyl)-4-((imidazo[1,2-a]pyridin-7-ylmethyl)carbamoyl)phenyl)sulfonyl)methyl)-1H-pyrazol-1-yl)piperidine-1-carboxylate

4-(((1-(1-(tert-butoxycarbonyl)piperidin-4-yl)-1H-pyrazol-3-yl)methyl)sulfonyl)-3-((4-fluorophenyl)ethynyl)benzoic acid (0.1 g, 0.172 mmol, 1 eq) was dissolved in MC, and then TBTU (0.083 g, 0.258 mmol, 1.5 eq), TEA (0.053 ml, 0.38 mmol, 2.2 eq), and 1-{imidazo[1,2-a]pyridin-7-yl}methanamine hydrochloride (0.035 g, 0.189 mmol, 1.1 eq) were added thereto and stirred at room temperature overnight. After completion of a reaction, a saturated NaHCO₃ aqueous solution was added, neutralized and extracted with MC. An organic layer was dried over MgSO₄, filtered, concentrated, and purified by column chromatography (silica gel, MeOH/MC). (0.112 g, 93.3%)

### Step 9) Synthesis of 3-((4-fluorophenyl)ethynyl)-N-(imidazo[1,2-a]pyridin-7-ylmethyl)-4-(((1-(piperidin-4-yl)-1H-pyrazol-3-yl)methyl)sulfonyl)benzamide

Tert-butyl 4-(3-(((2-((4-fluorophenyl)ethynyl)-4-((imidazo[1,2-a]pyridin-7-ylmethyl)carbamoyl)phenyl)sulfonyl)methyl)-1H-pyrazol-1-yl)piperidine-1-carboxylate (0.112 g, 0.16 mmol, 1 eq) was dissolved in DCM (1 ml, 0.2 M), and then 4 N HCl in dioxane (0.4 ml, 1.6 mmol, 10 eq) was added thereto and stirred at room temperature for two hours. After completion of a reaction, a resulting mixture was concentrated and purified by reverse phase column chromatography (0.1% formic acid in H₂O/ACN) to obtain the title compound (0.028 g, 32.5%).

¹H NMR (400 MHz, MeOD) δ 8.62 (s, 1H), 8.44 (d, *J* = 7.0 Hz, 1H), 8.09 (dd, *J* = 8.1, 1.1 Hz, 1H), 7.88 (d, *J* = 8.1 Hz, 1H), 7.83 (s, 1H), 7.76 (s, 1H), 7.56 (s, 1H), 7.38 (d, *J* = 1.9 Hz, 1H), 7.30 (dd, *J* = 8.7, 5.4 Hz, 2H), 7.07 - 6.98 (m, 4H), 5.91 (d, *J* = 1.9 Hz, 1H), 4.72 (s, 2H), 4.60 - 4.50 (m, 1H), 3.22 (d, *J =* 12.8 Hz, 2H), 2.79 (td, *J* = 15.0, 4.7 Hz, 2H), 2.68 (s, 2H), 2.22 - 2.10 (m, 3H), 1.97 (d, *J* = 10.1 Hz, 1H).

### Example 23: Synthesis of compound 148

### 4-((4-aminobenzyl)sulfonyl)-3-((4-fluorophenyl)ethynyl)-N-(imidazo[1,2-a]pyridin-7-ylmethyl)benzamide

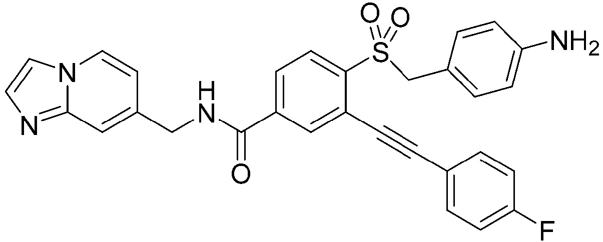

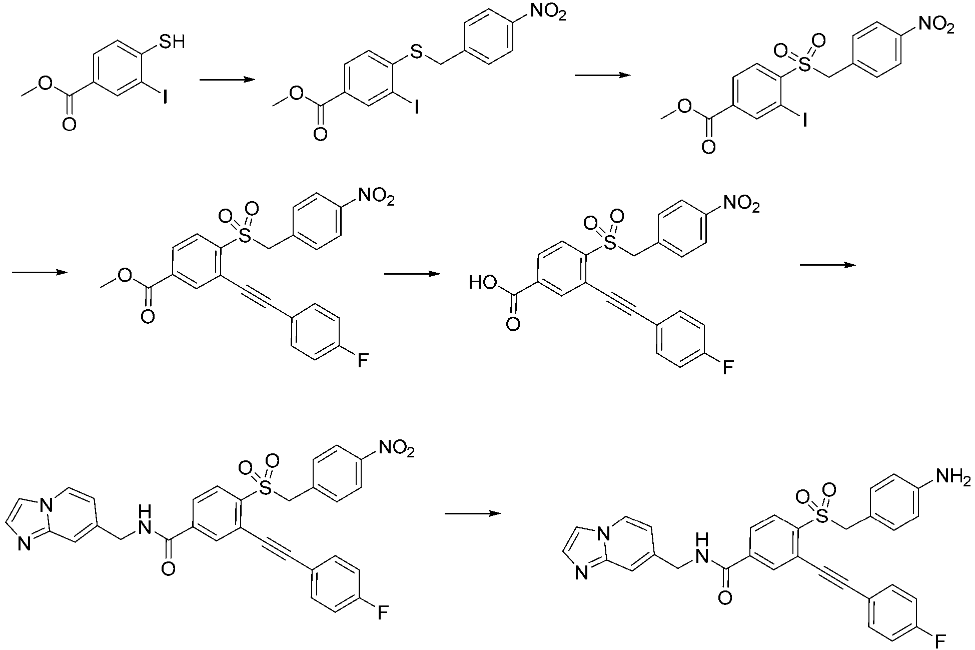

Reagents and conditions: (a) 1-(chloromethyl)-4-nitrobenzene, K₂CO₃, DMF, room temperature (r.t), 0.5 hours (0.5 h) (b) mCPBA, DCM, room temperature (r.t), 16 hours (16 h) (c) PdCl₂[PPh₃]₂, CuI, TEA, ACN, room temperature (r.t), 16 hours (16 h) (d) NaOH, H₂O, MeOH, 55°C, 12 hours (12 h) (e) 1-{imidazo[1,2-a]pyridin-7-yl}methanamine hydrochloride, TBTU, TEA, DCM, room temperature (rt), 12 hours (12 h) (f) Zn, NH₄Cl, dioxane, H₂O, room temperature (rt), two hours (2 h)

### Step 1) Synthesis of methyl 3-iodo-4-((4-nitrobenzyl)thio)benzoate

1-(chloromethyl)-4-nitrobenzene (0.24 g, 1.4 mmol, 1 eq) was dissolved in DMF (14 ml), and K₂CO₃ (0.39 g, 2.84 mmol, 2 eq) was added thereto. Then, methyl 3-iodo-4-mercaptobenzoate (0.5 g, 1.70 mmol, 1.2 eq) was added to a reaction product and stirred for 30 minutes. Water was added to a reaction product and extracted several times with EA. An organic layer was dried over magnesium sulfate and concentrated under reduced pressure to obtain a target compound (0.6 g, 99%) as a crude product.

### Step 2) Synthesis of methyl 3-iodo-4-((4-nitrobenzyl)sulfonyl)benzoate

Methyl 3-iodo-4-((4-nitrobenzyl)thio)benzoate (0.73 g, 1.7 mmol, 1 eq) was dissolved in DCM (8.5 ml, 0.2 M), and mCPBA (1.47 g, 8.5 mmol, 5 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure. A resulting residue was purified by column chromatography (silica gel, 40% EA/HX) to obtain a target compound (0.23 g, 29.1%).

### Step 3) Synthesis of methyl 3-((4-fluorophenyl)ethynyl)-4-((4-nitrobenzyl)sulfonyl)benzoate

MeCN (5 ml, 0.1 M) was added to methyl 3-iodo-4-((4-nitrobenzyl)sulfonyl)benzoate (0.23 g, 0.5 mmol, 1 eq) and TEA (0.15 ml, 1.1 mmol, 2.2 eq) was added thereto. PdCl₂[PPh₃]₂ (10.5 mg, 3 mol%) and CuI (4 mg, 3 mol%) were added to a reaction product and stirred at room temperature for five minutes under a nitrogen atmosphere. Subsequently, 4-fluorophenyl-acetylene (0.09 ml, 0.78 mmol, 1.5 eq) was added thereto. A reaction mixture was stirred at room temperature for one hour and concentrated under reduced pressure. A resulting residue was purified by column chromatography (silica gel, 40% EA/HX) to obtain a target compound (0.191 g, 84.1%).

¹H NMR (400 MHz, CDCl₃) δ 8.38 (d, *J* = 1.6 Hz, 1H), 8.09 (d, *J* = 8.7 Hz, 2H), 7.99 (dd, *J* = 8.3, 1.7 Hz, 1H), 7.85 (d, *J* = 8.3 Hz, 1H), 7.67 (dd, *J* = 8.9, 5.3 Hz, 2H), 7.36 (d, *J* = 8.7 Hz, 2H), 7.15 (t, *J* = 8.7 Hz, 2H), 4.80 (s, 2H), 3.98 (s, 3H).

### Step 4) Synthesis of 3-((4-fluorophenyl)ethynyl)-4-((4-nitrobenzyl)sulfonyl)benzoic acid

MeOH (8 ml), H₂O (0.3 ml), and NaOH (0.034 g, 0.842 mmol, 2 eq) were added to methyl 3-((4-fluorophenyl)ethynyl)-4-((4-nitrobenzyl)sulfonyl)benzoate (0.191 g, 0.421 mmol, 1 eq) and stirred at 55°C for one hour. When a reaction was completed by confirming the reaction through TLC, the pH of a reaction product was adjusted to 1 with 1 N HCl aqueous solution and extracted with EA. An organic layer was washed with water, dried over magnesium sulfate, and concentrated under reduced pressure to obtain a target compound (0.1 g, 56%).

### Step 5) Synthesis of 3-((4-fluorophenyl)ethynyl)-N-(imidazo[1,2-a]pyridin-7-ylmethyl)-4-((4-nitrobenzyl)sulfonyl)benzamide

DCM (2.3 ml), TBTU (0.1 g, 0.342 mmol, 1.5 eq), TEA (0.07 ml, 0.5 mmol, 2.2 eq), and 1-{imidazo[1,2-a]pyridin-7-yl}methanamine hydrochloride (0.046 g, 0.25 mmol, 1.1 eq) were added to 3-((4-fluorophenyl)ethynyl)-4-((4-nitrobenzyl)sulfonyl)benzoic acid (0.1 g, 0.228 mmol, 1 eq) and stirred for 12 hours. A reaction product was concentrated under reduced pressure, and a resulting residue was purified by column chromatography (silica gel, 10% MeOH/DCM) to obtain a target compound (0.053 g, 40.8%).

### Step 7) Synthesis of 4-((4-aminobenzyl)sulfonyl)-3-((4-fluorophenyl)ethynyl)-N-(imidazo[1,2-a]pyridin-7-ylmethyl)benzamide

3-((4-fluorophenyl)ethynyl)-N-(imidazo[1,2-a]pyridin-7-ylmethyl)-4-((4-nitrobenzyl)sulfonyl)benzamide (0.053 g, 0.084 mmol, 1 eq) was dissolved in 1,4-dioxane : water (H₂O) (3:1 (v:v), 1 ml), and then zinc (0.023 g, 0.42 mmol, 5 eq) and NH₄Cl (0.022 g, 0.42 mmol, 5 eq) were added thereto and stirred at room temperature for two hours. A reaction mixture was washed with MeOH and filtered under reduced pressure through celite. A filtrate was concentrated and purified by prep-LC (0.1% formic acid in H₂O/acetonitrile). (0.002 g)

¹H NMR (400 MHz, MeOD) δ 8.58 (s, 1H), 8.53 (s, 7H), 8.44 (d, *J =* 6.8 Hz, 1H), 8.06 (d, *J* = 9.0 Hz, 1H), 7.84 (d, *J* = 8.3 Hz, 2H), 7.70 (s, 1H), 7.55 (s, 2H), 7.42 - 7.36 (m, 2H), 6.99 (dd, *J =* 14.3, 7.7 Hz, 5H), 6.63 (d, *J =* 8.6 Hz, 1H), 4.71 (s, 2H).

MS(ESI) m/e MH⁺ 539

### Example 24: Synthesis of compound 234

### Synthesis of 4-(((1H-pyrazol-3-yl)methyl)sulfonyl)-3-((4-fluorophenyl)ethynyl)-N-(imidazo[1,2-a]pyridin-7-ylmethyl)benzamide

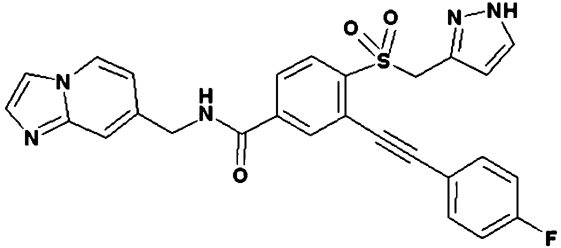

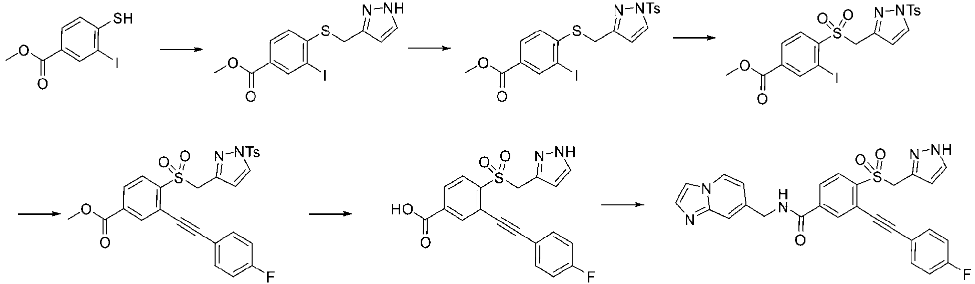

Reagents and conditions: (a) 3-(chloromethyl)pyrazole hydrochloride, K₂CO₃, DMF, room temperature (r.t), 0.5 hours (0.5 h) (b) TsCl, pyridine, DCM, room temperature (rt), 12 hours (12 h) (c) mCPBA, DCM, room temperature (r.t), 16 hours (16 h) (d) PdCl₂[PPh₃]₂, CuI, TEA, ACN, room temperature (r.t), 16 hours (16 h) (e) NaOH, H₂O, MeOH, 55°C, 12 hours (12 h) (f) 1-{imidazo[1,2-a]pyridin-7-yl}methanamine hydrochloride, TBTU, TEA, DCM, 12 hours (12 h)

### Step 1) Synthesis of methyl 4-(((1H-pyrazol-3-yl)methyl)thio)-3-iodobenzoate

3-(chloromethyl)pyrazole hydrochloride (0.5 g, 1.70 mmol, 1 eq) was dissolved in DMF (14 ml), and K₂CO₃ (0.39 g, 2.84 mmol, 2 eq) was added thereto. Then, methyl 3-iodo-4-mercaptobenzoate (0.5 g, 1.70 mmol, 1.2 eq) was added to a reaction product and stirred for 30 minutes. Water was added to a reaction product, and a resulting solid was filtered and dried to obtain a target compound.

### Step 2) Synthesis of methyl 3-iodo-4-(((1-tosyl-1H-pyrazol-3-yl)methyl)thio)benzoate

Methyl 4-(((1H-pyrazol-3-yl)methyl)thio)-3-iodobenzoate (0.05 g, 0.133 mmol, 1 eq) was dissolved in DCM (0.1 M, 1.33 ml), and pyridine (0.027 ml, 5.8 M) and TsCl (0.030g, 0.16 mmol, 1.2 eq) were added thereto and stirred at room temperature for 12 hours. A reaction product was concentrated, and then water was added and extracted with DCM. An organic layer was dried over magnesium sulfate and concentrated under reduced pressure, and a resulting residue was purified by column chromatography (silica gel, 20% EA/HX) to obtain a target compound (0.041 g, 0.08 mmol, 58.6%).

### Step 3) Synthesis of methyl 3-iodo-4-(((1-tosyl-1H-pyrazol-3-yl)methyl)sulfonyl)benzoate

Methyl 3-iodo-4-(((1-tosyl-1H-pyrazol-3-yl)methyl)thio)benzoate (0.041 g, 0.08 mmol, 1 eq) was dissolved in DCM (1 ml), and mCPBA (0.07 g, 0.39 mmol, 5 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure to obtain a resulting residue, which was used in the next reaction step without separation.

### Step 4) Synthesis of methyl 3-((4-fluorophenyl)ethynyl)-4-(((1-tosyl-1H-pyrazol-3-yl)methyl)sulfonyl)benzoate

MeCN (1 ml) was added to methyl 3-iodo-4-(((1-tosyl-1H-pyrazol-3-yl)methyl)sulfonyl)benzoate (0.03 g, 0.052 mmol, 1 eq) and TEA (0.016 ml, 0.114 mmol, 2.2 eq) was added thereto. PdCl₂[PPh₃]₂ (1 mg, 3 mol%) and CuI (0.4 mg, 3 mol%) were added to a reaction product and stirred at room temperature for five minutes under a nitrogen atmosphere. Subsequently, 4-fluorophenyl-acetylene (0.009 ml, 0.078 mmol, 1.5 eq) was added thereto. A reaction mixture was stirred at room temperature for one hour and concentrated under reduced pressure. A resulting residue was purified by column chromatography (silica gel, 40% EA/HX) to obtain a target compound (0.020 g, 69.7%).

### Step 5) Synthesis of 4-(((1H-pyrazol-3-yl)methyl)sulfonyl)-3-((4-fluorophenyl)ethynyl)benzoic acid

MeOH (1 ml), H₂O (1 ml), and NaOH (3 mg, 0.072 mmol, 2 eq) were added to methyl 3-((4-fluorophenyl)ethynyl)-4-(((1-tosyl-1H-pyrazol-3-yl)methyl)sulfonyl)benzoate (0.020 g, 0.036 mmol, 1 eq) and stirred at 55°C for one hour. When a reaction was completed by confirming the reaction through TLC, a resulting mixture was concentrated under reduced pressure to obtain a target compound as a crude product.

### Step 6) Synthesis of 4-(((1H-pyrazol-3-yl)methyl)sulfonyl)-3-((4-fluorophenyl)ethynyl)-N-(imidazo[1,2-a]pyridin-7-ylmethyl)benzamide

DCM (1 ml), TBTU (0.027 g, 0.09 mmol, 1.5 eq), TEA (0.02 ml, 0.125 mmol, 2.2 eq), and 1-{imidazo[1,2-a]pyridin-7-yl}methanamine hydrochloride (0.012 g, 0.063 mmol, 1.1 eq) were added to 4-(((1H-pyrazol-3-yl)methyl)sulfonyl)-3-((4-fluorophenyl)ethynyl)benzoic acid (0.022 g, 0.057 mmol, 1 eq) and stirred for two hours. A reaction product was concentrated under reduced pressure, and a resulting residue was purified by column chromatography (silica gel, 10% MeOH/DCM) to obtain a target compound.

¹H NMR (400 MHz, MeOD) δ 8.42 (d, *J* = 7.0 Hz, 1H), 8.24 (s, 1H), 7.89 (t, *J* = 6.4 Hz, 2H), 7.82 (s, 1H), 7.74 (dd, *J* = 8.6, 5.4 Hz, 2H), 7.54 (d, *J* = 7.0 Hz, 2H), 7.48 (s, 1H), 7.22 (t, *J* = 8.7 Hz, 2H), 6.96 (d, *J* = 7.1 Hz, 1H), 6.25 (s, 1H), 4.65 (s, 2H).

MS(ESI) m/e MH⁺ 514

### Example 25: Synthesis of compound 235

### Synthesis of N-(3-(1H-pyrazol-4-yl)propyl)-3-((3-methoxy-4-methylphenyl)ethynyl)-4-(((1-methyl-1H-pyrazol-3-yl)methyl)sulfonyl)benzamide

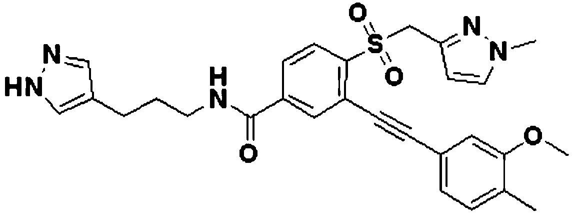

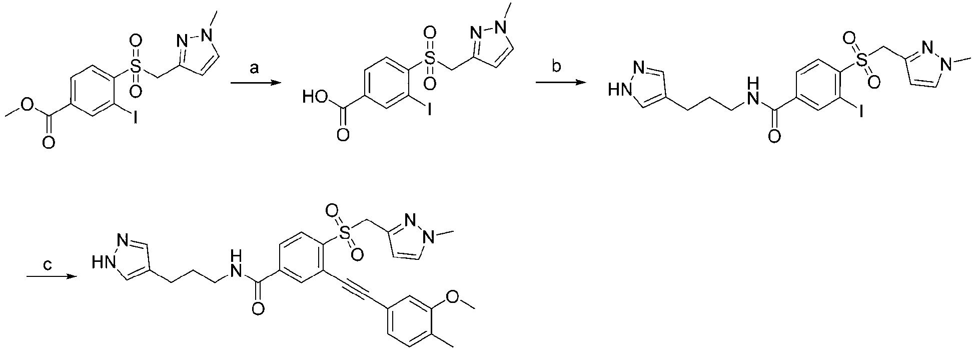

Reagents and conditions: (a) NaOH, H₂O, MeOH, 60°C, two hours (2 h); (b) 3-(1H-pyrazol-4-yl)propan-1-amine dihydrochloride, TBTU, TEA, DCM, 12 hours (12 h), (c) PdCl₂[PPh₃]₂, CuI, TEA, ACN, room temperature (r.t), one hour (1 h)

### Step 1) Synthesis of 3-iodo-4-(((1-methyl-1H-pyrazol-3-yl)methyl)sulfonyl)benzoic acid

MeOH (15 ml, 0.2 M), H₂O (2 ml), and NaOH (0.23 g, 5.79 mmol, 2 eq) were added to methyl 3-iodo-4-(((1-methyl-1H-pyrazol-3-yl)methyl)sulfonyl)benzoate (1.216 g, 2.9 mmol, 1 eq) and stirred at 60°C for two hours. When a reaction was completed by confirming the reaction through TLC, the pH of a reaction product was adjusted to 1 with 1 N HCl aqueous solution and extracted with EA. An organic layer was washed with water, separated, dried over magnesium sulfate, and concentrated under reduced pressure to obtain a target compound (1.18 g, 100%).

¹H NMR (400 MHz, DMSO) δ 13.74 (s, 1H), 8.57 (d, *J* = 1.3 Hz, 1H), 8.04 (dd, *J* = 8.2, 1.4 Hz, 1H), 7.93 - 7.88 (m, 1H), 7.59 (d, *J* = 2. 0 Hz, 1H), 6.06 (d, *J* = 2.1 Hz, 1H), 4.83 (s, 2H), 3.69 (s, 3H).

### Step 2) Synthesis of N-(3-(1H-pyrazol-4-yl)propyl)-3-iodo-4-(((1-methyl-1H-pyrazol-3-yl)methyl)sulfonyl)benzamide

DCM (10 ml), TBTU (0.48 g, 1.5 mmol, 1.5 eq), TEA (0.3 ml, 2.2 mmol, 2.2 eq), and 3-(1H-pyrazol-4-yl)propan-1-amine dihydrochloride (0.22 g, 1.1 mmol, 1.1 eq) were added to 3-iodo-4-(((1-methyl-1H-pyrazol-3-yl)methyl)sulfonyl)benzoic acid (0.4 g, 1 mmol, 1 eq) and stirred for 12 hours. A reaction product was concentrated, and a resulting residue was purified by column chromatography (silica gel, 10% MeOH/DCM) to obtain a target compound. (0.16 g, 31.3%)

¹H NMR (400 MHz, MeOD) δ 8.56 (d, *J =* 1.4 Hz, 1H), 7.92 (d, *J =* 8.2 Hz, 1H), 7.87 (dd, *J* = 8.2, 1.5 Hz, 1H), 7.51 - 7.45 (m, 3H), 6.18 (d, *J=* 2.2 Hz, 1H), 4.83 (s, 2H), 3.77 (s, 3H), 3.43 (t, *J* = 7.1 Hz, 2H), 3.37 (s, 1H), 2.83 (s, 1H), 2.61 (t, *J* = 7.5 Hz, 2H), 1.91 (p, *J* = 7.3 Hz, 2H).

### Step 3) Synthesis of N-(3-(1H-pyrazol-4-yl)propyl)-3-((3-methoxy-4-methylphenyl)ethynyl)-4-(((1-methyl-1H-pyrazol-3-yl)methyl)sulfonyl)benzamide

MeCN (1 ml, 0.1 M) was added to N-(3-(1H-pyrazol-4-yl)propyl)-3-iodo-4-(((1-methyl-1H-pyrazol-3-yl)methyl)sulfonyl)benzamide (0.03 g, 0.058 mmol, 1 eq) and TEA (0.018 ml, 0.128 mmol, 2.2 eq) was added thereto. PdCl₂[PPh₃]₂ (1.22 mg, 3 mol%) and CuI (0.44 mg, 3 mol%) were added to a reaction product and stirred at room temperature for five minutes under a nitrogen atmosphere. Subsequently, 4-ethynyl-2-methoxy-1-methylbenzene (13 mg, 0.088 mmol, 1.5 eq) was added thereto. A reaction mixture was stirred at room temperature for one hour and concentrated under reduced pressure. A resulting residue was purified by column chromatography (silica gel, 10% MeOH/DCM) to obtain a target compound (27 mg, 87.7%).

¹H NMR (400 MHz, CDCl₃) δ 8.16 (s, 1H), 7.90 (d, *J* = 8.2 Hz, 1H), 7.84 (d, *J* = 8.2 Hz, 1H), 7.50 (s, 2H), 7.44 (s, 1H), 7.19 (d, *J* = 4.8 Hz, 3H), 6.16 (s, 1H), 4.86 (s, 2H), 3.89 (s, 3H), 3.72 (s, 3H), 3.44 (t, *J* = 7.0 Hz, 2H), 2.62 (t, *J* = 7.5 Hz, 2H), 2.24 (s, 3H), 1.97 - 1.88 (m, 2H).

The reagents may be changed in substantially the same manner as in Example 25 to synthesize compounds 44, 45, 46, and 75 to 82.

### Example 26: Synthesis of compound 236

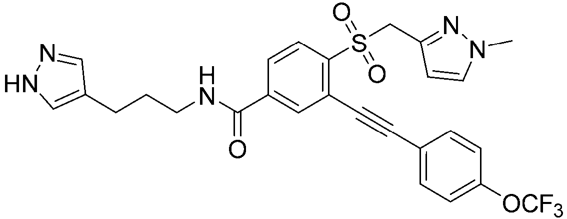

### Synthesis of N-(3-(1H-pyrazol-4-yl)propyl)-4-(((1-methyl-1H-pyrazol-3-yl)methyl)sulfonyl)-3-((4-(trifluoromethoxy)phenyl)ethynyl)benzamide

Compound 236 was prepared in substantially the same manner as in the synthesis of compound 235 of Example 25, except that 1-ethynyl-4-(trifluoromethoxy)benzene was used instead of 4-ethynyl-2-methoxy-1-methylbenzene in Step 3) in comparison with the synthesis of compound 235 of Example 25.

¹H NMR (400 MHz, CDCl₃) δ 8.18 (s, 1H), 7.89 (q, *J* = 8.2 Hz, 2H), 7.80 *(d, J=* 8.7 Hz, 2H), 7.48 (s, 2H), 7.44 (s, 1H), 7.39 (d, *J* = 8.2 Hz, 2H), 6.16 (d, J = 2.0 Hz, 1H), 4.82 (s, 2H), 3.72 (s, 3H), 3.44 (t, *J* = 7.0 Hz, 2H), 2.61 (t, *J* = 7.4 Hz, 2H), 1.97 - 1.85 (m, 2H).

### Example 27: Synthesis of compound 237

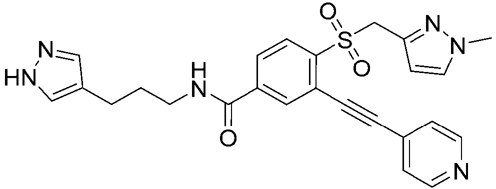

### Synthesis of N-(3-(1H-pyrazol-4-yl)propyl)-4-(((1-methyl-1H-pyrazol-3-yl)methyl)sulfonyl)-3-(pyridin-4-ylethynyl)benzamide

Compound 237 was prepared in substantially the same manner as in the synthesis of compound 235, except that 4-ethynylpyridine was used instead of 4-ethynyl-2-methoxy-1-methylbenzene in Step 3) in comparison with the synthesis of compound 235 of Example 25.

¹H NMR (400 MHz, MeOD) δ 8.67 (d, *J* = 5.9 Hz, 2H), 8.25 (s, 1H), 7.96 (s, 2H), 7.71 (d, *J* = 6.1 Hz, 2H), 7.50 (s, 2H), 7.46 (d, *J* = 2.1 Hz, 1H), 6.20 (d, *J* = 2.2 Hz, 1H), 4.82 (s, 2H), 3.73 (s, 3H), 3.46 (t, *J* = 7.1 Hz, 2H), 2.63 (t, *J* = 7.5 Hz, 2H), 1.99 - 1.89 (m, 2H), 1.33 (t, *J* = 7.3 Hz, 1H).

### Example 28: Synthesis of compound 238

### Synthesis of 3-((4-fluorophenyl)ethynyl)-N-(imidazo[1,2-a]pyrimidin-6-ylmethyl)-4-(((1-methyl-1H-pyrazol-3-yl)methyl)sulfonyl)benzamide

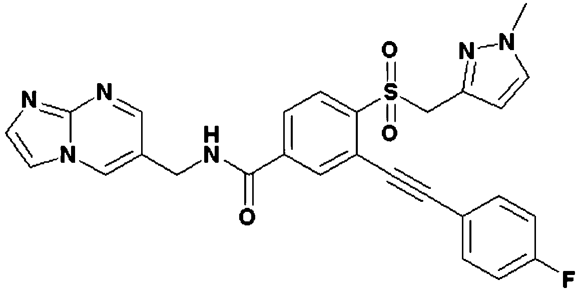

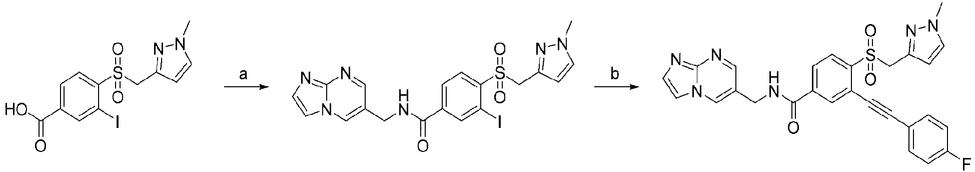

Reagents and conditions: (a) {imidazo[1,2-a]pyrimidin-6-yl}methanamine dihydrochloride, TBTU, TEA, DCM, room temperature (rt), overnight; (b) 1-ethynyl-4-fluorobenzene, PdCl₂(PPh₃)₂, CuI, TEA, ACN, 60°C, one hour (1 h)

### Step 1) Synthesis of N-(imidazo[1,2-a]pyrimidin-6-ylmethyl)-3-iodo-4-(((1-methyl-1H-pyrazol-3-yl)methyl)sulfonyl)benzamide

3-iodo-4-(((1-methyl-1H-pyrazol-3-yl)methyl)sulfonyl)benzoic acid (0.27 g, 0.67 mmol, 1 eq) was dissolved in DCM (6.7 ml, 0.1 M), and then {imidazo[1,2-a]pyrimidin-6-yl}methanamine dihydrochloride (0.147 g, 0.8 mmol, 1.2 eq), TBTU (0.257 g, 0.8 mmol, 1.2 eq), and TEA (0.37 ml, 2.68 mmol, 4 eq) were added thereto and stirred at room temperature overnight. A reaction mixture was diluted in DCM, and then a NaHCO₃ aqueous solution, H₂O, and brine were added thereto, and an organic layer was extracted. An organic layer was dried over MgSO₄, filtered, and concentrated and purified by reverse phase column chromatography (0.1% formic acid in H₂O/ACN) to obtain a target compound (0.092 g, 25.6%).

### Step 2) Synthesis of 3-((4-fluorophenyl)ethynyl)-N-(imidazo[1,2-a]pyrimidin-6-ylmethyl)-4-(((1-methyl-1H-pyrazol-3-yl)methyl)sulfonyl)benzamide

N-(imidazo[1,2-a]pyrimidin-6-ylmethyl)-3-iodo-4-(((1-methyl-1H-pyrazol-3-yl)methyl)sulfonyl)benzamide (0.046 g, 0.08 mmol, 1 eq) was dissolved in ACN (1 ml, 0.1 M), and then 1-ethynyl-4-fluorobenzene (0.014 g, 0.12 mmol, 1.5 eq), PdCl₂(PPh₃)₂ (0.001 g, 0.002 mmol, 0.03 eq), CuI (0.00045 g, 0.002 mmol, 0.03 eq), and TEA (0.016 g, 0.16 mmol, 2 eq) were added and degassed with nitrogen (N₂) gas. A reaction solution was heated to 60°C and stirred for one hour. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.016 g, 37.8%).

¹H NMR (400 MHz, DMSO) δ 9.45 (s, 1H), 8.96 (d, J = 2.2 Hz, 1H), 8.57 (d, J = 2.4 Hz, 1H), 8.28 (d, J = 1.4 Hz, 1H), 8.01 (dd, J = 8.3, 1.6 Hz, 1H), 7.92 (dd, J = 6.6, 4.8 Hz, 2H), 7.76 (dd, J = 8.7, 5.5 Hz, 2H), 7.71 (d, J = 1.1 Hz, 1H), 7.58 (d, J = 2.1 Hz, 1H), 7.37 (t, J = 8.9 Hz, 2H), 6.07 (d, J = 2.1 Hz, 1H), 4.85 (s, 2H), 4.55 (d, J = 4.1 Hz, 2H), 3.69 (s, 3H).

### Example 29: Synthesis of compound 239

### Synthesis of 4-((3-aminobenzyl)sulfonyl)-3-((4-fluorophenyl)ethynyl)-N-(imidazo[1,2-a]pyridin-7-ylmethyl)benzamide

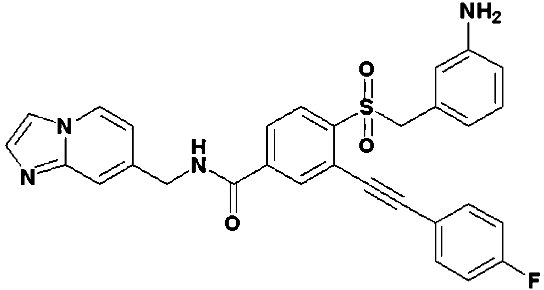

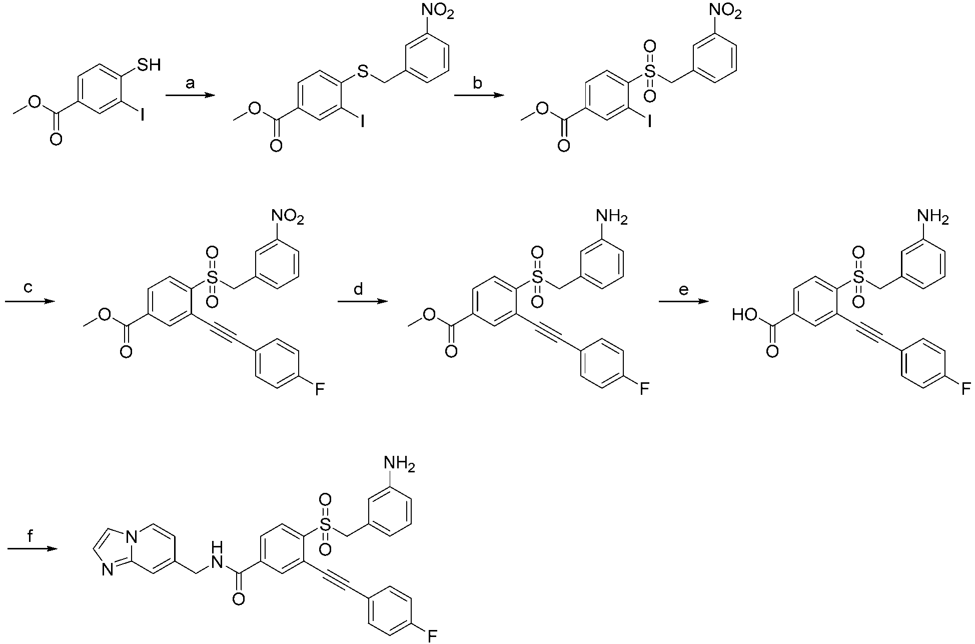

Reagents and conditions: (a) 3-nitrobenzyl chloride, K₂CO₃, DMF, room temperature (rt), two hours (2 h) (b) m-CPBA, DCM, 0°C to room temperature(0°C-rt), overnight; (c) 1-ethynyl-4-fluorobenzene, PdCl₂(PPh₃)₂, CuI, TEA, ACN, 60°C, one hour (1 h); (d) Zn, NH₄Cl, 1,4-dioxane, H₂O, room temperature (rt), 0.5 hours (0.5 h); (e) 1N NaOH (aq), THF, 70°C, overnight; (f) 1-{imidazo[1,2-a]pyridin-7-yl}methanamine dihydrochloride, TBTU, TEA, DCM, room temperature (rt), overnight

### Step 1) Synthesis of methyl 3-iodo-4-((3-nitrobenzyl)thio)benzoate

3-nitrobenzyl chloride (0.291 g, 1.7 mmol, 1 eq) and K₂CO₃ (0.469 g, 3.4 mmol, 2 eq) were dissolved in DMF (3.4 ml, 0.5 M), and then methyl 3-iodo-4-mercaptobenzoate (0.5 g, 1.7 mmol, 1 eq) was added dropwise and stirred at room temperature for two hours. Water was added to a reaction product and extracted with EA. An organic layer was dried over MgSO₄, filtered, concentrated, and purified by column chromatography (silica gel, EA/HEX) to obtain a target compound (0.661 g, 90.5%).

### Step 2) Synthesis of methyl 3-iodo-4-((3-nitrobenzyl)sulfonyl)benzoate

Methyl 3-iodo-4-((3-nitrobenzyl)thio)benzoate (0.661 g, 1.54 mmol, 1 eq) was dissolved in DCM (15.4 ml, 0.1 M), and then m-CPBA (1.063 g, 6.16 mmol, 4 eq) was added dropwise thereto at 0°C. A reaction product was slowly heated to room temperature and stirred overnight. After completion of a reaction, a NaHCO₃ aqueous solution was added and extracted with DCM. An organic layer was dried over MgSO₄, filtered and concentrated to obtain a target compound as a crude product.

### Step 3) Synthesis of methyl 3-((4-fluorophenyl)ethynyl)-4-((3-nitrobenzyl)sulfonyl)benzoate

Methyl 3-iodo-4-((3-nitrobenzyl)sulfonyl)benzoate (0.1 g, 0.22 mmol, 1 eq) was dissolved in ACN (2.2 ml, 0.1 M), and then 1-ethynyl-4-fluorobenzene (0.039 g, 0.33 mmol, 1.5 eq), PdCl₂(PPh₃)₂ (0.005 g, 0.007 mmol, 0.03 eq), CuI (0.001 g, 0.007 mmol, 0.03 eq), and TEA (0.045 g, 0.44 mmol, 2 eq) were added and degassed with nitrogen (N₂) gas. A reaction solution was heated to 60°C and stirred for one hour. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.083 g, 83%).

### Step 4) Synthesis of methyl 4-((3-aminobenzyl)sulfonyl)-3-((4-fluorophenyl)ethynyl)benzoate

Methyl 3-((4-fluorophenyl)ethynyl)-4-((3-nitrobenzyl)sulfonyl)benzoate (0.083 g, 0.18 mmol, 1 eq) was dissolved in 1,4-dioxane : H₂O (3:1 (v/v)) (1.8 ml, 0.1 M), and then Zn (0.058 g, 0.9 mmol, 5 eq) and NH₃Cl (0.048 g, 0.9 mmol, 5 eq) were added thereto and stirred at room temperature for 0.5 hours. After completion of a reaction, a resulting product was filtered and concentrated to obtain a target compound as a crude product.

### Step 5) Synthesis of 4-((3-aminobenzyl)sulfonyl)-3-((4-fluorophenyl)ethynyl)benzoic acid

Methyl 4-((3-aminobenzyl)sulfonyl)-3-((4-fluorophenyl)ethynyl)benzoate (0.076 g, 0.18 mmol, 1 eq) was dissolved in THF (1.8 ml, 0.1 M), and then a 1 N NaOH aqueous solution (0.36 ml, 0.36 mmol, 2 eq) was added thereto, heated to 70°C, and stirred overnight. After completion of a reaction, a reaction solution was concentrated to obtain a target compound as a crude product.

### Step 6) Synthesis of 4-((3-aminobenzyl)sulfonyl)-3-((4-fluorophenyl)ethynyl)-N-(imidazo[1,2-a]pyridin-7-ylmethyl)benzamide

4-((3-aminobenzyl)sulfonyl)-3-((4-fluorophenyl)ethynyl)benzoic acid (0.040 g, 0.098 mmol, 1 eq) was dissolved in DCM (1 ml, 0.1 M), and then 1-{imidazo[1,2-a]pyridin-7-yl}methanamine dihydrochloride (0.019 g, 0.108 mmol, 1.1 eq), TBTU (0.047 g, 0.147 mmol, 1.5 eq), and TEA (0.019 g, 0.196 mmol, 2 eq) were added thereto and stirred at room temperature overnight. After completion of a reaction, water was added to a reaction solution and extracted with DCM. An organic layer was washed with water, separated, dried over MgSO₄, filtered, concentrated and purified by reverse phase column chromatography (0.1% formic acid in H₂O/ACN) to obtain a target compound (0.005 g, 9.47%).

¹H NMR (400 MHz, MeOD) δ 8.38 (d, J = 7.1 Hz, 1H), 8.20 (d, J = 1.4 Hz, 1H), 7.86 - 7.75 (m, 3H), 7.69 (dd, J = 8.8, 5.4 Hz, 2H), 7.50 (s, 1H), 7.44 (s, 1H), 7.19 (t, J = 8.8 Hz, 2H), 6.95 - 6.84 (m, 2H), 6.57 - 6.49 (m, 2H), 6.40 (d, J = 7.6 Hz, 1H), 4.65 (s, 2H), 4.61 (s, 2H).

### Example 30: Synthesis of compound 240

### Synthesis of 3-((4-aminophenyl)ethynyl)-N-(imidazo[1,2-a]pyridin-6-ylmethyl)-4-(((1-methyl-1H-pyrazol-3-yl)methyl)sulfonyl)benzamide

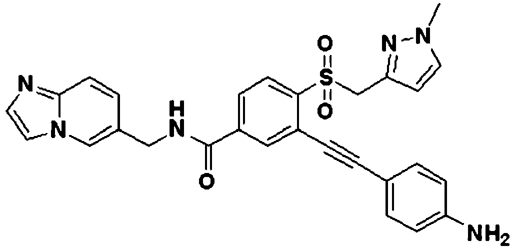

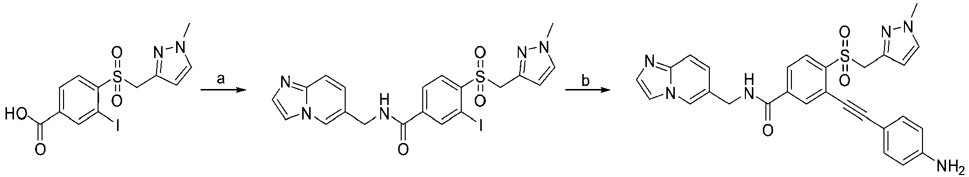

Reagents and conditions: (a) imidazo[1,2-a]pyridin-6-ylmethanamine dihydrochloride, TBTU, TEA, DCM, room temperature (rt), overnight; (b) 4-ethynylaniline, PdCl₂(PPh₃)₂, CuI, TEA, ACN, 60°C, three hours (3 h)

### Step 1) Synthesis of N-(imidazo[1,2-a]pyridin-6-ylmethyl)-3-iodo-4-(((1-methyl-1H-pyrazol-3-yl)methyl)sulfonyl)benzamide

3-iodo-4-(((1-methyl-1H-pyrazol-3-yl)methyl)sulfonyl)benzoic acid (0.1 g, 0.246 mmol, 1 eq) was dissolved in DCM (2.5 ml, 0.1 M), and then imidazo[1,2-a]pyridin-6-ylmethanamine dihydrochloride (0.065 g, 0.295 mmol, 1.2 eq), TBTU (0.158 g, 0.492 mmol, 2 eq) and TEA (0.074 g, 0.739 mmol, 3 eq) were added thereto and stirred at room temperature overnight. After completion of a reaction, water was added and extracted with DCM. An organic layer was washed with water, separated, dried over MgSO₄, filtered, concentrated, and purified by column chromatography (silica gel, MeOH/DCM) to obtain a target compound (0.033 g, 25%).

### Step 2) Synthesis of 3-((4-aminophenyl)ethynyl)-N-(imidazo[1,2-a]pyridin-6-ylmethyl)-4-(((1-methyl-1H-pyrazol-3-yl)methyl)sulfonyl)benzamide

N-(imidazo[1,2-a]pyridin-6-ylmethyl)-3-iodo-4-(((1-methyl-1H-pyrazol-3-yl)methyl)sulfonyl)benzamide (0.033 g, 0.062 mmol, 1 eq) was dissolved in ACN (1 ml, 0.1 M), and then 4-ethylnylaniline (0.0087 g, 0.074 mmol, 1.2 eq), PdCl₂(PPh₄)₂ (0.001 g, 0.002 mmol, 0.03 eq), CuI (0.0003 g, 0.002 mmol, 0.03 eq), and TEA (0.0125 g, 0.124 mmol, 2 eq) were added and degassed with nitrogen (N₂) gas. A reaction solution was heated to 60°C and stirred for three hours. After completion of a reaction, a resulting mixture was filtered through celite and extracted with EA. An organic layer was washed with water, separated, dried over MgSO₄, filtered, concentrated, and purified by column chromatography (silica gel, MeOH/DCM) to obtain a target compound (0.0089 g, 27.4%).

¹H NMR (400 MHz, MeOD) δ 8.53 (s, 1H), 8.10 (d, J = 1.4 Hz, 1H), 7.91 (s, 1H), 7.79 (dt, J = 8.3, 5.0 Hz, 2H), 7.65 (s, 1H), 7.61 (d, J = 9.4 Hz, 1H), 7.50 (d, J = 9.3 Hz, 1H), 7.38 (d, J = 2.2 Hz, 1H), 7.35 (d, J = 8.6 Hz, 2H), 6.65 (d, J = 8.6 Hz, 2H), 6.09 (d, J = 2.2 Hz, 1H), 4.82 (s, 2H), 4.59 (s, 2H), 3.68 (s, 3H).

### Example 31: Synthesis of compound 241

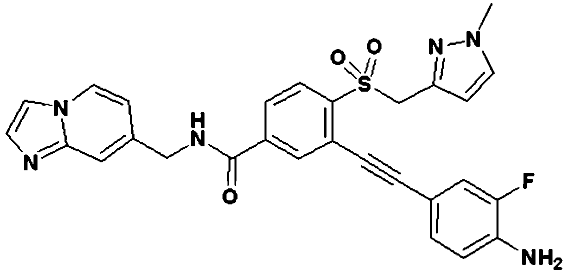

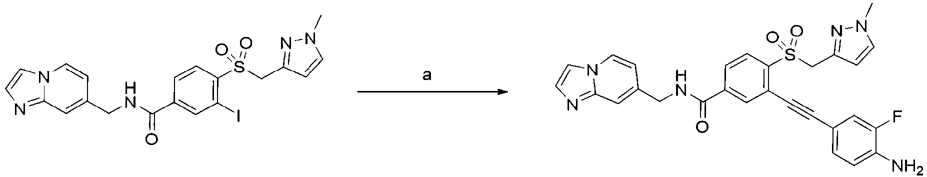

Reagents and conditions: (a) PdCl₂[PPh₃]₂, CuI, TEA, ACN, room temperature (r.t), 16 hours (16 h)

### Synthesis of 3-((4-amino-3-fluorophenyl)ethynyl)-N-(imidazo[1,2-a]pyridin-7-ylmethyl)-4-(((1-methyl-1H-pyrazol-3-yl)methyl)sulfonyl)benzamide

MeCN (1 ml, 0.2 M) was added to N-(imidazo[1,2-a]pyridin-7-ylmethyl)-3-iodo-4-(((1-methyl-1H-pyrazol-3-yl)methyl)sulfonyl)benzamide (0.1 g, 0.2 mmol, 1 eq) and TEA (0.08 ml, 0.6 mmol, 3 eq) was added thereto. PdCl₂[PPh₃]₂ (4 mg, 3 mol%) and CuI (1 mg, 3 mol%) were added to a reaction product and stirred at room temperature for five minutes under a nitrogen atmosphere. Subsequently, 4-ethynyl-2-fluoroaniline (35 mg, 1.2 eq) was added thereto. A reaction mixture was stirred at room temperature for 16 hours and concentrated under reduced pressure. A resulting residue was purified by reverse phase chromatography (water/acetonitrile) to obtain a target compound.

¹H NMR (400 MHz, DMSO) δ 9.43 (t, *J =* 5.8 Hz, 1H), 8.51 (d, *J =* 7.1 Hz, 1H), 8.23 (d, *J =* 1.5 Hz, 1H), 7.89-7.98 (m, 3H), 7.61 (d, *J =* 2.1 Hz, 1H), 7.54 (d, *J =* 1.0 Hz, 1H), 7.46 (s, 1H), 7.39 (dd, *J =* 12.0, 1.7 Hz, 1H), 7.25 (dd, *J* = 8.2, 1.6 Hz, 1H), 6.89 (dd, J= 7.0, 1.5 Hz, 1H), 6.85 - 6.78 (m, 1H), 6.10 (d, J = 2.2 Hz, 1H), 5.88 (s, 2H), 4.87 (s, 2H), 4.54 (d, *J =* 5.7 Hz, 2H), 3.73 (s, 3H).

### Example 32: Synthesis of compound 242

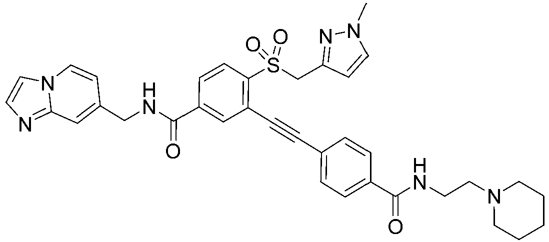

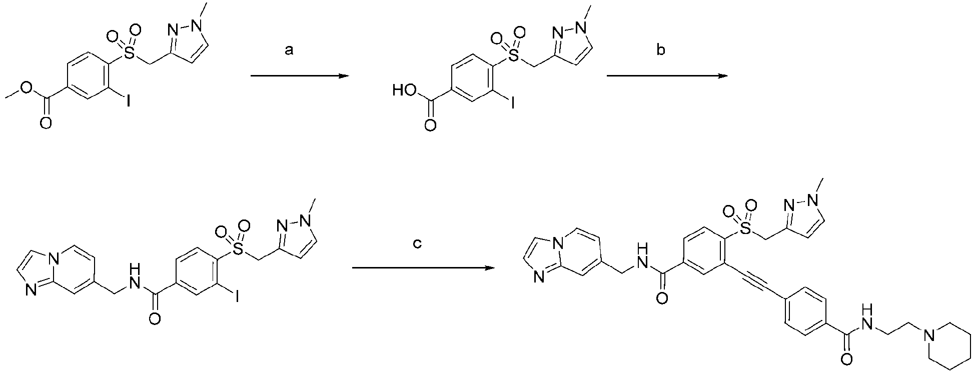

Reagents and conditions: (a) PdCl₂[PPh₃]₂, CuI, TEA, ACN, room temperature (r.t), 16 hours (16 h) (b) NaOH, H₂O, MeOH, 55°C, two hours (2 h) (c) 1-{imidazo[1,2-a]pyridin-7-yl}methanamine dihydrochloride, TBTU, TEA, DCM, 12 hours (12 h)

### Step 1) Synthesis of 3-iodo-4-(((1-methyl-1H-pyrazol-3-yl)methyl)sulfonyl)benzoic acid

MeOH (10 ml, 0.1 M), H₂O (5 ml, 0.2 M), and NaOH (0.1 g, 2.5 mmol, 2.5 eq) were added to methyl 3-iodo-4-(((1-methyl-1H-pyrazol-3-yl)methyl)sulfonyl)benzoate (0.42 g, 1 mmol, 1 eq) and stirred at 80°C for two hours. When a reaction was completed by confirming the reaction through TLC, the pH of a reaction product was adjusted to 1 with 1 N HCl aqueous solution and extracted with EA. An organic layer was washed with water, dried over magnesium sulfate, and concentrated under reduced pressure to obtain a target compound (0.35 g, 86%).

### Step 2) Synthesis of N-(imidazo[1,2-a]pyridin-7-ylmethyl)-3-iodo-4-(((1-methyl-1H-pyrazol-3-yl)methyl)sulfonyl)benzamide

DCM (10 ml) and TBTU (0.42 g, 1 mmol, 1.5 eq) were added to 3-iodo-4-(((1-methyl-1H-pyrazol-3-yl)methyl)sulfonyl)benzoic acid (0.35 g, 0.86 mmol, 1 eq) and stirred at room temperature for 50 minutes. Subsequently, TEA (0.45 ml, 1.3 mmol, 3.7 eq) and 1-{imidazo[1,2-a]pyridin-7-yl}methanamine dihydrochloride (92 mg, 0.42 mmol, 1.2 eq) were added to a reaction product and stirred for 12 hours. A solid produced in a reaction product was filtered to obtain a target compound (0.3 g, 65%).

### Step 3) Synthesis of N-(imidazo[1,2-a]pyridin-7-ylmethyl)-4-(((1-methyl-1H-pyrazol-3-yl)methyl)sulfonyl)-3-((4-((2-(piperidin-1-yl)ethyl)carbamoyl)phenyl)ethynyl)benzamide

MeCN (1.4 ml, 0.2 M) was added to N-(imidazo[1,2-a]pyridin-7-ylmethyl)-3-iodo-4-(((1-methyl-1H-pyrazol-3-yl)methyl)sulfonyl)benzamide (0.15 g, 0.28 mmol, 1 eq) and TEA (0.12 ml, 0.84 mmol, 3 eq) was added thereto. PdCl₂[PPh₃]₂ (6 mg, 3 mol%) and CuI (1.6 mg, 3 mol%) were added to a reaction product and stirred at room temperature for five minutes under a nitrogen atmosphere. Subsequently, 4-ethynyl-N-(2-(piperidin-1-yl)ethyl)benzamide (86 mg, 1.2 eq) was added. A reaction mixture was stirred at room temperature for 16 hours and concentrated under reduced pressure. A resulting residue was purified by prep-LC to obtain a target compound.

¹H NMR (400 MHz, MeOD) δ 8.46 (d, J = 6.1 Hz, 2H), 8.29 (s, 1H), 7.97 (s, 4H), 7.88 - 7.78 (m, 3H), 7.56 (m, 2H), 7.46 (s, 1H), 7.01 (d, J = 6.5 Hz, 1H), 6.19 (s, 1H), 4.86 (s, 2H), 4.69 (s, 2H), 3.80 (s, 2H), 3.74 (m, 3H), 3.31 - 3.25 (m, 2H), 2.68 (s, 3H), 1.90 (s, 4H), 1.71 (s, 2H), 1.31 (s, 1H).

### Example 33: Synthesis of compound 243

### Synthesis of 3-((6-aminopyridin-3-yl)ethynyl)-N-(imidazo[1,2-a]pyridin-7-ylmethyl)-4-(((1-methyl-1H-pyrazol-3-yl)methyl)sulfonyl)benzamide

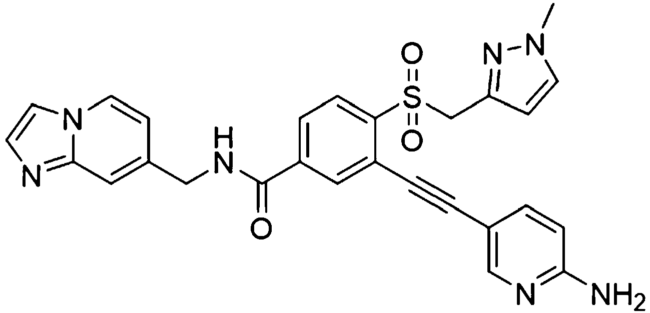

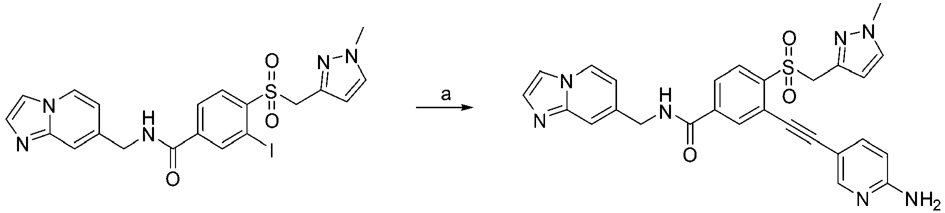

Reagents and conditions (a) 5-ethynylpyridin-2-amine, TEA, CuI, PdCl₂(PPh₃)₂, ACN, reflux, three hours (3 h)

N-(imidazo[1,2-a]pyridin-7-ylmethyl)-3-iodo-4-(((1-methyl-1H-pyrazol-3-yl)methyl)sulfonyl)benzamide (50 mg, 0.09 mmol, 1 eq) was dissolved in ACN (1 ml, 0.1 M), and then TEA (0.025 ml, 0.18 mmol, 2 eq), PdCl₂(PPh₃)₂ (1.89 mg, 0.0027 mmol, 3 mol %), and CuI (0.51 mg, 0.0027 mmol, 3 mol%) were added thereto and stirred at room temperature for five minutes. 5-ethynylpyridin-2-amine (22 mg, 0.186 mmol, 2 eq) was added thereto and stirred under reflux for three hours. After cooling to room temperature, a resulting mixture was concentrated and purified by reverse phase column chromatography (0.1% formic acid in H₂O/ACN) to obtain a title compound (0.004 g, 8.4%).

¹H NMR (400 MHz, MeOD) δ 8.38 (d, J = 7.0 Hz, 1H), 8.21 (d, J = 2.0 Hz, 1H), 8.15 (d, J = 0.9 Hz, 1H), 7.86 (dd, J = 8.9, 4.8 Hz, 2H), 7.78 (s, 1H), 7.66 (dd, J = 8.7, 2.2 Hz, 1H), 7.51 (s, 1H), 7.46 (s, 1H), 7.41 (d, J = 2.1 Hz, 1H), 6.92 (dd, J = 7.0, 1.2 Hz, 1H), 6.58 (d, J = 8.7 Hz, 1H), 6.12 (d, J = 2.2 Hz, 1H), 4.80 (s, 2H), 4.62 (s, 2H), 3.70 (s, 3H).

### Example 34: Synthesis of compound 244

### Synthesis of N-(3-(1H-pyrazol-4-yl)propyl)-3-((4-(dimethylamino)phenyl)ethynyl)-4-(((1-methyl-1H-pyrazol-3-yl)methyl)sulfonyl)benzamide

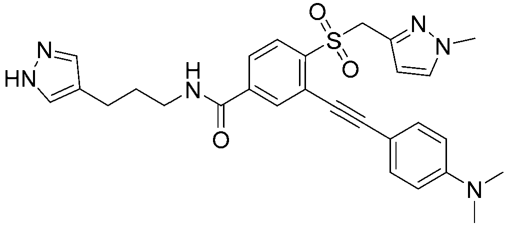

Compound 244 was prepared in substantially the same manner as in the synthesis of compound 235 of Example 25, except that 4-ethynyl-N,N-dimethylaniline was used instead of 4-ethynyl-2-methoxy-1-methylbenzene in Step 3) in comparison with the synthesis of compound 235 of Example 25.

¹H NMR (400 MHz, MeOD) δ 8.11 (d, *J =* 1.5 Hz, 1H), 7.87 (d, *J =* 8.3 Hz, 1H), 7.77 (dd, *J =* 8.3, 1.7 Hz, 1H), 7.54 (d, *J =* 8.9 Hz, 2H), 7.50 (s, 2H), 7.45 (d, *J =* 2.1 Hz, 1H), 6.79 (d, *J =* 8.9 Hz, 2H), 6.15 (d, *J =* 2.2 Hz, 1H), 4.88 (s, 2H), 3.75 (s, 3H), 3.45 (t, *J =* 7.1 Hz, 2H), 3.05 (s, 6H), 2.63 (t, *J =* 7.5 Hz, 2H), 1.97 - 1.88 (m, 2H).

### Example 35: Synthesis of compound 162

### Synthesis of N-(3-(1H-pyrazol-4-yl)propyl)-3-(2-(4-fluorophenyl)thiazol-5-y1)-4-(((1-methyl-1H-pyrazol-3-yl)methyl)sulfonyl)benzamide

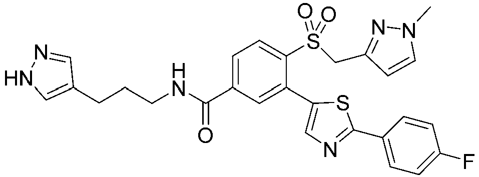

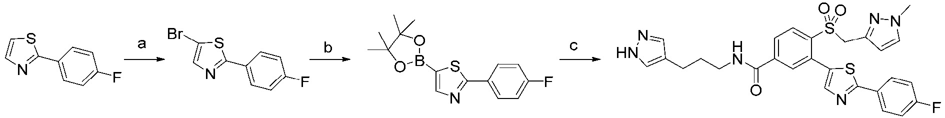

Reagents and conditions: (a)NBS, DMF, room temperature (r.t), one hour (1 h ) (b) bis(pinacolato)diboron, KOAc, PdCl₂(dppf), 1,2-dimethoxyethane, 80°C, 1.5 hours (1.5 h) (c) N-(3-(1H-pyrazol-4-yl)propyl)-3-iodo-4-(((1-methyl-1H-pyrazol-3-yl)methyl)sulfonyl)benzamide, PdCl₂(dppf), K₂CO₃, 1,4-dioxane, H₂O, 100°C, 1 hour (1 h)

### Step 1) Synthesis of 5-bromo-2-(4-fluorophenyl)thiazole

2-(4-fluorophenyl)thiazole (0.27 g, 1.5 mmol, 1 eq) was dissolved in DMF (3 ml), and then N-bromosuccinimide (0.402 g) was added thereto and stirred at room temperature for one hour. After completion of a reaction, water was added thereto, filtered and dried to obtain a target compound as a crude product.

### Step 2) Synthesis of 2-(4-fluorophenyl)-5-(4,4,5,5-tetramethyl-1,3,2-dioxaborolan-2-yl)thiazole

5-bromo-2-(4-fluorophenyl)thiazole (0.05 g, 0.194 mmol, 1 eq) was dissolved in 1,2-dimethoxyethane (0.2 M, 1ml), and then bis(pinacolato)diboron (0.058 g, 0.23 mmol, 1.2 eq), KOAc (0.057 g, 0.582 mmol, 3 eq), and PdCl₂(dppf) (0.014 g, 0.0194 mmol, 0.1 eq) were added thereto and stirred at 80°C for one hour and 30 minutes. After completion of a reaction, a resulting product was filtered through celite and concentrated to obtain a target compound as a crude product.

### Step 3) Synthesis of N-(3-(1H-pyrazol-4-yl)propyl)-3-(2-(4-fluorophenyl)thiazol-5-yl)-4-(((1-methyl-1H-pyrazol-3-yl)methyl)sulfonyl)benzamide

N-(3-(1H-pyrazol-4-yl)propyl)-3-iodo-4-(((1-methyl-1H-pyrazol-3-yl)methyl)sulfonyl)benzamide (0.03 g, 0.058 mmol, 1 eq), 2-(4-fluorophenyl)-5-(4,4,5,5-tetramethyl-1,3,2-dioxaborolan-2-yl)thiazole (0.059 g, 0.194 mmol, 1.5 eq), PdCl₂(dppf) (8.5 mg, 0.011 mmol, 0.2 eq), and K₂CO₃ (17.6 mg, 0.128 mmol, 2.2 eq) were dissolved in 1,4-dioxane : H₂O (3:1 (v/v)) (0.1 M, 0.6 ml), and then stirred at 100°C for one hour. After completion of a reaction, a resulting product was filtered through celite, concentrated, and purified by column chromatography (silica gel, MeOH/DCM) to obtain a target compound.

¹H NMR (400 MHz, MeOD) δ 8.11 - 8.05 (m, 2H), 8.03 (s, 1H), 7.99 (d, J = 1.8 Hz, 1H), 7.62 (d, J = 3.3 Hz, 2H), 7.45 (d, J = 2.2 Hz, 1H), 7.32 - 7.27 (m, 2H), 7.27 - 7.23 (m, 2H), 6.13 (d, J = 2.2 Hz, 1H), 4.41 (s, 2H), 3.74 (s, 3H), 3.43 (t, J = 7.2 Hz, 2H), 2.61 (t, J = 7.5 Hz, 2H), 1.95 - 1.86 (m, 2H).

The reagents may be changed in substantially the same manner as in Example 35 to synthesize compounds 92, 93, 94, 95, 96, 97, 98, 99, 100, 101, 151, 152, 159, 160, 161, 162, 163, 164, 165, 166, 167, and 168.

### Example 36: Synthesis of compound 246

### Synthesis of 3-(3-(2-aminoethyl)-2-methyl-4-oxo-3,4-dihydroquinazolin-7-yl)-N-(imidazo[1,2-a]pyridin-7-ylmethyl)-4-(((1-methyl-1H-pyrazol-3-yl)methyl)sulfonyl)benzamide

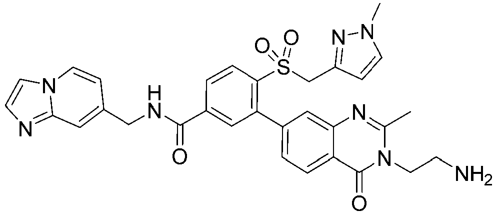

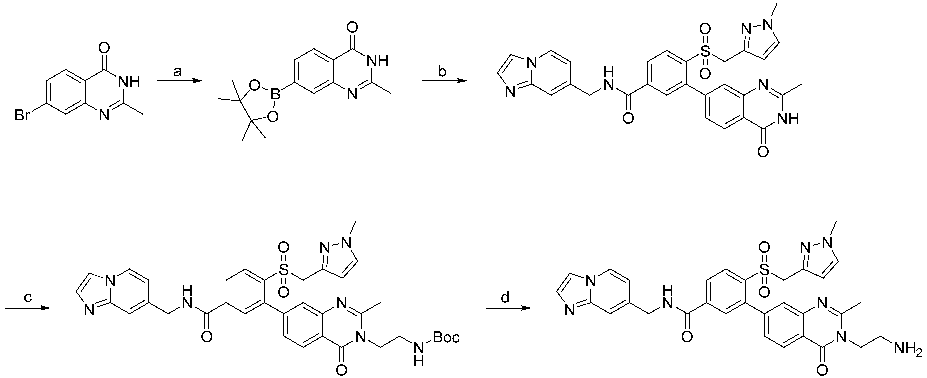

Reagents and conditions: (a) bis(pinacolato)diboron, KOAc, Pd(dppf)Cl₂, 1,4-dioxane, reflux, 0.5 hours (0.5 h); (b) N-(imidazo[1,2-a]pyridin-7-ylmethyl)-3-iodo-4-(((1-methyl-1H-pyrazol-3-yl)methyl)sulfonyl)benzamide, PdCl2(dppf)₂, K₂CO₃, 1,4-dioxane, H2O, reflux, overnight; (c) tert-butyl (2-bromoethyl)carbamate, Cs₂CO₃, DMF, 80°C, overnight; (d) 4 N HCl in dioxane, 4 N HCl in dioxane, room temperature (rt), overnight

### Step 1) Synthesis of 2-methyl-7-(4,4,5,5-tetramethyl-1,3,2-dioxaborolan-2-yl)quinazolin-4(3H)-one

7-bromo-2-methylquinazolin-4(3H)-one (0.5 g, 2.1 mmol, 1.0 eq) was dissolved in 1,4-dioxane (21 ml, 0.1 M), and then bis(pinacolato)diboron (1.06 g, 4.2 mmol, 2 eq), Pd(dppf)Cl₂ (0.307 g, 0.42 mmol, 0.2 eq), and KOAc (0.412 g, 4.2 mmol, 2 eq) were added thereto. A reaction product was heated under reflux and stirred for 0.5 hours. After completion of a reaction, a resulting product was cooled to room temperature and concentrated to obtain a target compound as a crude product.

### Step 2) Synthesis of N-(imidazo[1,2-a]pyridin-7-ylmethyl)-4-(((1-methyl-1H-pyrazol-3-yl)methyl)sulfonyl)-3-(2-methyl-4-oxo-3,4-dihydroquinazolin-7-yl)benzamide

N-(imidazo[1,2-a]pyridin-7-ylmethyl)-3-iodo-4-(((1-methyl-1H-pyrazol-3-yl)methyl)sulfonyl)benzamide (0.2 g, 0.374 mmol, 1 eq) was dissolved in 1,4-dioxane : H₂O (3:1 (v:v), 3 ml, 0.1 M), and then 2-methyl-7-(4,4,5,5-tetramethyl-1,3,2-dioxaborolan-2-yl)quinazolin-4(3H)-one (0.214 g, 0.747 mmol, 2 eq), PdCl₂(dppf)₂ (0.054 g, 0.075 mmol, 0.2 eq) and K₂CO₃ (0.114 g, 0.822 mmol, 2.2 eq) were added thereto. A reaction solution was heated under reflux and stirred overnight. After completion of a reaction, a resulting mixture was filtered through celite and extracted with EA. An organic layer was washed with water, separated, dried over MgSO₄, filtered, concentrated and purified by reverse phase column chromatography (0.1% formic acid in H₂O/ACN) to obtain a target compound (0.019 g, 8.9%).

### Step 3) Synthesis of tert-butyl (2-(7-(5-((imidazo[1,2-a]pyridin-7-ylmethyl)carbamoyl)-2-(((1-methyl-1H-pyrazol-3-yl)methyl)sulfonyl)phenyl)-2-methyl-4-oxoquinazolin-3(4H)-yl)ethyl)carbamate

N-(imidazo[1,2-a]pyridin-7-ylmethyl)-4-(((1-methyl-1H-pyrazol-3-yl)methyl)sulfonyl)-3-(2-methyl-4-oxo-3,4-dihydroquinazolin-7-yl)benzamide (0.019 g, 0.03 mmol, 1.0 eq) was dissolved in DMF (1 ml, 0.03 M), and then tert-butyl (2-bromoethyl) carbamate (0.008 g, 0.036 mmol, 1.2 eq) and Cs₂CO₃ (0.019 g, 0.06 mmol, 2 eq) were added thereto. A reaction product was heated to 80°C and stirred overnight. After completion of a reaction, a resulting mixture was cooled to room temperature and extracted with EA. An organic layer was washed with water, separated, dried over MgSO₄, filtered and concentrated to obtain a target compound as a crude product.

### Step 4) Synthesis of 3-(3-(2-aminoethyl)-2-methyl-4-oxo-3,4-dihydroquinazolin-7-yl)-N-(imidazo[1,2-a]pyridin-7-ylmethyl)-4-(((1-methyl-1H-pyrazol-3-yl)methyl)sulfonyl)benzamide

Tert-butyl (2-(7-(5-((imidazo[1,2-a]pyridin-7-ylmethyl)carbamoyl)-2-(((1-methyl-1H-pyrazol-3-yl)methyl)sulfonyl)phenyl)-2-methyl-4-oxoquinazolin-3(4H)-yl)ethyl)carbamate (0.019 g, 0.03 mmol, 1 eq) was dissolved in 1,4-dioxane (1 ml, 0.03 M), and then 4 N HCl in 1,4-dioxane (0.05 ml, 0.2 mmol, 6.6 eq) was added thereto and stirred at room temperature overnight. After completion of a reaction, a resulting mixture was concentrated and purified by reverse phase column chromatography (0.1% formic acid in H₂O/ACN) to obtain a title compound (0.001 g, 5.4%).

¹H NMR (400 MHz, MeOD) δ 8.75 (s, 1H), 8.29 (d, J = 8.1 Hz, 1H), 8.18 (s, 1H), 8.12 (s, 1H), 8.08 (d, J = 8.0 Hz, 1H), 8.03 (d, J = 8.1 Hz, 1H), 7.99 (s, 1H), 7.92 (s, 1H), 7.85 (s, 1H), 7.69 (s, 1H), 7.59 (d, J = 7.9 Hz, 1H), 7.48 (d, J = 10.3 Hz, 2H), 6.10 (s, 1H), 4.78 (s, 2H), 4.72 (s, 2H), 4.34 - 4.29 (m, 2H), 3.76 (s, 3H), 3.41 (d, J = 6.0 Hz, 2H), 2.73 (s, 3H).

MS(ESI) m/e MH+ 611.08

### Example 37: Synthesis of compound 247

### Synthesis of N-(3-(1H-pyrazol-4-yl)propyl)-3-((4-fluorophenyl)ethynyl)-4-((oxazol-5-ylmethyl)sulfonyl)benzamide

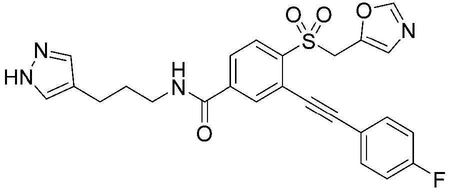

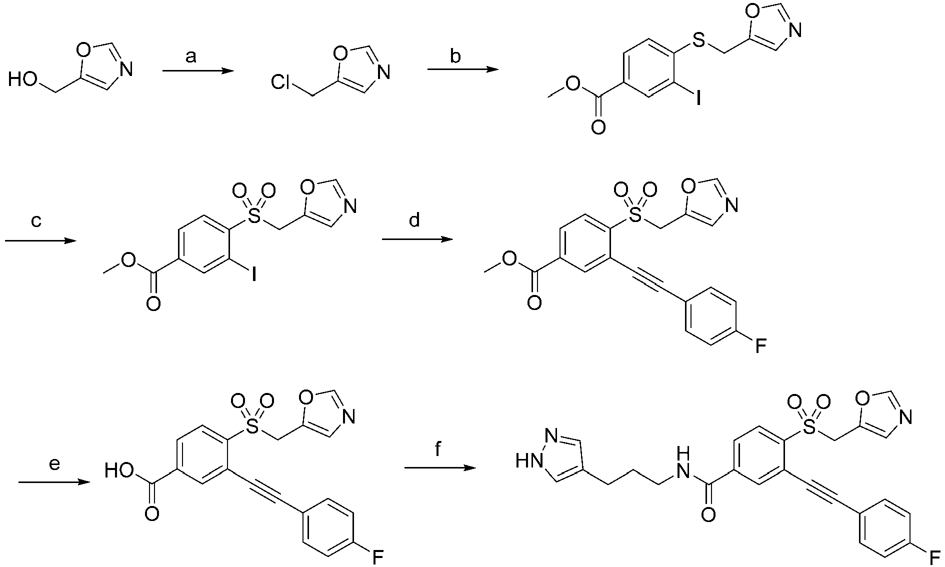

Reagents and conditions: (a) oxazol-5-ylmethanol, SOCl₂, DCM, two hours (2 h) (b) methyl 3-iodo-4-mercaptobenzoate, K₂CO₃, DMF, room temperature (r.t), one hour (1 h) (c) mCPBA, DCM, room temperature (r.t) 16 hours (16 h) (d) PdCl₂[PPh₃]₂, CuI, TEA, ACN, room temperature (r.t), 16 hours (16 h) (e) NaOH, H₂O, MeOH, room temperature (rt), four hours (4 h) (f) 3-(1H-pyrazol-4-yl)propan-1-amine dihydrochloride, TBTU, TEA, DCM, 12 hours (12 h).

### Step 1) Synthesis of 5-(chloromethyl)oxazole

Oxazol-5-ylmethanol (1 g, 10 mmol, 1 eq) was dissolved in DCM (20 ml) and 1.0 M thionyl chloride in dichloromethane (11 ml, 11 eq) was slowly added dropwise in an ice bath. A temperature of a reaction product was raised to room temperature and stirred for two hours. A reaction product was concentrated under reduced pressure to obtain a target compound as a crude product.

### Step 2) Synthesis of methyl 3-iodo-4-((oxazol-5-ylmethyl)thio)benzoate

5-(chloromethyl)oxazole (1 g, 6.5 mmol, 1 eq) was dissolved in DMF (13 ml) and K₂CO₃ (1.79 g, 13 mmol, 2 eq) was added thereto. Then, methyl 3-iodo-4-mercaptobenzoate (1.9 g, 6.5 mmol, 1 eq) was added to a reaction product and stirred for one hour. Water was added to a reaction product and extracted several times with EA. An organic layer was dried over magnesium sulfate and concentrated under reduced pressure. A resulting residue was purified by column chromatography (silica gel, 40% EA/HX) to obtain a target compound (0.22 g, 9%).

### Step 3) Synthesis of methyl 3-iodo-4-((oxazol-5-ylmethyl)sulfonyl)benzoate

Methyl 3-iodo-4-((oxazol-5-ylmethyl)thio)benzoate (0.22 g, 0.59 mmol, 1 eq) was dissolved in DCM (5 ml), and mCPBA (0.3 g, 1.17 mmol, 2 eq) was added thereto and stirred for 16 hours. When a reaction was completed by confirming the reaction through TLC, a resulting product was diluted with water, neutralized with a saturated NaHCO₃ aqueous solution, and extracted several times with DCM. An organic layer was dried over magnesium sulfate and concentrated under reduced pressure. A resulting residue was concentrated under reduced pressure to obtain a target compound as a crude product.

### Step 4) Synthesis of methyl 3-((4-fluorophenyl)ethynyl)-4-((oxazol-5-ylmethyl)sulfonyl)benzoate

MeCN (2 ml, 0.2 M) was added to methyl 3-iodo-4-((oxazol-5-ylmethyl)sulfonyl)benzoate (0.17 g, 0.41 mmol, 1 eq) and TEA (0.074 ml, 0.53 mmol, 0.53 eq) was added thereto. PdCl₂[PPh₃]₂ (8.4 mg, 3 mol%) and CuI (2.3 mg, 3 mol%) were added to a reaction product and stirred at room temperature for five minutes under a nitrogen atmosphere. Subsequently, 4-fluorophenyl-acetylene (0.06 g, 0.49 mmol, 1.2 eq) was added thereto. A reaction mixture was stirred at room temperature for 16 hours and concentrated under reduced pressure. A resulting residue was purified by column chromatography (silica gel, 30% EA/HX) to obtain a target compound (0.05 g, 30.5%).

### Step 5) Synthesis of 3-((4-fluorophenyl)ethynyl)-4-((oxazol-5-ylmethyl)sulfonyl)benzoic acid

MeOH (0.6 ml), H₂O (0.3 ml), and NaOH (0.012 g, 0.31 mmol, 2.5 eq) were added to methyl 3-((4-fluorophenyl)ethynyl)-4-((oxazol-5-ylmethyl)sulfonyl)benzoate (0.05 g, 0.125 mmol, 1 eq) and stirred for four hours. When a reaction was completed by confirming the reaction through TLC, the pH of a reaction product was adjusted to 1 with 1 N HCl aqueous solution and extracted with EA. An organic layer was washed with water, dried over magnesium sulfate, and concentrated under reduced pressure to obtain a target compound.

### Step 6) Synthesis of N-(3-(1H-pyrazol-4-yl)propyl)-3-((4-fluorophenyl)ethynyl)-4-((oxazol-5-ylmethyl)sulfonyl)benzamide

DCM (2 ml), TBTU (0.06 g, 0.19 mmol, 1.5 eq), TEA (0.064 ml, 0.46 mmol, 3.7 eq), and 1-{imidazo[1,2-a]pyridin-7-yl}methanamine hydrochloride (0.03 g, 0.15 mmol, 1.2 eq) were added to 3-((4-fluorophenyl)ethynyl)-4-((oxazol-5-ylmethyl)sulfonyl)benzoic acid (0.05 g, 0.125 mmol, 1 eq) and stirred for 12 hours. A reaction product was concentrated under reduced pressure, and a resulting residue was purified by column chromatography (silica gel, 5-10% MeOH/DCM) to obtain a target compound (0.053 g, 40.8%).

¹H NMR (400 MHz, DMSO) δ 12.55 (s, 1H), 8.89 (t, *J =* 5.6 Hz, 1H), 8.32 (s, 1H), 8.27 (d, *J =* 1.2 Hz, 1H), 8.06 - 8.00 (m, 1H), 7.96-7.94 (m, 1H), 7.74 (dd, *J =* 8.6, 5.5 Hz, 2H), 7.53 (s, 1H), 7.39 (t, *J =* 8.8 Hz, 2H), 7.09 (s, 1H), 5.19 (s, 2H), 3.34 - 3.28 (m, 2H), 2.48 (m, 2H), 1.80 (p, *J =* 7.2 Hz, 2H).

MS(ESI) m/e MH⁺ 493.08

### <Experimental Example>

### Experimental Example 1: NAMPT enzyme inhibition assay

The NAMPT enzyme activity inhibition ability of the compound of the present invention was evaluated using the NAMPT Inhibitor Screening Assay Kit (# 7176-1) from BPS bioscience. Recombinant NAMPT protein was added to each well of a black 96-well plate, and dilution buffer was added to a blank well instead of NAMPT protein. A 5 x concentrated compound solution was added and pre-incubated at room temperature (approximately 22°C) for 30 minutes before the start of the reaction so that the compound and protein might bind before the enzyme reaction. At this time, the concentration of NAMPT protein was adjusted and used according to the enzyme activity of each protein lot within the final concentration range of 4-10 ng/µl, and the test compound was serially diluted at a ratio of 1:3 from 1000 nM to 3 nM, and experiments were conducted with n=2 for about seven concentrations. A 2 x concentrated test buffer containing ATP (final concentration of 20 µM), nicotinamide (final concentration of 20 µM), phosphoribosyl pyrophosphate (PRPP, final concentration of 40 µM), and ethanol (final concentration of 1.5%) was added, and the plate was incubated at 30°C for two hours, after which the fluorescence of a resulting reaction product was measured with excitation of 340 nm and emission of 460 nM. The NAMPT enzyme activity (%) was calculated by subtracting the measurement value of the blank well without NAMPT protein from the measurement value of all wells, and then taking the measurement value of the control well without the compound as 100%. IC₅₀ was calculated using GraphPad Prism 9 software, and the results are shown in table 2.

### <Experimental Example 2> Cellular NAD measurement assay

With regard to HCT-116 cell lines, the decrease in intracellular NAD+ and NADH levels due to the inhibition of NAMPT enzyme activity by compounds was evaluated using the product of NAD/NADH-Glo^{™} Assay (Promega, G9072) from Promega. The cells were incubated using DMEM (Gibco, 11995-065) medium containing 10% FBS. The HCT-116 cells were incubated for 24 hours after being distributed at a density of 2,500 cells per well in a 96-well plate, and were treated with a drug using a serum-free DMEM. First, a compound was dissolved in 100% dimethyl sulfoxide (DMSO), and then diluted in the culture medium so that the final concentration of DMSO might reach 0.5%, and treated with the drug. The final concentration range of the compounds was 0.1 nM to 100 nM. After that, they were further incubated for 24 hours at 37°C and CO₂ 5%. To conduct the NAD/NADH-Glo^{™} Assay, the culture medium of drug-treated cells was removed, and 50 µL of DPBS was treated per well, and left at room temperature for five minutes. The required amount of NAD/NADH-Glo^{™} Detection Reagent (Reconstituted Luciferin Detection Reagent 1 mL, Reductase 5 µL, Reductase Substrate 5 µL, NAD Cycling Enzyme 5 µL, NAD Cycling Substrate 25 µL) was prepared according to the manufacturer's manual and treated in an amount of 50 µL per well. After incubating for two minutes at 400 rpm in a plate shaker for cell lysis, the cells were incubated for 30 minutes at room temperature, and luminescence was measured. NAD+/NADH levels were calculated by subtracting the measurement value of the blank well without cells from the measurement value of all wells and taking the 0.5% DMSO-treated control as 100%. IC₅₀ was calculated using GraphPad Prism 9 software and the results are shown in table 2 below.

**[Table 2]**

| NO | Compound | NAMPT IC₅₀ | NAD, IC50 (HCT-116) |
|---|---|---|---|
| 6 | 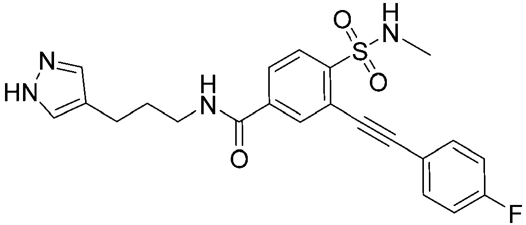 | C | - |
| 1 | 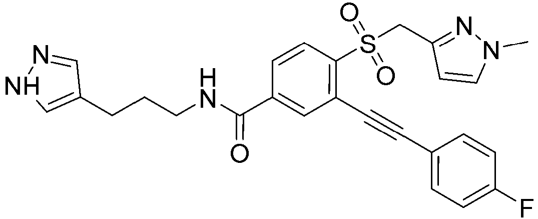 | A | A |
| 2 | 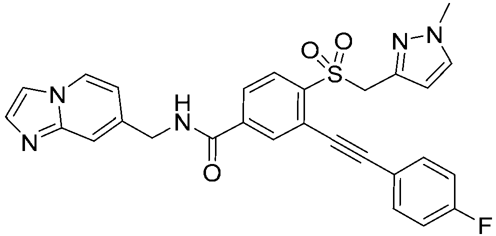 | A | A |
| 7 | 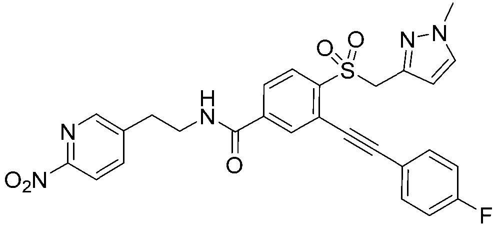 | C | - |
| 8 | 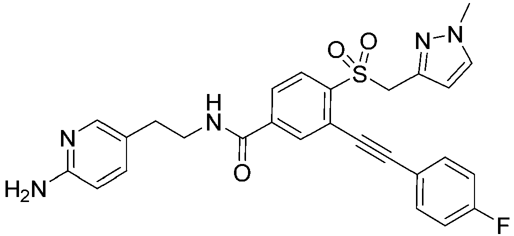 | C | - |
| 3 | 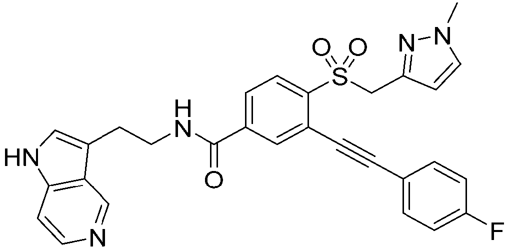 | A | - |
| 4 | 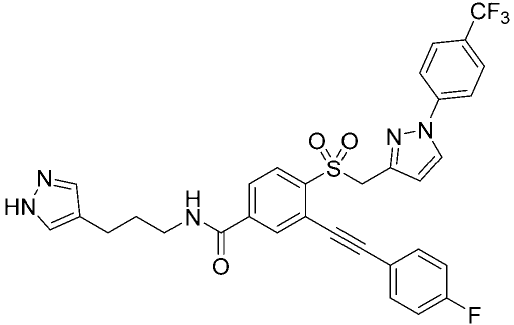 | B | - |
| 5 | 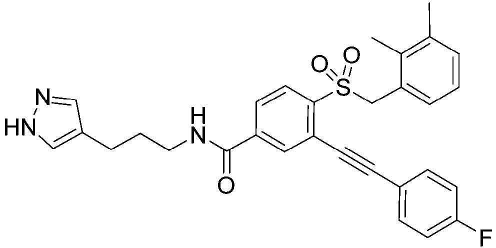 | C | - |
| 9 | 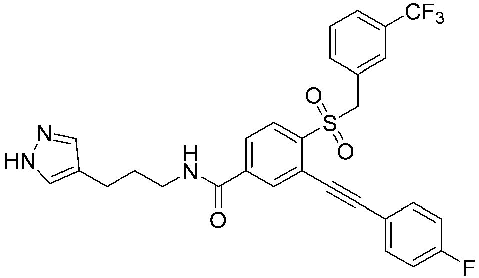 | B | - |
| 10 | 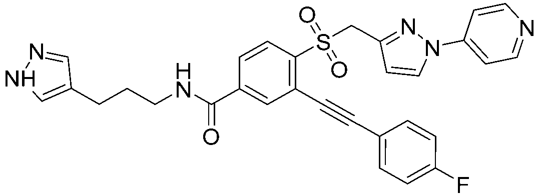 | A | A |
| 242 | 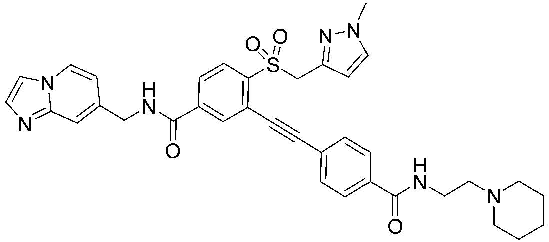 | B | - |
| 193 | 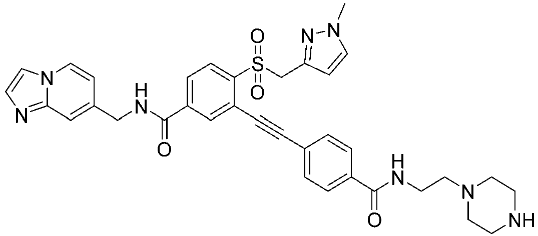 | C | - |
| 115 | 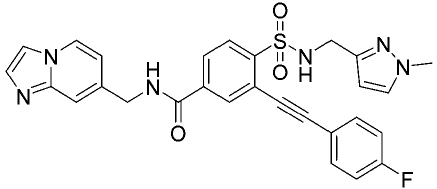 | A | - |
| 245 | 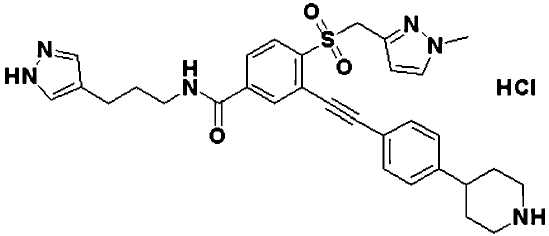 | A | - |
| 228 | 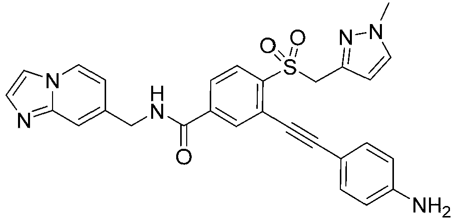 | A | A |
| 229 | 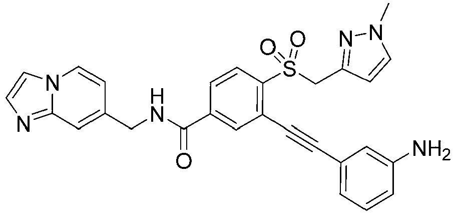 | A | A |
| 230 | 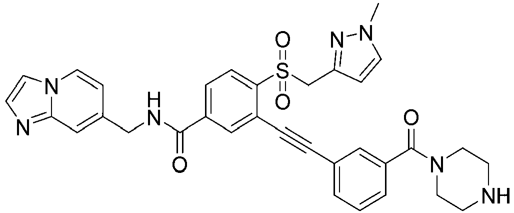 | A | B |
| 231 | 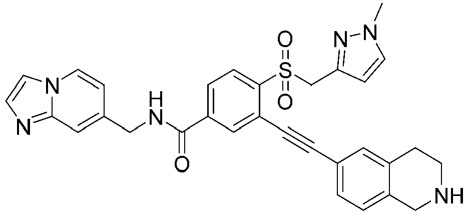 | A | - |
| 232 | 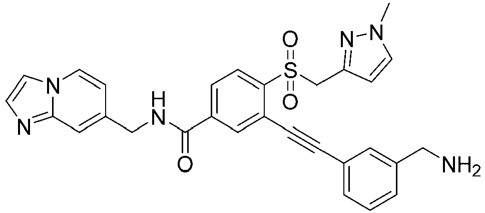 | A | |
| 233 | 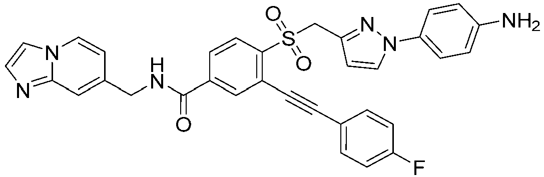 | A | |
| 241 | 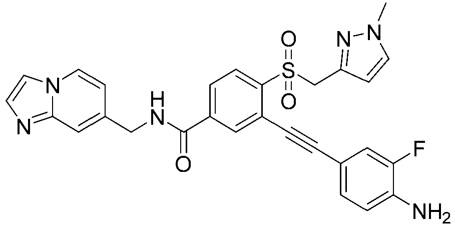 | A | |
| 122 | 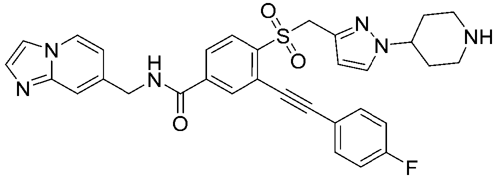 | C | |
| 148 | 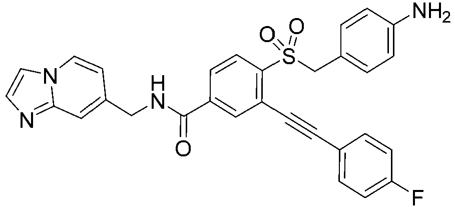 | A | |
| 238 | 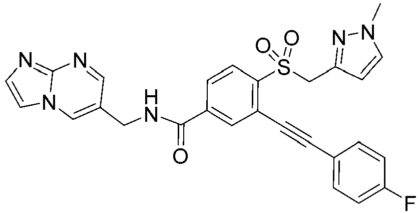 | A | |
| 234 | 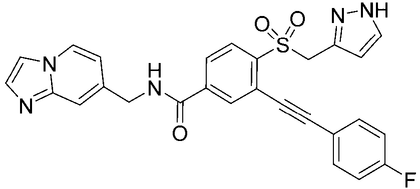 | A | |
| 147 | 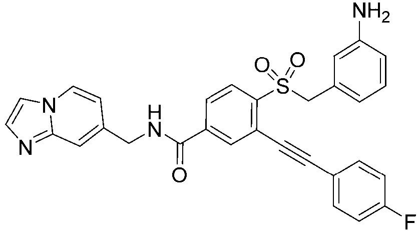 | A | |
| 240 | 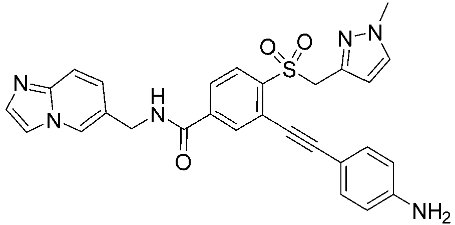 | A | |
| 235 | 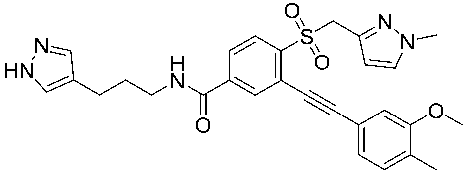 | A | |
| 236 | 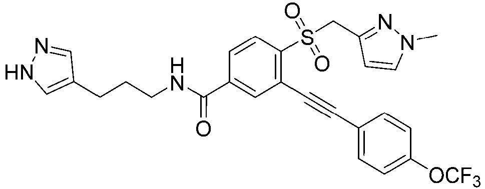 | C | |
| 237 | 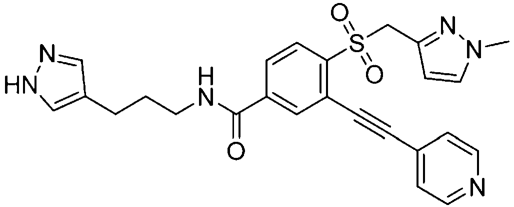 | A | |
| 244 | 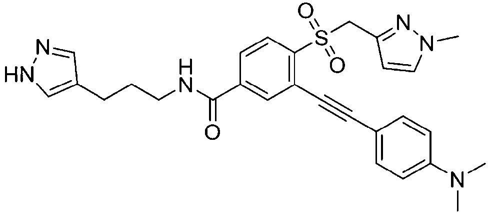 | C | |
| 243 | 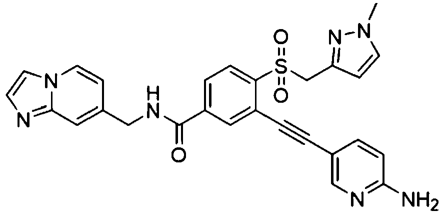 | A | |

In above table, A, B and C are as follows.
NAMPT IC₅₀ A: <0.5 µM (less than 0.5 µM)/ B: <1 µM (0.5 µM or more and less than 1 µM)/ C: ≥1 µM (1 µM or more)
NAD IC₅₀ A: ≤10 nM (10 nM or less) / B: ≤1 µM (more than 10 nM, 1 µM or less)/ C: >1 µM (more than 1 µM)

As can be seen from the above table, it can be found that the compounds of the present invention exhibit excellent inhibitory activity against NAMPT and show sufficient inhibitory activity even at low concentrations.

Thus, the compounds of the present invention may have excellent preventive or therapeutic effects on NAMPT-associated diseases.

As can be seen from the above table, it was found that the compounds of the present invention remarkably inhibit NAD production in HCT-116 cell lines and sufficiently inhibit the concentration of intracellular NAD even at low concentrations.

Thus, the compounds of the present invention may inhibit NAMPT and sufficiently lower the concentration of NAD in cells even at low concentrations, and thus may have an excellent preventive or therapeutic effect on diseases (e.g., cancer) that may be treated by inhibiting NAMPT.

### <Experimental Example 3> Tumor cytotoxicity assay

The anticancer efficacy of the compound of the present invention was evaluated using the MTT analysis method in HCT116 colorectal cancer cell lines. The cells were incubated using DMEM medium containing 10% FBS. The cells were distributed in a 96-well plate at a density of 2,500 cells per well, the HCT116 cells were incubated for 24 hours, and then treated with a drug using a serum free DMEM. First, a compound was prepared in 100% dimethyl sulfoxide (DMSO) at a concentration of 0.02 µM to 200 µM, diluted in the culture medium so that the final concentration of DMSO might reach 0.5%, and treated with the drug so that the final concentration of the compound might range from 0.1 nM to 1000 nM. After that, they were further incubated for 72 hours at 37°C and CO₂ 5% and treated with MTT (Sigma-Aldrich, M2128) to confirm cytotoxicity. Formazan produced from reduction by enzymes in the mitochondria of living cells was measured using Spark^{®} Multimode Microplate Reader TECAN equipment. A cell viability (%) was relatively determined by subtracting a reference 650 nm optical density (O.D.) from 570 nm O.D., in which the 0.5% DMSO-treated control was set as a standard of 100%. The resulting values were plotted as a function of compound concentration using GraphPad Prism 9 software to calculate a CC₅₀ (50% cytotoxic concentration) value, and the results are shown in table 3 below.

**[Table 3]**

| NO | Compound | Cytotoxicity, CC₅₀ |
|---|---|---|
| | | HCT-116 |
| 1 | 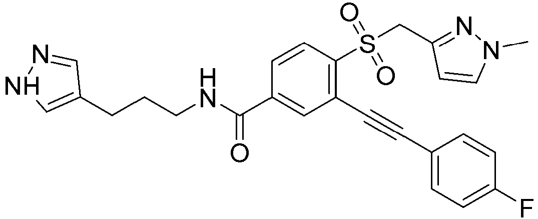 | A |
| 2 | 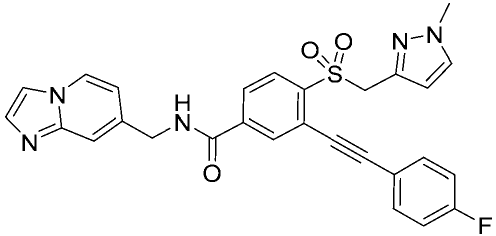 | A |
| 10 | 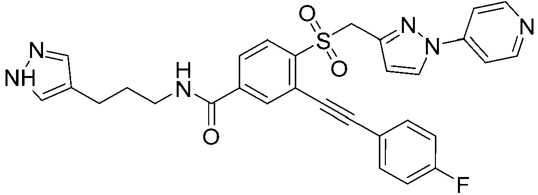 | A |
| 228 | 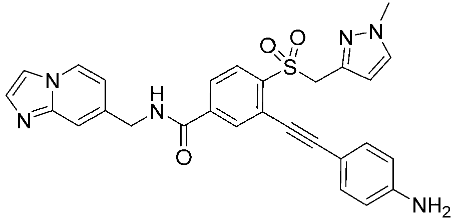 | A |
| 229 | 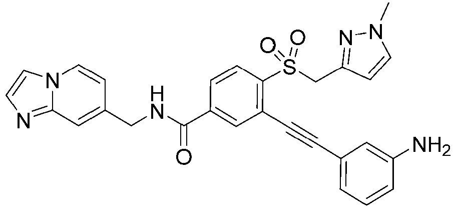 | A |
| 230 | 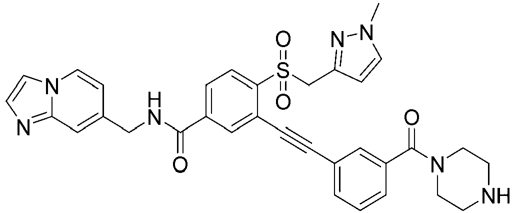 | C |
| 232 | 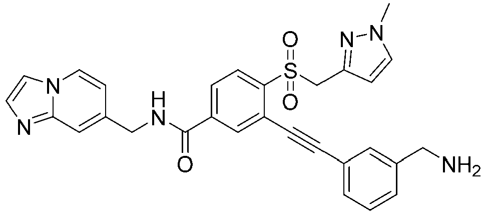 | A |
| 241 | 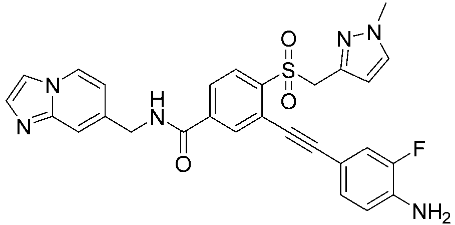 | A |
| 148 | 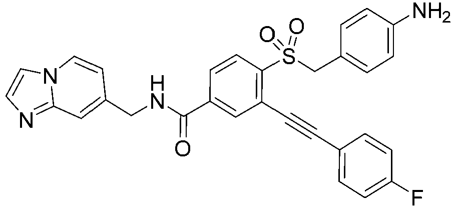 | C |
| 238 | 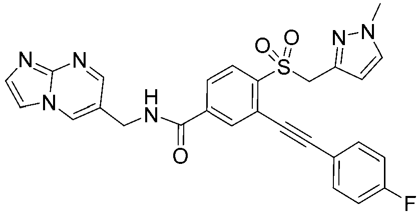 | C |
| 234 | 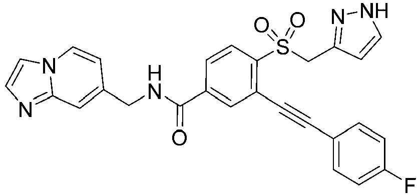 | A |

In above table 3, A, B and C are as follows.
A : CC₅₀ ≤30 nM (30 nM or less)
B: 30 nM < CC₅₀ ≤100 nM (more than 30 nM and 100 nM or less)
C: 100 nM <CC₅₀ ≤1 µM (more than 100 nM and 1 µM or less)

As can be seen from the above table, it can be found that the compounds of the present invention exhibit excellent cytotoxicity even at low concentrations against colorectal cancer cell lines.

Thus, it can be seen that the compounds of the present invention have excellent preventive or therapeutic effects against various diseases, such as cancer, by inhibiting NAMPT.

The present invention has been described with reference to one exemplary embodiment of the present invention, but it will be understood by those skilled in the art that the present invention may be variously changed and modified without departing from the spirit and field of the present invention, as described in the following scope of patent claims.

## Claims

1. A benzamide derivative compound represented by formula I below, a stereoisomer thereof, a pharmaceutically acceptable salt of them, or a hydrate or solvate of them: 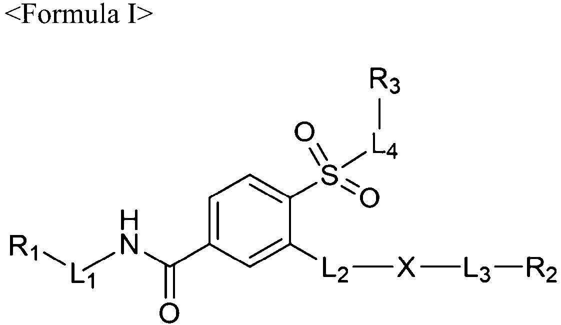 wherein in above formula I,
R₁ is 6- to 14-membered aryl; C2-C8 heterocycloalkenyl; or 5- to 12-membered heteroaryl, in which -H of said R₁ is unsubstituted or at least one H thereof is substituted with halogen, NRaRb, NO₂ or OH;
L₁ is C1-C6 alkylene;
L₂ is a single bond; or C2-C6 alkynylene;
X is 6- to 14-membered arylene; C2-C8 heterocycloalkenylene; or 5- to 12-membered heteroarylene, in which at least one -H of said X is unsubstituted or at least one H of X is substituted with C1-C6 alkyl, CF₃, halogen, -C1-C6 alkoxy, or OH, and -CH₂- of said X is unsubstituted or at least one -CH₂- of X is each independently substituted with -(C=O)-;
L₃ is a single bond; C1-C6 alkylene; C2-C6 alkenylene; C=O; (C=O)NRc; (C=O)NRd-(C1-C6 alkylene); NRy(C=O)-(C1-C6 alkylene); or O(C=O)-(C1-C6alkylene);
R₂ is H, halogen, C1-C6 alkyl, C1-C6 alkoxy, C3-C8 cycloalkyl, C1-C8 heterocycloalkyl, -NRz₁Rz₂, 6- to 14-membered aryl, or 5- to 12-membered heteroaryl, in which -H of said R₂ is unsubstituted or at least one -H thereof is substituted with C1-C6 alkyl, OH, COOH, (C1-C6 alkylene)-NReRf, NRgRh, NO₂, CF₃, (C=O)NRiRj or halogen;
L₄ is a single bond, C1-C6 alkylene, -NRm-(C1-C6 alkylene)-, or NRn;
R₃ is H, C1-C6 alkyl, C3-C8 cycloalkyl, 6- to 14-membered aryl, C1-C8 heterocycloalkyl, C2-C8 heterocycloalkenyl, 5- to 12-membered heteroaryl, 6- to 14-membered arylene-C1-C8 heterocycloalkyl, 6- to 14-membered arylene-5- to 12-membered heteroaryl, 5- to 12-membered heteroarylene-6- to 14-membered aryl, 5- to 12-membered heteroarylene-C1-C8 heterocycloalkyl or 5- to 12-membered heteroarylene-5- to 12-membered heteroaryl, in which -H of said R₃ is unsubstituted or at least one -H thereof is substituted with C1-C6 alkyl, C1-C6 alkoxy, phenoxy, OH, (C1-C6 alkylene)-OH, COOH, (C1-C6 alkylene)-COOH, (C1-C6 alkylene)-CN, NRoRp, (C1-C6 alkylene)-NRqRr, (C=O)-(C1-C6 alkyl), (C=O)-(C3-C8 cycloalkyl), (C=O)-(6- to 14-membered aryl), 6- to 14-membered aryl, 6- to 14-membered heteroaryl, 6- to 14-membered heterocycloaryl, halogen-substitutable (C=O)NRsRt, (C=O)-(C1-C6 alkylene)-NRuRv, NRwRx, (C=O)O-(C1-C6 alkyl), NO₂, CHF₂, CF₃, OCF₃ or halogen, and -CH₂- of heterocycloalkenyl of said R₃ is unsubstituted or substituted with -(C=O)-; and
said Ra to Rj, Rm to Ry, Rz₁ and Rz₂ are each independently H or C1-C6 alkyl.

2. The benzamide derivative compound represented by above formula I, the stereoisomer thereof, the pharmaceutically acceptable salt of them, or the hydrate or solvate of them of claim 1, wherein
said R₁ is 6- to 14-membered aryl; C2-C8 heterocycloalkenyl including at least one heteroatom or heterofunctional group selected from the group consisting of N, O, S, S(=O), and S(=O)₂; or 5- to 12-membered heteroaryl including at least one heteroatom selected from the group consisting of N, O, and S, in which -H of said R₁ is unsubstituted or at least one H thereof is each independently substituted with halogen, -NRaRb, -NO₂, or -OH;
Li is C1-C6 alkylene;
L₂ is a single bond; or C2-C6 alkynylene;
X is 6- to 14-membered arylene; C2-C8 heterocycloalkenylene including at least one heteroatom or heterofunctional group selected from the group consisting of N, O, S, S(=O), and S(=O)₂; or 5- to 12-membered heteroarylene including at least one heteroatom selected from the group consisting of N, O and S, in which when -H of X is unsubstituted or said X is 6- to 14-membered arylene or C2-C8 heterocycloalkenylene, at least one -H thereof is each independently substituted with C1-C6 alkyl, -CF₃, halogen, -C1-C6 alkoxy or OH, and when said X is C2-C8 heterocycloalkenylene, -CH₂- is unsubstituted or at least one - CH₂- is substituted with -(C=O)-;
L₃ is a single bond; C1-C6 alkylene; C2-C6 alkenylene; -C=O; - C(=O)NRc; -C(=O)NRd-(C1-C6 alkylene); -NRyC(=O)-(C1-C6 alkylene); - NHC(=O); or -OC(=O)-(C1-C6alkylene);
R₂ is H; halogen; -NRz₁Rz₂; C1-C6 alkyl; C1-C6 alkoxy; C3-C8 cycloalkyl; C1-C8 heterocycloalkyl including at least one heteroatom or heterofunctional group selected from the group consisting of N, O, S, SO and SO₂; 6- to 14-membered aryl, or 5- to 12-membered heteroaryl including at least one heteroatom selected from the group consisting of N, O and S, in which -H of said R₂ is unsubstituted or at least one -H of R₂ is each independently substituted with -C1-C6 alkyl, -C1-C6 alkoxy, -OH, -COOH, -(C1-C6 alkylene)-NReRf, -NRgRh, -NO₂, -CF₃, -C(=O)NRiRj or halogen;
L₄ is a single bond; -C1-C6 alkylene-; -NRm-(C1-C6 alkylene)-; or -NRn-, in which -H of said L4 is unsubstituted or at least one -H of R₂ is each independently substituted with -C1-C6 alkyl;
R₃ is H; C1-C6 alkyl; C3-C8 cycloalkyl; 6- to 14-membered aryl; C1-C8 heterocycloalkyl including at least one heteroatom or heterofunctional group selected from the group consisting of N, O, S, SO and SO₂; C2-C8 heterocycloalkenyl including at least one heteroatom or heterofunctional group selected from the group consisting of N, O, S, SO and SO₂; 5- to 12-membered heteroaryl including at least one heteroatom selected from the group consisting of N, O and S; 6- to 14-membered arylene-C1-C8 heterocycloalkyl (in which said heterocycloalkyl includes at least one heteroatom or heterofunctional group selected from the group consisting of N, O, S, SO and SO₂); 6- to 14-membered arylene-5- to 12-membered heteroaryl (in which said heteroaryl includes at least one heteroatom selected from the group consisting of N, O and S); 5- to 12-membered heteroarylene-6- to 14-membered aryl (in which said heteroarylene includes at least one heteroatom selected from the group consisting of N, O and S); 5- to 12-membered heteroarylene-C1-C8 heterocycloalkyl (in which said heteroarylene includes at least one heteroatom selected from the group consisting of N, O and S, and said heterocycloalkyl includes at least one heteroatom or heterofunctional group selected from the group consisting of N, O, S, SO and SO₂); or 5- to 12-membered heteroarylene-5- to 12-membered heteroaryl (in which said heteroarylene or heteroaryl includes at least one heteroatom selected from the group consisting of N, O and S), in which -H of said R₃ is unsubstituted or at least one H of R₃ is each independently substituted with -C1-C6 alkyl, -C1-C6 alkoxy, -phenoxy, -OH, -(C1-C6 alkylene)-OH, -COOH, -(C1-C6 alkylene)-COOH, (C1-C6 alkylene)-CN, -NRoRp, (C1-C6 alkylene)-NRqRr, -C(=O)-(C1-C6 alkyl), -C(=O)-(C3-C8 cycloalkyl), -C=(O)-(6- to 14-membered aryl), -NO₂, -CHF₂, -CF₃, -OCF₃, 6- to 14-membered aryl, 6- to 14-membered heteroaryl including at least one heteroatom selected from the group consisting of N, O and S, 6- to 14-membered heterocycloaryl including at least one heteroatom or heterofunctional group selected from the group consisting of N, O, S, SO and SO₂, -C(=O)NRsRt (in which -H of -C(=O)NRsRt is unsubstituted or at least one -H thereof is each independently substituted with halogen), -C(=O)-(C1-C6 alkylene)-NRuRv, -NRwRx, or halogen, and when said R₃ is heterocycloalkenyl, -CH₂- is unsubstituted or at least one -CH₂- is each independently substituted with -(C=O)-; and
said Ra to Rj, Rm to Ry, Rz₁ and Rz₂ are each independently H or C1-C6 alkyl in each case.

3. The benzamide derivative compound represented by above formula I, the stereoisomer thereof, the pharmaceutically acceptable salt of them, or the hydrate or solvate of them of claim 1, wherein R₁ is 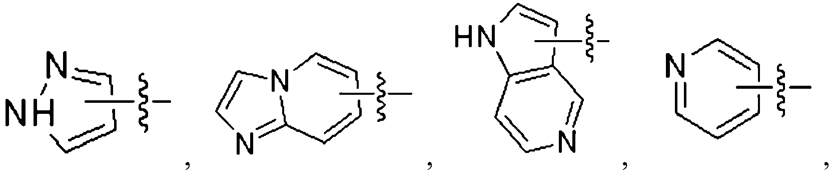 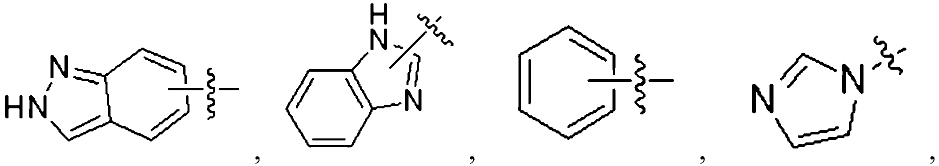 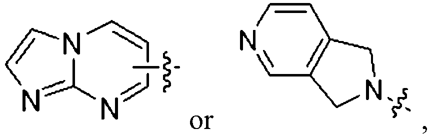 and
-H of R₁ is unsubstituted or at least one H of R₁ is each independently substituted with halogen, -NRaRb, -NO₂ or -OH, in which Ra or Rb is each independently -H or C1-C6 alkyl.

4. The benzamide derivative compound represented by above formula I, the stereoisomer thereof, the pharmaceutically acceptable salt of them, or the hydrate or solvate of them of claim 3, wherein -H of R₁ is unsubstituted or at least one - H of R₁ is each independently substituted with -NH₂, -NO₂, -NHCH₃, -N(CH₃)₂ or -OH.

5. The benzamide derivative compound represented by above formula I, the stereoisomer thereof, the pharmaceutically acceptable salt of them, or the hydrate or solvate of them of claim 1, wherein L₁ is methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), sec-butylene (-CHCH₃CH₂CH₂-), isobutylene (-CH₂CHCH₃CH₂-), n-butylene (-CH₂CH₂CH₂CH₂-), sec-butylene (-CHCH₃CH₂CH₂-), tert-butylene (-CCH₃CH₃CH₂-), n-pentylene (-CH₂CH₂CH₂CH₂CH₂-), or n-hexylene (-CH₂CH₂CH₂CH₂CH₂CH₂-).

6. The benzamide derivative compound represented by above formula I, the stereoisomer thereof, the pharmaceutically acceptable salt of them, or the hydrate or solvate of them of claim 1, wherein L₂ is a single bond or ethynylnene

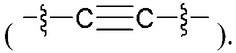

7. The benzamide derivative compound represented by above formula I, the stereoisomer thereof, the pharmaceutically acceptable salt of them, or the hydrate or solvate of them of claim 1 or 2, wherein X is 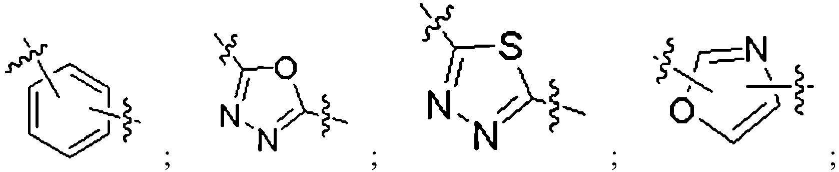 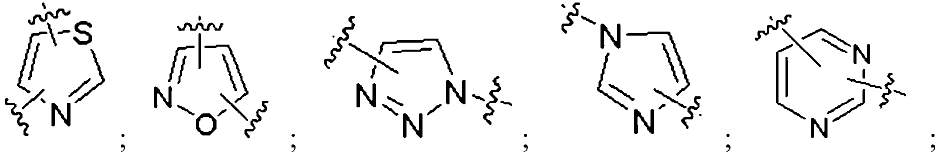 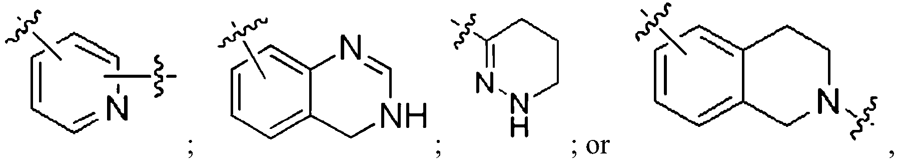 in which -H of said X is unsubstituted or when X is 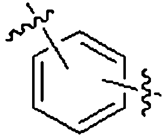 or 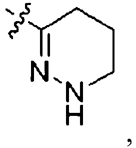 -H is unsubstituted or at least one -H thereof is each independently substituted with C1-C6 alkyl, halogen, -C1-C6 alkoxy, -OH, or -CF₃, and when X is 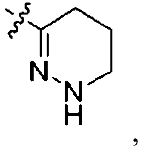 -CH₂- is unsubstituted or at least one -CH₂- is each independently substituted with -(C=O)-.

8. The benzamide derivative compound represented by above formula I, the stereoisomer thereof, the pharmaceutically acceptable salt of them, or the hydrate or solvate of them of claim 1, wherein L₃ is a single bond; C1-C6 alkylene; C2-C6 alkenylene; -(C=O)-; -(C=O)-NRc-; -C(=O)NRd-(C1-C6 alkylene)-; -NRyC(=O)-(C1-C6 alkylene); -NHC(=O); or -O(C=O)-(C1-C6 alkylene), in which Rc, Rd or Ry is each independently -H or C1-C6 alkyl.

9. The benzamide derivative compound represented by above formula I, the stereoisomer thereof, the pharmaceutically acceptable salt of them, or the hydrate or solvate of them of claim 8, wherein L₃ is a single bond; -(CH₂)-; -(CH₂)₂-; -(CH₂)₃-; -(CH₂)₄-; -(CH₂)₅-; -(C=O)-NH(CH₂)n; -NH(C=O)-; - NH(C=O)-(CH₂)m; -(C=O)NH-; -(CH=CH)-CH₂-; -O(C=O)CH₂; or -(C=O)-, in which n and m are each independently 1, 2, 3 or 4.

10. The benzamide derivative compound represented by above formula I, the stereoisomer thereof, the pharmaceutically acceptable salt of them, or the hydrate or solvate of them of claim 1, wherein said R₂ is H; halogen; C1-C6 alkyl; C1-C6 alkoxy; -OH; -NH₂; 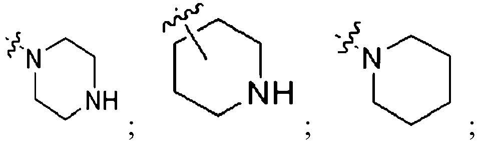 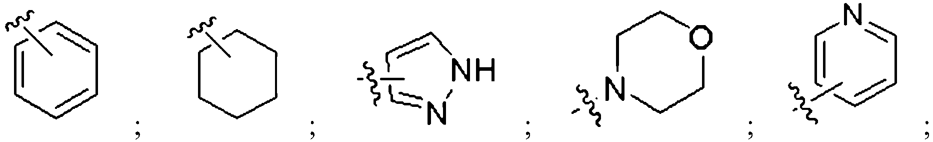 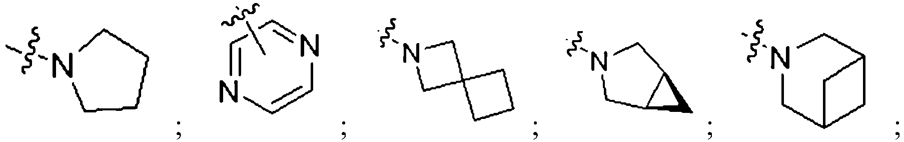 or 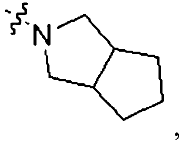 in which -H of said R₂ is unsubstituted or at least one -H of R₂ is each independently substituted with halogen, -C1-C6 alkoxy, -NH₂, -OH, -CF₃, -NO₂, -C1-C6 alkyl, -(CH₂)₃-NH₂, -(CH₂)₂-NH₂, -(CH₂)-NH₂, -(C=O)NH₂, -NHCH₃, - NHCH(CH₃)₂, or -COOH.

11. The benzamide derivative compound represented by above formula I, the stereoisomer thereof, the pharmaceutically acceptable salt of them, or the hydrate or solvate of them of claim 1 or 2, wherein L₄ is a single bond; - C1-C6 alkylene-; -NRm-(C1-C6 alkylene)-; or -NRn-, in which Rm and Rn are each independently -H or C1-C6.

12. The benzamide derivative compound represented by above formula I, the stereoisomer thereof, the pharmaceutically acceptable salt of them, or the hydrate or solvate of them of claim 11, wherein L₄ is a single bond; -(CH₂)-; -(CH₂)₂-; -(CH₂)₃-; -(CH₂)₄-; -(CH₂)₅-; -NH-; -NHCH₂-; -NH(CH₂)₂-; - NH(CH₂)₃-; -CH(CH₃)-; -CH₂NH-; -(CH₂)₂NH-; or -(CH₂)₃NH-, in which -H of said L₄ is unsubstituted or at least one -H of L₄ is each independently substituted with -C1-C6 alkyl.

13. The benzamide derivative compound represented by above formula I, the stereoisomer thereof, the pharmaceutically acceptable salt of them, or the hydrate or solvate of them of claim 1, wherein R₃ is
H; C1-C6 alkyl; 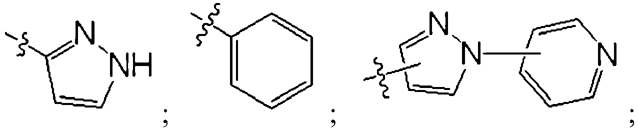 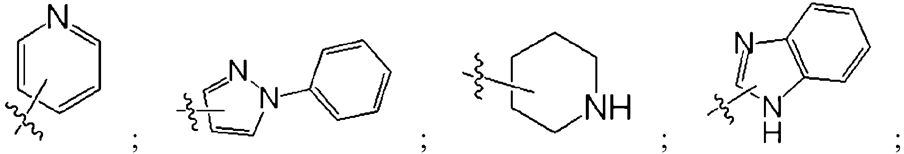 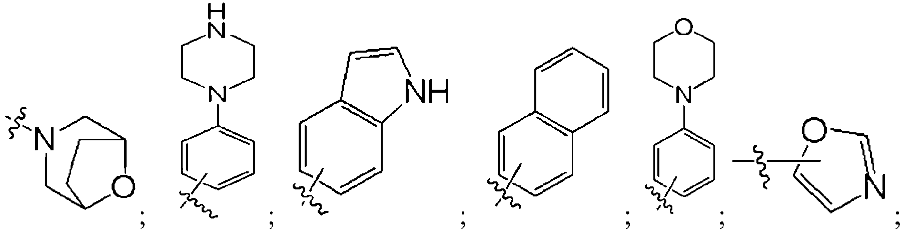 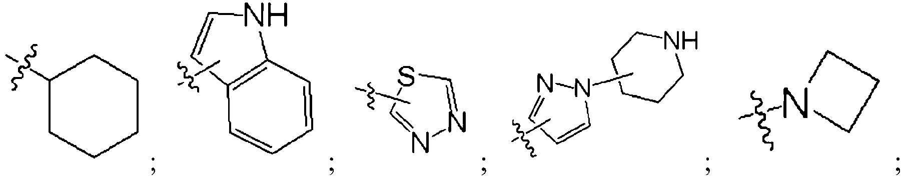 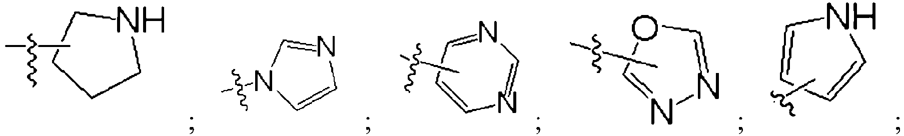 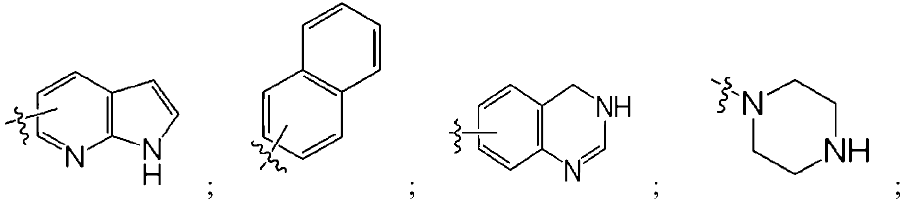 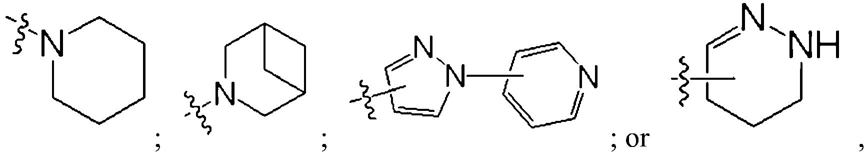
in which -H of said R₃ is unsubstituted or at least one H of R₃ is each independently substituted with -C1-C6 alkyl, -C1-C6 alkoxy, -phenoxy, -OH, - (C1-C6 alkylene)-OH, -COOH, -(C1-C6 alkylene)-COOH, (C1-C6 alkylene)-CN, -NRoRp, (C1-C6 alkylene)-NRqRr, -C(=O)-(C1-C6 alkyl), -C(=O)-(C3-C8 cycloalkyl), -C=(O)-(6- to 14-membered aryl), -NO₂, -CHF₂, -CF₃, -OCF₃, - C(=O)NRsRt (in which -H of -C(=O)NRsRt is unsubstituted or at least one -H thereof is each independently substituted with halogen), -C(=O)-(C1-C6 alkylene)-NRuRv, -NRwRx, or halogen, and when said R₃ is heterocycloalkenyl, -CH₂- is unsubstituted or at least one -CH₂- is each independently substituted with -(C=O)-, in which Ro, Rp, Rq, Rr, Rs, Rt, Ru, Rv, Rw or Rx is each independently H or C1-C6 alkyl in each case.

14. The benzamide derivative compound represented by above formula I, the stereoisomer thereof, the pharmaceutically acceptable salt of them, or the hydrate or solvate of them of claim 13, wherein -H of said R₃ is unsubstituted or at least one -H of R₃ is each independently substituted with -C1-C6 alkyl -C1-C6 alkoxy, -phenoxy, -OH, -CH₂F, -CHF₂, -CF₃, -OCF₃, halogen, - (C=O)OCH₂CH₃, -(C=O)CH₃, -(C=O)CH(CH₃)₂, -(C=O)CHCH₃NH₂, -NH₂, - NHCH₃, -N(CH₃)₂, -COOH, -(C=O)NH₂, -(C=O)NHCH₃, -(C=O)N(CH₃)₂, -NO₂, -(C=O)NHCH₂CF₃, -CH(CH₃)OH, -CH₂(CH₃)OH, -CH₂CN, -CH(CH₃)CN, - C(CH₃)CH₂CN, -(CH₂)₂NH₂, -(CH₂)NH₂, -(CH₂)₃NH₂, -(CH₂)₄NH₂, -(CH₂)₂OH, -(CH₂)OH, -(CH₂)₃OH, -(CH₂)₄OH, -(CH₂)₂COOH, -(CH₂)COOH, - (CH₂)₃COOH, 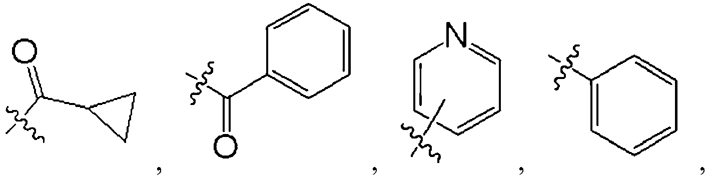 or 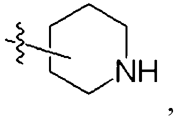 and when said R₃ is 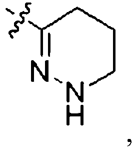 -CH₂- is unsubstituted or at least one -CH₂- is substituted with -(C=O).

15. The benzamide derivative compound represented by above formula I, the stereoisomer thereof, the pharmaceutically acceptable salt of them, or the hydrate or solvate of them of claim 1, wherein above formula I is a compound represented by formula I-1 below: 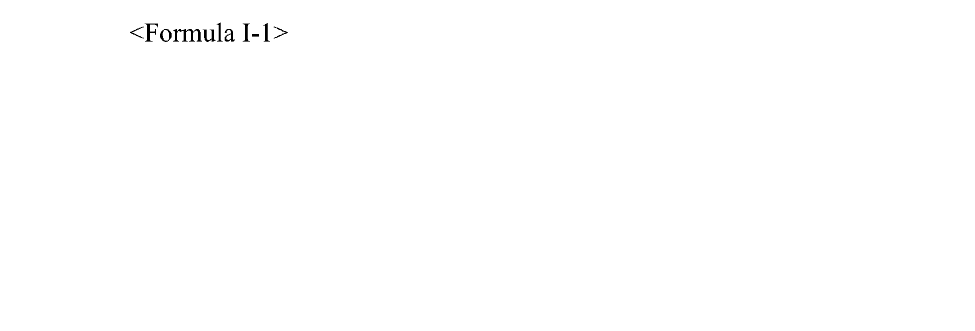 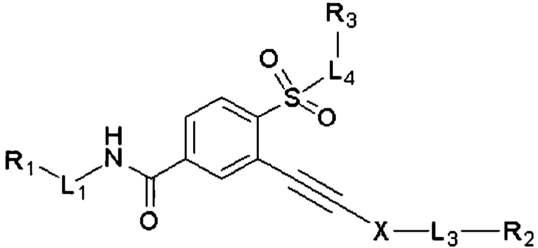 wherein in above formula I-1, R₁, L₁, X, L₃, R₂, L₄, R₃ are as defined in claim 1.

16. The benzamide derivative compound represented by above formula I, the stereoisomer thereof, the pharmaceutically acceptable salt of them, or the hydrate or solvate of them of claim 15, wherein said R₁ is 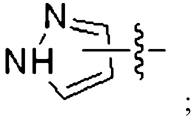 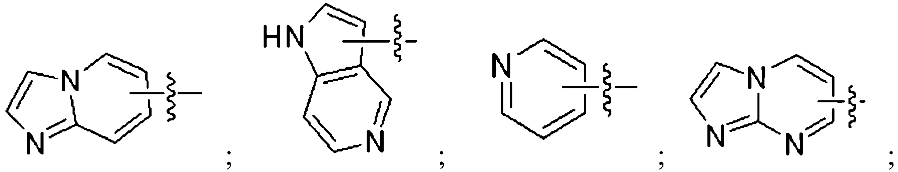 or 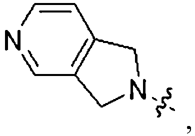 in which -H of said R₁ is unsubstituted or at least one H thereof is each independently substituted with halogen, -NH₂, -NO₂, -NHCH₃, -N(CH₃)₂ or -OH.

17. The benzamide derivative compound represented by above formula I, the stereoisomer thereof, the pharmaceutically acceptable salt of them, or the hydrate or solvate of them of claim 15, wherein L₁ is methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), sec-butylene (-CHCH₃CH₂CH₂-), isobutylene (-CH₂CHCH₃CH₂-), tert-butylene (-CCH₃CH₃CH₂-), n-pentylene (-CH₂CH₂CH₂CH₂CH₂-), n-hexylene (-CH₂CH₂CH₂CH₂CH₂CH₂-) or the like, specifically methylene (-CH₂-), ethylene (-CH₂CH₂-), n-propylene (-CH₂CH₂CH₂-), isopropylene (-CHCH₃ CH₂), n-butylene (-CH₂CH₂CH₂CH₂-), n-pentylene (-CH₂CH₂CH₂CH₂CH₂-) or n-hexylene (-CH₂CH₂CH₂CH₂CH₂CH₂-).

18. The benzamide derivative compound represented by above formula I, the stereoisomer thereof, the pharmaceutically acceptable salt of them, or the hydrate or solvate of them of claim 15, wherein X is 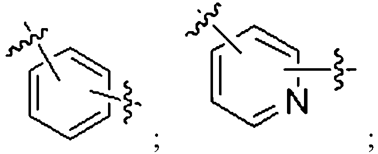 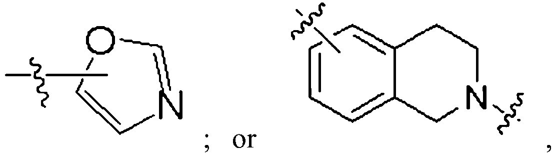 in which -H of said X is unsubstituted or at least one -H thereof is each independently substituted with Cl-C6 alkyl, halogen, -OH, -C1-C6 alkoxy, or -CF₃.

19. The benzamide derivative compound represented by above formula I, the stereoisomer thereof, the pharmaceutically acceptable salt of them, or the hydrate or solvate of them of claim 15, wherein when said X is 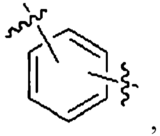 -H is unsubstituted or at least one -H thereof is each independently substituted with C1-C6 alkyl, halogen, -C1-C6 alkoxy, -OH, or -CF₃.

20. The benzamide derivative compound represented by above formula I, the stereoisomer thereof, the pharmaceutically acceptable salt of them, or the hydrate or solvate of them of claim 15, wherein L₃ is a single bond, -(CH₂)-, -(CH₂)₂-, - (CH₂)₃-, -(CH₂)₄-, -(CH₂)₅-, -(C=O)-NH(CH₂)n, -(C=O)NH-, or -(C=O)-, in which n is 0, 1, 2, 3 or 4.

21. The benzamide derivative compound represented by above formula I, the stereoisomer thereof, the pharmaceutically acceptable salt of them, or the hydrate or solvate of them of claim 15, wherein R₂ is H; halogen; C1-C6 alkyl; C1-C6 alkoxy; -NH₂; 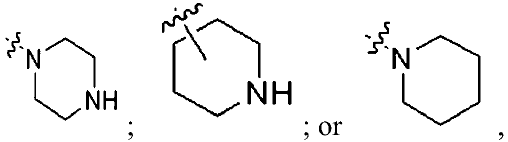 in which -H of said R₂ is unsubstituted or at least one -H of R₂ is each independently substituted with halogen, -C1-C6 alkoxy, -NH₂, or -C1-C6 alkyl.

22. The benzamide derivative compound represented by above formula I, the stereoisomer thereof, the pharmaceutically acceptable salt of them, or the hydrate or solvate of them of claim 15, wherein L₄ is a single bond; -(CH₂)-; -(CH₂)₂-; - (CH₂)₃-; -(CH₂)₄-; -(CH₂)₅-; -NH-; -NHCH₂-; -NH(CH₂)₂-; or -NH(CH₂)₃-,
in which -H of said L₄ is unsubstituted or at least one -H of L₄ is each independently substituted with -C1-C6 alkyl.

23. The benzamide derivative compound represented by above formula I, the stereoisomer thereof, the pharmaceutically acceptable salt of them, or the hydrate or solvate of them of claim 15, wherein R₃ is H; C1-C6 alkyl; 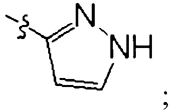 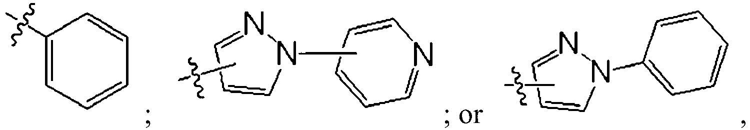 in which -H of said R₃ is unsubstituted or at least one -H of R₃ is each independently substituted with -C1-C6 alkyl, -C1-C6 alkoxy, -NH₂, -CF₃, -OCF₃, halogen, or

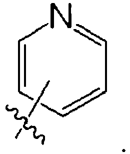

24. The benzamide derivative compound represented by above formula I, the stereoisomer thereof, the pharmaceutically acceptable salt of them, or the hydrate or solvate of them of claim 1, wherein above formula I is a compound represented by formula I-2 below: 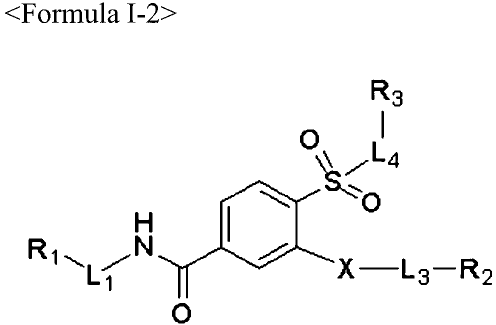 wherein in above formula I-2, R₁, L₁, X, L₃, R₂, L₄, R₃ are as defined in claim 1.

25. The benzamide derivative compound represented by above formula I, the stereoisomer thereof, the pharmaceutically acceptable salt of them, or the hydrate or solvate of them of claim 24, wherein in above formula I-2,
said R₁ is 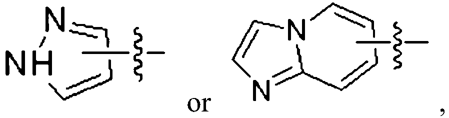 in which -H of said R₁ is unsubstituted or at least one H thereof is each independently substituted with halogen (F, Cl, Br, or I), -NH₂, -NO₂, -NHCH₃, -N(CH₃)₂ or -OH;
said L₁ is C1-C6 alkylene;
said X is 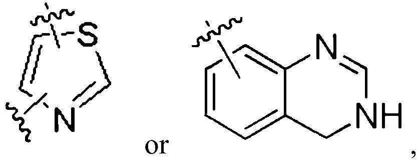 in which -H of said X is unsubstituted or at least one -H thereof is each independently substituted with C1-C6 alkyl, halogen (F, Br, Cl, or I), -OH, -C1-C6 alkoxy, or -CF₃, and when said X is 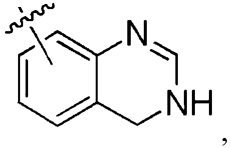 -CH₂- is unsubstituted or at least one -CH₂- is each independently substituted with -(C=O)-;
said L₃ is a single bond or C1-C6 alkylene;
said R₂ is H, halogen, -NH₂, or 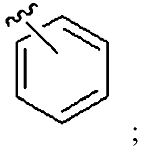 in which -H of said R₂ is unsubstituted or at least one -H of R₂ is each independently substituted with halogen (F, Cl, Br or I), -C1-C6 alkoxy, -NH₂, or -C1-C6 alkyl;
L₄ is a single bond, -C1-C6 alkylene-; and
said R₃ is 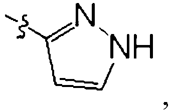 in which -H of said R₃ is unsubstituted or at least one -H of R₃ is each independently substituted with -C1-C6 alkyl, -C1-C6 alkoxy, or halogen.

26. A compound of table A below, a stereoisomer thereof, a pharmaceutically acceptable salt of them, or a hydrate or solvate of them:
**[Table A]**
| NO | Compound |
|---|---|
| 1 | 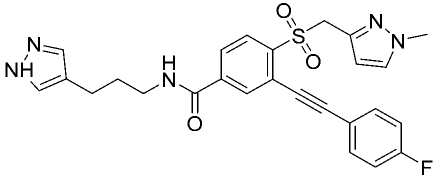 |
| 2 | 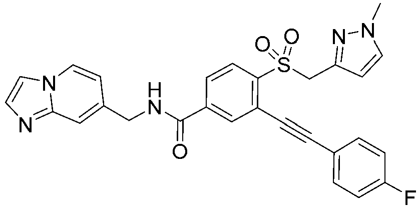 |
| 3 | 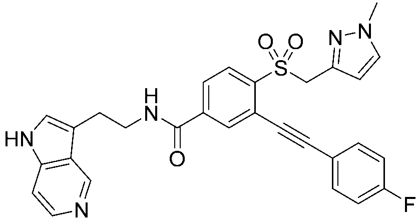 |
| 4 | 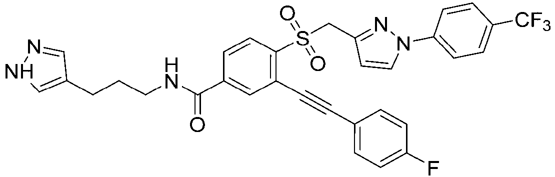 |
| 5 | 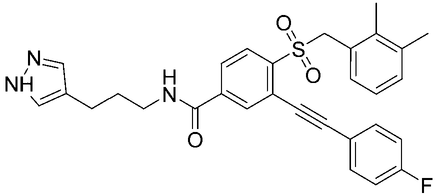 |
| 6 | 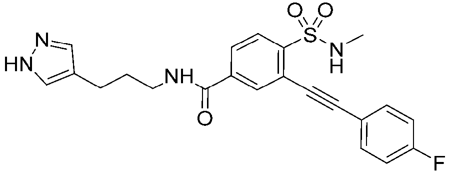 |
| 7 | 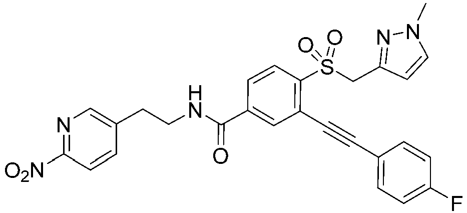 |
| 8 | 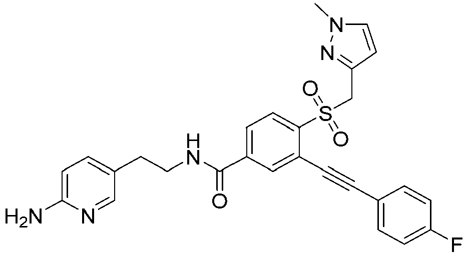 |
| 9 | 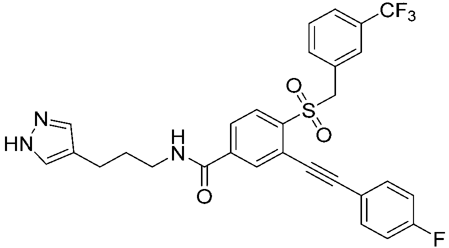 |
| 10 | 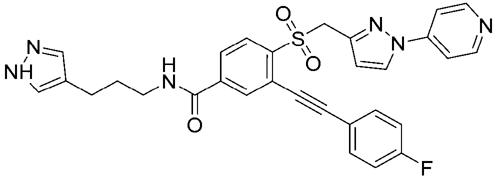 |
| 11 | 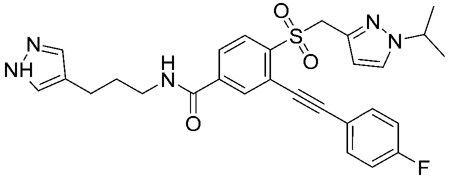 |
| 12 | 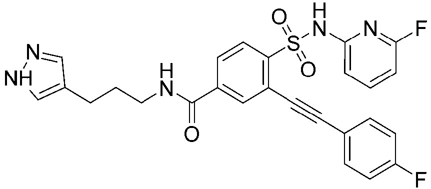 |
| 13 | 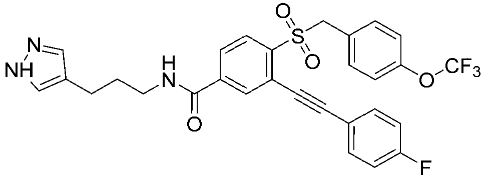 |
| 14 | 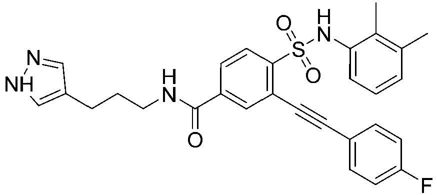 |
| 15 | 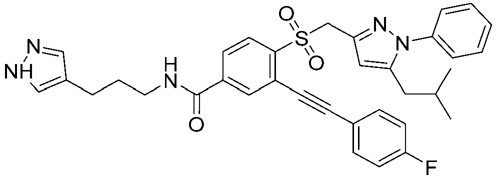 |
| 16 | 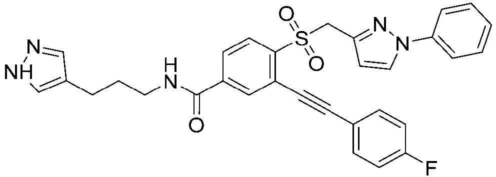 |
| 17 | 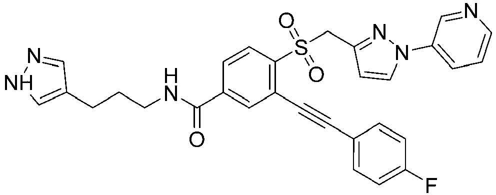 |
| 18 | 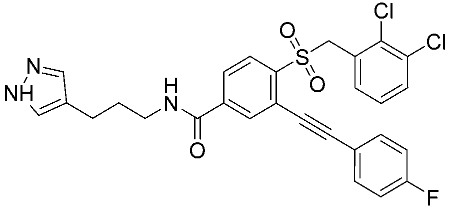 |
| 19 | 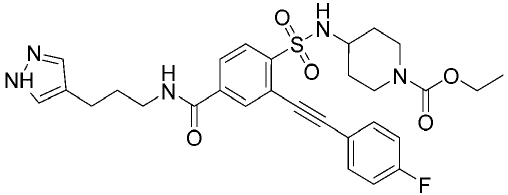 |
| 20 | 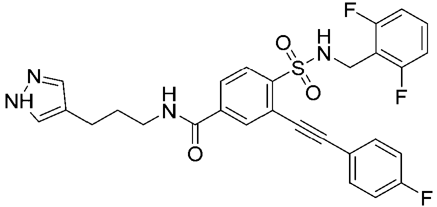 |
| 21 | 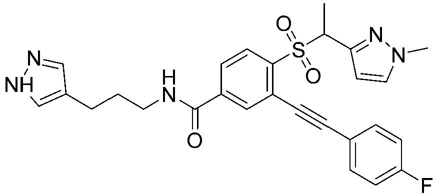 |
| 22 | 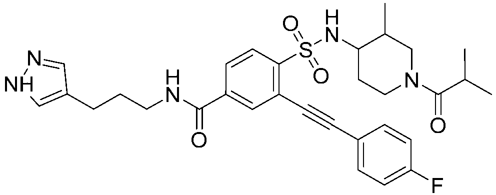 |
| 23 | 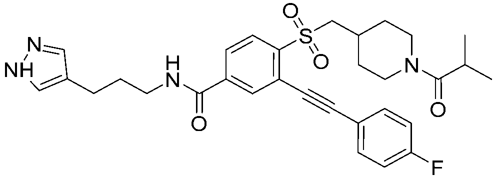 |
| 24 | 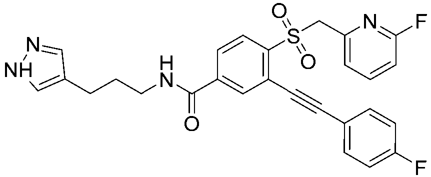 |
| 25 | 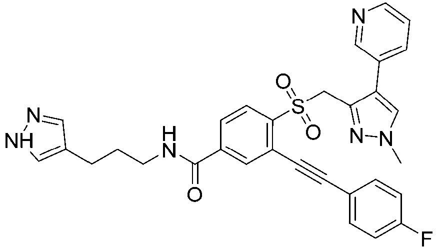 |
| 26 | 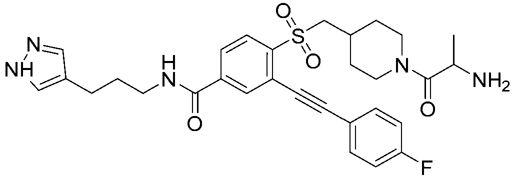 |
| 27 | 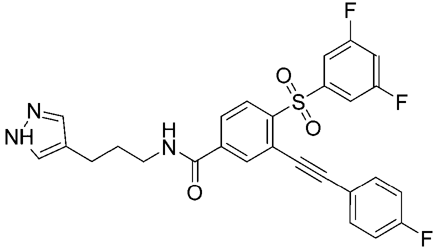 |
| 28 | 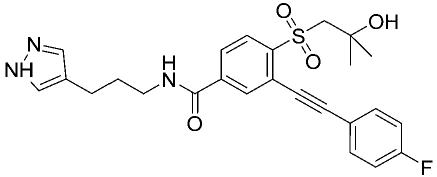 |
| 29 | 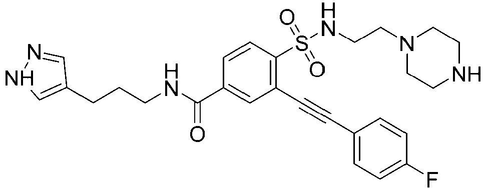 |
| 30 | 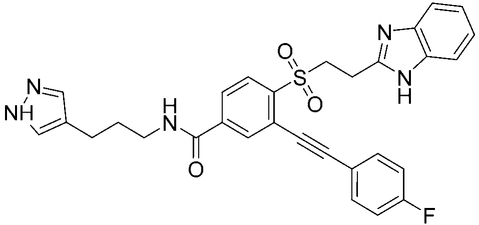 |
| 31 | 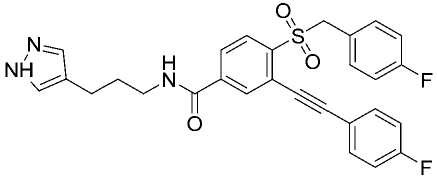 |
| 32 | 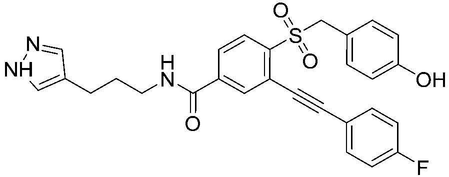 |
| 33 | 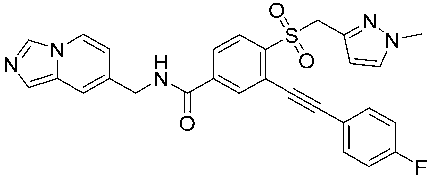 |
| 34 | 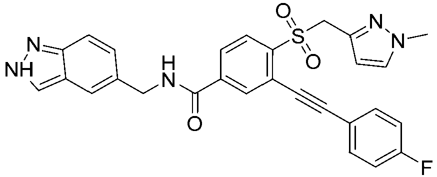 |
| 35 | 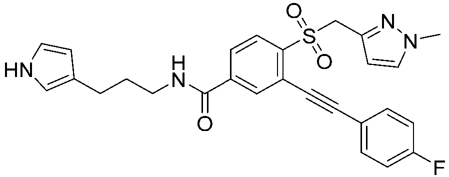 |
| 36 | 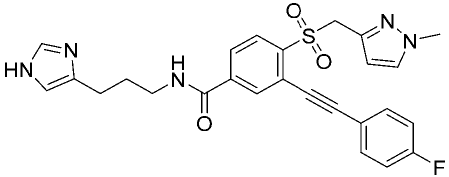 |
| 37 | 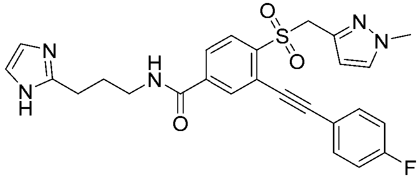 |
| 38 | 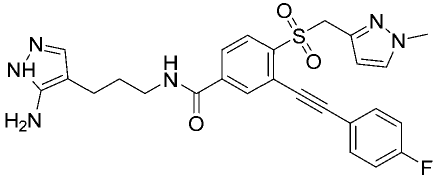 |
| 39 | 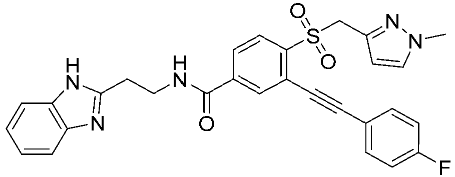 |
| 40 | 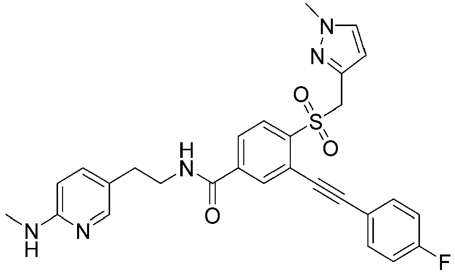 |
| 41 | 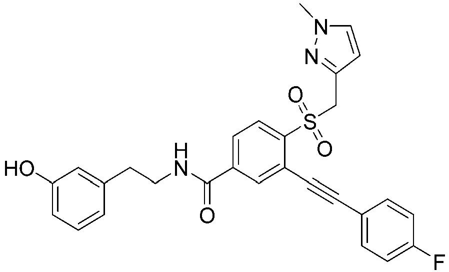 |
| 42 | 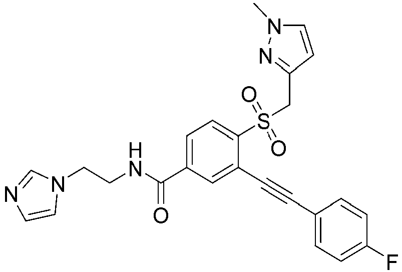 |
| 43 | 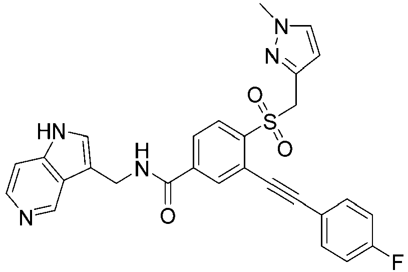 |
| 44 | 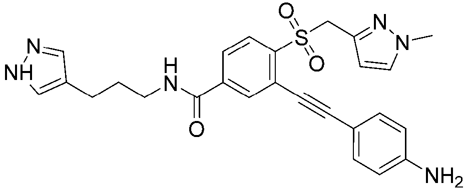 |
| 45 | 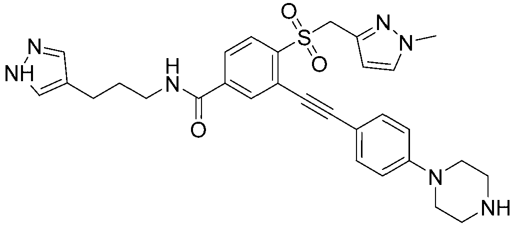 |
| 46 | 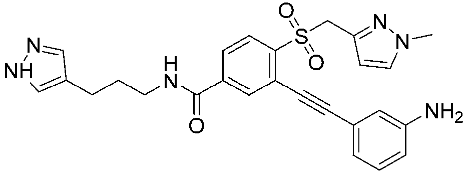 |
| 47 | 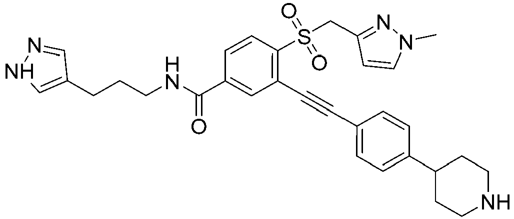 |
| 48 | 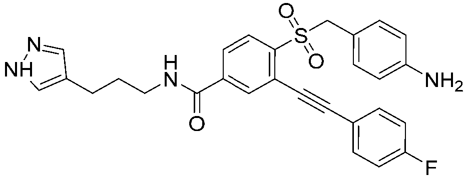 |
| 49 | 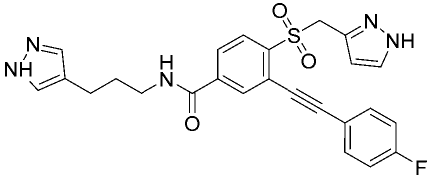 |
| 50 | 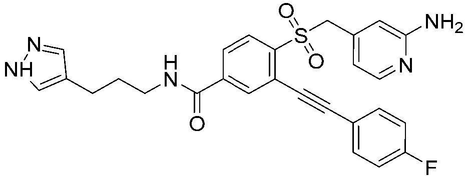 |
| 51 | 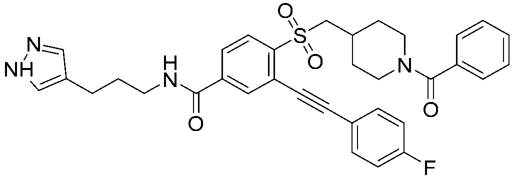 |
| 52 | 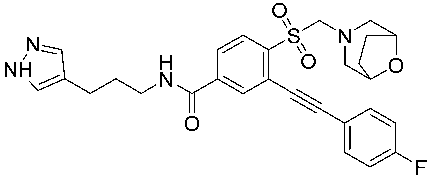 |
| 53 | 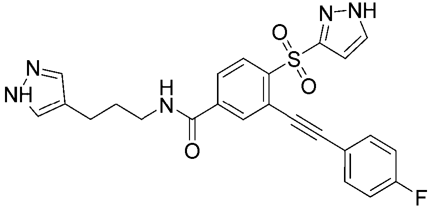 |
| 54 | 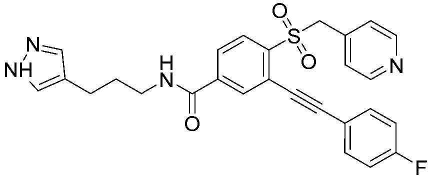 |
| 55 | 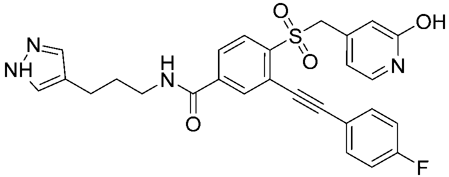 |
| 56 | 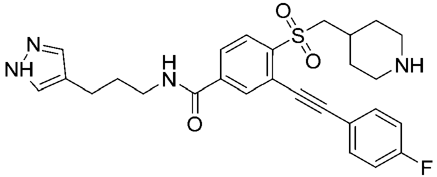 |
| 57 | 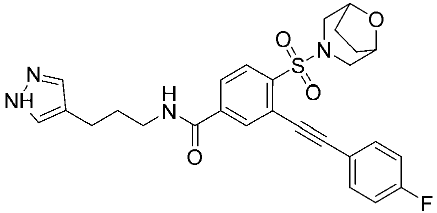 |
| 58 | 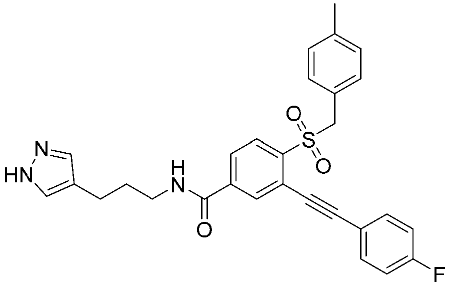 |
| 59 | 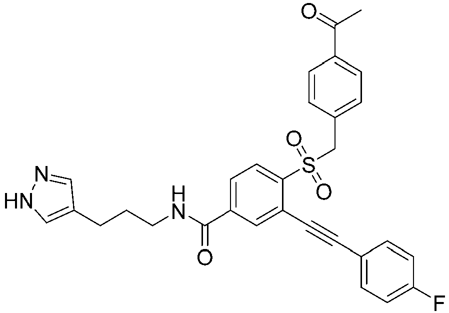 |
| 60 | 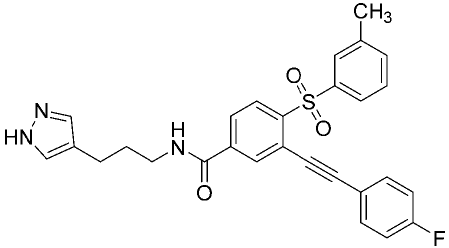 |
| 61 | 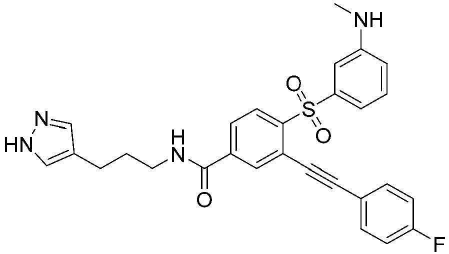 |
| 62 | 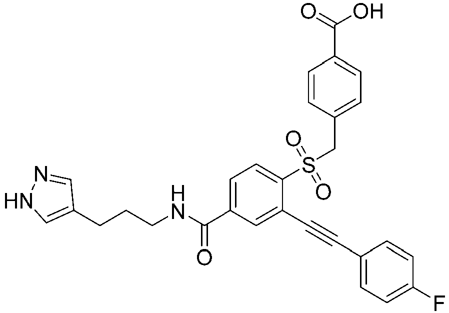 |
| 63 | 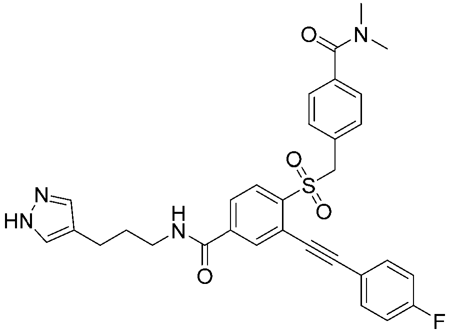 |
| 64 | 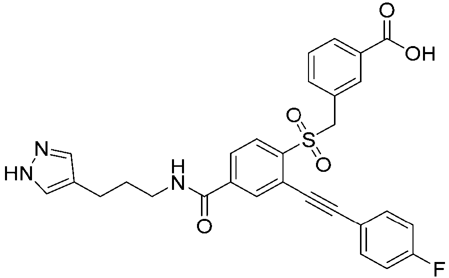 |
| 65 | 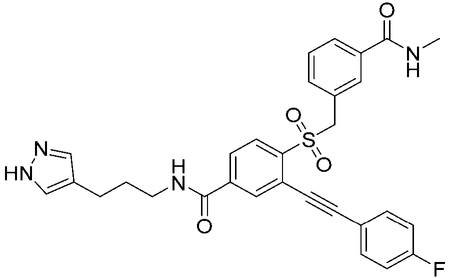 |
| 66 | 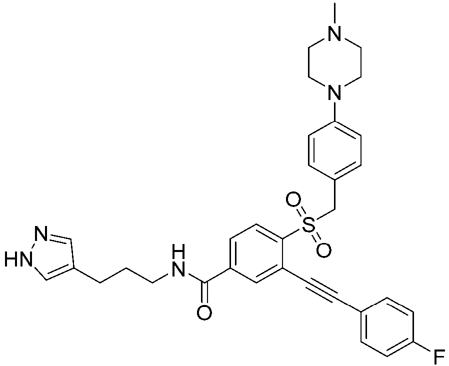 |
| 67 | 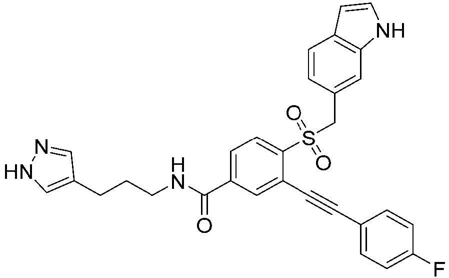 |
| 68 | 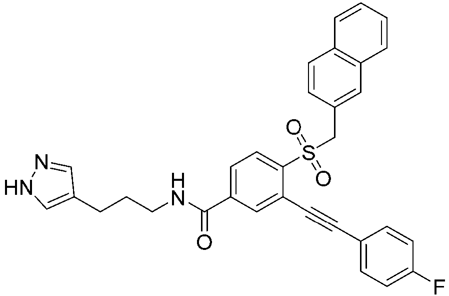 |
| 69 | 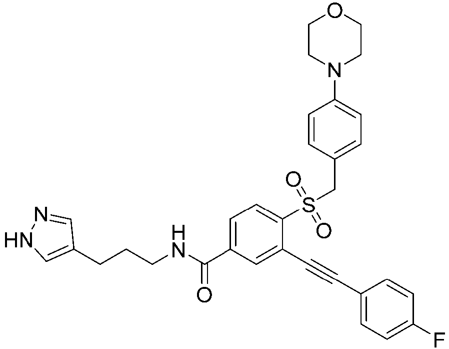 |
| 70 | 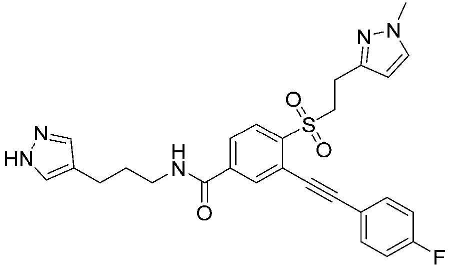 |
| 71 | 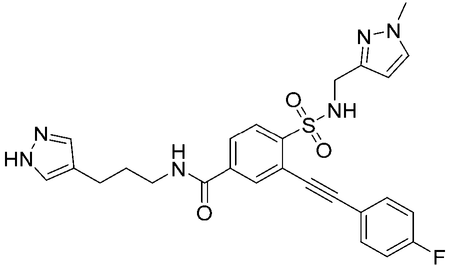 |
| 72 | 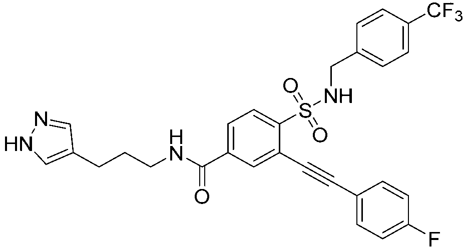 |
| 73 | 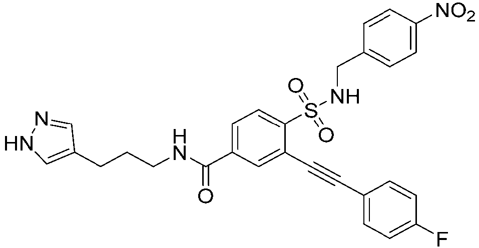 |
| 74 | 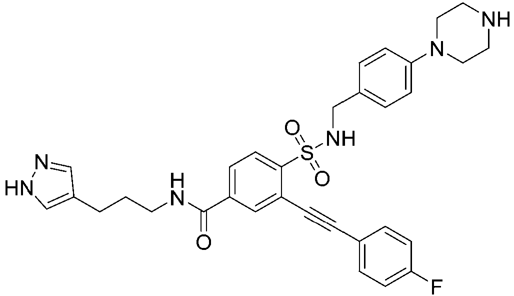 |
| 75 | 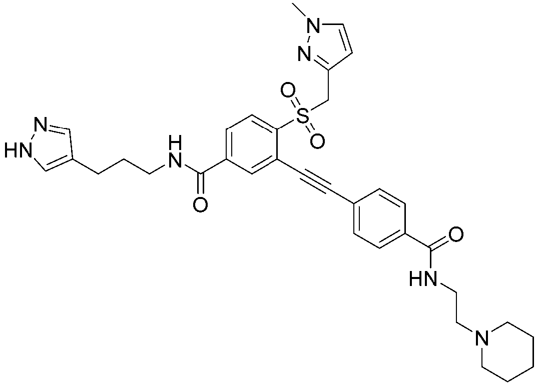 |
| 76 | 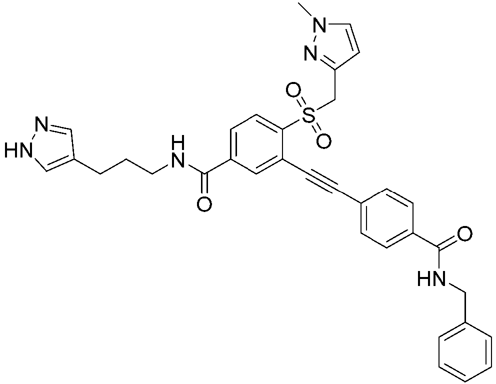 |
| 77 | 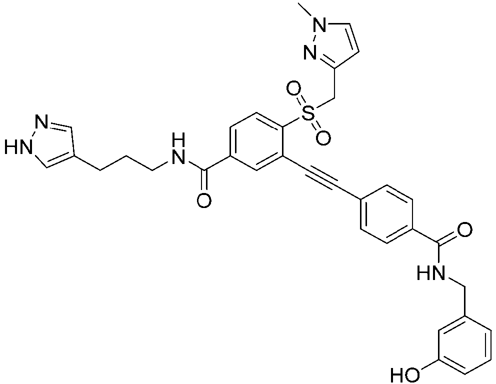 |
| 78 | 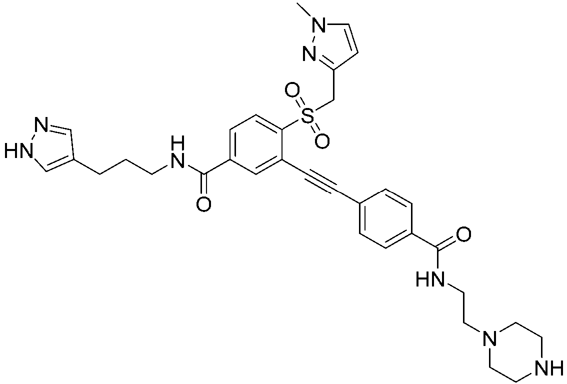 |
| 79 | 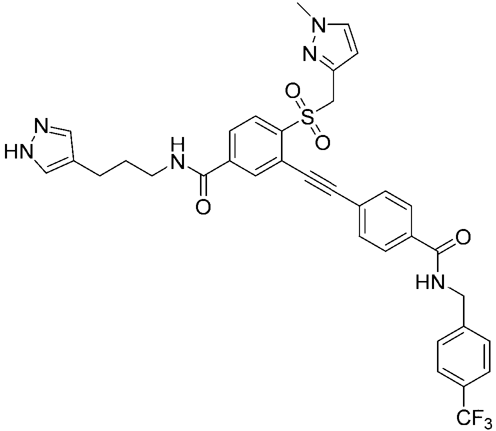 |
| 80 | 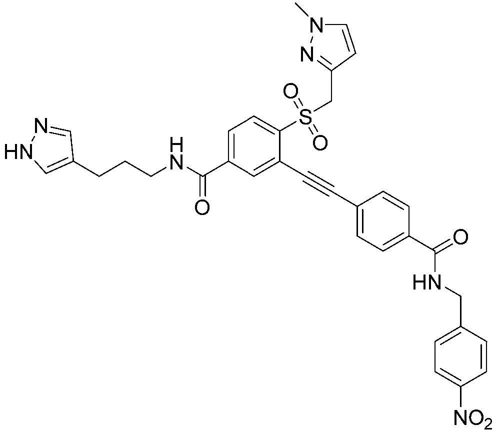 |
| 81 | 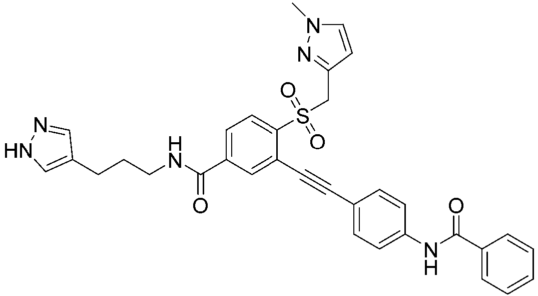 |
| 82 | 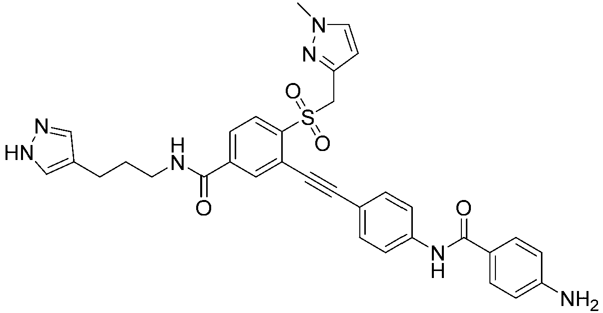 |
| 83 | 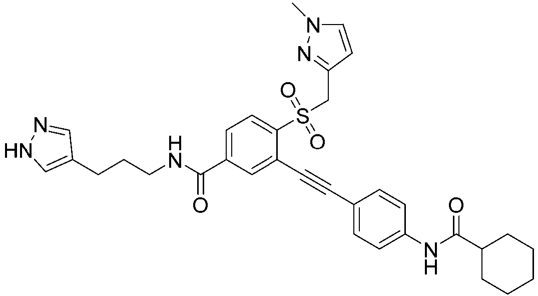 |
| 84 | 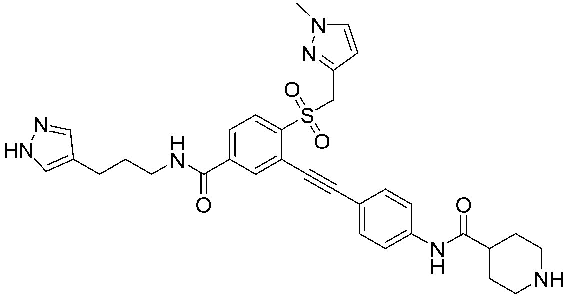 |
| 85 | 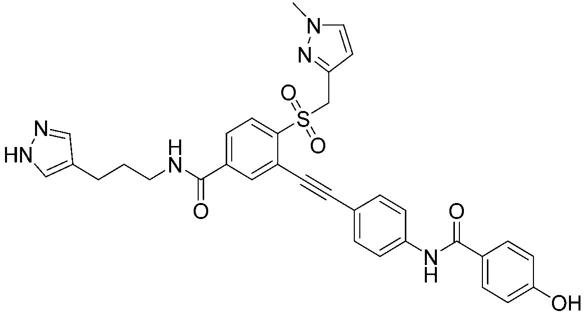 |
| 86 | 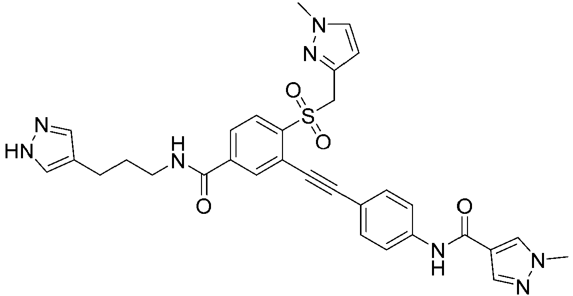 |
| 87 | 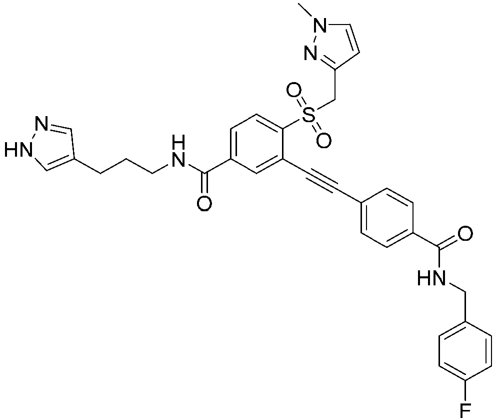 |
| 88 | 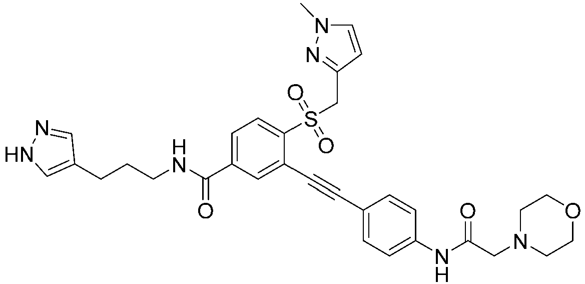 |
| 89 | 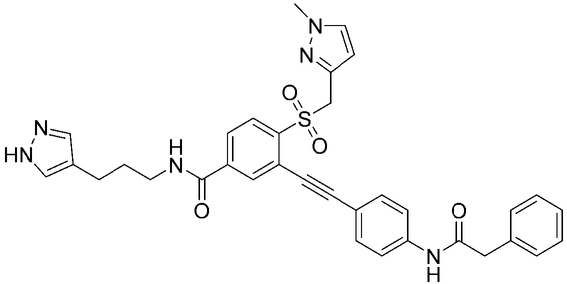 |
| 90 | 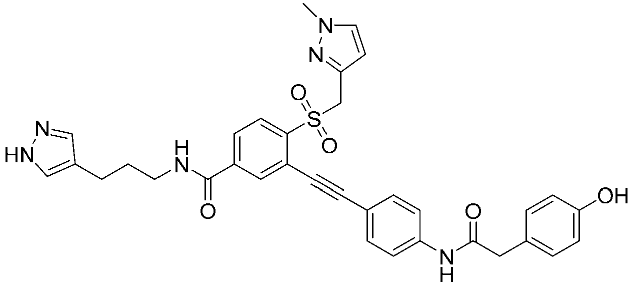 |
| 91 | 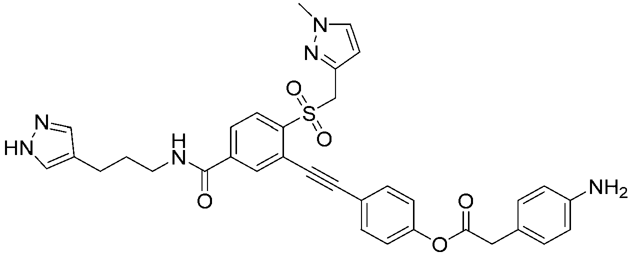 |
| 92 | 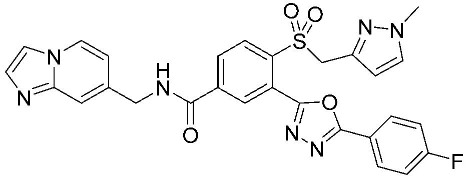 |
| 93 | 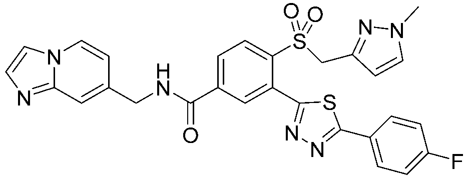 |
| 94 | 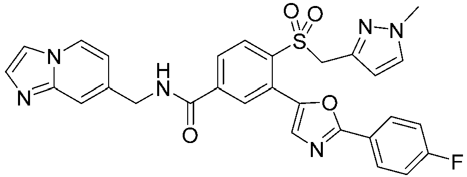 |
| 95 | 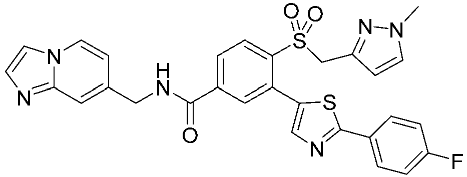 |
| 96 | 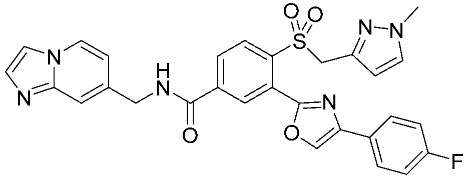 |
| 97 | 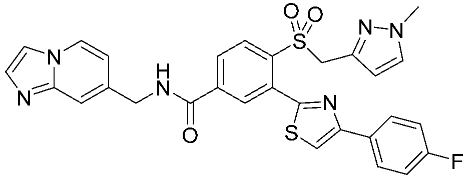 |
| 98 | 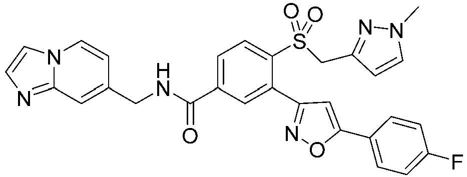 |
| 99 | 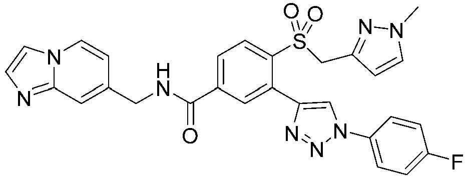 |
| 100 | 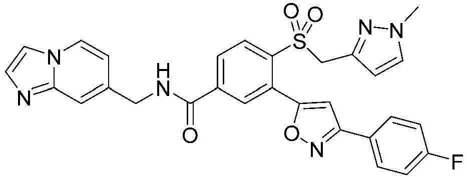 |
| 101 | 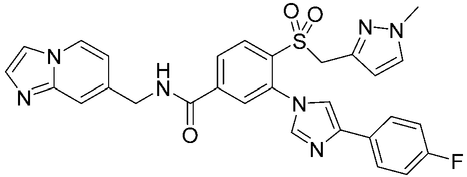 |
| 102 | 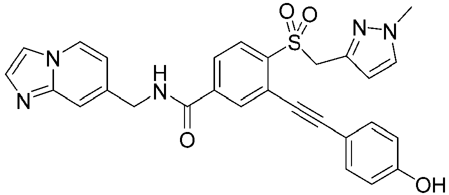 |
| 103 | 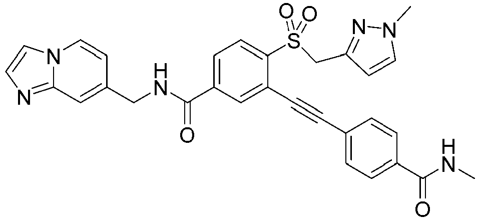 |
| 104 | 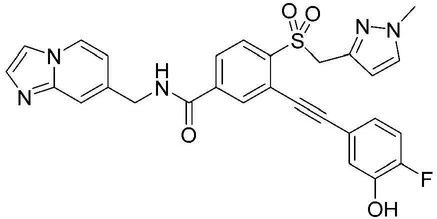 |
| 105 | 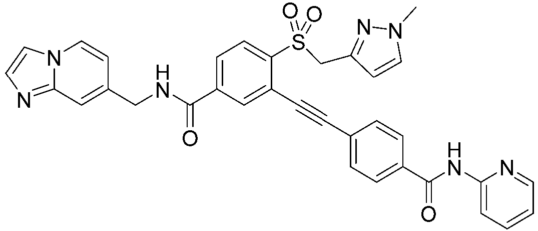 |
| 106 | 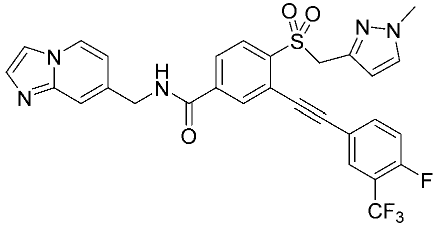 |
| 107 | 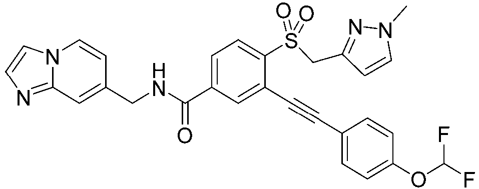 |
| 108 | 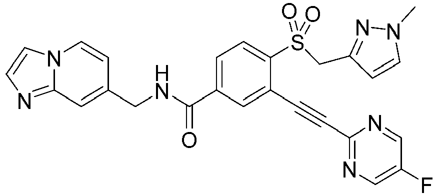 |
| 109 | 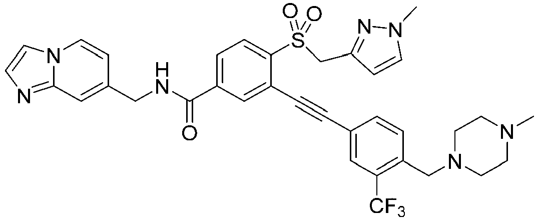 |
| 110 | 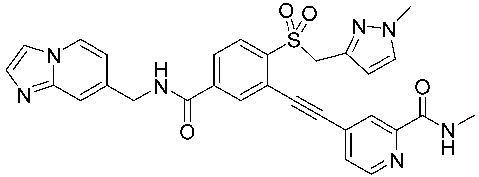 |
| 111 | 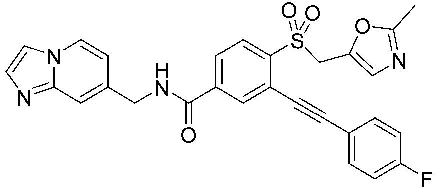 |
| 112 | 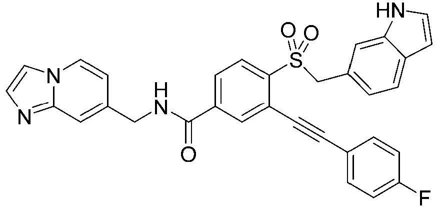 |
| 113 | 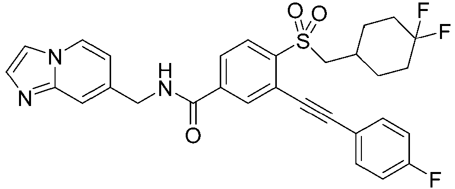 |
| 114 | 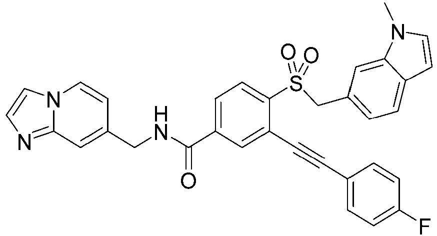 |
| 115 | 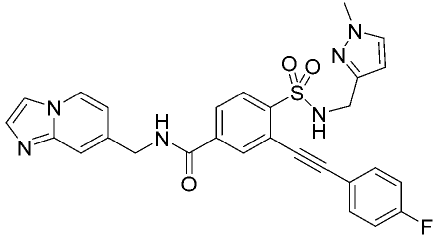 |
| 116 | 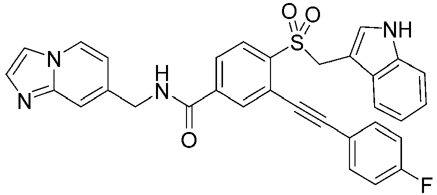 |
| 117 | 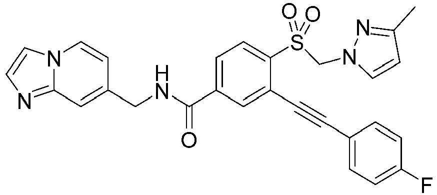 |
| 118 | 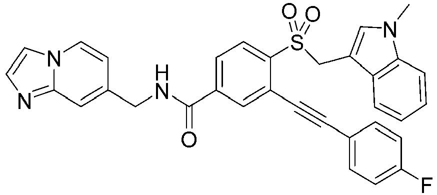 |
| 119 | 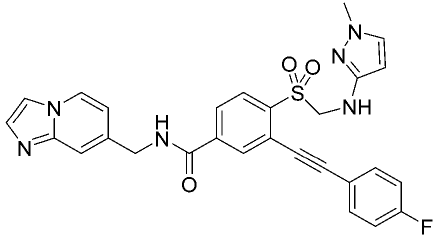 |
| 120 | 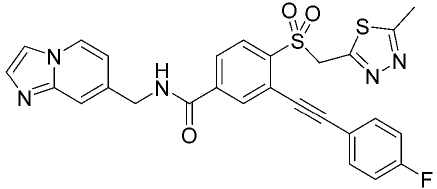 |
| 121 | 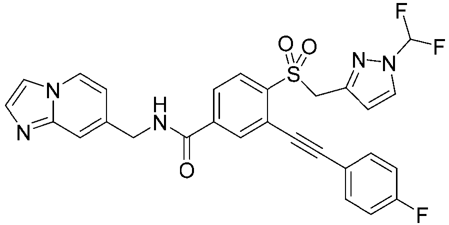 |
| 122 | 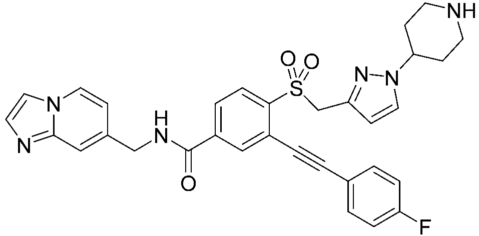 |
| 123 | 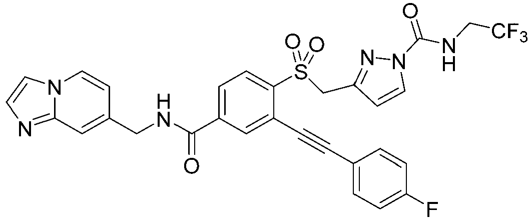 |
| 124 | 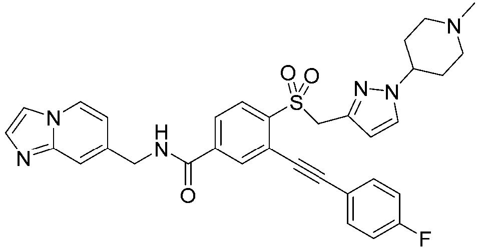 |
| 125 | 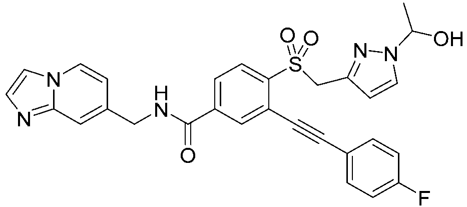 |
| 126 | 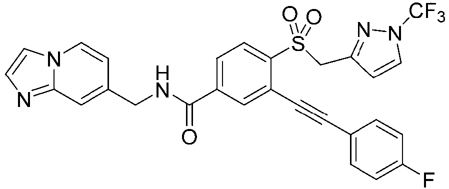 |
| 127 | 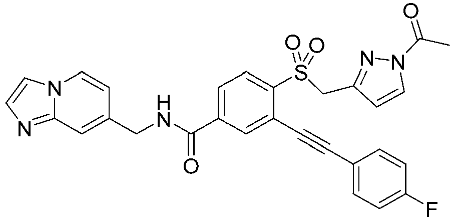 |
| 128 | 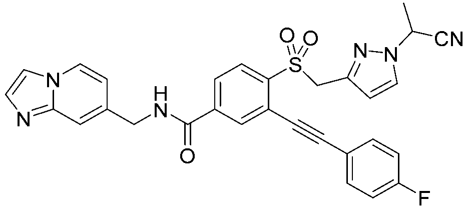 |
| 129 | 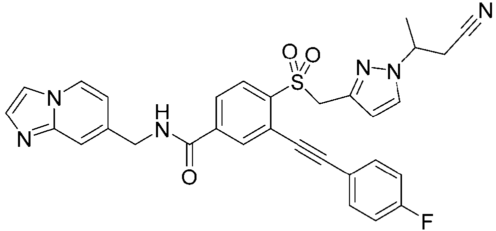 |
| 130 | 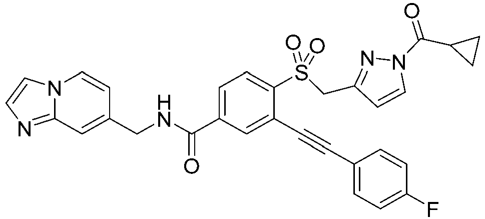 |
| 131 | 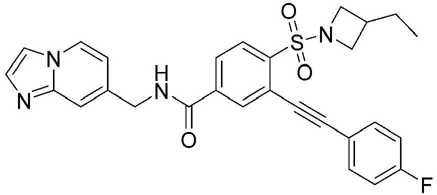 |
| 132 | 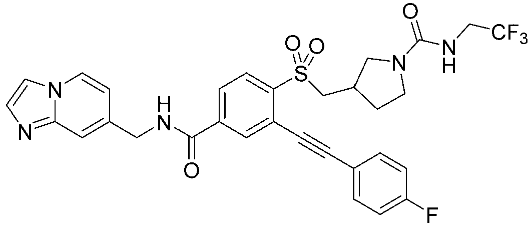 |
| 133 | 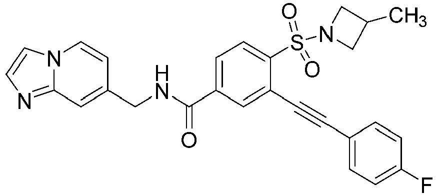 |
| 134 | 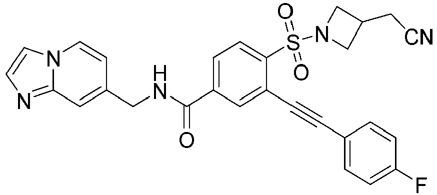 |
| 135 | 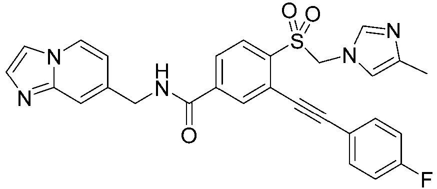 |
| 136 | 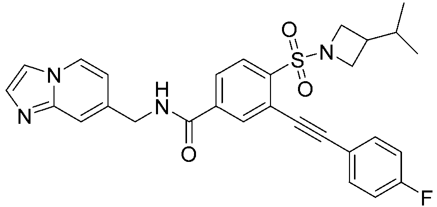 |
| 137 | 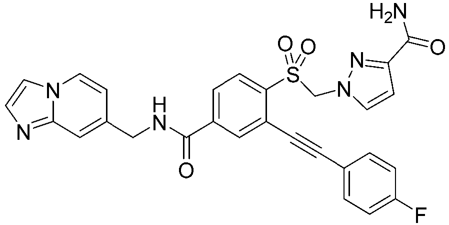 |
| 138 | 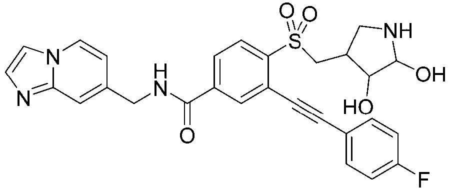 |
| 139 | 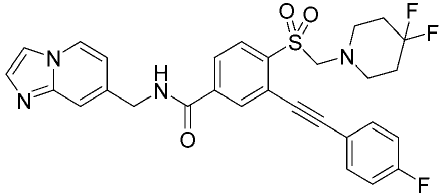 |
| 140 | 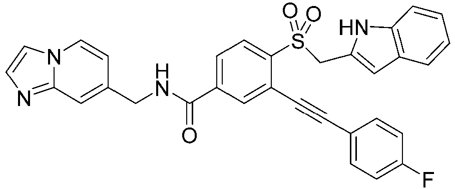 |
| 141 | 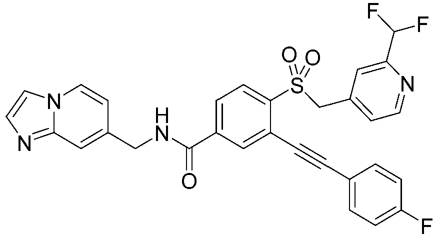 |
| 142 | 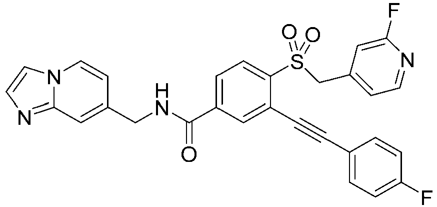 |
| 143 | 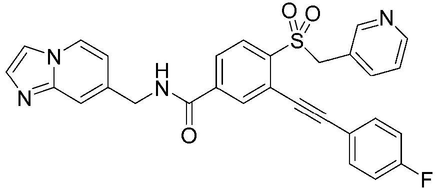 |
| 144 | 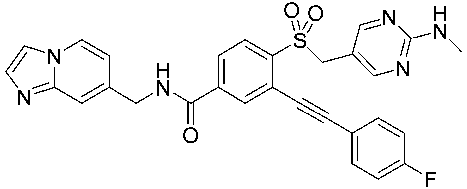 |
| 145 | 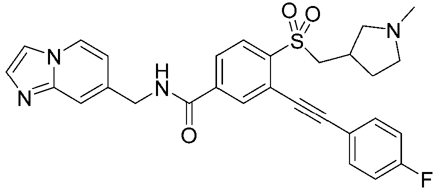 |
| 146 | 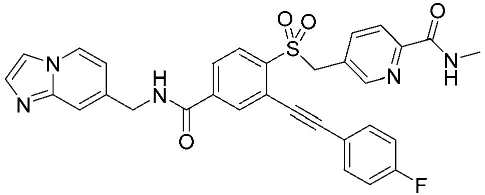 |
| 147 | 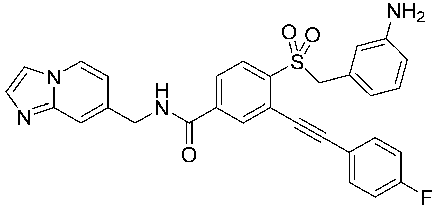 |
| 148 | 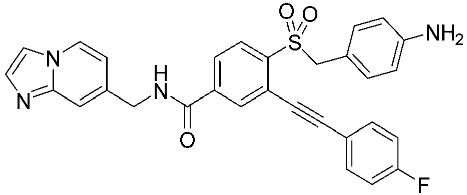 |
| 149 | 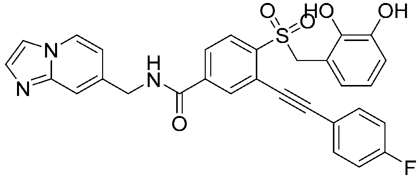 |
| 150 | 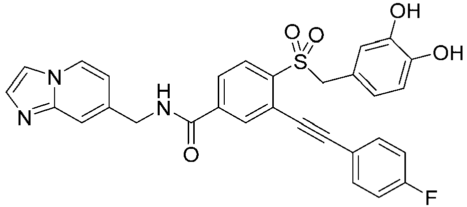 |
| 151 | 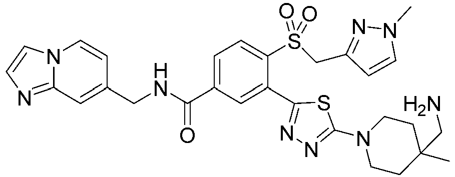 |
| 152 | 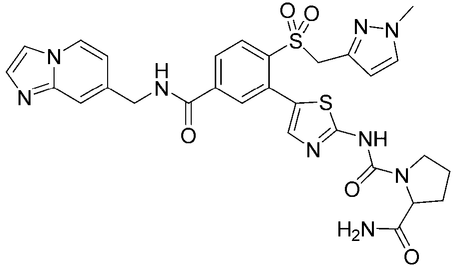 |
| 153 | 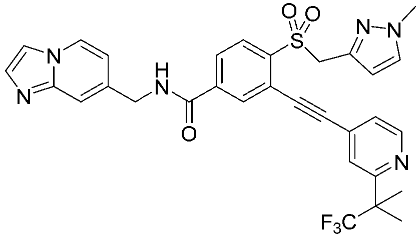 |
| 154 | 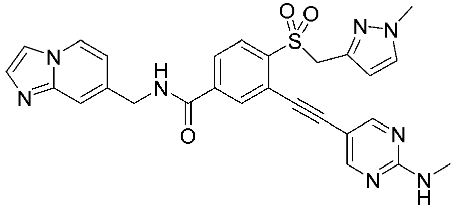 |
| 155 | 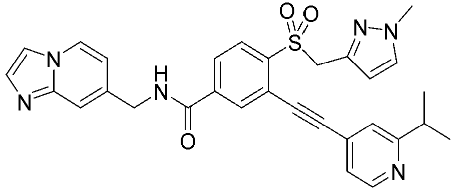 |
| 156 | 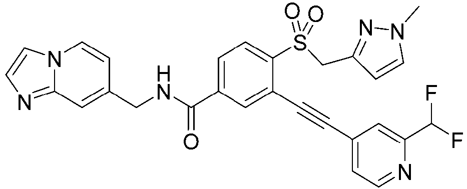 |
| 157 | 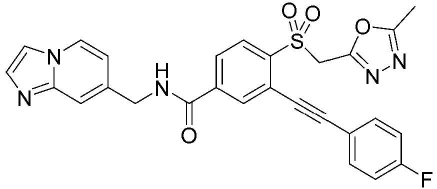 |
| 158 | 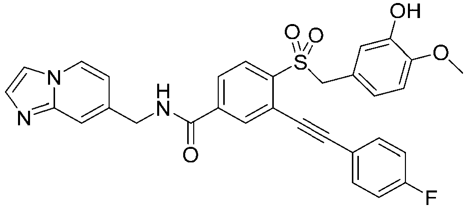 |
| 159 | 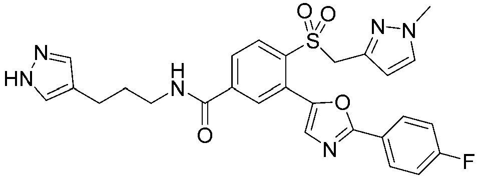 |
| 160 | 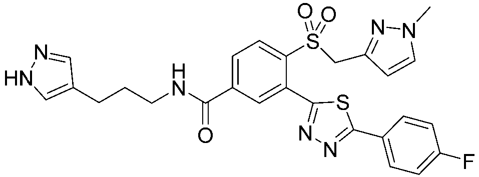 |
| 161 | 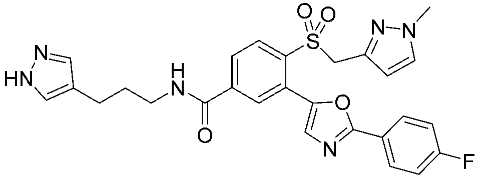 |
| 162 | 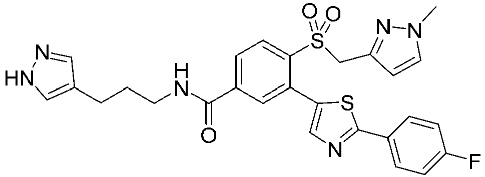 |
| 163 | 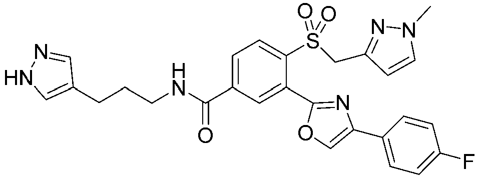 |
| 164 | 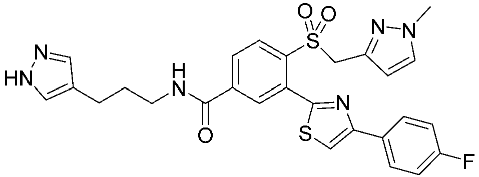 |
| 165 | 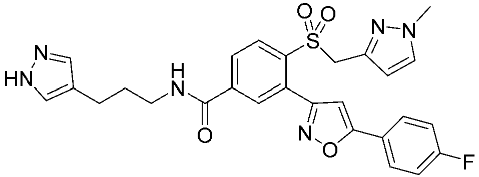 |
| 166 | 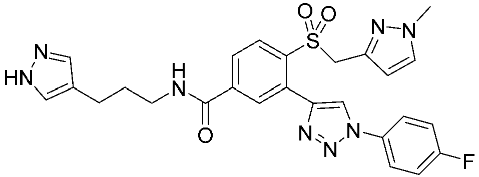 |
| 167 | 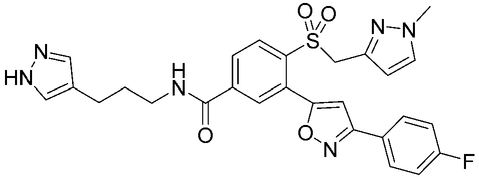 |
| 168 | 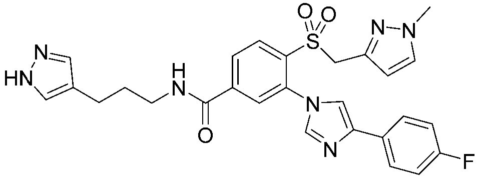 |
| 169 | 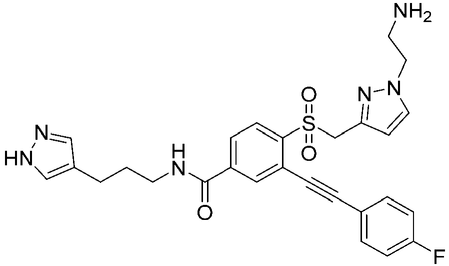 |
| 170 | 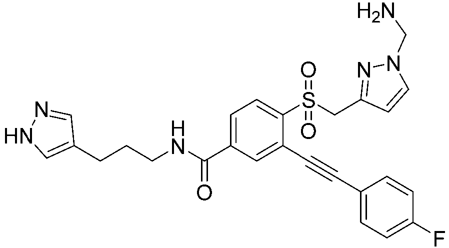 |
| 171 | 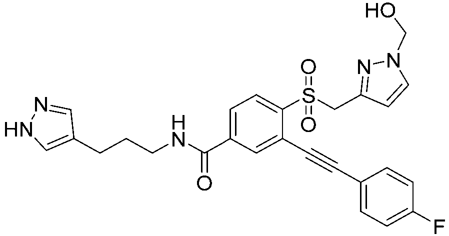 |
| 172 | 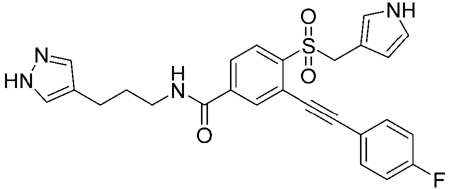 |
| 173 | 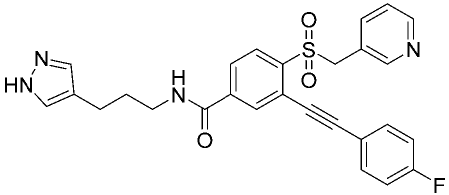 |
| 174 | 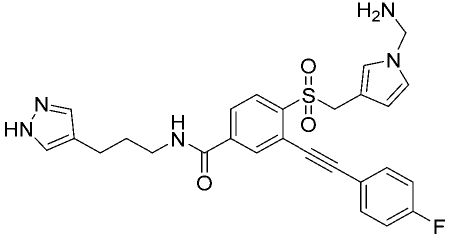 |
| 175 | 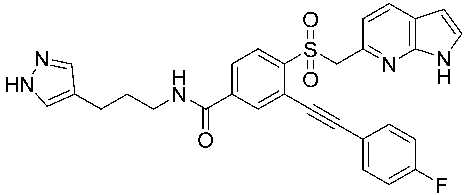 |
| 176 | 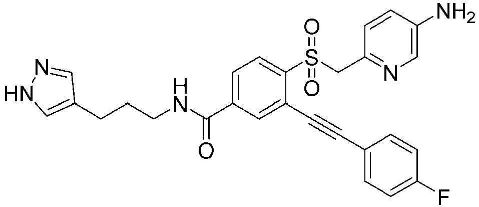 |
| 177 | 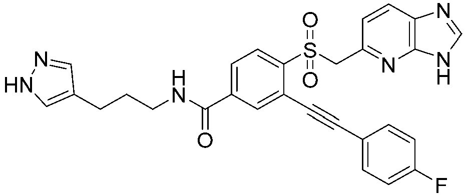 |
| 178 | 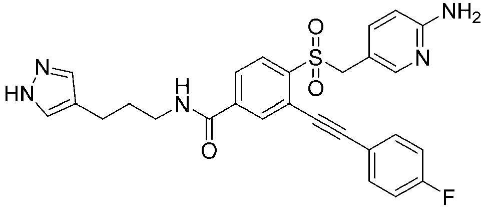 |
| 179 | 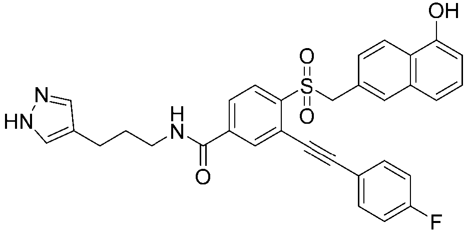 |
| 180 | 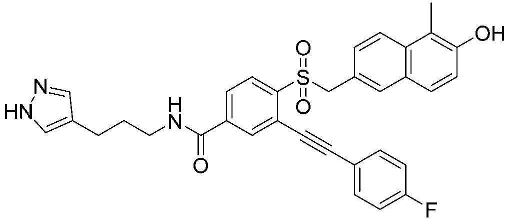 |
| 181 | 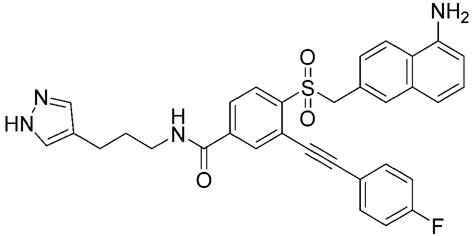 |
| 182 | 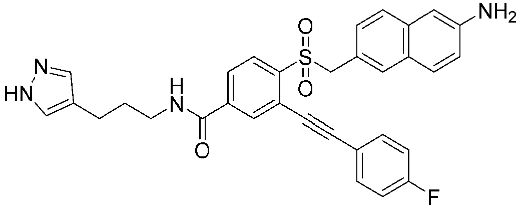 |
| 183 | 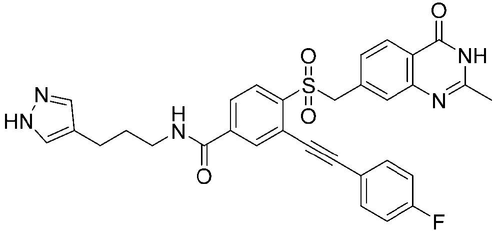 |
| 184 | 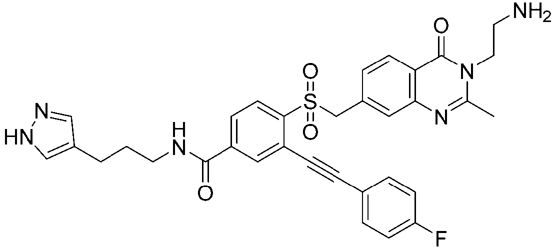 |
| 185 | 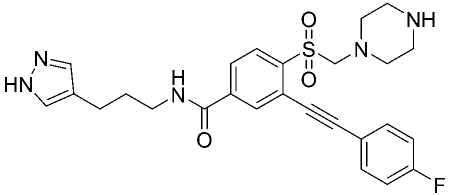 |
| 186 | 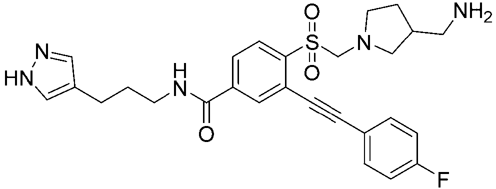 |
| 187 | 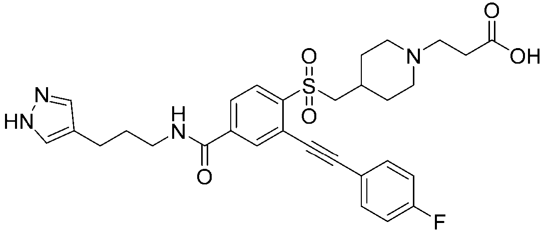 |
| 188 | 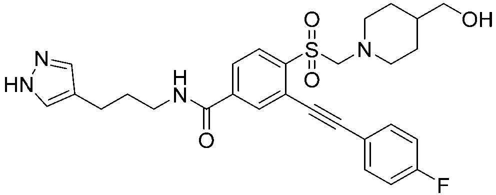 |
| 189 | 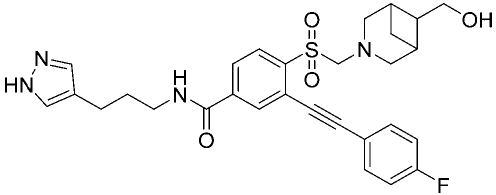 |
| 190 | 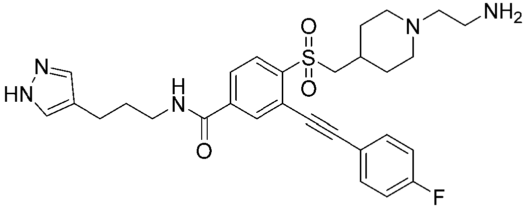 |
| 191 | 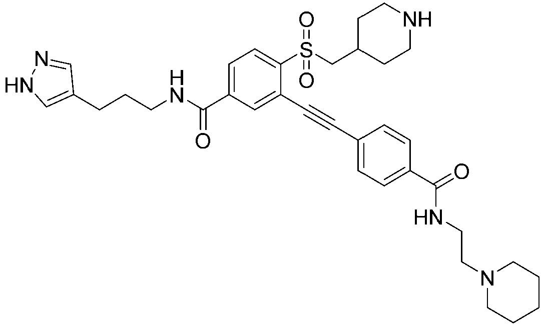 |
| 192 | 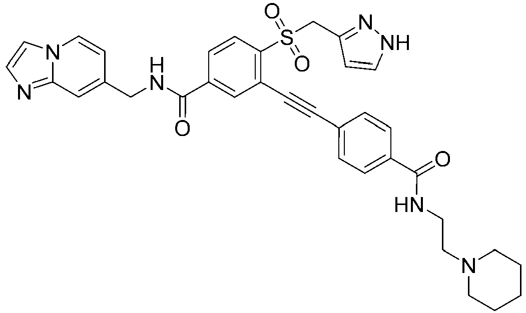 |
| 193 | 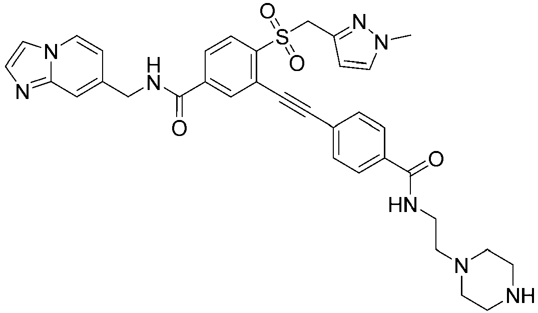 |
| 194 | 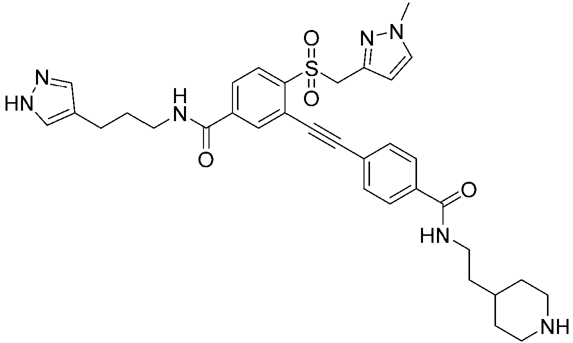 |
| 195 | 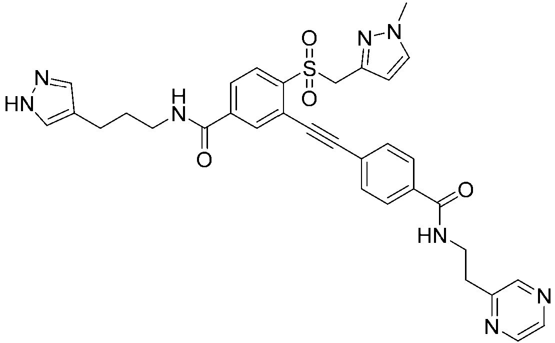 |
| 196 | 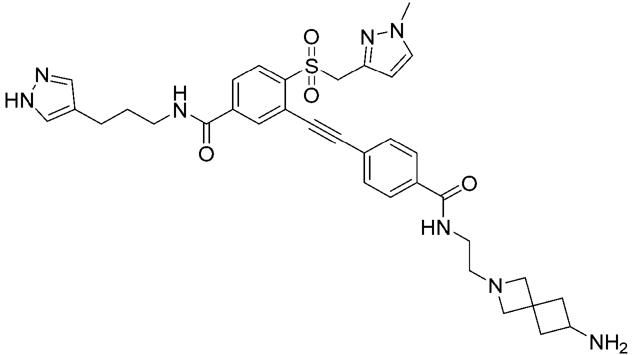 |
| 197 | 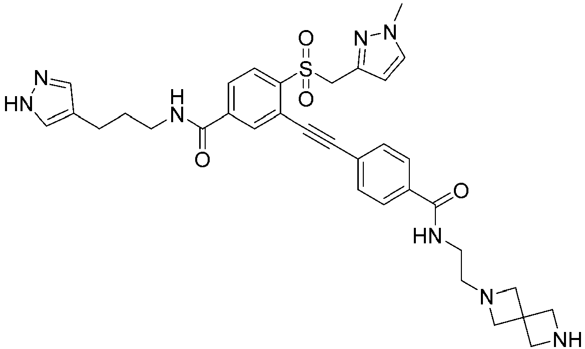 |
| 198 | 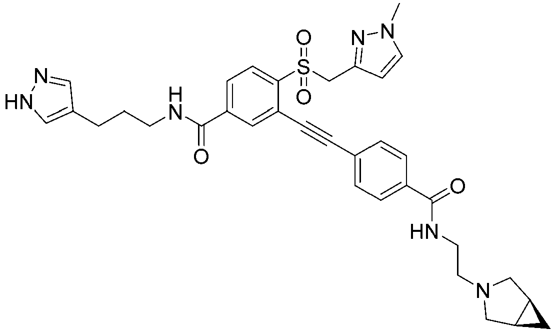 |
| 199 | 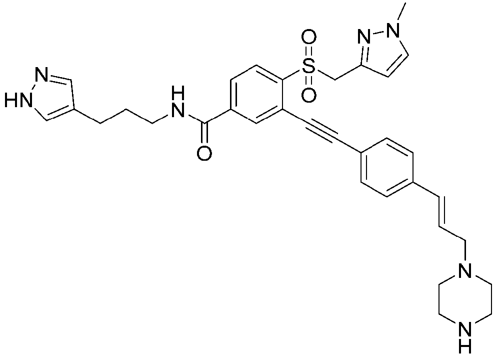 |
| 200 | 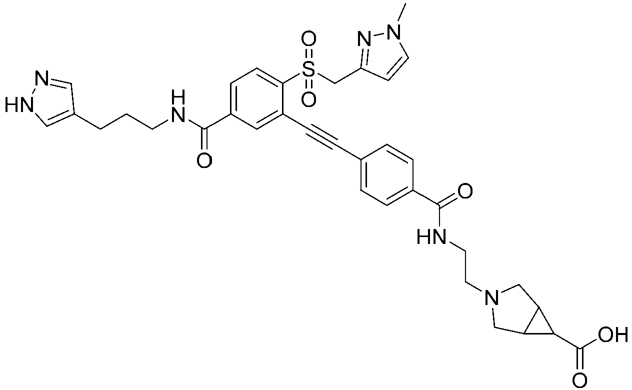 |
| 201 | 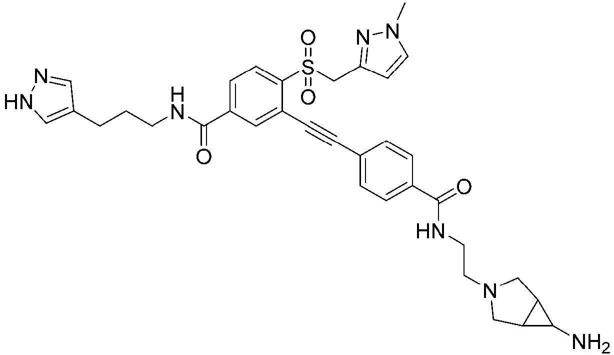 |
| 202 | 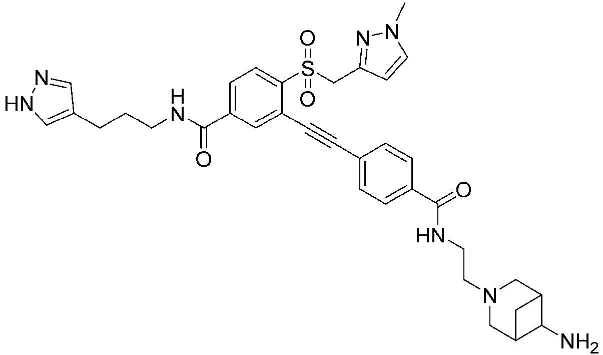 |
| 203 | 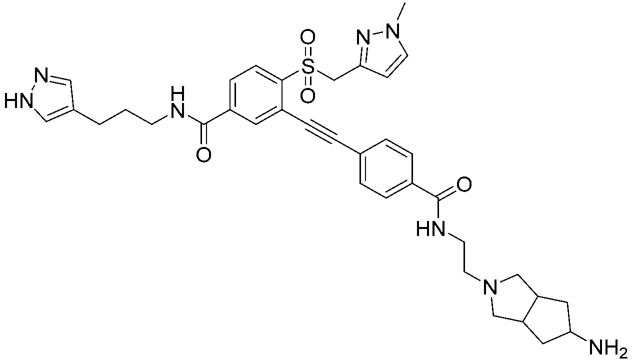 |
| 204 | 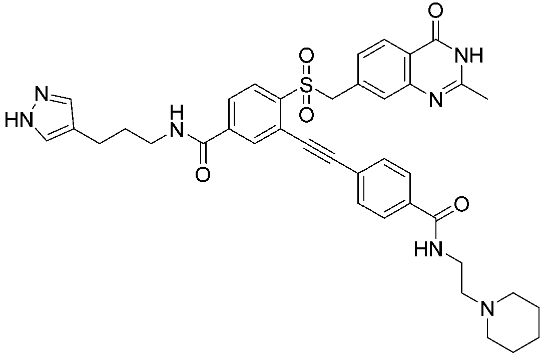 |
| 205 | 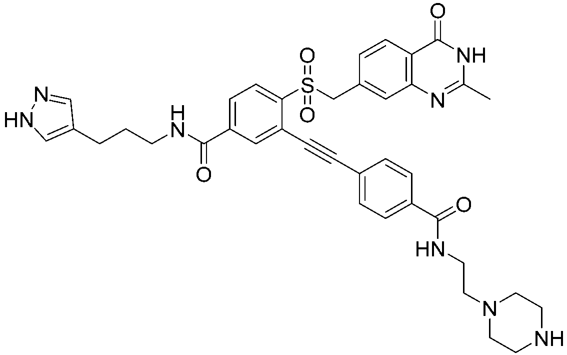 |
| 206 | 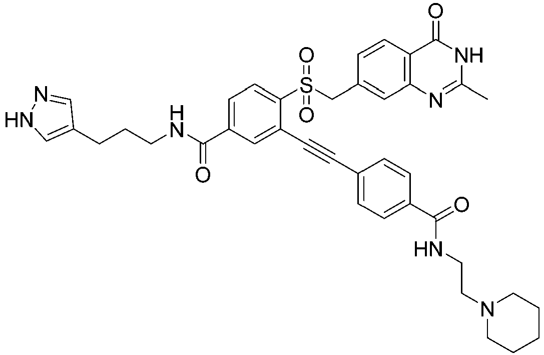 |
| 207 | 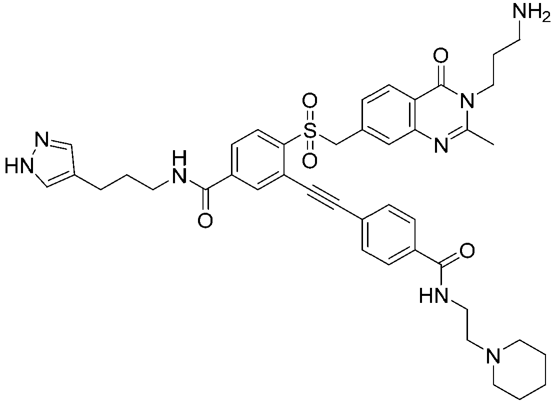 |
| 208 | 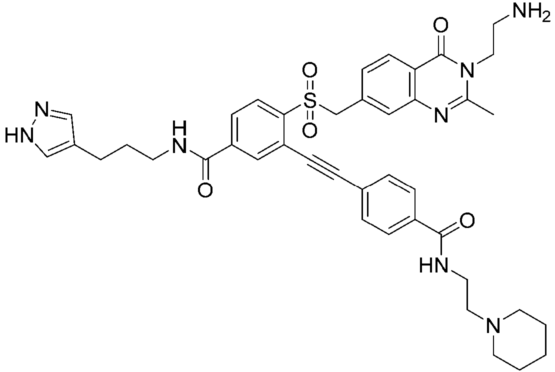 |
| 209 | 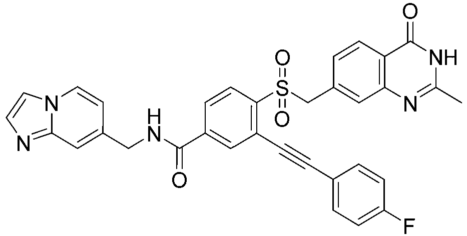 |
| 210 | 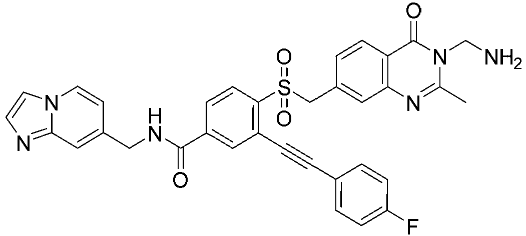 |
| 211 | 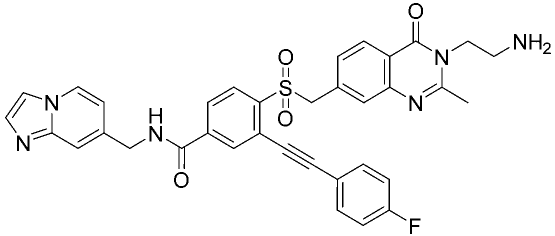 |
| 212 | 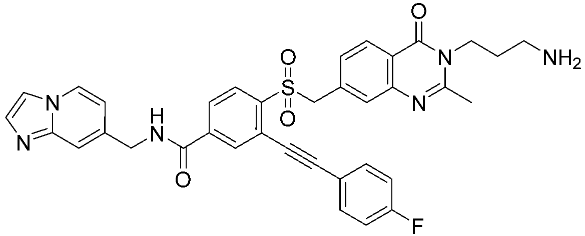 |
| 213 | 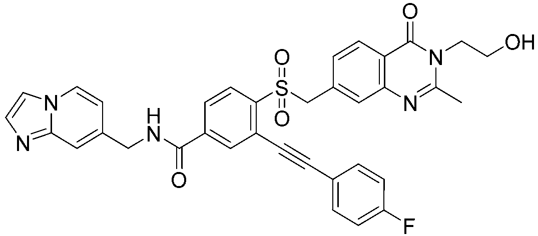 |
| 214 | 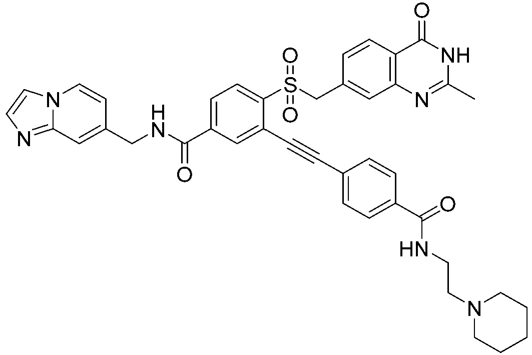 |
| 215 | 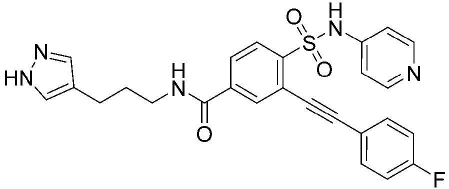 |
| 216 | 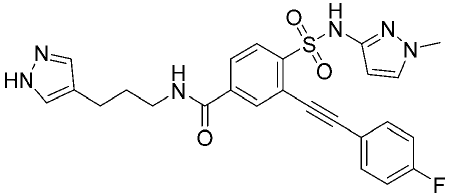 |
| 217 | 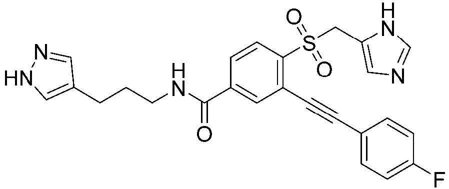 |
| 218 | 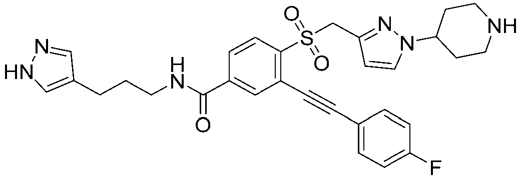 |
| 219 | 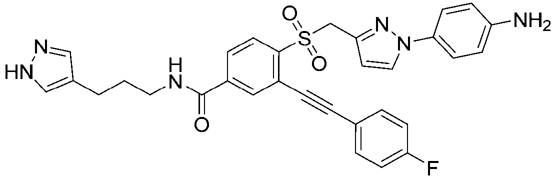 |
| 220 | 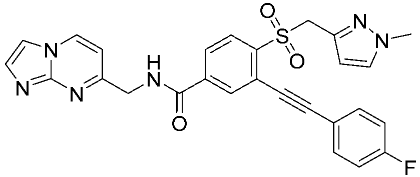 |
| 221 | 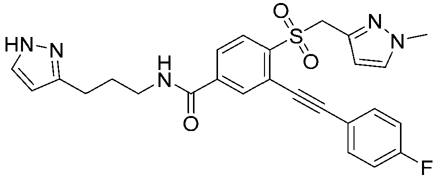 |
| 222 | 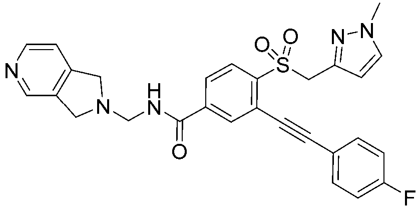 |
| 223 | 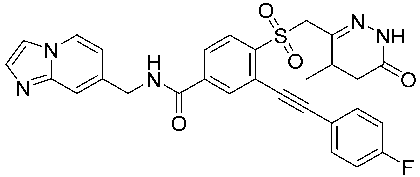 |
| 224 | 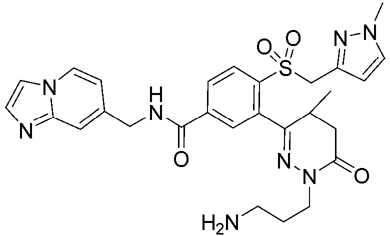 |
| 225 | 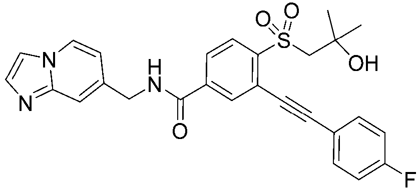 |
| 226 | 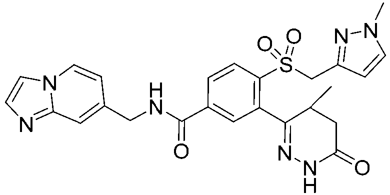 |
| 227 | 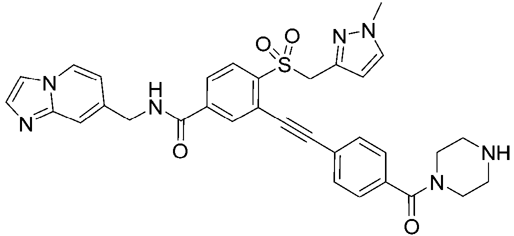 |
| 228 | 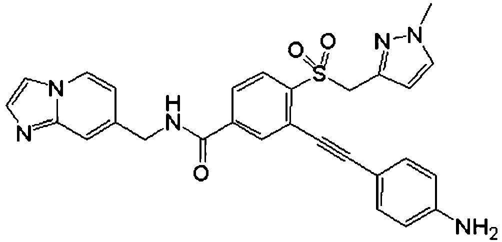 |
| 229 | 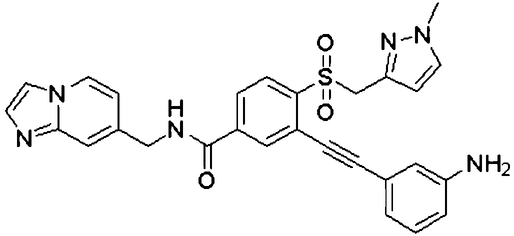 |
| 230 | 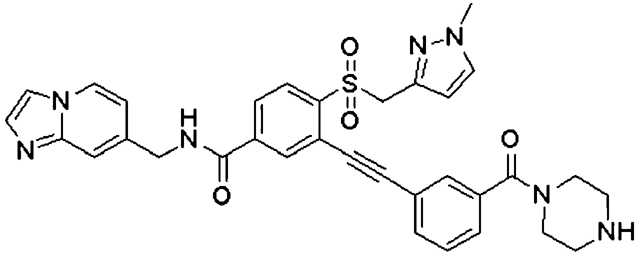 |
| 231 | 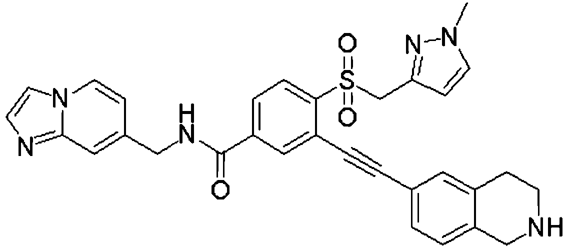 |
| 232 | 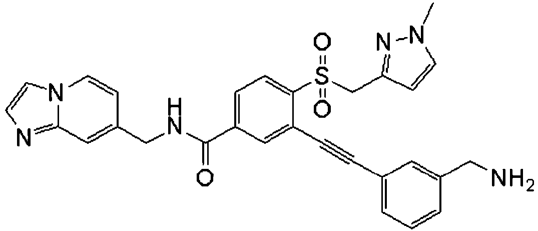 |
| 233 | 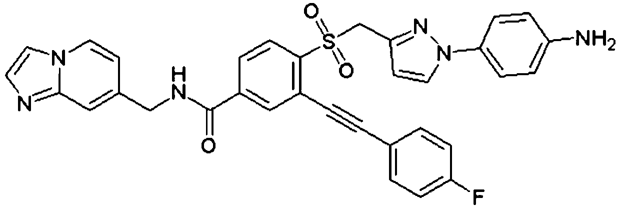 |
| 234 | 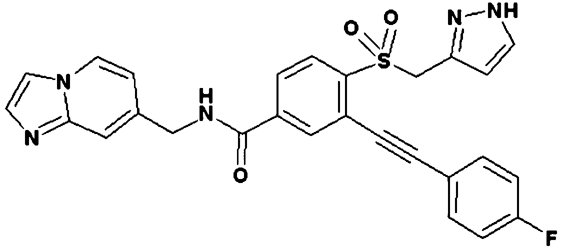 |
| 235 | 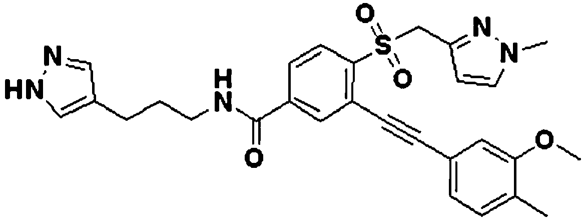 |
| 236 | 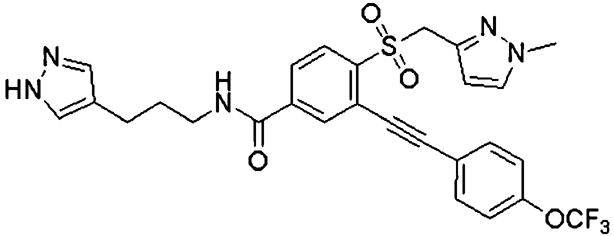 |
| 237 | 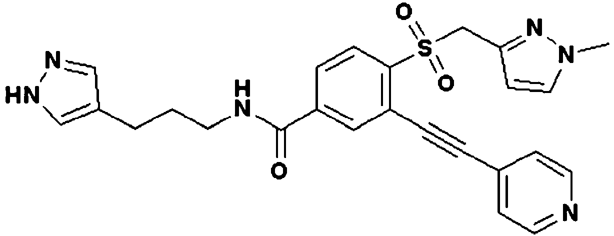 |
| 238 | 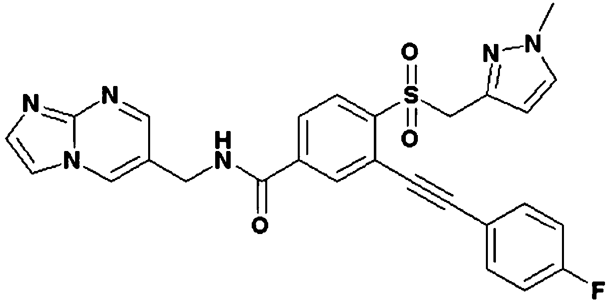 |
| 240 | 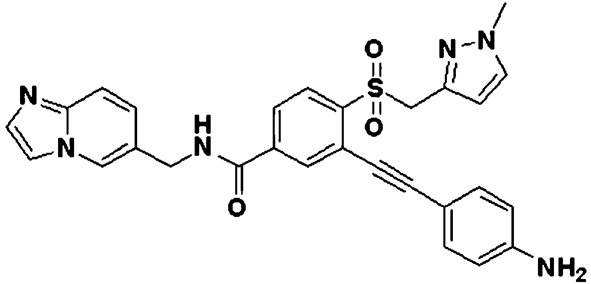 |
| 241 | 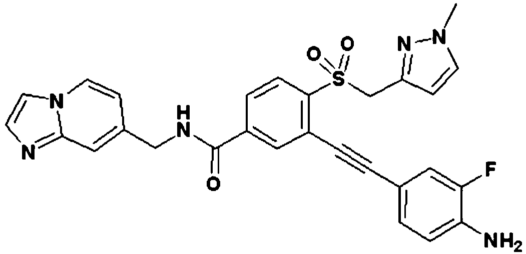 |
| 242 | 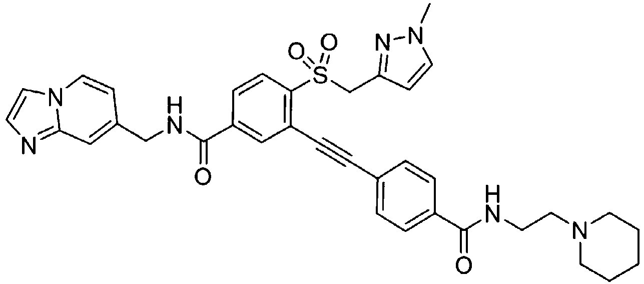 |
| 243 | 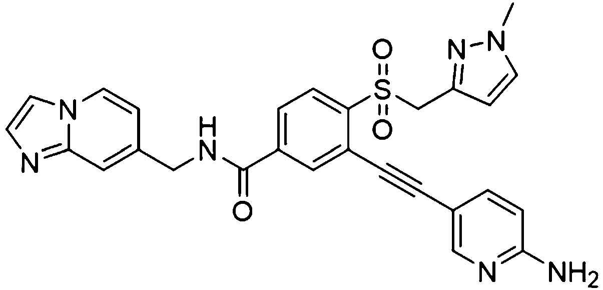 |
| 244 | 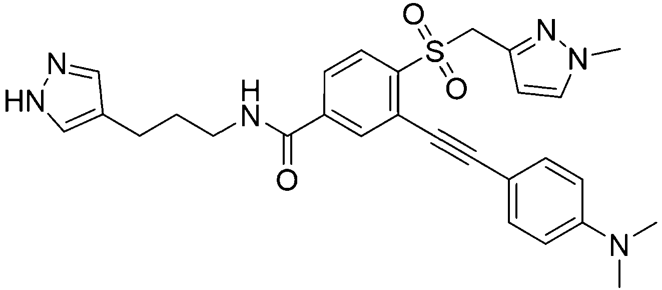 |
| 245 | 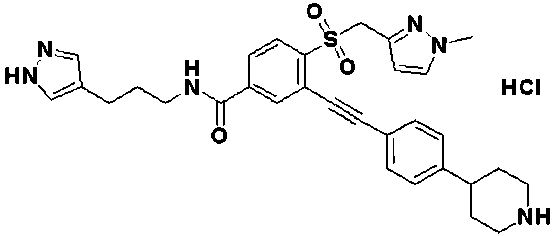 |
| 246 | 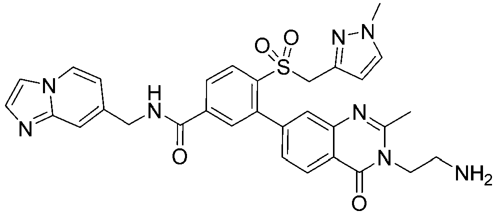 |
| 247 | 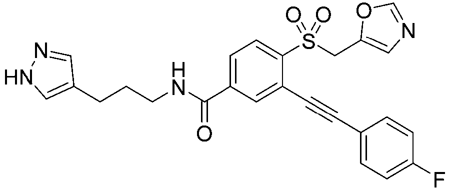 |
| 248 | 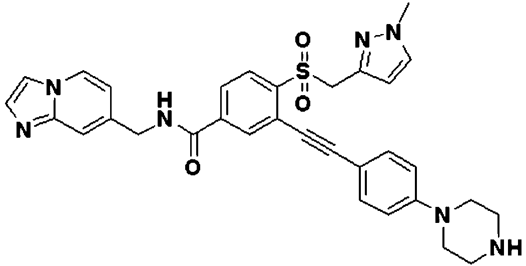 |
| 249 | 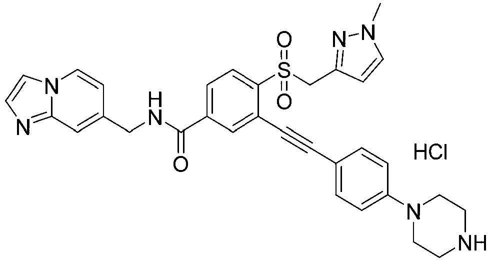 |

27. A pharmaceutical composition comprising a compound represented by above formula I according to claim 1, a stereoisomer thereof, a pharmaceutically acceptable salt of them, or a hydrate or solvate of them.

28. The pharmaceutical composition of claim 27, wherein said pharmaceutical composition exhibits NAMPT inhibitory activity.

29. The pharmaceutical composition of claim 28, wherein said pharmaceutical composition is used for preventing or treating cancer.

30. The pharmaceutical composition of claim 29, wherein said cancer is solid cancer or blood cancer.

31. The pharmaceutical composition of claim 30, wherein said cancer is one selected from the group consisting of skin cancer, melanoma, lymphoma, breast cancer, cervical cancer, uterine cancer, gastrointestinal cancer, lung cancer, ovarian cancer, prostate cancer, colorectal cancer, colon cancer, rectal cancer, oral cancer, brain cancer, head and neck cancer, throat cancer, testicular cancer, kidney cancer, pancreatic cancer, bone cancer, spleen cancer, liver cancer, bladder cancer, larynx cancer, nasal cavity cancer, AIDS-related cancer, esophageal cancer, gastric cancer, stomach cancer, pancreatic cancer, colorectal cancer, virus-related cancer, nasopharyngeal cancer, vaginal cancer, vulvar cancer, penile cancer, Kaposi sarcoma, Burkitt lymphoma, T-cell lymphoma and Merkel cell carcinoma, multiple myeloma, acute and chronic leukemia, lymphoblastic leukemia, myeloid leukemia, lymphocytic leukemia, and myelocytic leukemia.

32. A method for preventing or treating cancer, the method comprising: administering a compound represented by above formula I according to claim 1, a stereoisomer thereof, a pharmaceutically acceptable salt of them, a hydrate or solvate of them, or a pharmaceutical composition according to claim 27 into an individual.

33. A use of a compound represented by above formula I according to claim 1, a stereoisomer thereof, a pharmaceutically acceptable salt of them, a hydrate or solvate of them, or a pharmaceutical composition according to claim 27 for preventing or treating cancer.

34. A use of a compound represented by above formula I according to claim 1, a stereoisomer thereof, a pharmaceutically acceptable salt of them, a hydrate or solvate of them, or a pharmaceutical composition according to claim 27 for preparing a medication for preventing or treating cancer.
